(19) Europäisches Patentamt
European Patent Office
Office européen des brevets

(11) EP 4 349 372 A1

(12) EUROPEAN PATENT APPLICATION
published in accordance with Art. 153(4) EPC

(43) Date of publication:
**10.04.2024 Bulletin 2024/15**

(21) Application number: **22815082.7**

(22) Date of filing: **23.05.2022**

(51) International Patent Classification (IPC):
***A61K 47/68*** (2017.01) ***A61P 35/00*** (2006.01)
***C07K 16/28*** (2006.01)

(52) Cooperative Patent Classification (CPC):
**A61K 47/68; A61P 35/00; C07K 16/28**

(86) International application number:
**PCT/CN2022/094559**

(87) International publication number:
**WO 2022/253035 (08.12.2022 Gazette 2022/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.06.2021 CN 202110615214**
**16.08.2021 CN 202110941134**

(71) Applicants:
- **Sichuan Kelun-Biotech Biopharmaceutical Co., Ltd.**
  **Chengdu, Sichuan 611138 (CN)**
- **Klus Pharma Inc.**
  **Princeton, New Jersey 08540 (US)**

(72) Inventors:
- **TIAN, Haijun**
  **Princeton, NJ 08540 (US)**
- **TIAN, Qiang**
  **Chengdu, Sichuan 611138 (CN)**
- **YUAN, Xiaoxi**
  **Chengdu, Sichuan 611138 (CN)**
- **CHANG, Ying-Hua**
  **Princeton, NJ 08540 (US)**
- **LI, Deliang**
  **Chengdu, Sichuan 611138 (CN)**
- **HU, Jiangjiang**
  **Chengdu, Sichuan 611138 (CN)**
- **ZHANG, Yitao**
  **Chengdu, Sichuan 611138 (CN)**
- **WANG, Xiaobei**
  **Chengdu, Sichuan 611138 (CN)**
- **ZHENG, Yong**
  **Chengdu, Sichuan 611138 (CN)**
- **YE, Jian**
  **Chengdu, Sichuan 611138 (CN)**
- **WANG, Bo**
  **Chengdu,, Sichuan 611138 (CN)**
- **MIAO, Yu**
  **Chengdu, Sichuan 611138 (CN)**
- **KANG, Bingqiang**
  **Chengdu, Sichuan 611138 (CN)**
- **LI, Fen**
  **Chengdu,, Sichuan 611138 (CN)**
- **TANG, Zujian**
  **Chengdu, Sichuan 611138 (CN)**
- **DENG, Hanwen**
  **Chengdu, Sichuan 611138 (CN)**
- **SONG, Hongmei**
  **Chengdu,Sichuan 611138 (CN)**
- **GE, Junyou**
  **Chengdu, Sichuan 611138 (CN)**
- **WANG, Jingyi**
  **Chengdu,, Sichuan 611138 (CN)**

(74) Representative: **Cabinet Nony**
**11 rue Saint-Georges**
**75009 Paris (FR)**

Remarks:
The complete document including Reference Table(s) and the Sequence Listing(s) can be downloaded from the EPO website

# (54) ANTIBODY DRUG CONJUGATE, AND PREPARATION METHOD THEREFOR AND USE THEREOF

(57) Provided in the present application are an antibody-drug conjugate, and a preparation method therefor and the use thereof. The antibody drug conjugate has a structure as represented by the formula Ab-[M-L-E-D]$_x$, and the drug is selected from an anti-tubulin agent, a DNA intercalator, a DNA topoisomerase inhibitor and a

RNA polymerase inhibitor. The prepared antibody-drug conjugate has a better drug-to-antibody ratio, and has a good targeted killing effect on colon cancer and non-small cell lung cancer (for example, lung adenocarcinoma).

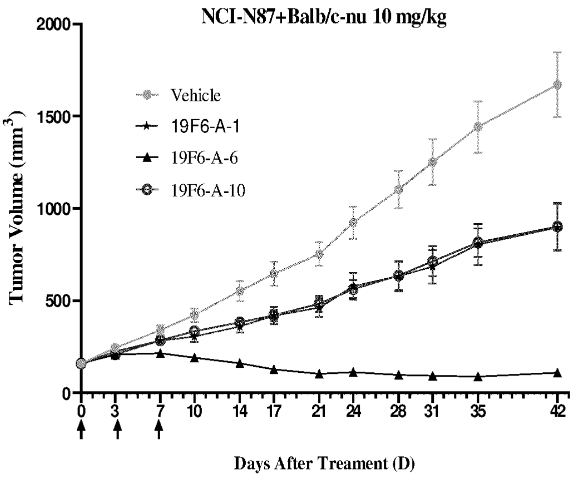


Fig. 2

## Description

The present application is based on CN application number 202110615214.X and application date of June 2, 2021, and CN application number 202110941134.3 and application date of August 16, 2021, and claims their priorities, and the disclosed contents of these CN applications are hereby incorporated into the present application in their entirety.

## Technical Field

The present application relates to the field of targeted therapy, in particular to an antibody-drug conjugate for treating a cancer with high expression of ROR1. Specifically, the present application provides a humanized ROR1 antibody, which has excellent binding activity to ROR1-positive cells, and can efficiently deliver a drug to cells with high expression of ROR1 in individuals. The present application also provides a drug-linker molecule for coupling to the antibody, in which the drug is selected from the group consisting of an anti-tubulin agent, a DNA intercalator, a DNA topoisomerase inhibitor and a RNA polymerase inhibitor. The prepared antibody-drug conjugate has a better drug-to-antibody ratio, and has good targeted killing effects on colon cancer, gastric cancer, breast cancer, lung cancer (e.g., non-small cell lung cancer, specifically lung adenocarcinoma), and lymphoma. Therefore, the present application further provides a preparation method of the antibody-drug conjugate and use thereof in the treatment of a cancer with high expression of ROR1.

## Background Art

Cancer is a class of diseases caused by the malignant transformation of healthy cells resulted from genetic changes, such as chromosomal translocation, mutations in tumor suppressor genes and growth factor receptors, that lead to malignant proliferation of cells. Defective apoptosis or programmed cell death further promotes the malignant transformation of cells that leads to cancer.

ROR1 (Receptor Tyrosine kinase-like Orphan Receptor 1), or neurotrophic tyrosine kinase receptor-related 1 (NTRKR1), is a member of receptor tyrosine kinase-like orphan receptor (ROR) family, also known as tyrosine-protein kinase transmembrane receptor, and belongs to type I membrane proteins.

The extracellular domains contains an immunoglobulin (IgG)-like domain, a CRD or frizzled domain, and a Kringle domain. From the membrane to the intracellular region, the intracellular domains are respectively a pseudokinase domain, a serine/threonine-rich domain 1, a proline-rich domain, and a serine/threonine-rich domain 2.

Under physiological conditions, ROR1 is highly expressed during embryonic development and plays an important role in regulating muscle and bone development of embryo. In recent years, studies have found that ROR1 is also expressed in a small amount in adipose tissue, pancreas, lung, and a small amount of B precursor cells, but it is highly expressed in various tumor tissues. Studies have shown that ROR1 can bind to the ligand molecule Wnt5a (mainly) or EGF, and participate in the regulation of cell proliferation, survival and migration. Overexpression of ROR1 is associated with poor prognosis of tumors, and studies have found that targeting ROR1 can effectively inhibit the growth of transplanted tumors.

Currently, the ROR1 target indications under clinical research cover hematological tumors and solid tumors. The main therapeutic strategies focus on monoclonal antibodies, CAR-T, and antibody-drug conjugates (ADCs). Among them, there are 3 anti-ROR1 antibody-conjugate drugs currently under research internationally, 2 of which have entered the clinical research stages (1 in phase II (VLS-101), and 1 in phase I/II (NBE-002)) for the treatment of MCL, BCL, NHL, NSCLC, TNBC, etc., and 3 in the preclinical/discovery stages. Companies involved in the researches include VelosBio, NBE-Therapeutics, LegoChem Biosciences, Almac, etc.

Wherein, the ADC drug is composed of an antibody, a bioactive molecule and a linker. The bioactive molecule is covalently coupled to the antibody through the linker; the antibody (e.g., monoclonal antibody) can specifically recognize a specific target on the surface of a cancer cell, and then guides the ADC to the surface of the cancer cell, and enters the cancer cell through endocytosis effect; then the bioactive molecule is released in the cancer cell to kill the cancer cell without damaging the normal tissue cells as much as possible.

Regarding the international clinical progress of Anti-ROR1 antibody-drug conjugates, VLS-101 developed by VelosBio has the fastest clinical progress, it adopts maleimide connector (MC) and enzyme-cleavable linker valine-citrulline (Val-Cit), tubulin inhibitor MMAE is used as the toxin, VLS-101 is formed by non-specific coupling, and has a DAR of about 4. It has been reported in the literature that under physiological conditions, MC connector is prone to exchange with a thiol through retro-Michael reaction, resulting in reduced efficacy and increased toxicity (Nat Biotechnol. 2012, 30(2): 184-9; Bioconjugate Chem. 2015, 26, 145-152).

NBE-002 was developed by NBE-Therapeutics, it adopts SarA sortase linker technology, uses five glycines as enzyme-cleavable linker, and topoisomerase PNU-159682 as the toxin. According to literature reports, the linker has multiple potential enzyme-cleavable sites, and PNU-159682 is sensitive to light and acidic conditions, and has certain

cardiotoxicity under physiological conditions (Bioorg. Med. Chem. Lett. 30 (2020) 127640).

## Contents of the Invention

The present application relates to an antibody-drug conjugate for the treatment of a cancer with high expression of ROR1, and exemplarily discloses an antibody-drug conjugate with a structure represented by the general formula Ab-$[M\text{-}L\text{-}E\text{-}D]_x$ that uses a humanized antibody 19F6-Hu35V1 as a targeting moiety. The results show that the conjugate has a better drug-to-antibody ratio (e.g., 1.5 to 2.5, 3.5 to 4.5, 6.5 to 8.5), and the conjugate has excellent binding activity to ROR1-positive cells, and good targeted killing effect on ROR1-positive cancers, such as colon cancer, gastric cancer, breast cancer, lung cancer (e.g., non-small cell lung cancer, specifically lung adenocarcinoma), or lymphoma. Therefore, the present application provides an antibody-drug conjugate for treating a cancer with high expression of ROR1, a pharmaceutical composition containing the antibody-drug conjugate, and use thereof in treating a cancer with high expression of ROR1.

Antibody-Drug Conjugate

In one aspect, the present application provides an antibody-drug conjugate, which has a structure represented by the formula Ab-$[M\text{-}L\text{-}E\text{-}D]_x$, wherein:
Ab represents an antibody or antigen-binding fragment thereof that specifically binds to receptor tyrosine kinase-like orphan receptor (ROR) family member 1 (ROR1):

M represents a linking site connecting the antibody or antigen-binding fragment thereof;

L represents a connector connecting the linking site M and E;

E represents a structural fragment connecting L and D;

D represents a cytotoxic drug moiety;

x is selected from 1 to 10.

In the antibody-drug conjugate, the cytotoxic drug can be linked to the antibody or antigen-binding fragment thereof through a linker (e.g., the "M-L-E" fragment as shown in the present application).
In some embodiments, M is selected from the following structures:

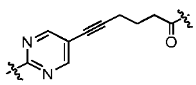 and 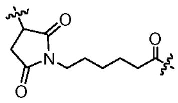.

In some embodiments, M is

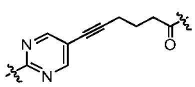.

In some embodiments, L is a divalent structure composed of one or more of the following groups: $C_{1-6}$ alkylene, -N(R')-, carbonyl, -O-, Val, Cit, Phe, Lys, D-Val, Leu, Gly, Ala, Asn, Val-Cit, Val-Ala, Val-Lys, Val-Lys(Ac), Phe-Lys, Phe-Lys(Ac), D-Val-Leu-Lys, Gly-Gly-Arg, Ala-Ala-Asn, Ala-Ala-Ala, Val-Lys-Ala, Gly-Gly-Gly, Gly-Gly-Phe-Gly, Gly-Gly-Gly-Gly-Gly,

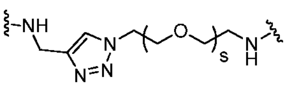, 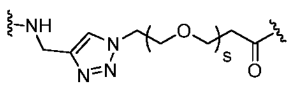, 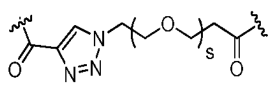, 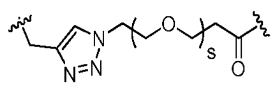,

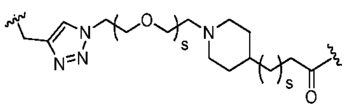, 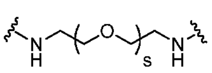, 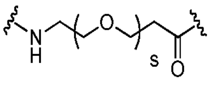, 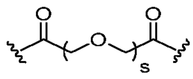, 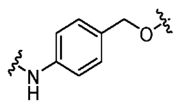,

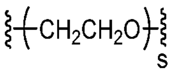, 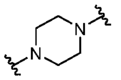, 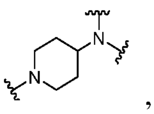, 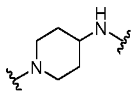, 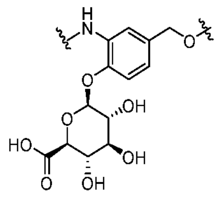, 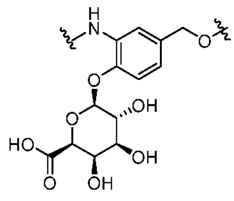,

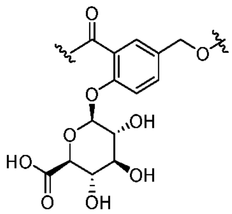, 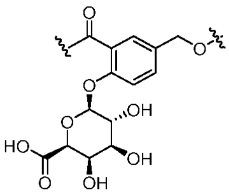 and 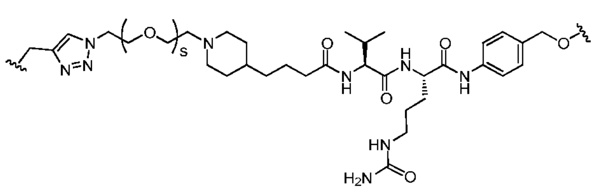,

wherein R' represents hydrogen, $C_{1-6}$ alkyl or a $-(CH_2CH_2O)_r$--containing alkyl; r is an integer selected from 1-10; s is an integer selected from 1-10.

In some embodiments, L is a divalent structure composed of one or more of the following groups: $C_{1-6}$ alkylene, -N(R')-, carbonyl, -O-, Val, Cit, Phe, Lys, D-Val, Leu, Gly, Ala, Asn, Val-Cit, Val-Ala, Val-Lys, Val-Lys(Ac), Phe-Lys, Phe-Lys(Ac), D-Val-Leu-Lys, Gly-Gly-Arg, Ala-Ala-Asn, Gly-Gly-Gly, Gly-Gly-Phe-Gly, Gly-Gly-Gly-Gly-Gly,

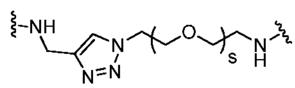,

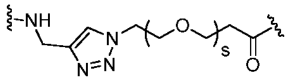, 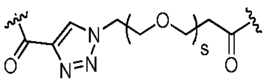, 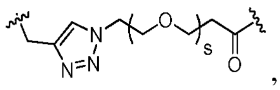, 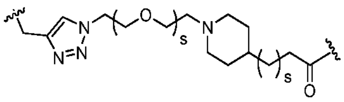,

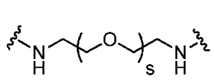, 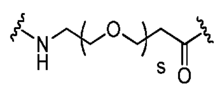, 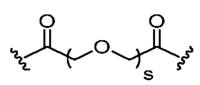, 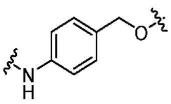, 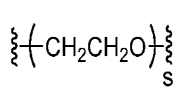, 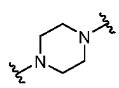,

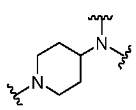, 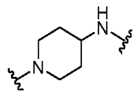, 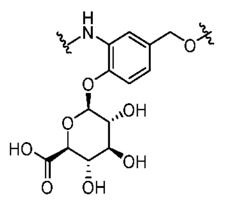, 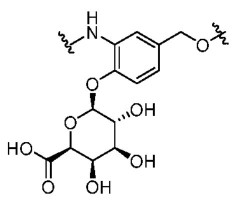, 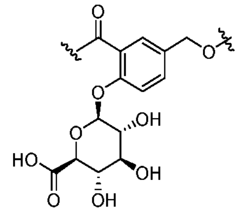,

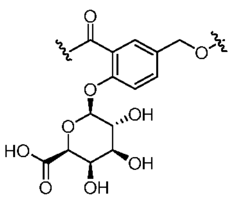 and 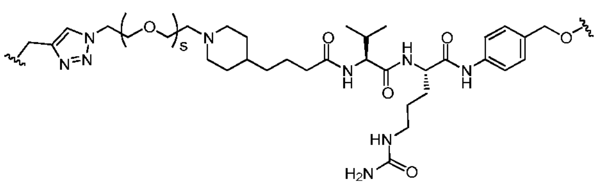,

wherein R' represents hydrogen, $C_{1-6}$ alkyl or a $-(CH_2CH_2O)_r$--containing alkyl; r is an integer selected from 1-10; s is an integer selected from 1-10.

In some embodiments, L is a structure composed of one or more of the following groups:

$C_{1-6}$ alkylene, -N(R')-, carbonyl, -O-, Val, Cit, Phe, Lys, D-Val, Leu, Gly, Ala, Asn, Val-Cit, Val-Ala, Val-Lys, Val-Lys(Ac), Phe-Lys, Phe-Lys(Ac), D-Val-Leu-Lys, Gly-Gly-Arg, Ala-Ala-Asn, Ala-Ala-Ala, Val-Lys-Ala, Gly-Gly-Gly, Gly-Gly-Phe-Gly, Gly-Gly-Gly-Gly-Gly,

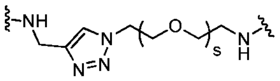, 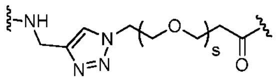, 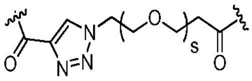, 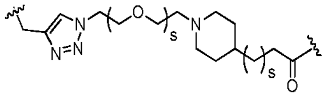,

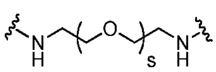, 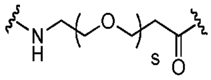, 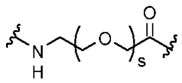, 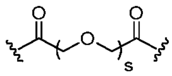, 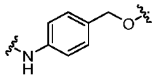, 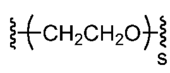,

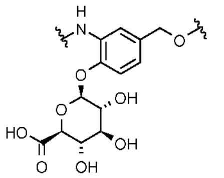 and 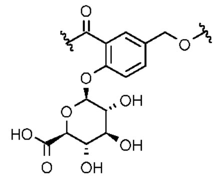 ;

where R' represents hydrogen, $C_{1-6}$ alkyl or a - $(CH_2CH_2O)_r$--containing alkyl; r is an integer selected from 1-10; s is an integer selected from 1-20; preferably, s is an integer selected from 1-10.

In some embodiments, L is a structure composed of one or more of the following groups: Val, Cit, Phe, Lys, D-Val, Leu, Gly, Ala, Asn, Val-Cit, Val-Ala, Val- Lys, Val-Lys(Ac), Phe-Lys, Phe-Lys(Ac), D-Val-Leu-Lys, Gly-Gly-Arg, Ala-Ala-Asn, Gly-Gly-Gly, Gly-Gly- Phe-Gly, Gly-Gly-Gly-Gly-Gly,

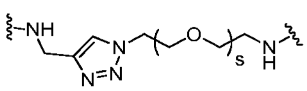, 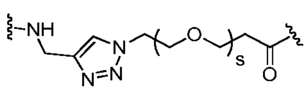, 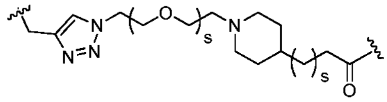,

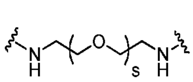, 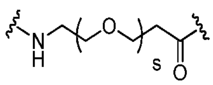, 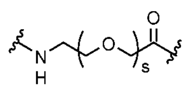, 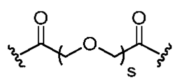, 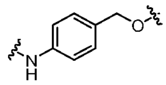, 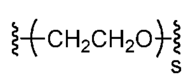,

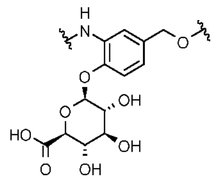 and 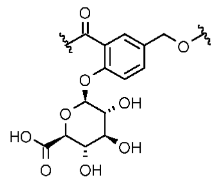 ;

wherein s is an integer selected from 1-10.

In some embodiments, L is selected from the following structures:

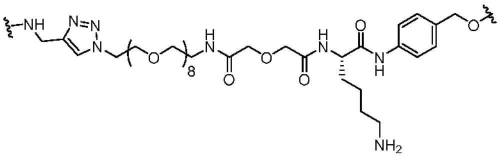, 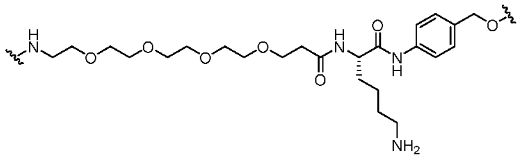,

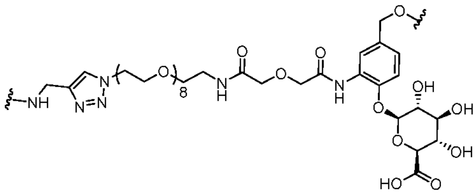 , 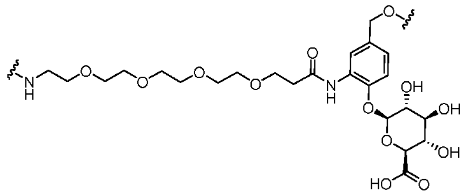 ,

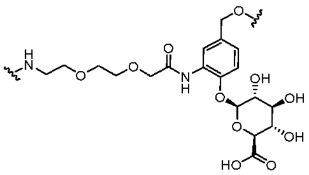 , 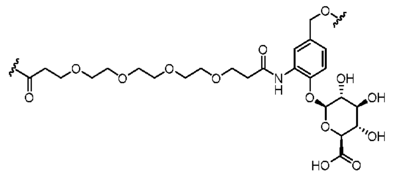 , 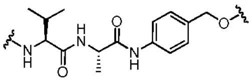 ,

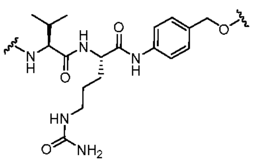 , 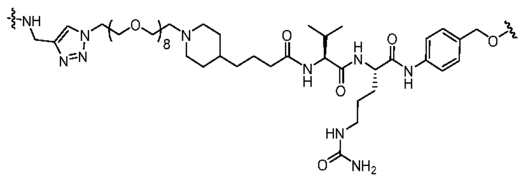 ,

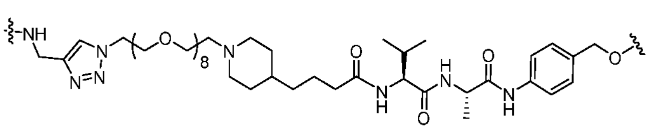 , 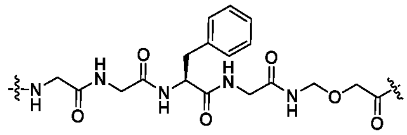 ,

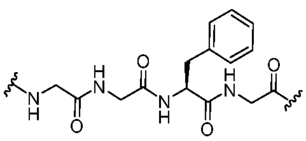 , 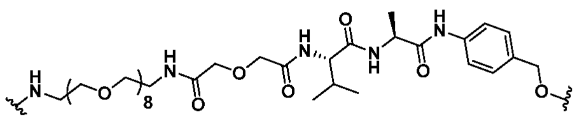 ,

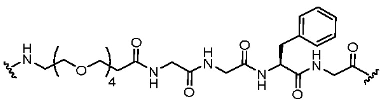 , 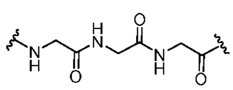 , 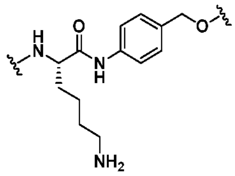 , 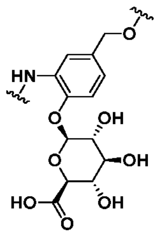 ,

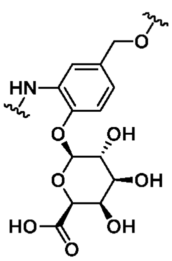 , 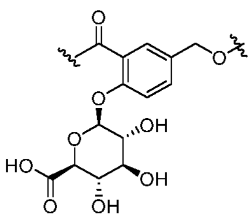 , 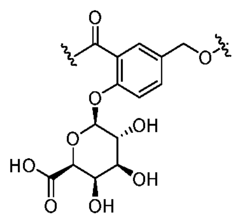 , 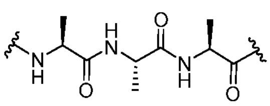 ,

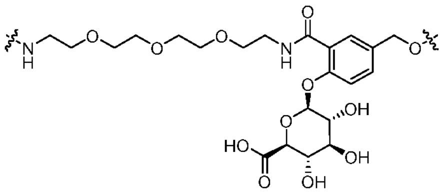,
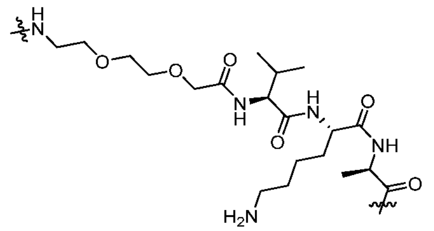,

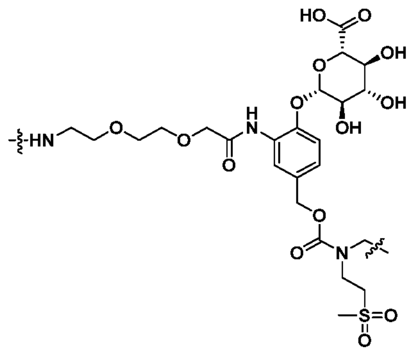 and 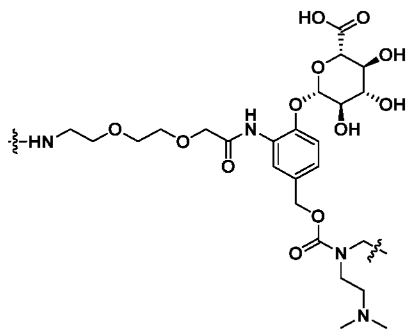.

In some embodiments, L is selected from the following structures:

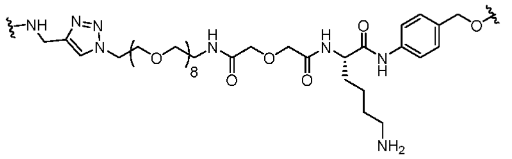,
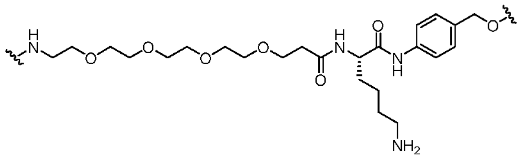,

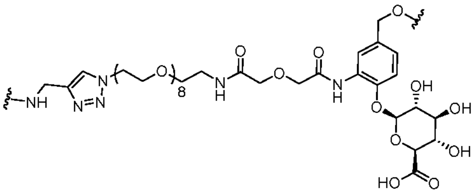,
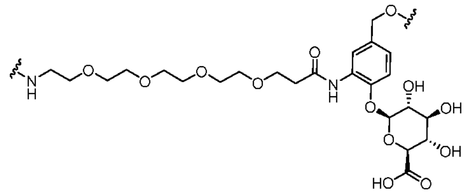,

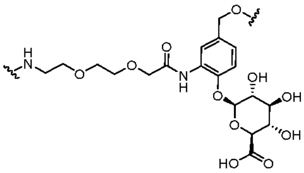, 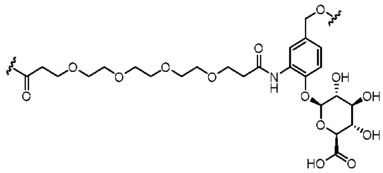, 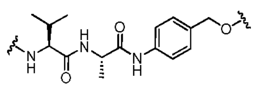, 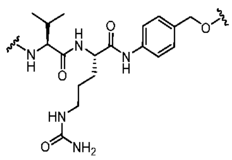,

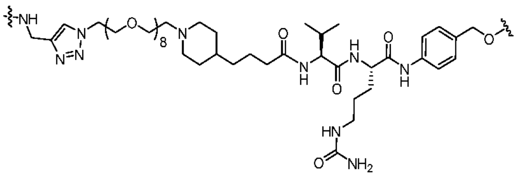,
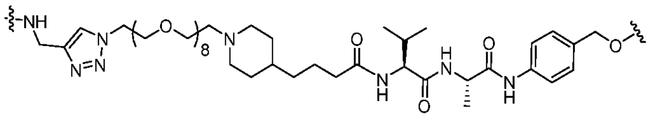,

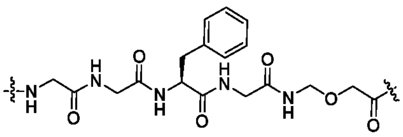,
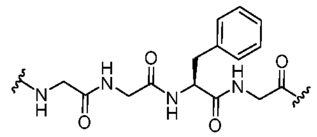,

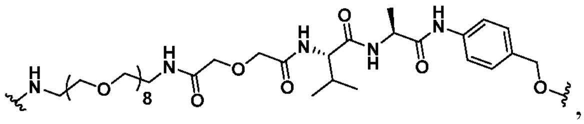, 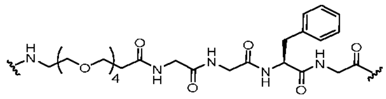, 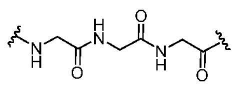,

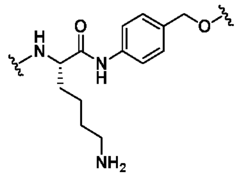, 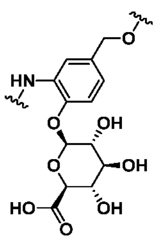, 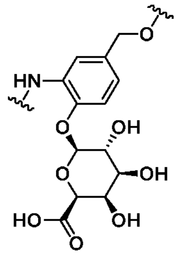, 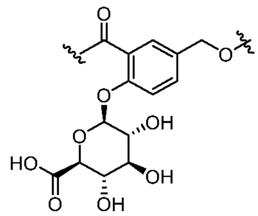 and 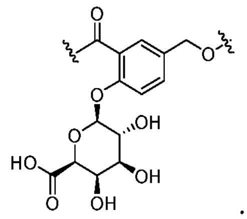.

In some embodiments, L is selected from the following structures:

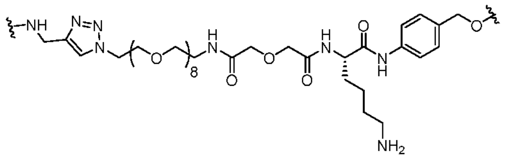, 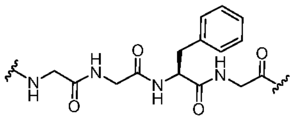, 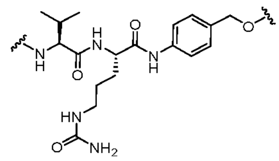,

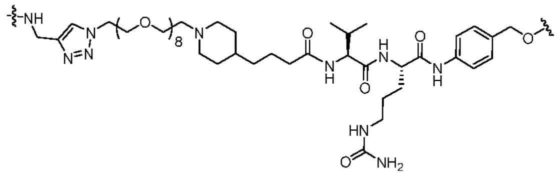, 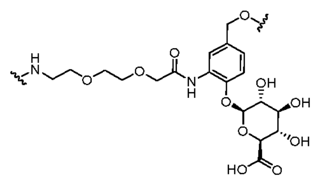,

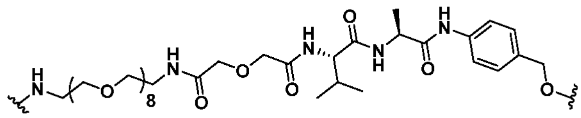

and

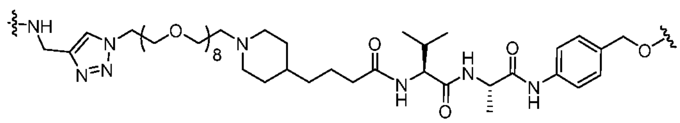.

In some embodiments, L is selected from the following structures:

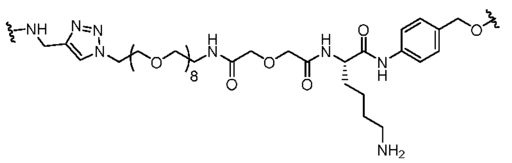, 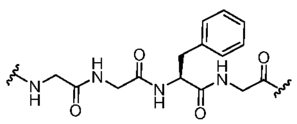, 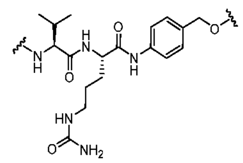,

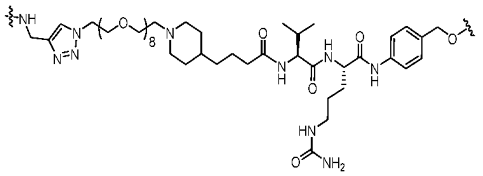, 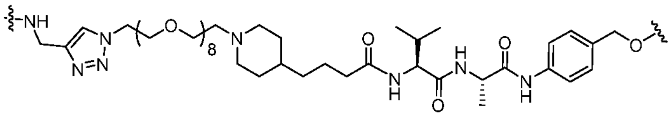,

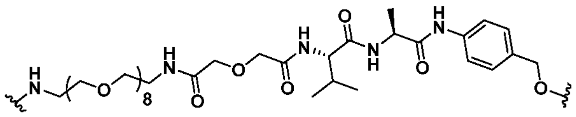, 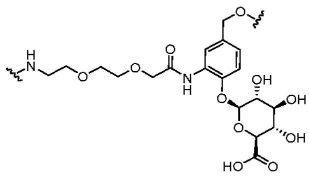,

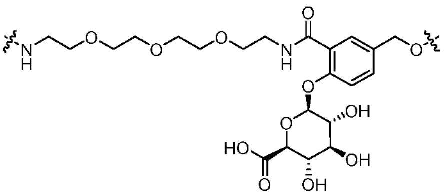 and 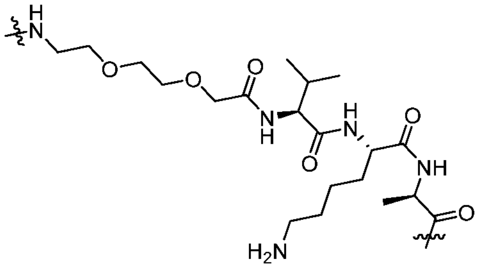.

In some embodiments, L is selected from the following structures:

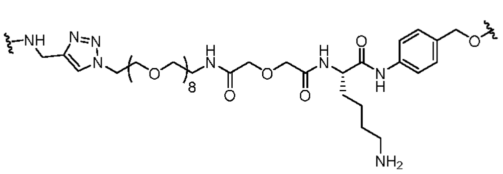, 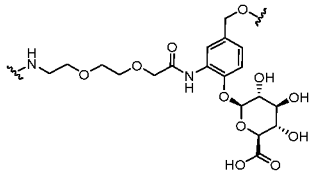, 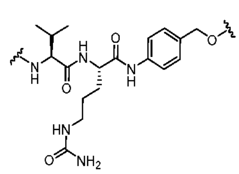,

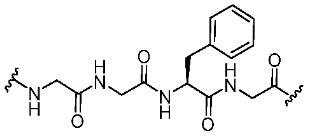, 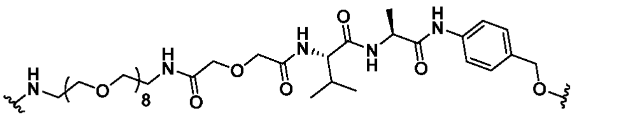,

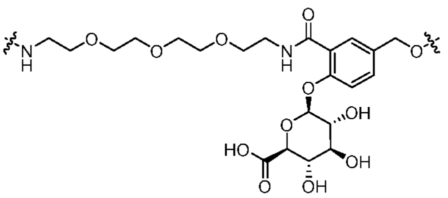 and 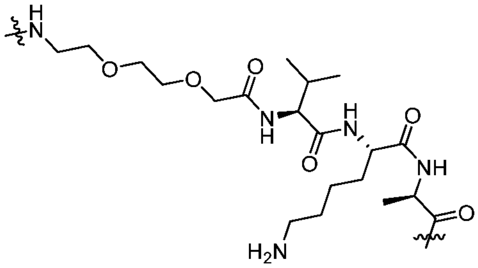.

In some embodiments, L is selected from the following structures:

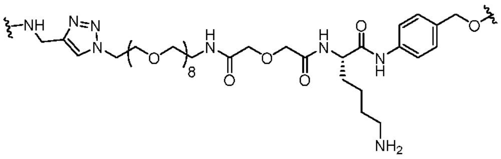, 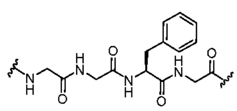, and 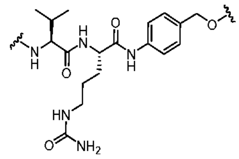.

In some embodiments, L is selected from the following structures:

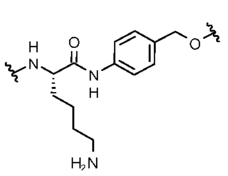 , 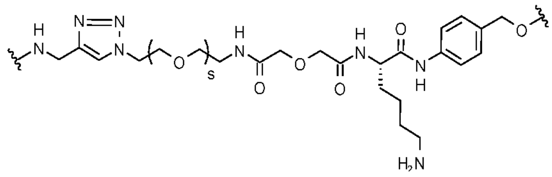 , 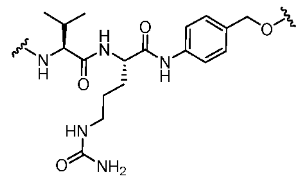 ,

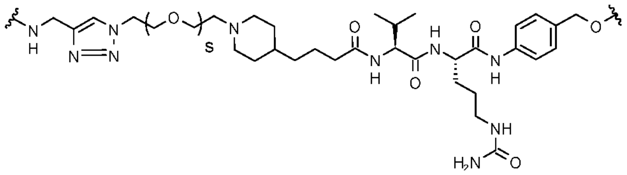 , 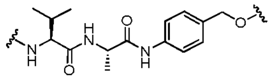 ,

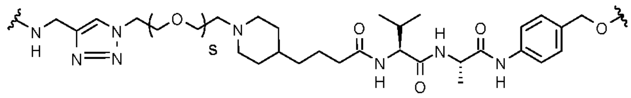 , 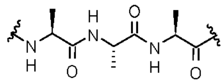 , 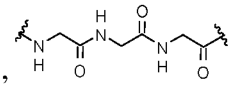 ,

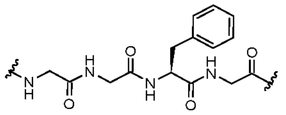 , 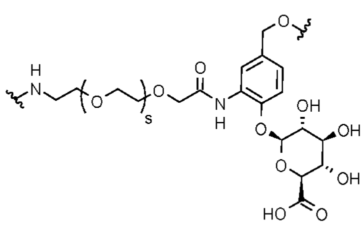 and 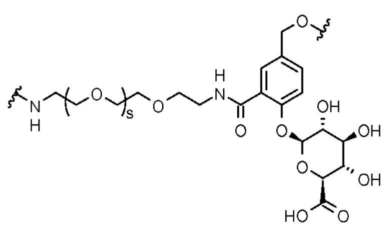 ;

wherein s is an integer selected from 1-20.

In some embodiments, L is selected from the following structures:

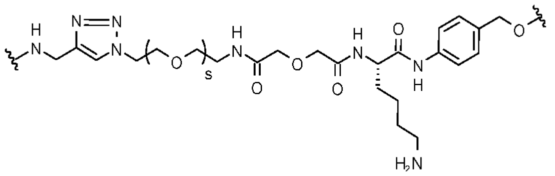 , 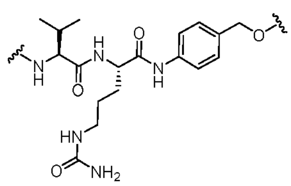 ,

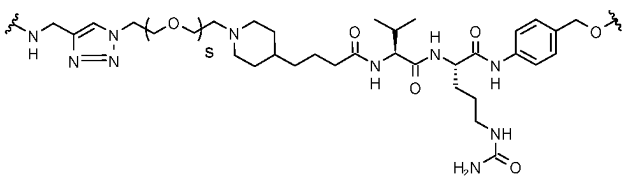 ,

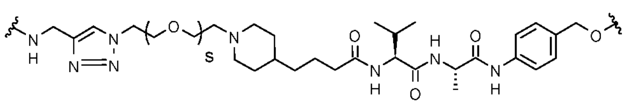 , 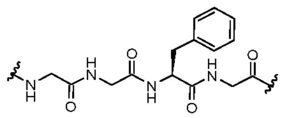 ,

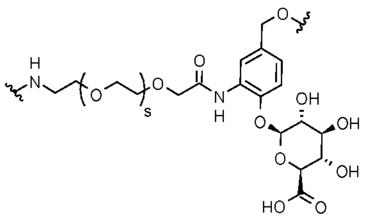 and 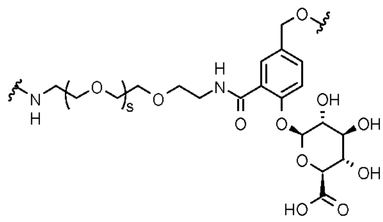;

wherein s is an integer selected from 1-10; preferably, s is selected from 1, 2 and 3.

In some embodiments, L is selected from the following structures:

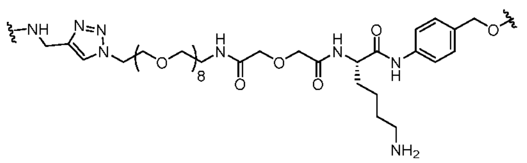, 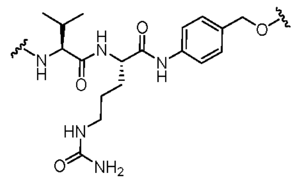,

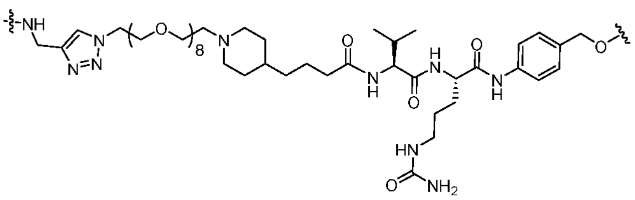,

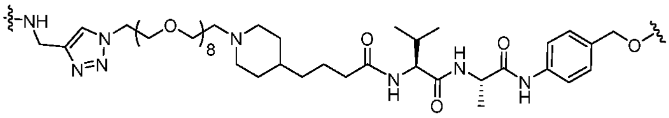, 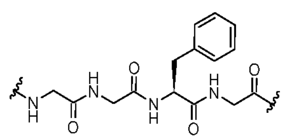,

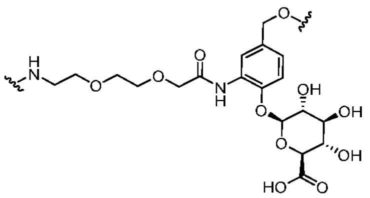 and 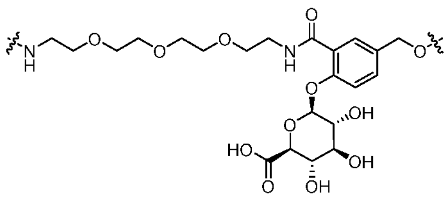.

In some embodiments, E is a single bond or -NH-$CH_2$-, or is selected from the following structures:

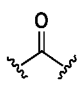, 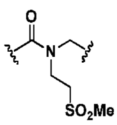, 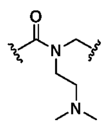, and 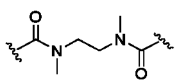.

In some embodiments, E is a single bond, -NH-$CH_2$- or

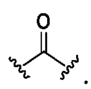.

In some embodiments, E is-NH-$CH_2$- or

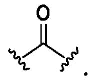

In some embodiments, M is

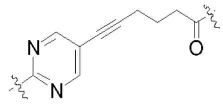;

L is selected from the following structures:

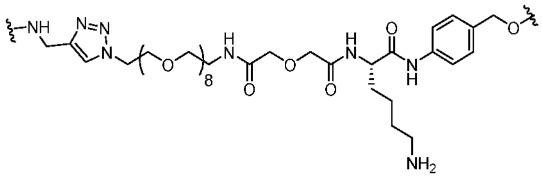, 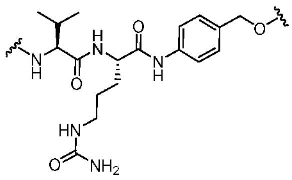,

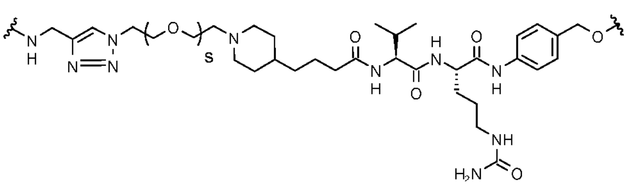, 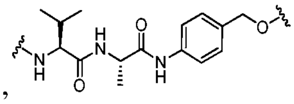,

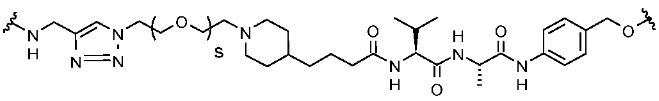, 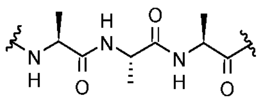,

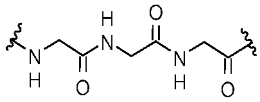,

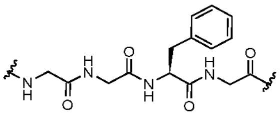, 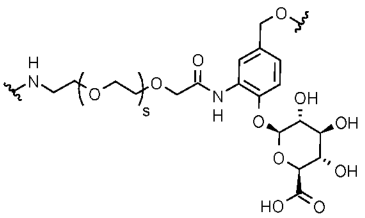 and 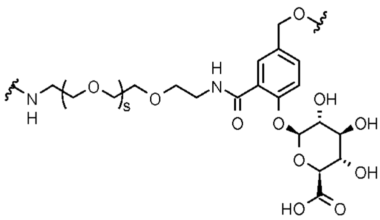;

wherein s is an integer selected from 1-10;
E is $-NH-CH_2-$ or

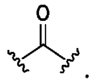

In some embodiments,

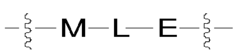

is selected from the following structures:

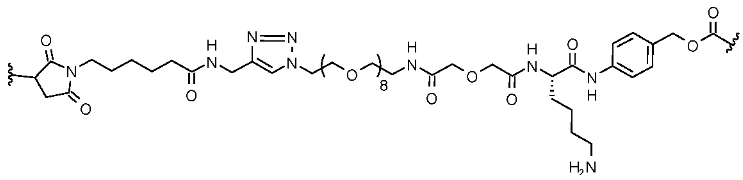

,

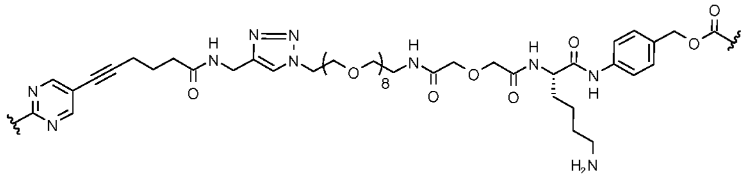

,

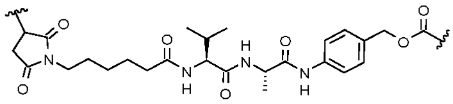

,

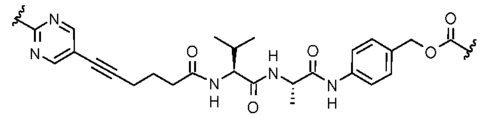

,

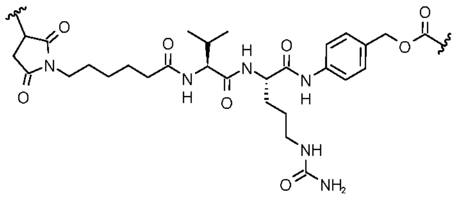

,

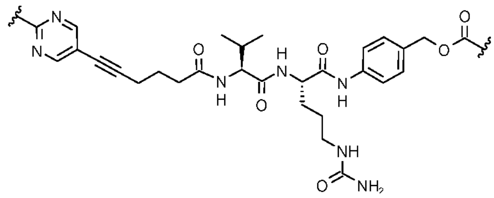

,

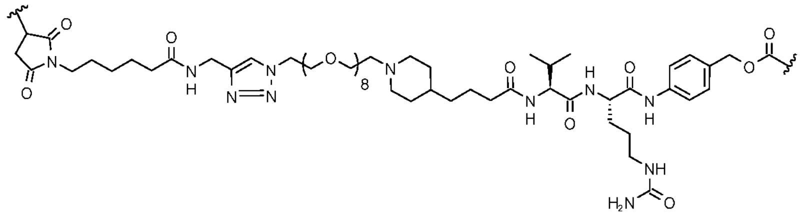

,

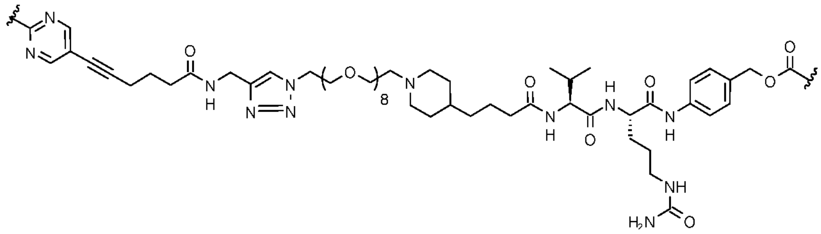

,

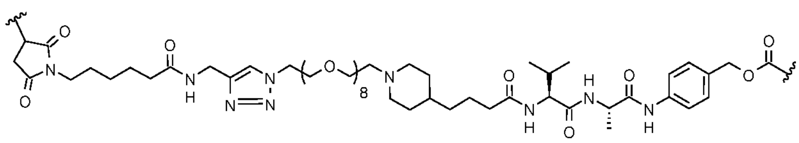

,

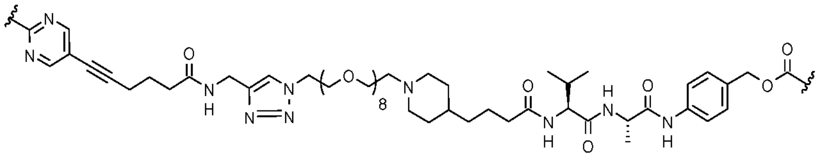

,

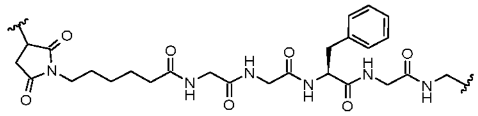

,

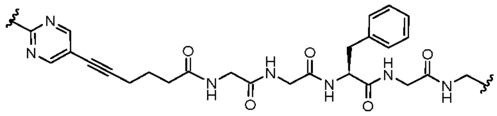

,

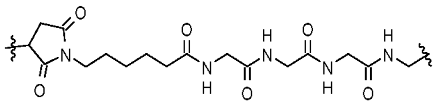

,

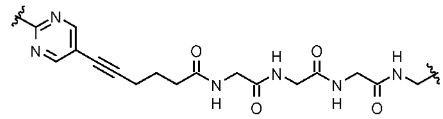

,

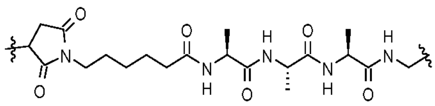

,

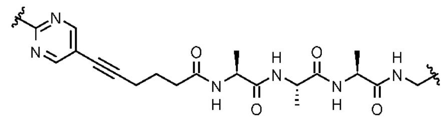

,

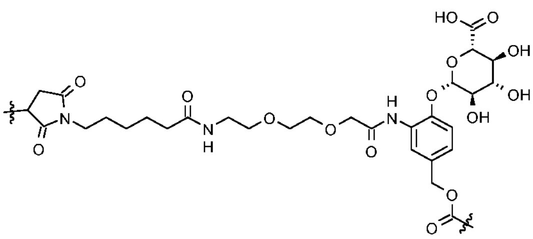

,

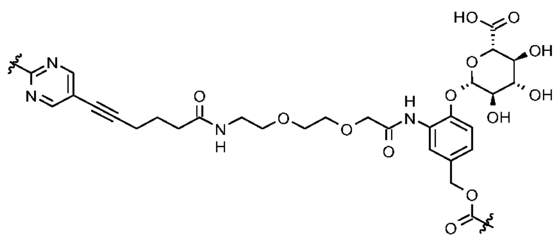

,

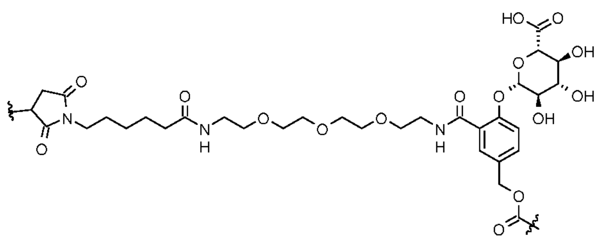

and

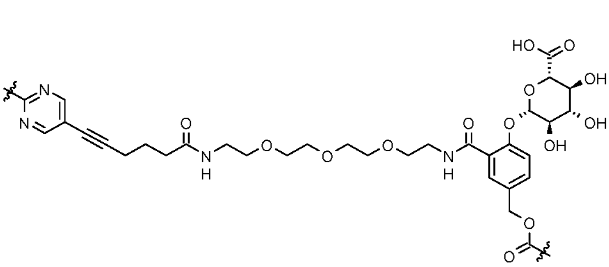

.

In some embodiments,

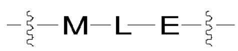

is selected from the following structures:

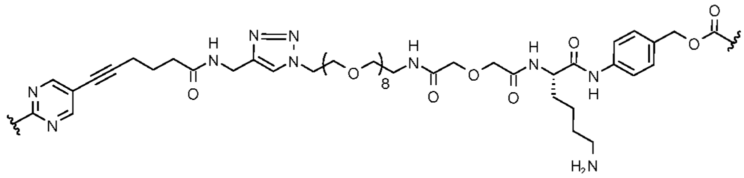

,

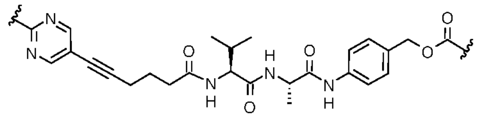

,

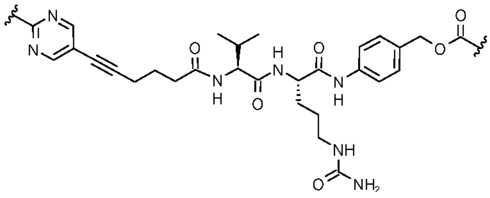

,

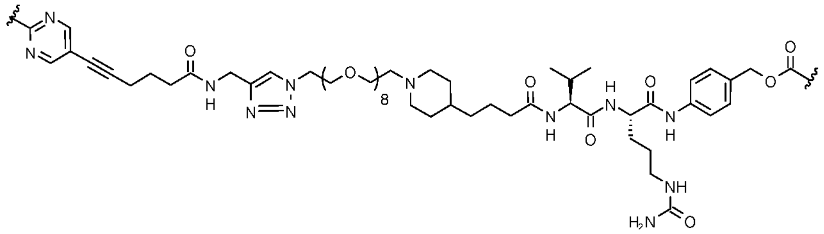

,

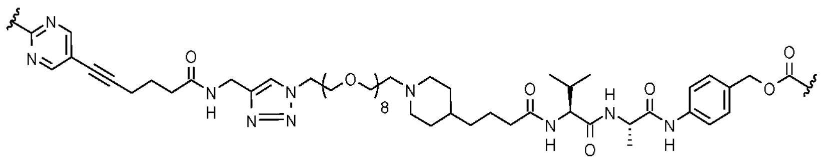

,

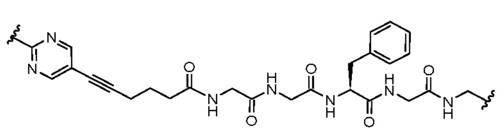

,

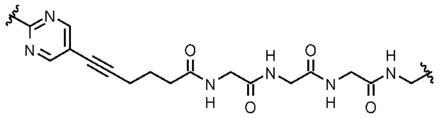

,

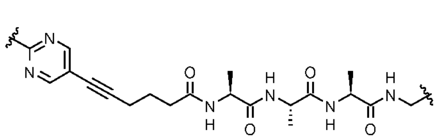

,

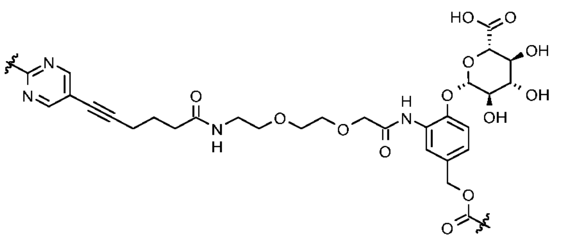

and

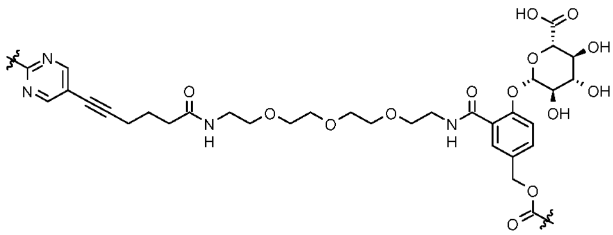
.

In some embodiments, the cytotoxic drug is selected from the group consisting of a tubulin inhibitor, a DNA intercalator, a DNA topoisomerase inhibitor, and a RNA polymerase inhibitor. In some embodiments, the tubulin inhibitor is an auristatin compound or a maytansine compound. In some embodiments, the DNA intercalator is pyrrolobenzodiazepine (PBD). In some embodiments, the DNA topoisomerase inhibitor is a topoisomerase I inhibitor (e.g., camptothecin, hydroxycamptothecin, 9-aminocamptothecin, SN-38, irinotecan, topotecan, belotecan, or rubitecan) or a topoisomerase II inhibitor (e.g., doxorubicin, PNU-159682, duocarmycin, daunorubicin, mitoxantrone, podophyllotoxin, or etoposide). In some embodiments, the RNA polymerase inhibitor is $\alpha$-amanitin or a pharmaceutically acceptable salt, ester or analog thereof.

The cytotoxic drugs disclosed in the present application usually contain a variety of functional groups, such as hydroxyl (-OH), carboxyl (-COOH), primary amino ($-NH_2$), secondary amine ($-NR_1H$), tertiary amine ($-NR_2R_3$), wherein $R_1$, $R_2$, $R_3$ here only represent non-hydrogen substituents on N, or sulfhydryl (-SH), and these functional groups can react with a suitable functional group in the rest of the conjugate to achieve the connection with the drug molecule.

In some embodiments, the cytotoxic drug is connected to E in the antibody-drug conjugate through -OH, primary amino group, secondary amino group or tertiary amino group, or -SH thereon.

In some embodiments, the cytotoxic drug is selected from the following compounds:

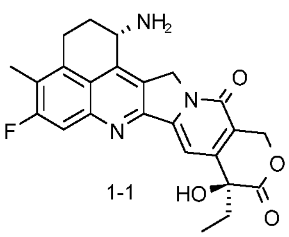
1-1

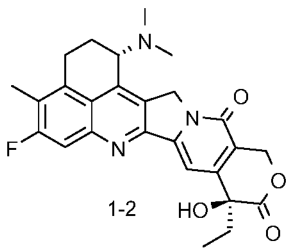
1-2

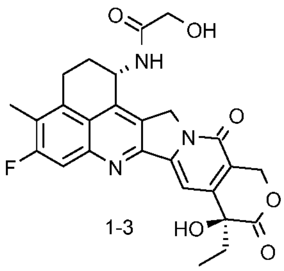
1-3

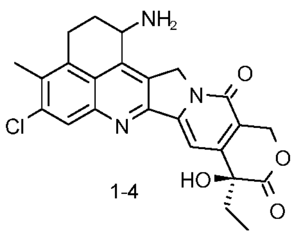
1-4

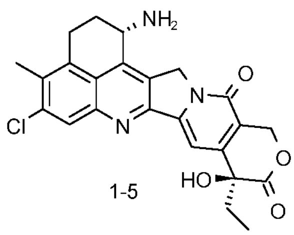
1-5

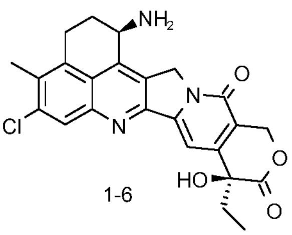
1-6

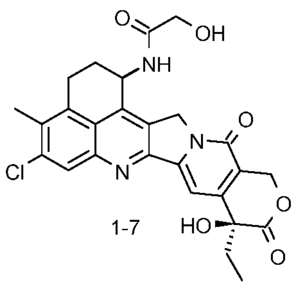
1-7

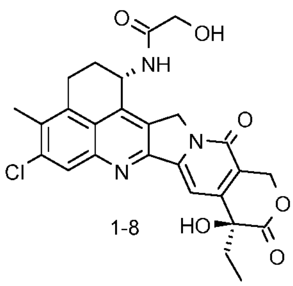
1-8

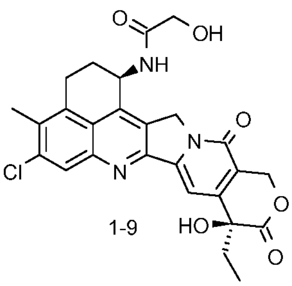
1-9

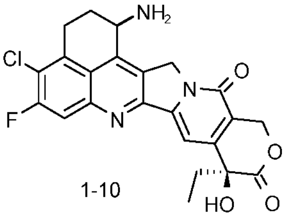
1-10

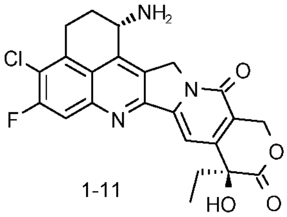
1-11

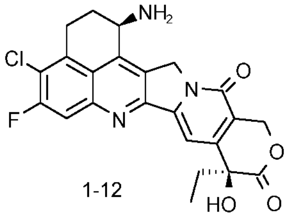
1-12

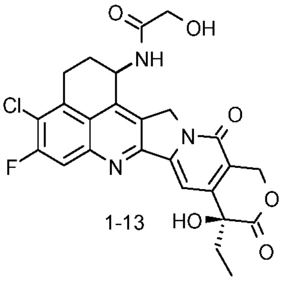
1-13

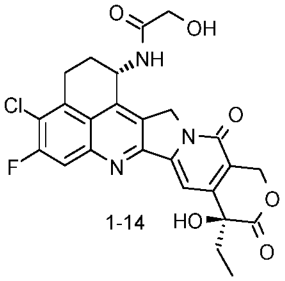
1-14

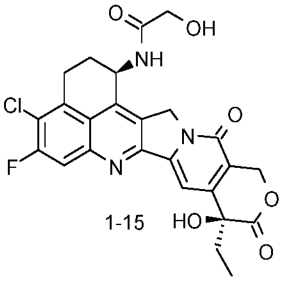
1-15

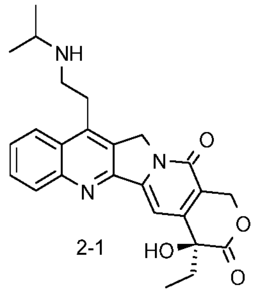
2-1

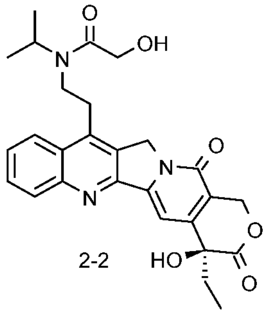
2-2

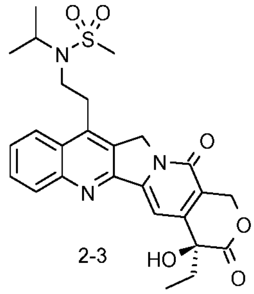
2-3

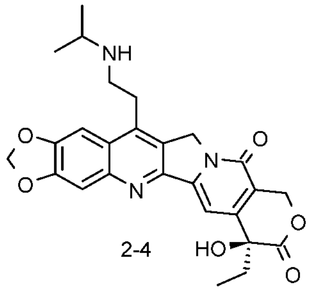
2-4

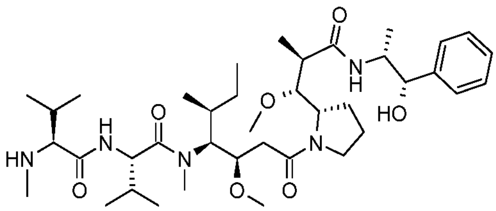
3-1

and

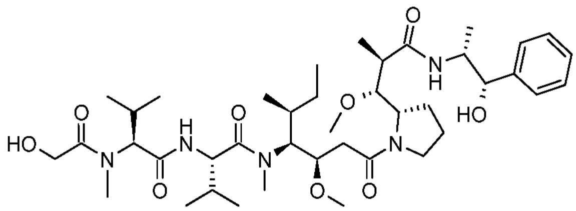
3-2

.

In some embodiments, D is selected from the following structures:

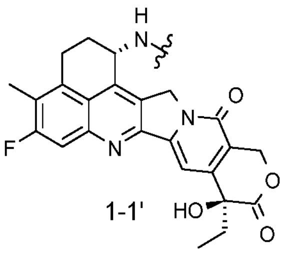
1-1'

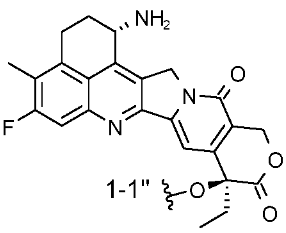
1-1''

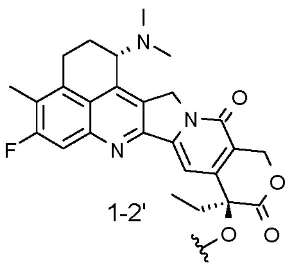
1-2'

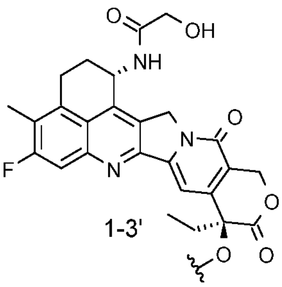
1-3'

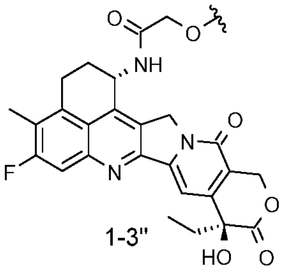
1-3''

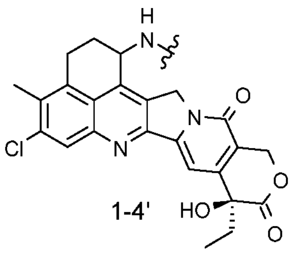
1-4'

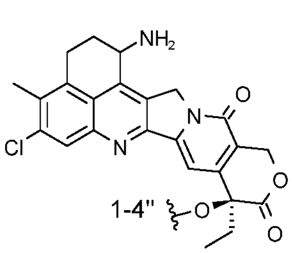
1-4''

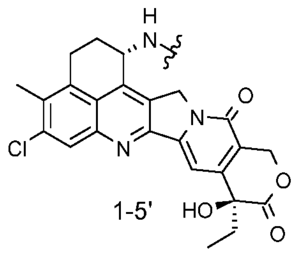
1-5'

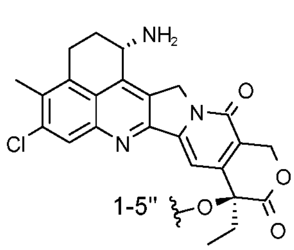
1-5''

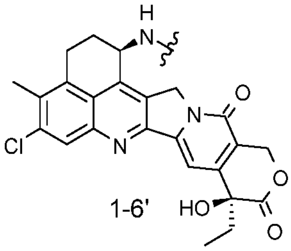
1-6'

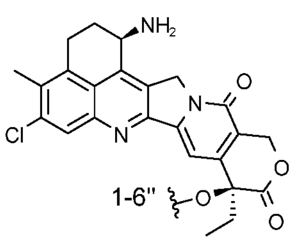
1-6''

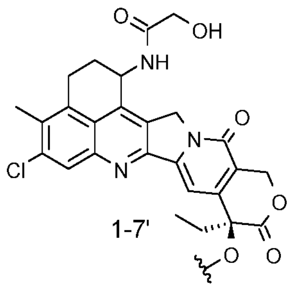
1-7'

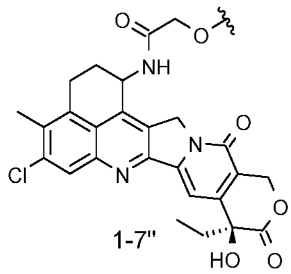
1-7''

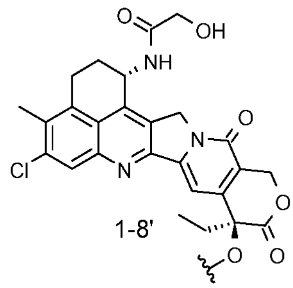
1-8'

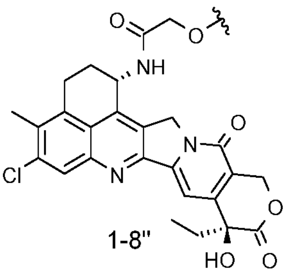
1-8''

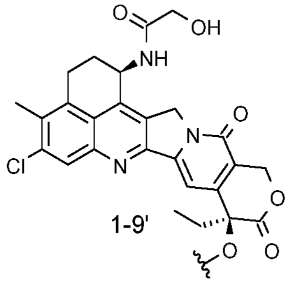
1-9'

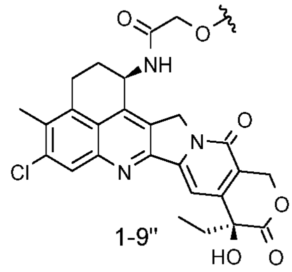
1-9''

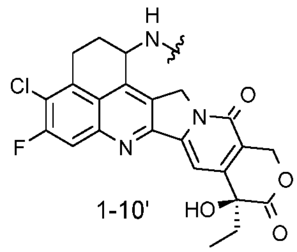
1-10'

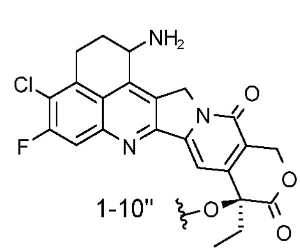
1-10''

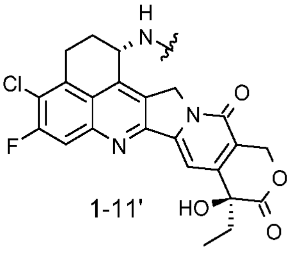
1-11'

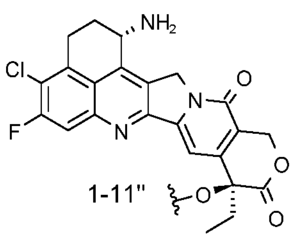
1-11''

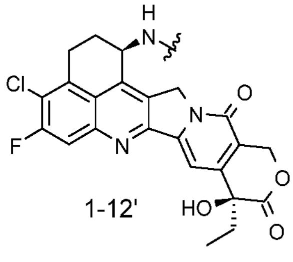
1-12'

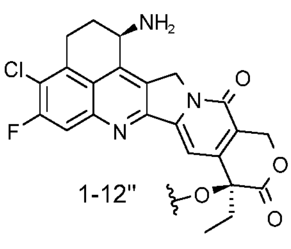
1-12''

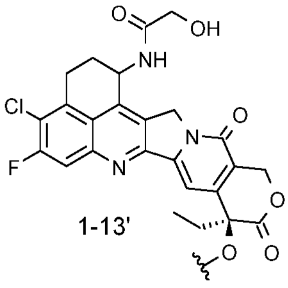
1-13'

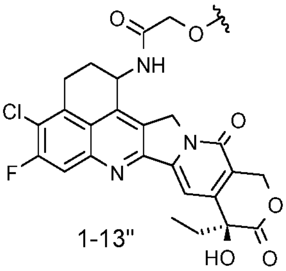
1-13''

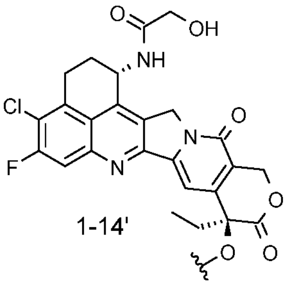
1-14'

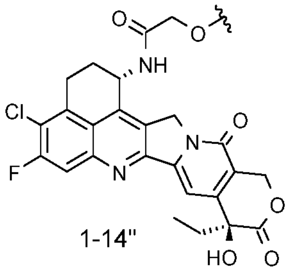
1-14''

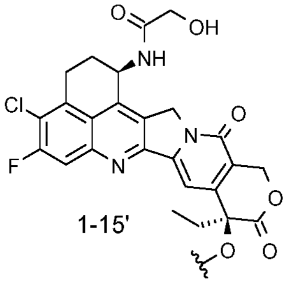
1-15'

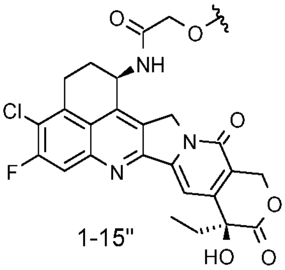
1-15''

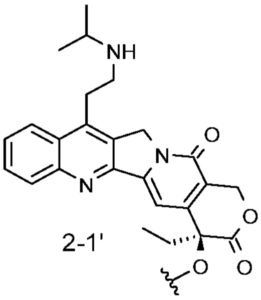
2-1'

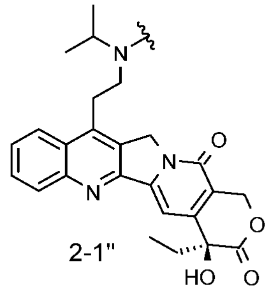
2-1''

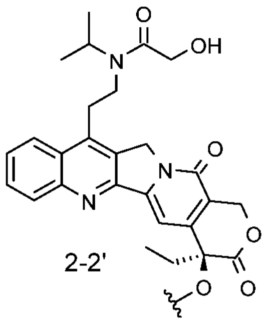
2-2'

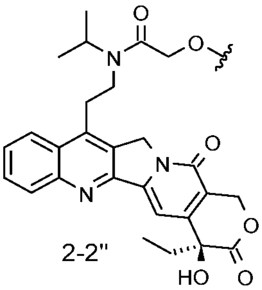
2-2''

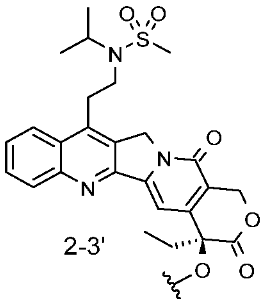
2-3'

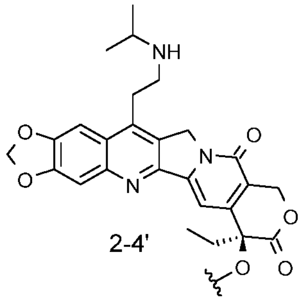
2-4'

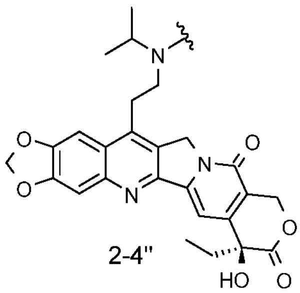
2-4''

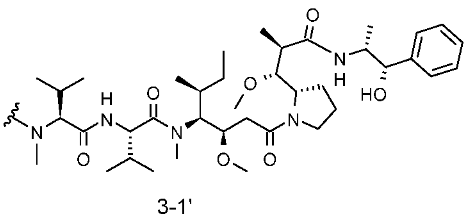
3-1'

and

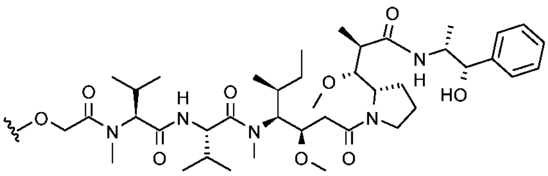
3-2' .

Those skilled in the art should understand that the antibody-drug conjugate described in the present application can be prepared modularly. For example, a free form of "drug-linker" (which can be understood as M'-L-E-D, wherein

M' is the form of M before it is covalently linked to the antibody or antigen-binding fragment thereof) can be firstly obtained, and then the "drug-linker" is covalently linked to the antibody or antigen-binding fragment thereof to obtain the antibody-drug conjugate as described in the present application. Correspondingly, M' in the free form of "drug-linker" is linked to one or more sulfhydryl (-SH), amino ($-NH_2$) or carboxyl (-COOH) groups on the antibody or antigen-binding fragment thereof through substitution reaction (e.g., removal of a structure such as-$SO_2Me$ or -Br thereon) or through addition reaction.

In some implementations, the free form of "drug-linker" is selected from the following A-1 to A-31 and B-1 to B-3:

A-1: 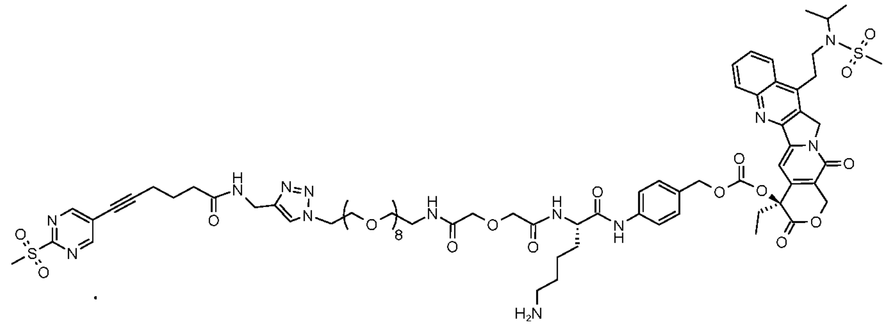 ,

A-2: 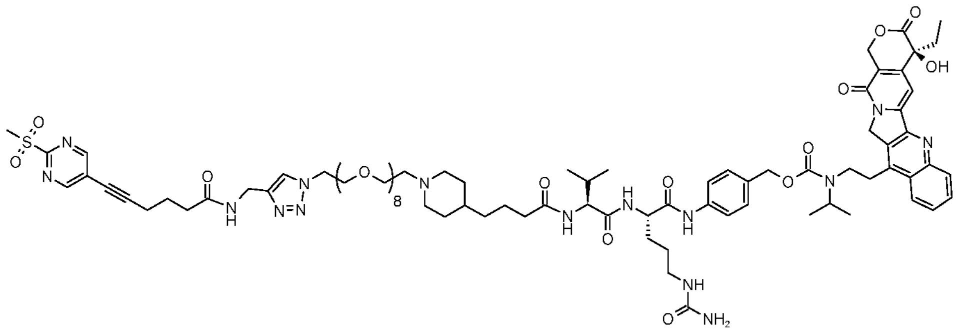 ,

A-3: 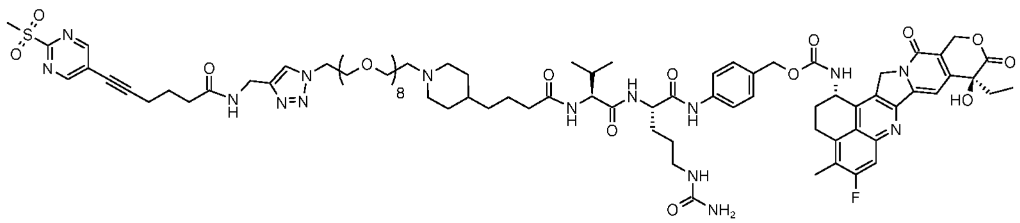 ,

A-4: 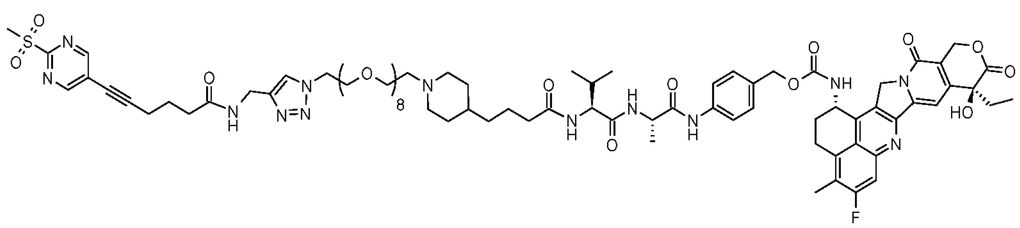 ,

A-5: 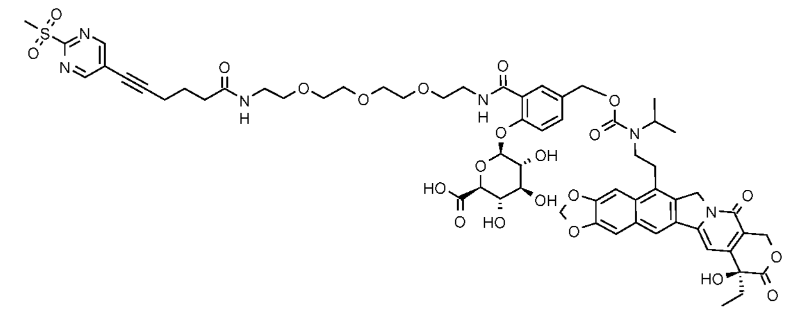,

A-6: 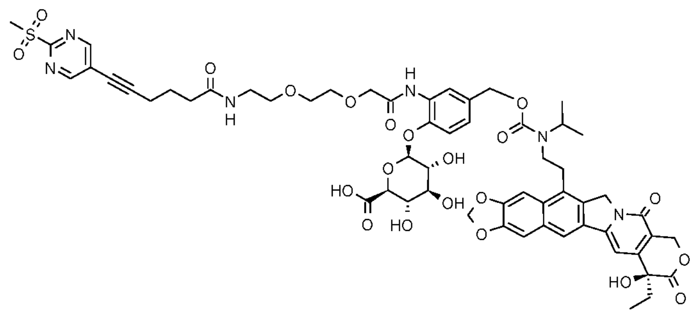,

A-7: 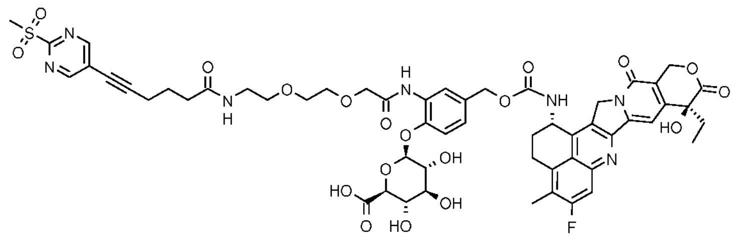,

A-8: 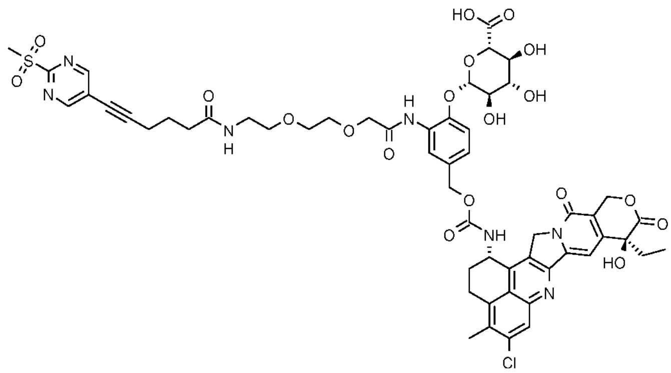,

A-9: 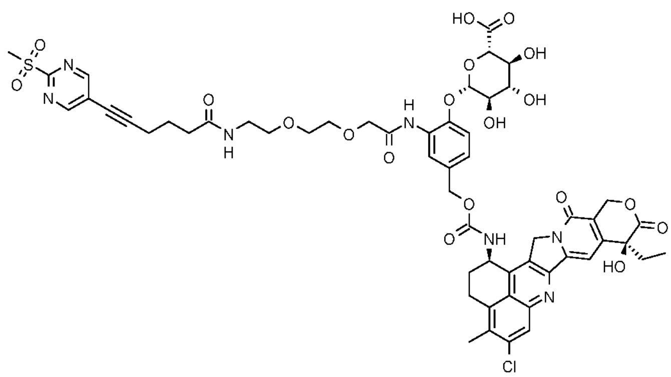,

A-10: 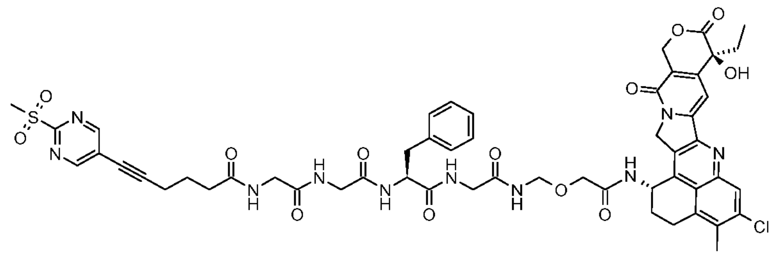 ,

A-11: 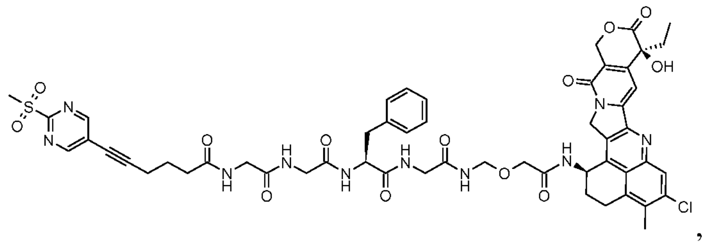 ,

A-12: 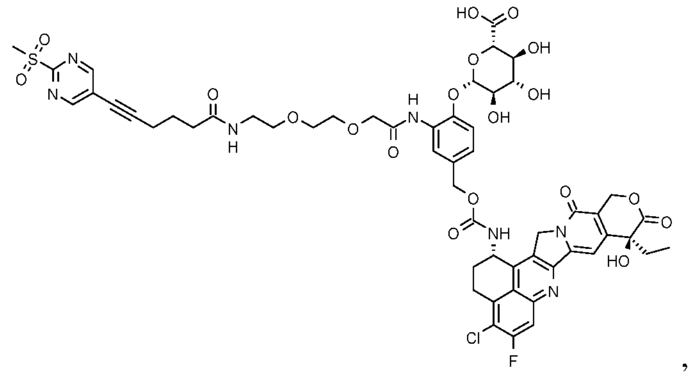 ,

A-13: 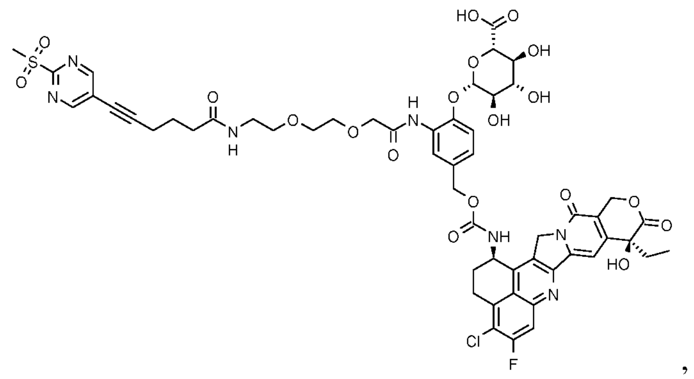 ,

A-14: 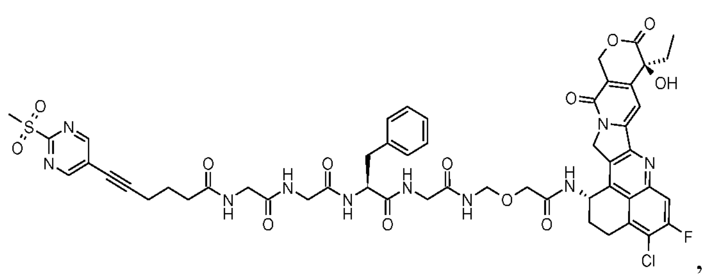 ,

A-15: 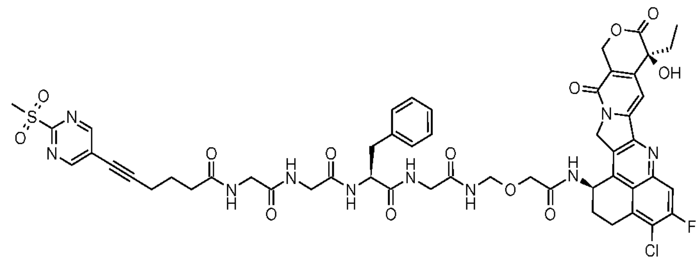 ,

A-16: 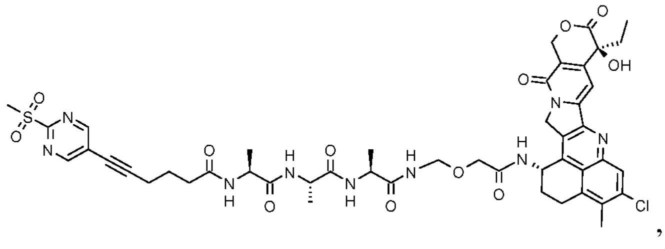 ,

A-17: 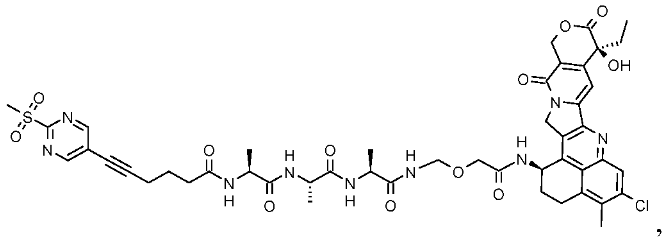 ,

A-18: 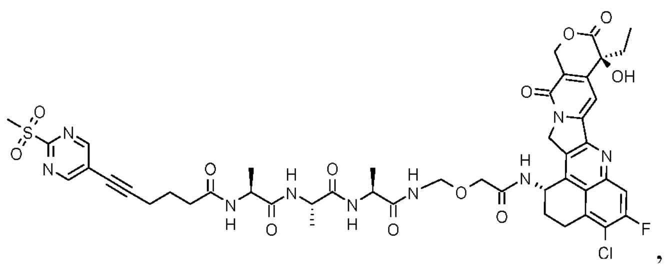 ,

A-19: 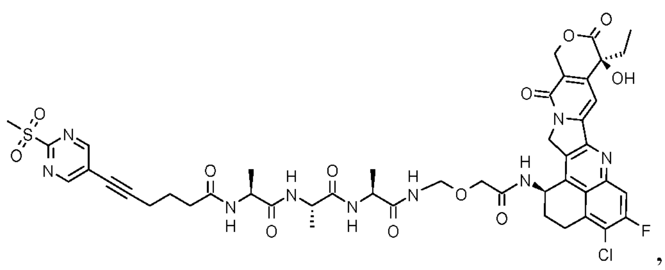 ,

A-20: 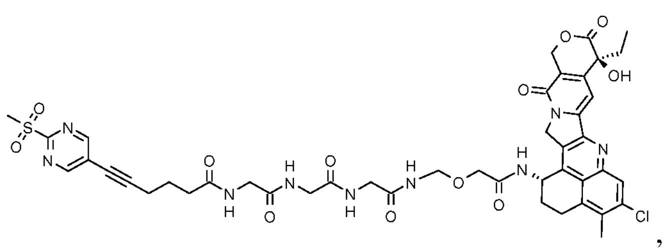 ,

A-21: 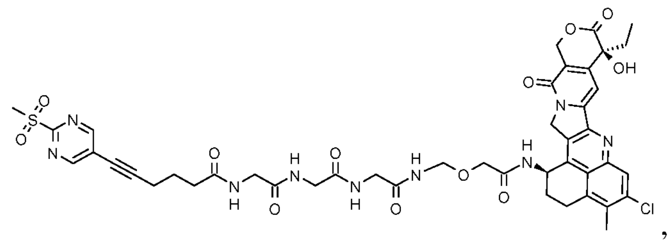,

A-22: 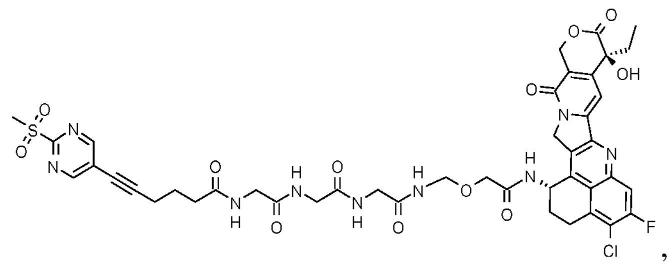,

A-23: 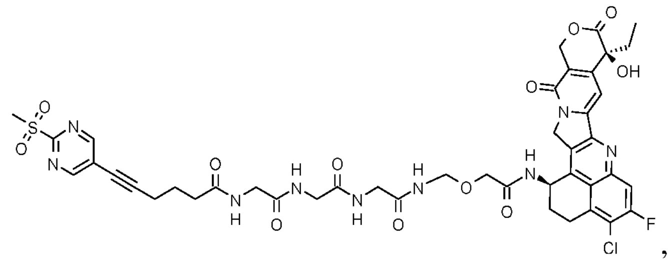,

A-24: 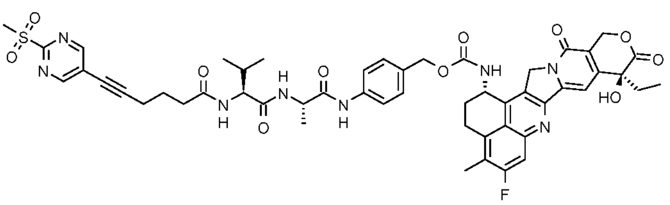,

A-25: 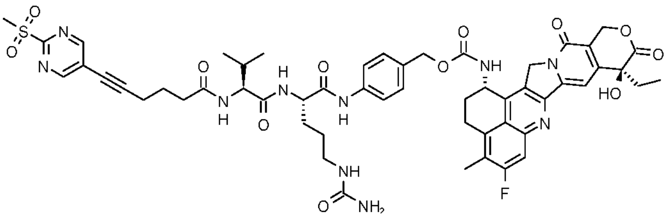,

A-26: 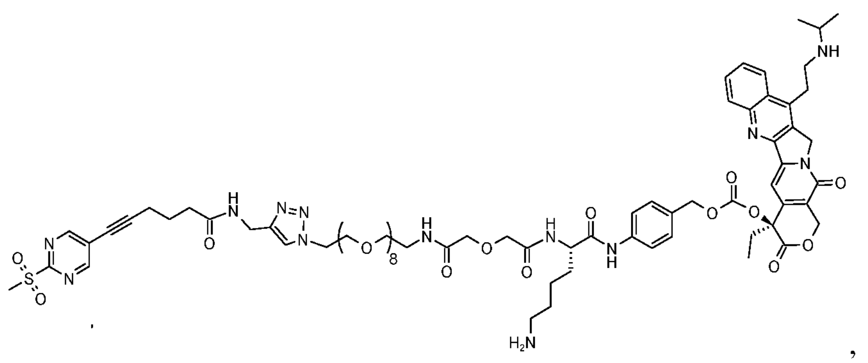,

A-27: 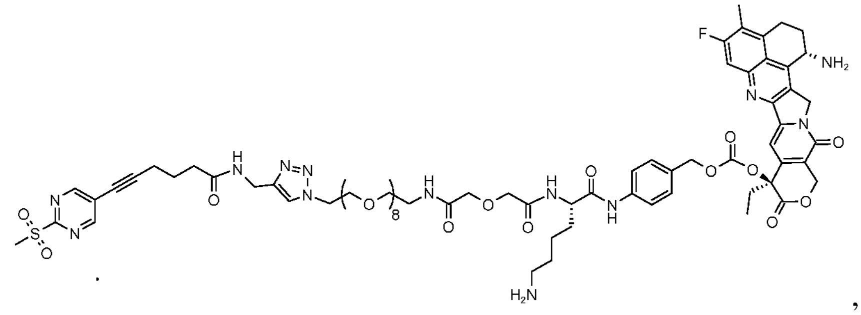 ,

A-28: 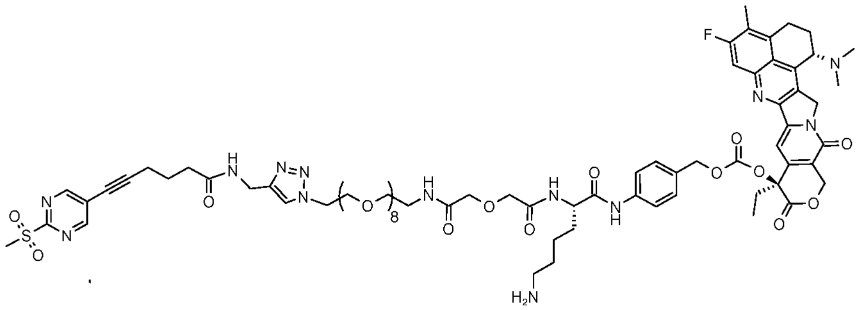 ,

A-29: 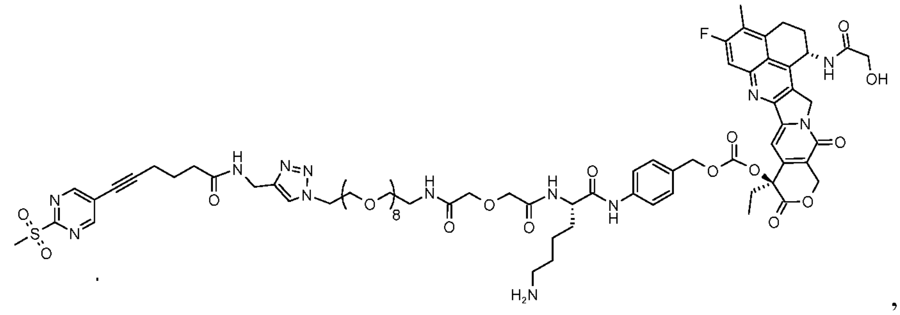 ,

A-30: 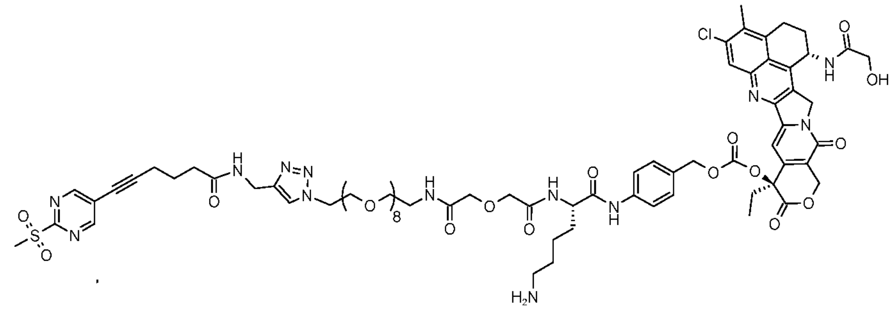 ,

A-31: 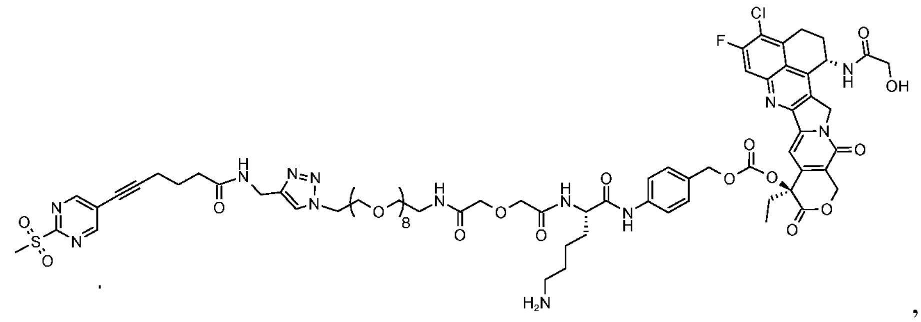 ,

B-1: 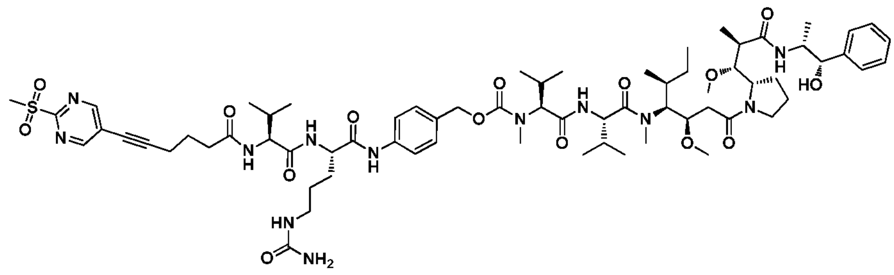 ,

B-2: 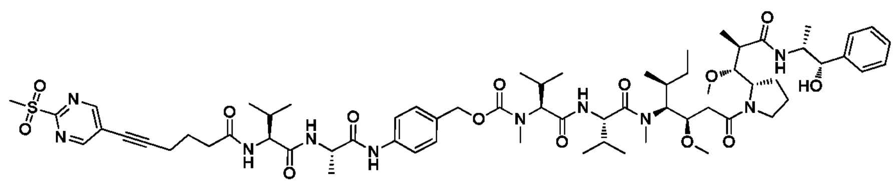 ,

B-3: 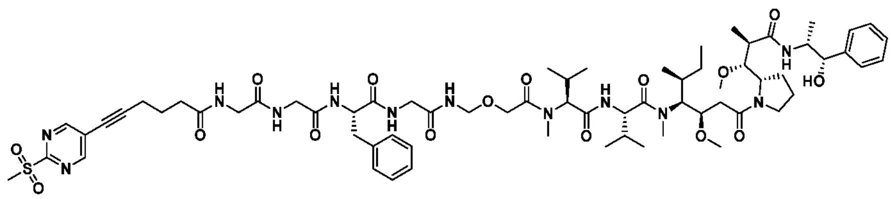 .

In some embodiments, the antibody or antigen-binding fragment thereof comprises:

(1) the following heavy chain variable region (VH) and/or light chain variable region (VL), wherein the CDRs are defined by the Chothia numbering system:

(1a) a heavy chain variable region (VH) comprising the following 3 CDRs: CDR-H1 having a sequence as set forth in SEQ ID NO: 3 or a variant thereof, CDR-H2 having a sequence as set forth in SEQ ID NO: 4 or a variant thereof, CDR-H3 having a sequence as set forth in SEQ ID NO: 5 or a variant thereof; and/or, a light chain variable region (VL) comprising the following 3 CDRs: CDR-L1 having a sequence as set forth in SEQ ID NO: 6 or a variant thereof, CDR-L2 having a sequence as set forth in SEQ ID NO: 7 or a variant thereof, CDR-L3 having a sequence as set forth in SEQ ID NO: 8 or a variant thereof; or,

(1b) a heavy chain variable region (VH) comprising the following 3 CDRs: CDR-H1 having a sequence as set forth in SEQ ID NO: 11 or a variant thereof, CDR-H2 having a sequence as set forth in SEQ ID NO: 12 or a variant thereof, CDR-H3 having a sequence as set forth in SEQ ID NO: 13 or a variant thereof; and/or, a light chain variable region (VL) comprising the following 3 CDRs: CDR-L1 having a sequence as set forth in SEQ ID NO: 14 or a variant thereof, CDR-L2 having a sequence as set forth in SEQ ID NO: 15 or a variant thereof, CDR-L3 having a sequence as set forth in SEQ ID NO: 16 or a variant thereof; or,

(1c) a heavy chain variable region (VH) comprising the following 3 CDRs: CDR-H1 having a sequence as set

forth in SEQ ID NO: 19 or a variant thereof, CDR-H2 having a sequence as set forth in SEQ ID NO: 20 or a variant thereof, CDR-H3 having a sequence as set forth in SEQ ID NO: 21 or a variant thereof; and/or, a light chain variable region (VL) comprising the following 3 CDRs: CDR-L1 having a sequence as set forth in SEQ ID NO: 14 or a variant thereof, CDR-L2 having a sequence as set forth in SEQ ID NO: 15 or a variant thereof, and CDR-L3 having a sequence as set forth in SEQ ID NO: 16 or a variant thereof;

wherein, the variant described in any one of items (1a), (1b) and (1c) has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletion or addition of one or several amino acids (e.g., a substitution, deletion or addition of 1, 2 or 3 amino acids) as compared to the sequence from which it is derived; preferably, the substitution is a conservative substitution;
or,

(2) the following heavy chain variable region (VH) and/or light chain variable region (VL), wherein the CDRs are defined by the AbM numbering system:

(2a) a heavy chain variable region (VH) comprising the following 3 CDRs: CDR-H1 having a sequence as set forth in SEQ ID NO: 29 or a variant thereof, CDR-H2 having a sequence as set forth in SEQ ID NO: 30 or a variant thereof, CDR-H3 having a sequence as set forth in SEQ ID NO: 5 or a variant thereof; and/or, a light chain variable region (VL) comprising the following 3 CDRs: CDR-L1 having a sequence as set forth in SEQ ID NO: 6 or a variant thereof, CDR-L2 having a sequence as set forth in SEQ ID NO: 7 or a variant thereof, CDR-L3 having a sequence as set forth in SEQ ID NO: 8 or a variant thereof; or,

(2b) a heavy chain variable region (VH) comprising the following 3 CDRs: CDR-H1 having a sequence as set forth in SEQ ID NO: 36 or a variant thereof, CDR-H2 having a sequence as set forth in SEQ ID NO: 37 or a variant thereof, CDR-H3 having a sequence as set forth in SEQ ID NO: 13 or a variant thereof; and/or, a light chain variable region (VL) comprising the following 3 CDRs: CDR-L1 having a sequence as set forth in SEQ ID NO: 14 or a variant thereof, CDR-L2 having a sequence as set forth in SEQ ID NO: 15 or a variant thereof, CDR-L3 having a sequence as set forth in SEQ ID NO: 16 or a variant thereof; or,

(2c) a heavy chain variable region (VH) comprising the following 3 CDRs: CDR-H1 having a sequence as set forth in SEQ ID NO: 45 or a variant thereof, CDR-H2 having a sequence as set forth in SEQ ID NO: 46 or a variant thereof, CDR-H3 having a sequence as set forth in SEQ ID NO: 21 or a variant thereof; and/or, a light chain variable region (VL) comprising the following 3 CDRs: CDR-L1 having a sequence as set forth in SEQ ID NO: 14 or a variant thereof, CDR-L2 having a sequence as set forth in SEQ ID NO: 15 or a variant thereof, and CDR-L3 having a sequence as set forth in SEQ ID NO: 16 or a variant thereof;

wherein, the variant described in any one of items (2a), (2b) and (2c) has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletion or addition of one or several amino acids (e.g., a substitution, deletion or addition of 1, 2 or 3 amino acids) as compared to the sequence from which it is derived; preferably, the substitution is a conservative substitution;
or,

(4) the following heavy chain variable region (VH) and/or light chain variable region (VL), wherein the CDRs are defined according to the Kabat numbering system:

(3a) a heavy chain variable region (VH) comprising the following 3 CDRs: CDR-H1 having a sequence as set forth in SEQ ID NO: 31 or a variant thereof, CDR-H2 having a sequence as set forth in SEQ ID NO: 32 or a variant thereof, CDR-H3 having a sequence as set forth in SEQ ID NO: 5 or a variant thereof; and/or, a light chain variable region (VL) comprising the following 3 CDRs: CDR-L1 having a sequence as set forth in SEQ ID NO: 6 or a variant thereof, CDR-L2 having a sequence as set forth in SEQ ID NO: 7 or a variant thereof, CDR-L3 having a sequence as set forth in SEQ ID NO: 8 or a variant thereof; or,

(3b) a heavy chain variable region (VH) comprising the following 3 CDRs: CDR-H1 having a sequence as set forth in SEQ ID NO: 38 or a variant thereof, CDR-H2 having a sequence as set forth in SEQ ID NO: 39 or a

variant thereof, CDR-H3 having a sequence as set forth in SEQ ID NO: 13 or a variant thereof; and/or, a light chain variable region (VL) comprising the following 3 CDRs: CDR-L1 having a sequence as set forth in SEQ ID NO: 14 or a variant thereof, CDR-L2 having a sequence as set forth in SEQ ID NO: 15 or a variant thereof, CDR-L3 having a sequence as set forth in SEQ ID NO: 16 or a variant thereof; or,

(3c) a heavy chain variable region (VH) comprising the following 3 CDRs: CDR-H1 having a sequence as set forth in SEQ ID NO: 47 or a variant thereof, CDR-H2 having a sequence as set forth in SEQ ID NO: 48 or a variant thereof, CDR-H3 having a sequence as set forth in SEQ ID NO: 21 or a variant thereof; and/or, a light chain variable region (VL) comprising the following 3 CDRs: CDR-L1 having a sequence as set forth in SEQ ID NO: 14 or a variant thereof, CDR-L2 having a sequence as set forth in SEQ ID NO: 15 or a variant thereof, and CDR-L3 having a sequence as set forth in SEQ ID NO: 16 or a variant thereof;

wherein, the variant described in any one of items (3a), (3b) and (3c) has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletion or addition of one or several amino acids (e.g., a substitution, deletion or addition of 1, 2 or 3 amino acids) as compared to the sequence from which it is derived; preferably, the substitution is a conservative substitution;
or,

(4) the following heavy chain variable region (VH) and/or light chain variable region (VL), wherein the CDRs are defined by the IMGT numbering system:

(4a) a heavy chain variable region (VH) comprising the following 3 CDRs: CDR-H1 having a sequence as set forth in SEQ ID NO: 24 or a variant thereof, CDR-H2 having a sequence as set forth in SEQ ID NO: 25 or a variant thereof, CDR-H3 having a sequence as set forth in SEQ ID NO: 26 or a variant thereof; and/or, a light chain variable region (VL) comprising the following 3 CDRs: CDR-L1 having a sequence as set forth in SEQ ID NO: 27 or a variant thereof, CDR-L2 having a sequence as set forth in SEQ ID NO: 28 or a variant thereof, CDR-L3 having a sequence as set forth in SEQ ID NO: 8 or a variant thereof; or,

(4b) a heavy chain variable region (VH) comprising the following 3 CDRs: CDR-H1 having a sequence as set forth in SEQ ID NO: 33 or a variant thereof, CDR-H2 having a sequence as set forth in SEQ ID NO: 34 or a variant thereof, CDR-H3 having a sequence as set forth in SEQ ID NO: 35 or a variant thereof; and/or, a light chain variable region (VL) comprising the following 3 CDRs: CDR-L1 having a sequence as set forth in SEQ ID NO: 43 or a variant thereof, CDR-L2 having a sequence as set forth in SEQ ID NO: 44 or a variant thereof, CDR-L3 having a sequence as set forth in SEQ ID NO: 16 or a variant thereof; or,

(4c) a heavy chain variable region (VH) comprising the following 3 CDRs: CDR-H1 having a sequence as set forth in SEQ ID NO: 40 or a variant thereof, CDR-H2 having a sequence as set forth in SEQ ID NO: 41 or a variant thereof, CDR-H3 having a sequence as set forth in SEQ ID NO: 42 or a variant thereof; and/or, a light chain variable region (VL) comprising the following 3 CDRs: CDR-L1 having a sequence as set forth in SEQ ID NO: 43 or a variant thereof, CDR-L2 having a sequence as set forth in SEQ ID NO: 44 or a variant thereof, and CDR-L3 having a sequence as set forth in SEQ ID NO: 16 or a variant thereof;

Wherein, the variant described in any one of items (4a), (4b) of (4c) has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletion or addition of one or several amino acids (e.g., a substitution, deletion or addition of 1, 2 or 3 amino acids) as compared to the sequence from which it is derived; preferably, the substitution is a conservative substitution.

In some embodiments, the antibody or antigen-binding fragment thereof comprises:

(a) a VH as set forth in SEQ ID NO: 1 or a variant thereof, and/or, a VL as set forth in SEQ ID NO: 2 or a variant thereof;

(b) a VH as set forth in SEQ ID NO: 9 or a variant thereof, and/or, a VL as set forth in SEQ ID NO: 10 or a variant thereof; or

(c) a VH as set forth in SEQ ID NO: 17 or a variant thereof, and/or, a VL as set forth in SEQ ID NO: 18 or a variant thereof;

wherein the variant has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletion or addition of one or several amino acids (e.g., a substitution, deletion or addition of 1, 2, 3, 4 or 5 amino acids) as compared to the sequence from which it is derived; preferably, the substitution is a conservative substitution.

In some embodiments, the antibody or antigen-binding fragment thereof further comprises:

(a) a human immunoglobulin heavy chain constant region (CH) or a variant thereof, the variant has a substitution, deletion or addition of one or more amino acids (e.g., a substitution, deletion or addition of up to 20, up to 15, up to 10, or up to 5 amino acids; for example, a substitution, deletion or addition of 1, 2, 3, 4 or 5 amino acids) as compared to the wild-type sequence from which it is derived; and

(b) a human immunoglobulin light chain constant region (CL) or a variant thereof, the variant has a substitution, deletion or addition of one or more amino acids (e.g., a substitution, deletion or addition of up to 20, up to 15, up to 10, or up to 5 amino acids; for example, a substitution, deletion or addition of 1, 2, 3, 4 or 5 amino acids) as compared to the wild-type sequence from which it is derived.

In some embodiments, the heavy chain constant region is an IgG heavy chain constant region, for example, an IgG1, IgG2, IgG3 or IgG4 heavy chain constant region, for example, a human IgG1 heavy chain constant region or a human IgG4 heavy chain constant region. In some embodiments, the antibody or antigen-binding fragment thereof comprises a heavy chain constant region (CH) as set forth in SEQ ID NO: 22 or a variant thereof, the variant has a conservative substitution of one or more amino acids (e.g., a conservative substitution of up to 20, up to 15, up to 10, or up to 5 amino acids; for example, a conservative substitution of 1, 2, 3, 4 or 5 amino acids) as compared to SEQ ID NO: 22.

In some embodiments, the light chain constant region is a κ light chain constant region. In some embodiments, the antibody or antigen-binding fragment thereof comprises a light chain constant region (CL) as set forth in SEQ ID NO: 23 or a variant thereof, the variant has a conservative substitution of one or more amino acids (e.g., a conservative substitution of up to 20, up to 15, up to 10, or up to 5 amino acids; for example, a conservative substitution of 1, 2, 3, 4 or 5 amino acids) as compared to SEQ ID NO: 23.

In some embodiments, the antibody or antigen-binding fragment thereof comprises a heavy chain constant region (CH) as set forth in SEQ ID NO:22 and a light chain constant region (CL) as set forth in SEQ ID NO:23.

In some embodiments, the antibody or antigen-binding fragment thereof comprises:

(1) a heavy chain comprising a VH having a sequence as set forth in SEQ ID NO: 1 and a heavy chain constant region (CH) as set forth in SEQ ID NO: 22, and, a light chain comprising a VL having a sequence as set forth in SEQ ID NO: 2 and a light chain constant region (CL) as set forth in SEQ ID NO: 23;

(2) a heavy chain comprising a VH having a sequence as set forth in SEQ ID NO: 9 and a heavy chain constant region (CH) as set forth in SEQ ID NO: 22, and, a light chain comprising a VL having a sequence as set forth in SEQ ID NO: 10 and a light chain constant region (CL) as set forth in SEQ ID NO: 23; or

(3) a heavy chain comprising a VH having a sequence as set forth in SEQ ID NO: 17 and a heavy chain constant region (CH) as set forth in SEQ ID NO: 22, and, a light chain comprising a VL having a sequence as set forth in SEQ ID NO: 18 and a light chain constant region (CL) as set forth in SEQ ID NO: 23.

In some embodiments, the antibody-drug conjugate is selected from ADC A-1 to ADC A-31 and ADC B-1 to ADC B-3 as shown below:

ADC A-1:

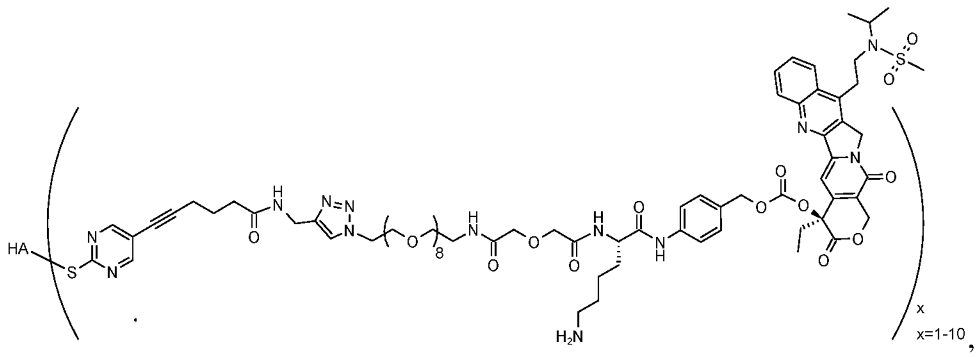

ADC A-2:

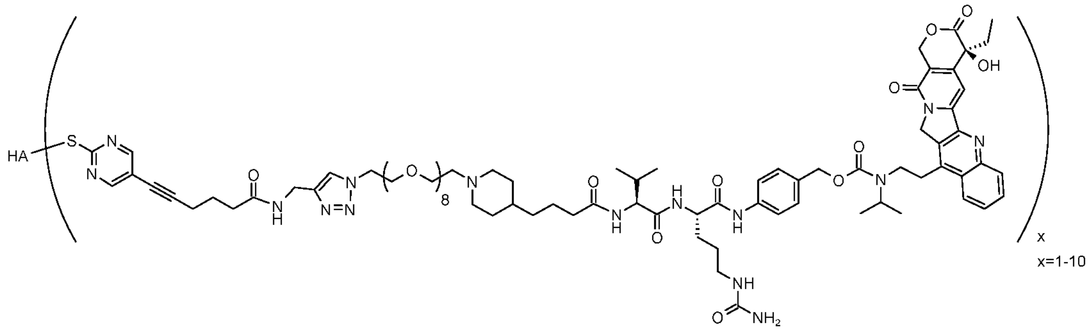

ADC A-3:

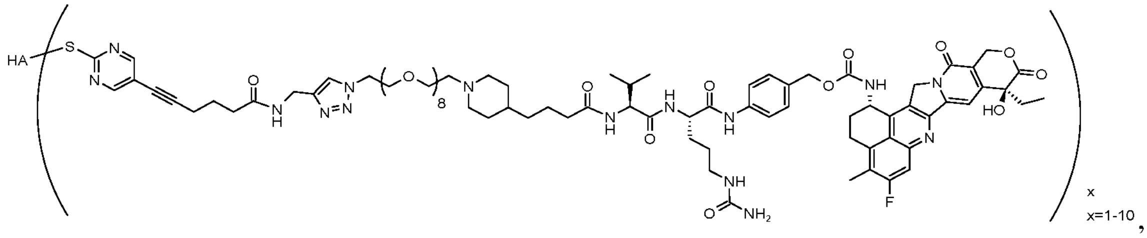

ADC A-4:

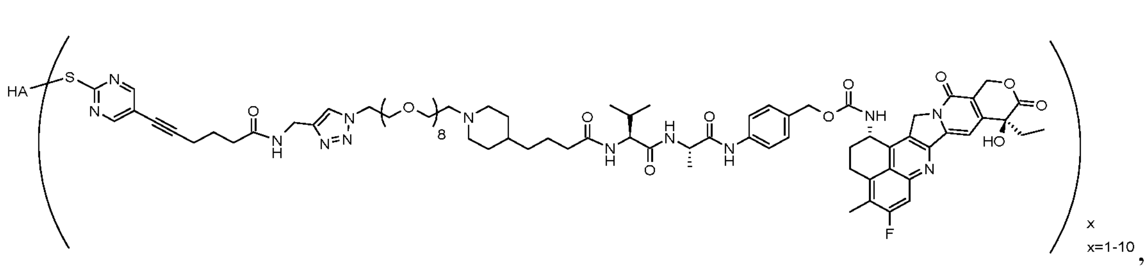

ADC A-5:

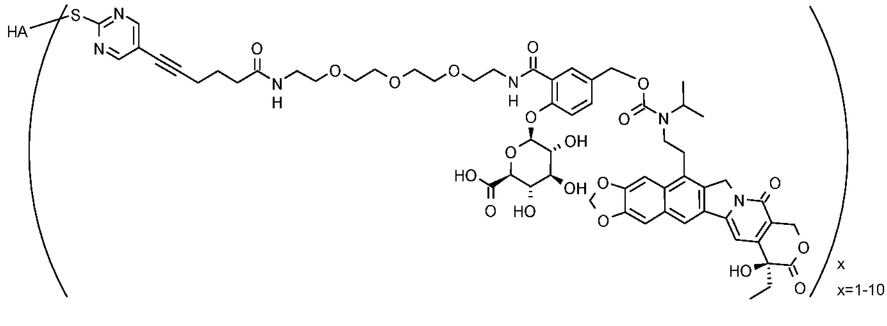

,

ADC A-6:

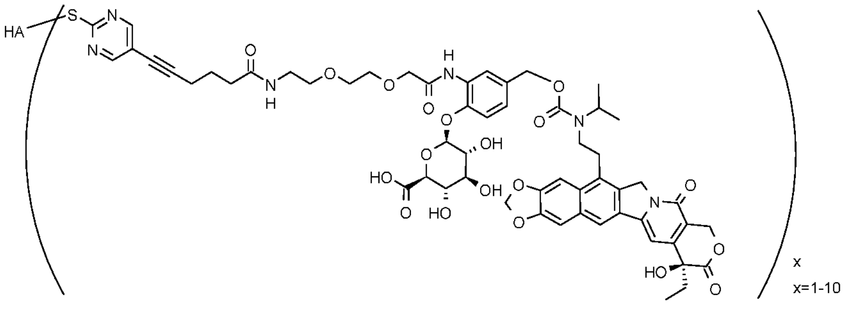

,

ADC A-7:

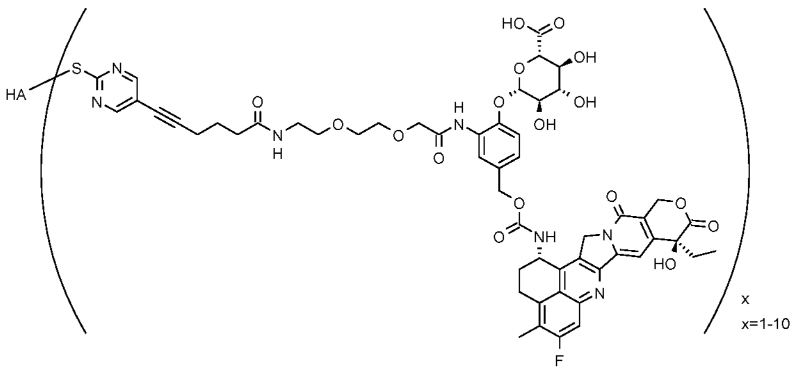

,

ADC A-8:

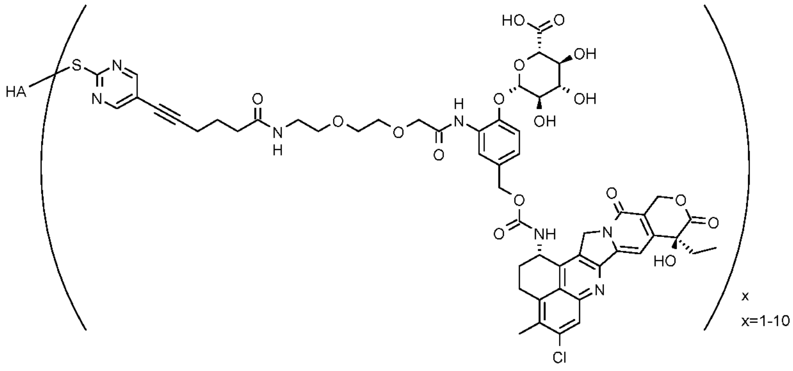

,

ADC A-9:

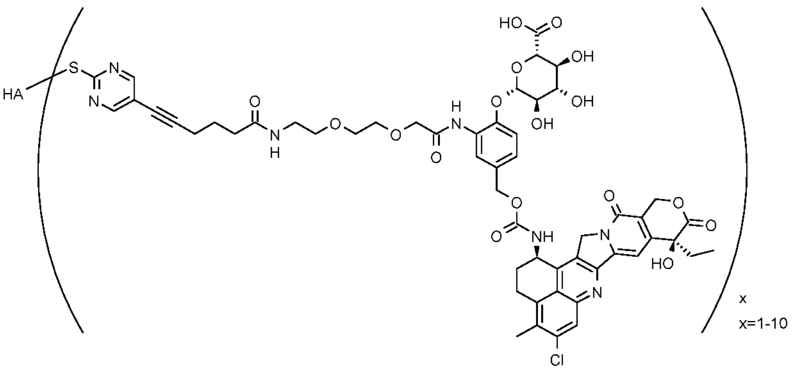

ADC A-10:

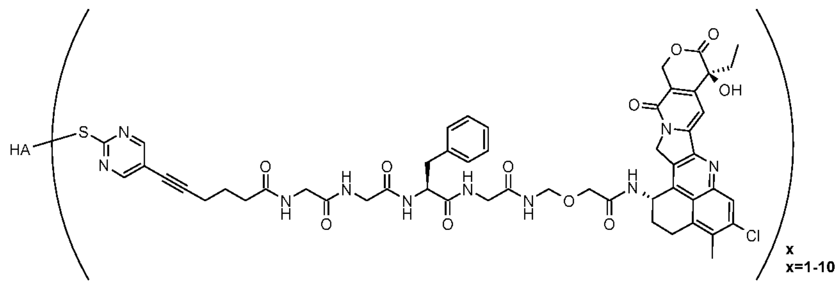

,

ADC A-11:

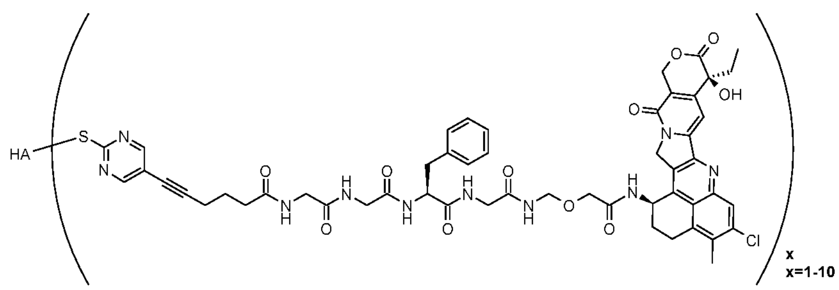

,

ADC A-12:

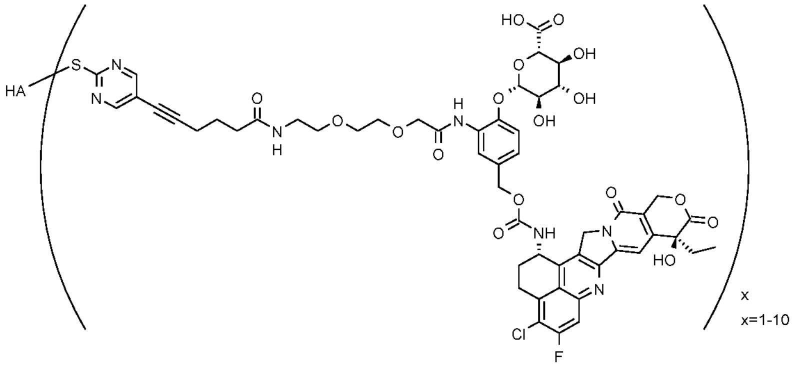

,

ADC A-13:

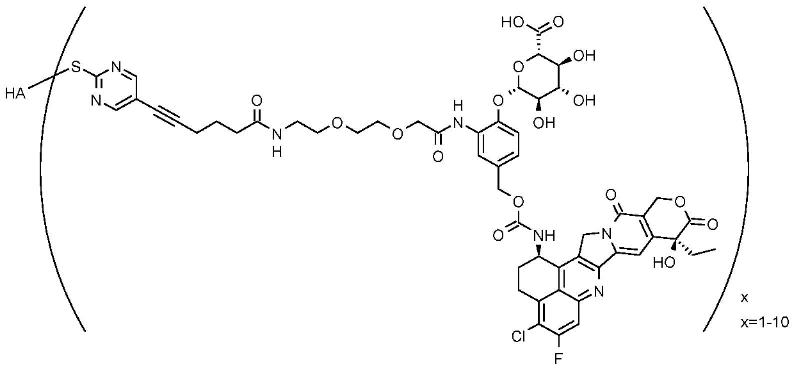

,

ADC A-14:

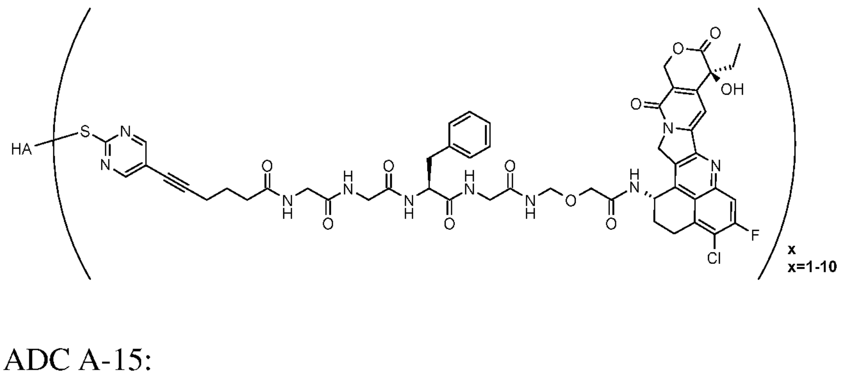

,

ADC A-15:

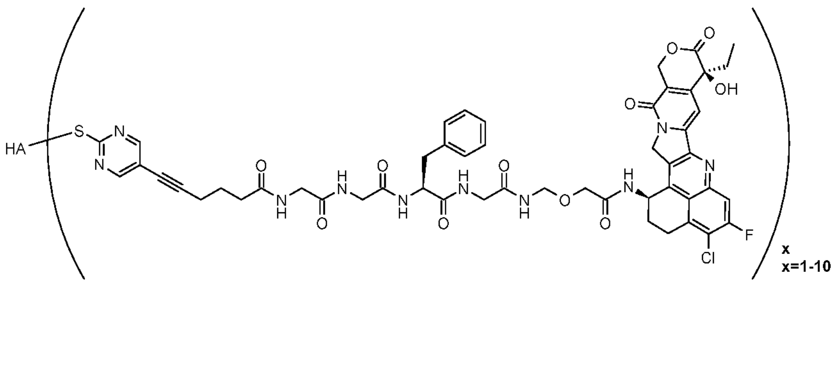

,

ADC A-16:

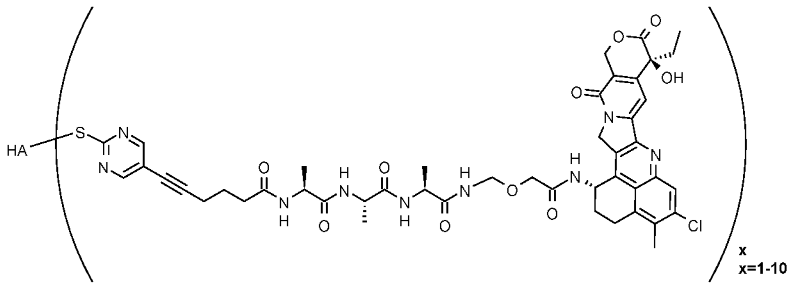

,

ADC A-17:

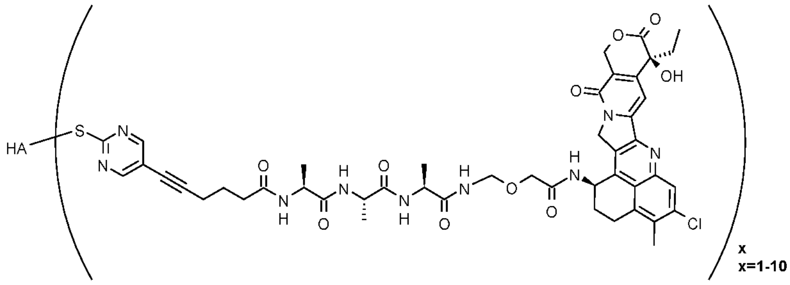

,

ADC A-18:

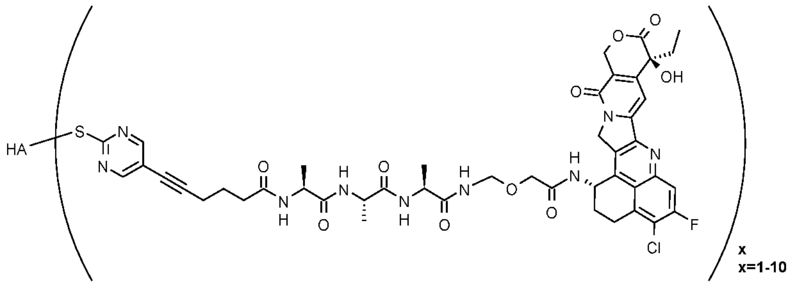

,

ADC A-19:

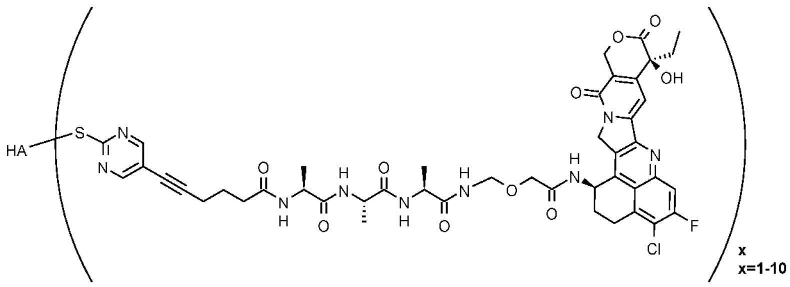

,

ADC A-20:

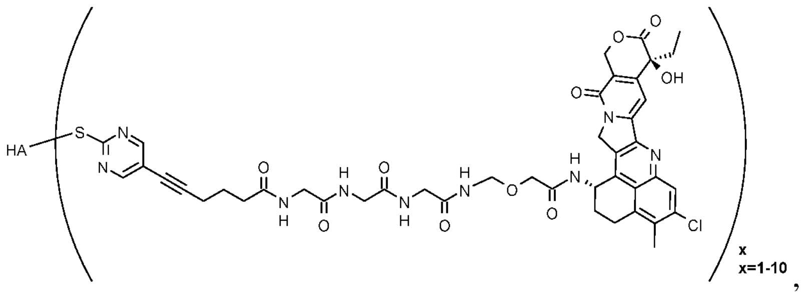

ADC A-21:

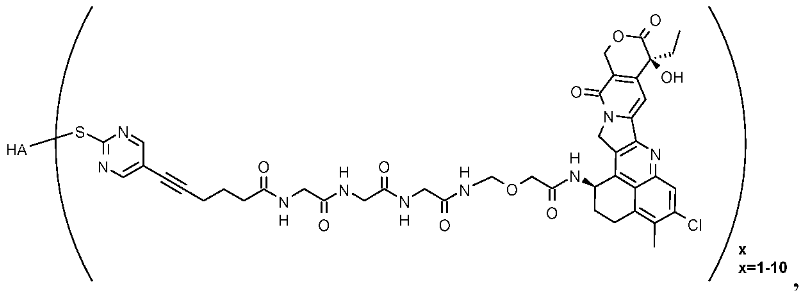

ADC A-22:

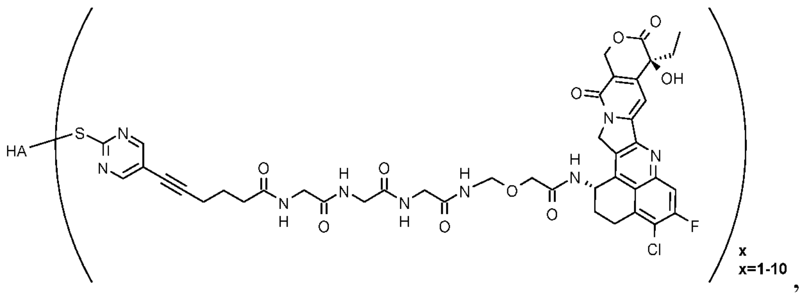

ADC A-23:

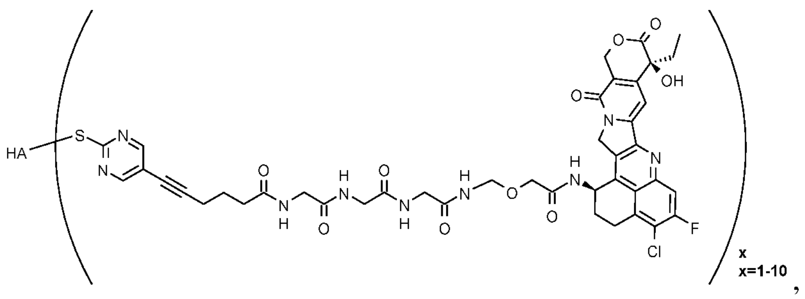

ADC A-24:

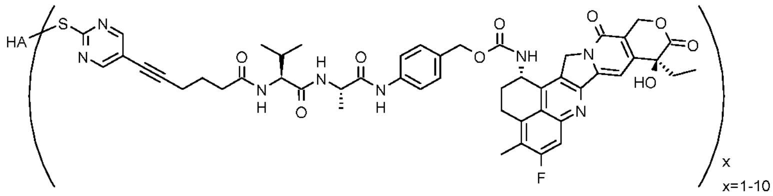

,

ADC A-25:

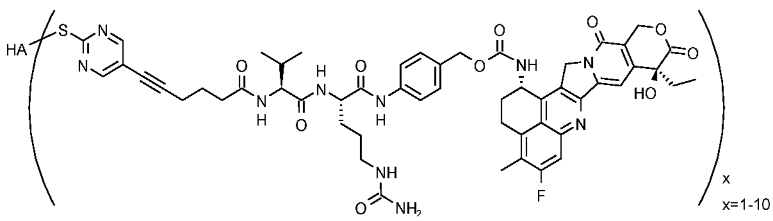

,

ADC A-26:

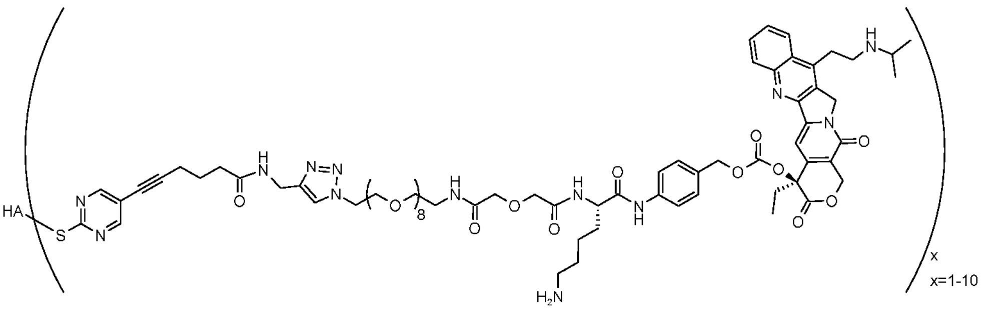

,

ADC A-27:

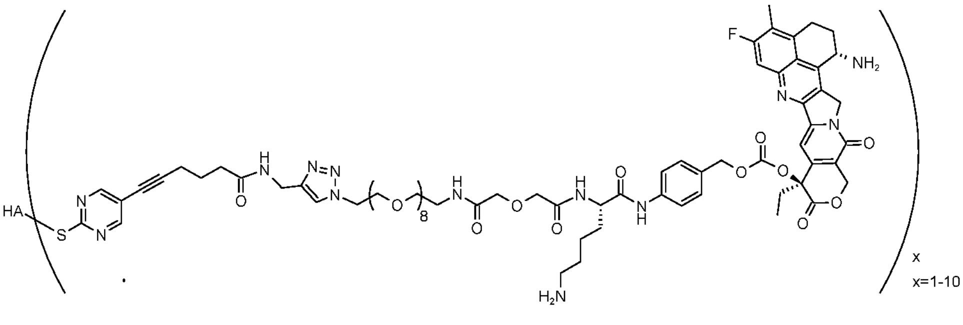

,

ADC A-28:

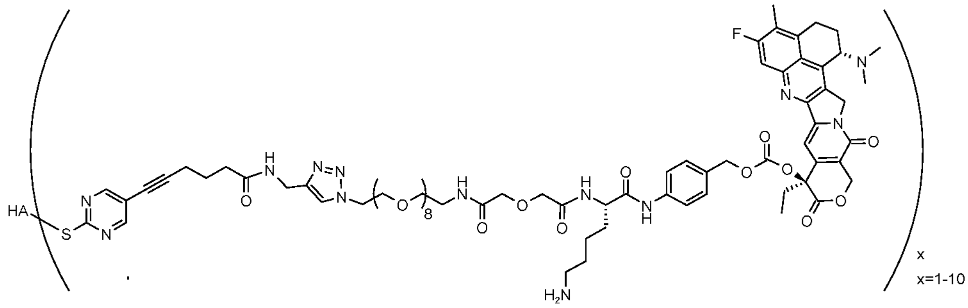

,

ADC A-29:

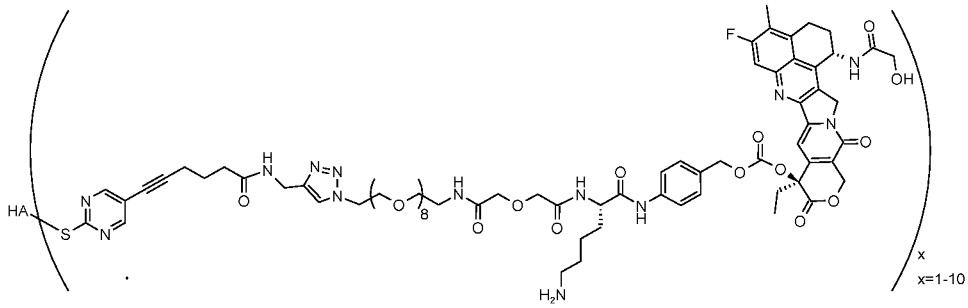

,

ADC A-30:

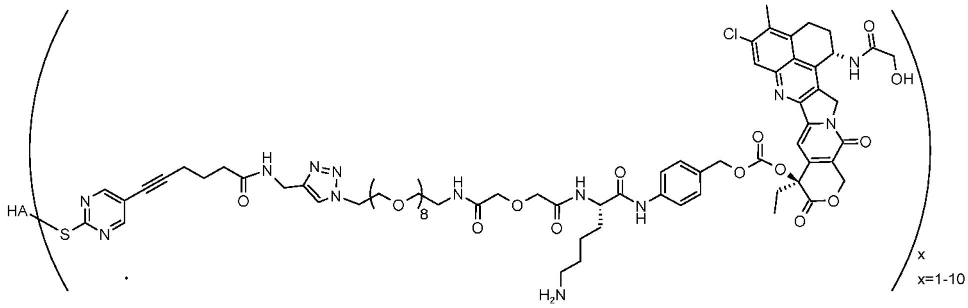

,

ADC A-31:

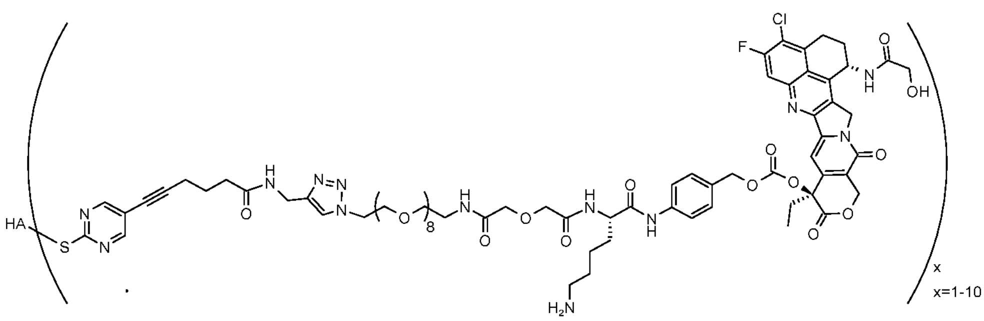

,

ADC B-1:

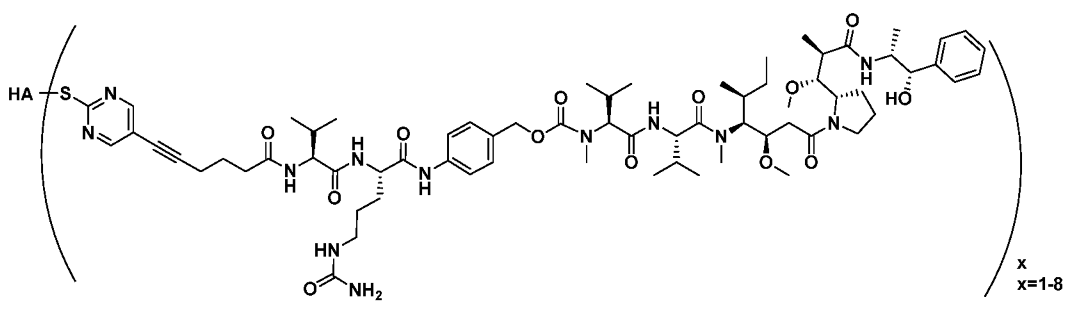

,

ADC B-2:

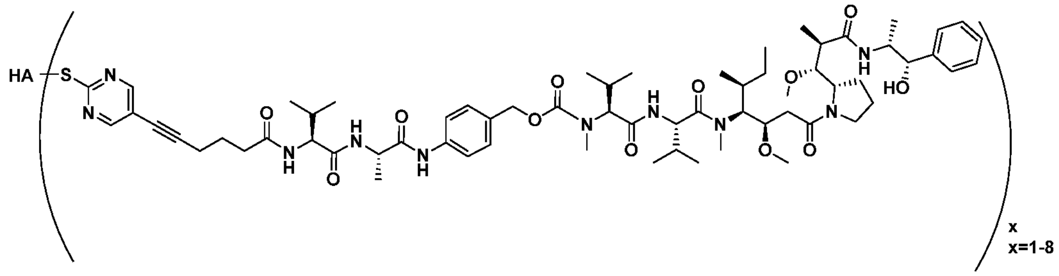

,

ADC B-3:

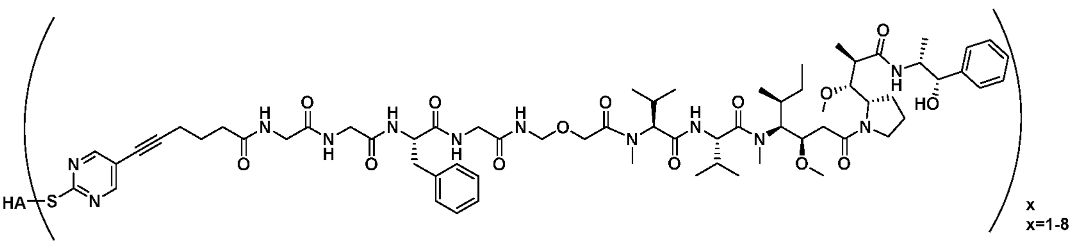

,

wherein, HA in each antibody-drug conjugate represents an antibody or antigen-binding fragment thereof comprising a VH as set forth in SEQ ID NO: 1 and a VL as set forth in SEQ ID NO: 2, for example, 19F6_Hu35v1 (which comprises a heavy chain comprising a VH as set forth in SEQ ID NO: 1 and a CH as set forth in SEQ ID NO: 22, and a light chain comprising a VL as set forth in SEQ ID NO: 2 and a CL as set forth in SEQ ID NO: 23); wherein,

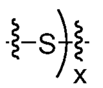

represents the specific connection mode between a sulfhydryl group of the antibody or antigen-binding fragment thereof and the connector.

In some embodiments, the antibody-drug conjugate has a DAR value (drug-to-antibody ratio) of 1-10, for example: 1 to 2, 1 to 3, 1 to 4, 1 to 5, 1 to 6, 1 to 7, 1 to 8, 1 to 9, 1 to 10, 2 to 3, 2 to 4, 2 to 5, 2 to 6, 2 to 7, 2 to 8, 2 to 9, 2 to 10, 3 to 4, 3 to 5, 3 to 6, 3 to 7, 3 to 8, 3 to 9, 3 to 10, 4 to 5, 4 to 6, 4 to 7, 4 to 8, 4 to 9, 4 to 10, 5 to 6, 5 to 7, 5 to 8, 5 to 9, 5 to 10, 6 to 7, 6 to 8, 6 to 9, 6 to 10, 7 to 8, 7 to 9, 7 to 10, 8 to 9, 8 to 10, or 9 to 10, preferably 3 to 8, such as 3.0 to 3.5, 3.0 to 4.0, 3.0 to 4.5, 3.0 to 5.0, 3.0 to 5.5, 3.0 to 6.0, 3.5 to 4.0, 3.5 to 4.5, 3.5 to 5.0, 3.5 to 5.5, 3.5 to 6.0, 3.5 to 6.5, 3.5 to 7.0, 3.5 to 7.5, 3.5 to 8.0, 4.0 to 4.5, 4.0 to 5.0, 4.0 to 5.5, 4.0 to 6.0, 4.0 to 6.5, 4.0 to 7.0, 4.0 to 7.5, 4.0 to 8.0, 4.5 to 5.0, 4.5 to 5.5, 4.5 to 6.0, 4.5 to 6.5, 4.5 to 7.0, 4.5 to 7.5, 4.5 to 8.0, 5.0 to 5.5, 5.0 to 6.0, 5.0 to 6.5, 5.0 to 7.0, 5.0 to 7.5, 5.0 to 8.0, 5.5 to 6.0, 5.5 to 6.5, 5.5 to 7.0, 5.5 to 7.5, 5.5 to 8.0, 6.0 to 6.5, 6.0 to 7.0, 6.0 to 7.5, 6.0 to 8.0, 6.5 to 7.0, 6.5 to 7.5, 6.5 to 8.0, 7.0 to 7.5, 7.0 to 8.0, or 7.5 to 8.0.

Drug-linker Conjugate

In another aspect, the present application provides a drug-linker, which can be used to prepare the aforementioned antibody-drug conjugate, and the drug-linker has a structure as shown in the formula M'-L-E-D, wherein: M' represents a structure of M before it is connected to the aforementioned antibody or antigen-binding fragment thereof, and M, L, E and D are as defined in any one of the aforementioned items of the first aspect.

In some embodiments, M' is selected from the following structures:

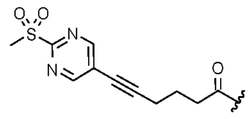 and 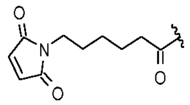 .

In some embodiments, M' is

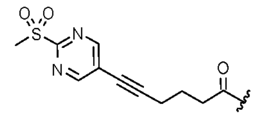 .

In some embodiments, L is a divalent structure composed of one or more of the following groups: $C_{1-6}$ alkylene, -N(R')-, carbonyl, -O-, Val, Cit, Phe, Lys, D-Val, Leu, Gly, Ala, Asn, Val-Cit, Val-Ala, Val-Lys, Val-Lys(Ac), Phe-Lys, Phe-Lys(Ac), D-Val-Leu-Lys, Gly-Gly-Arg, Ala-Ala-Asn, Ala-Ala-Ala, Val-Lys-Ala, Gly-Gly-Gly, Gly-Gly-Phe-Gly, Gly-Gly-Gly-Gly-Gly,

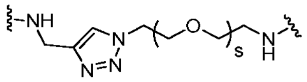 , 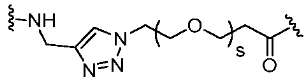 , 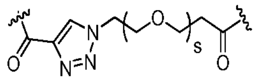 , 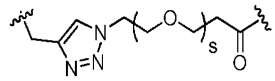 ,

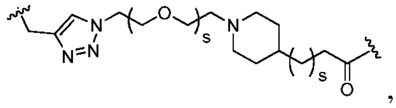 , 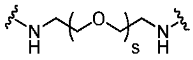 , 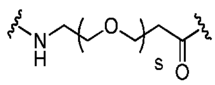 , 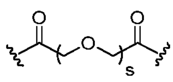 , 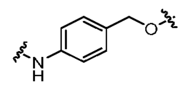 ,

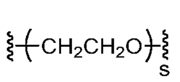 , 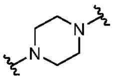 , 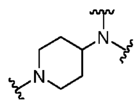 , 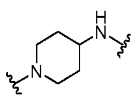 , 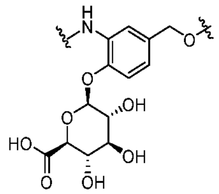 , 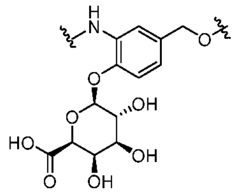 ,

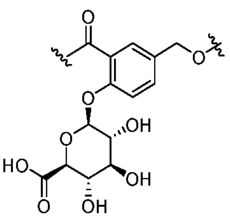 , 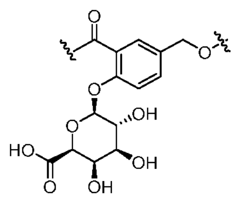 and 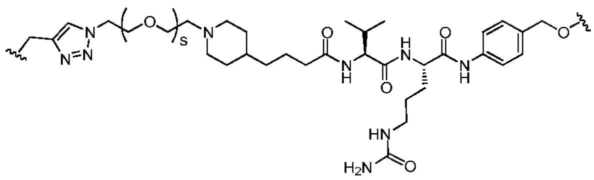 ,

wherein R' represents hydrogen, $C_{1-6}$ alkyl or a $-(CH_2CH_2O)_r-$-containing alkyl; r is an integer selected from 1-10; s is an integer selected from 1-10.

In some embodiments, L is a divalent structure composed of one or more of the following groups: $C_{1-6}$ alkylene, -N(R')-, carbonyl, -O-, Val, Cit, Phe, Lys, D-Val, Leu, Gly, Ala, Asn, Val-Cit, Val-Ala, Val-Lys, Val-Lys(Ac), Phe-Lys, Phe-Lys(Ac), D-Val-Leu-Lys, Gly-Gly-Arg, Ala-Ala-Asn, Gly-Gly-Gly, Gly-Gly-Phe-Gly, Gly-Gly-Gly-Gly-Gly,

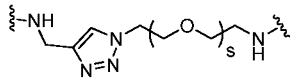,

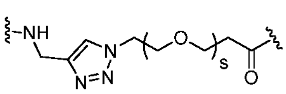, 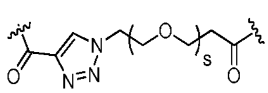, 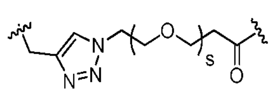, 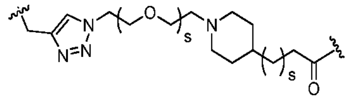,

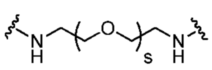, 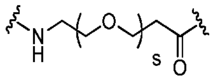, 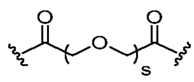, 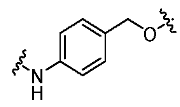, 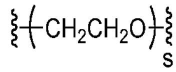, 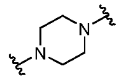,

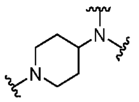, 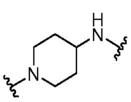, 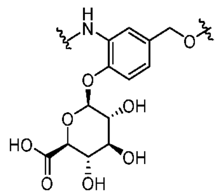, 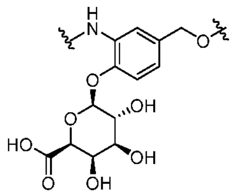, 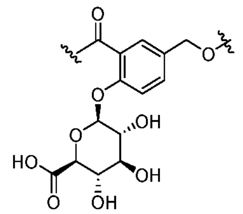,

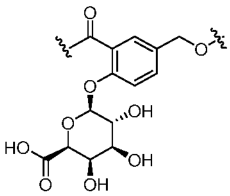 and 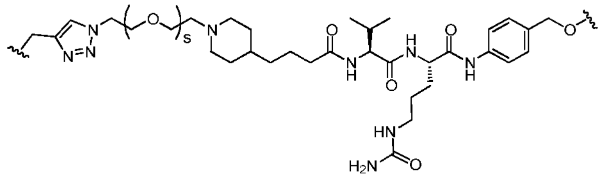,

wherein R' represents hydrogen, $C_{1-6}$ alkyl or a -$(CH_2CH_2O)_r$--containing alkyl; r is an integer selected from 1-10; s is an integer selected from 1-10.

In some embodiments, L is a structure composed of one or more of the following groups: $C_{1-6}$ alkylene, -N(R')-, carbonyl, -O-, Val, Cit, Phe, Lys, D-Val, Leu, Gly, Ala, Asn, Val-Cit, Val-Ala, Val-Lys, Val-Lys(Ac), Phe-Lys, Phe-Lys(Ac), D-Val-Leu-Lys, Gly-Gly-Arg, Ala-Ala-Asn, Ala-Ala-Ala, Val-Lys-Ala, Gly-Gly-Gly, Gly-Gly-Phe-Gly, Gly-Gly-Gly-Gly-Gly,

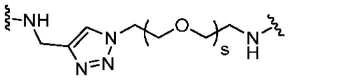, 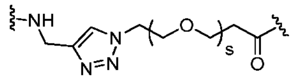, 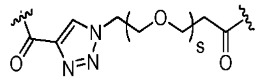,

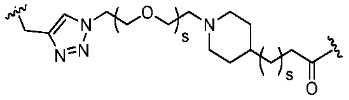, 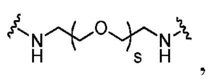, 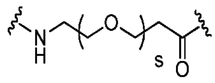, 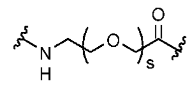, 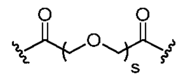,

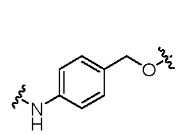, 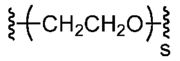, 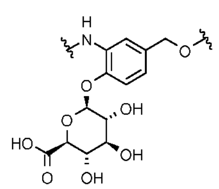 and 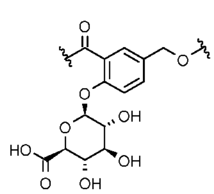;

wherein R' represents hydrogen, $C_{1-6}$ alkyl or a -$(CH_2CH_2O)_r$--containing alkyl; r is an integer selected from 1-10; s is an integer selected from 1-20; preferably, s is an integer selected from 1-10.

In some embodiments, L is a structure composed of one or more of the following groups: Val, Cit, Phe, Lys, D-Val, Leu, Gly, Ala, Asn, Val-Cit, Val-Ala, Val- Lys, Val-Lys(Ac), Phe-Lys, Phe-Lys(Ac), D-Val-Leu-Lys, Gly-Gly-Arg, Ala-Ala-Asn, Gly-Gly-Gly, Gly-Gly- Phe-Gly, Gly-Gly-Gly-Gly-Gly,

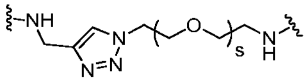, 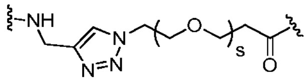, 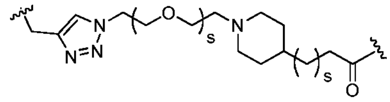,

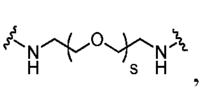, 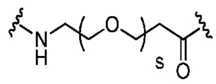, 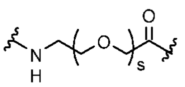, 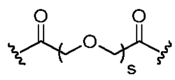, 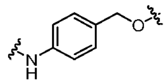, 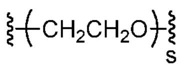,

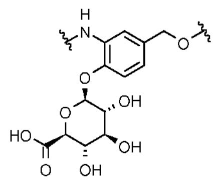 and 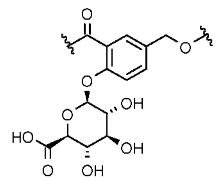 ;

wherein s is an integer selected from 1-10.

In some embodiments, L is selected from the following structures:

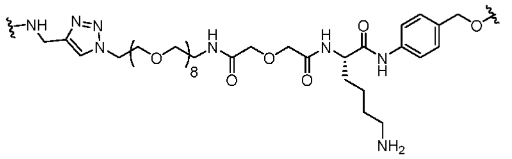, 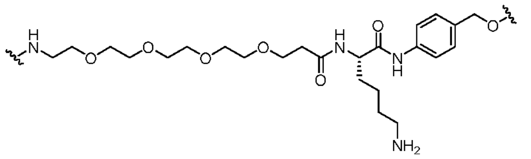,

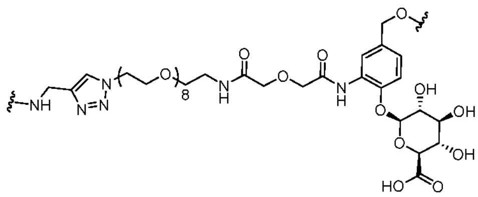, 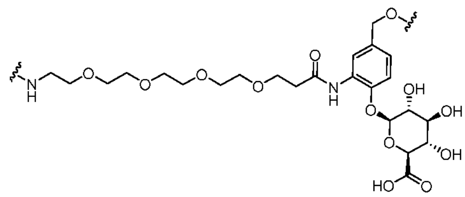,

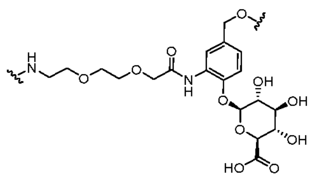, 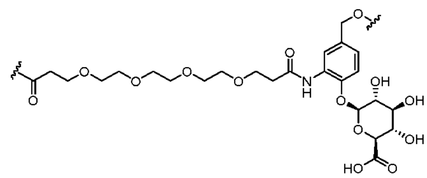, 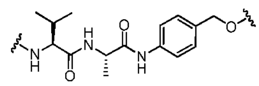,

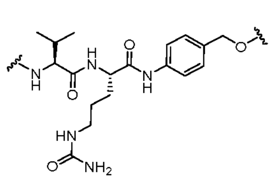, 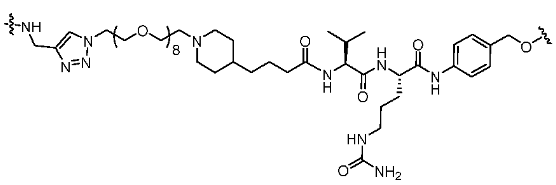,

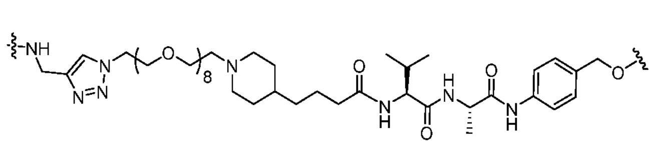, 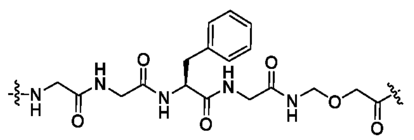,

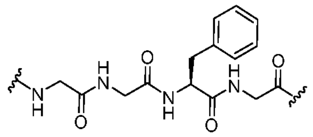 , 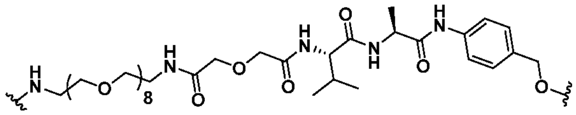 ,

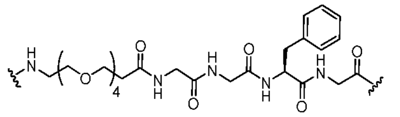 , 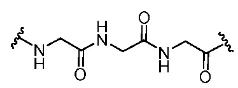 , 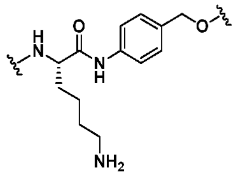 , 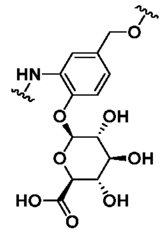 ,

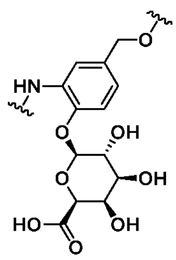 , 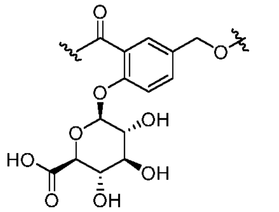 , 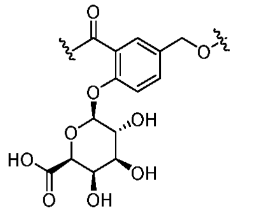 , 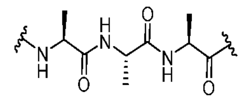 ,

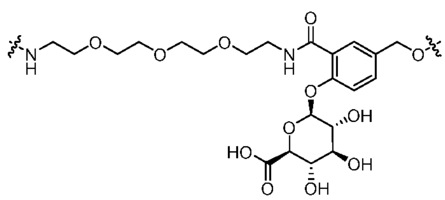 , 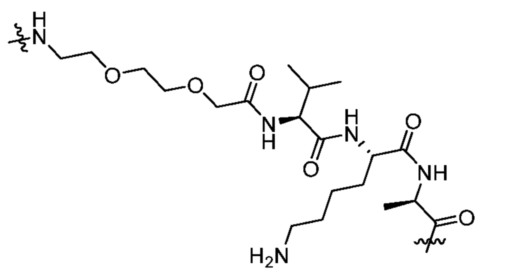 ,

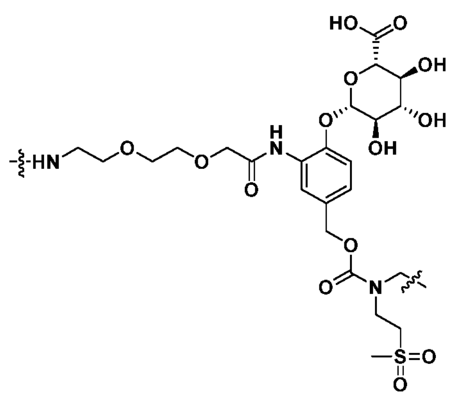 and 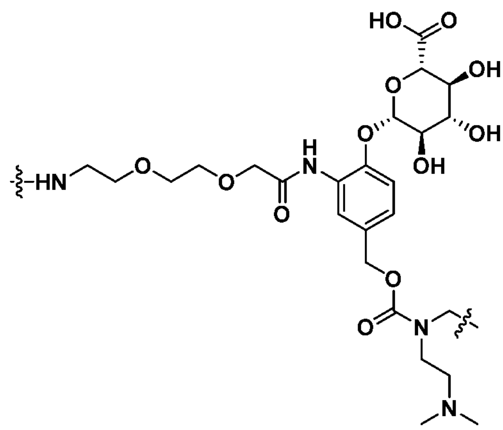 .

In some embodiments, L is selected from the following structures:

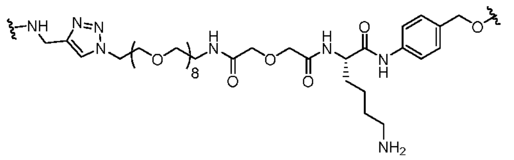 , 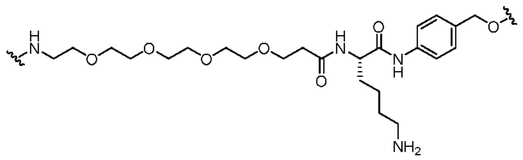 ,

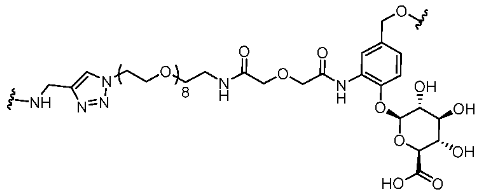,
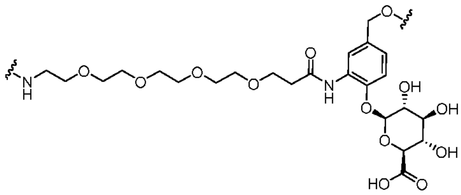,
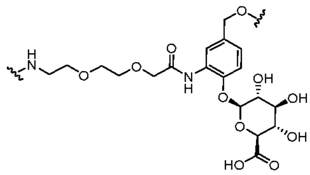,
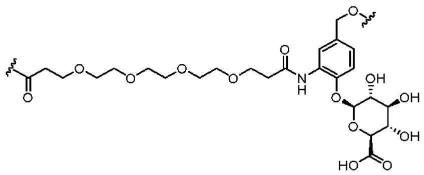,
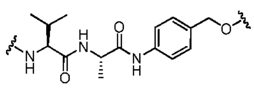,
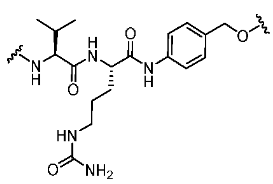,
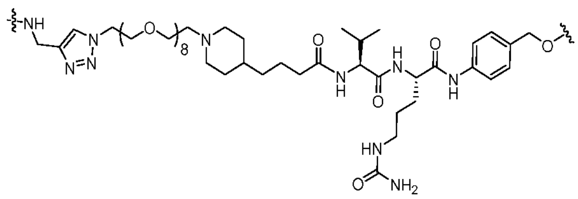,
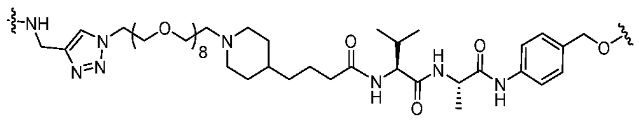,
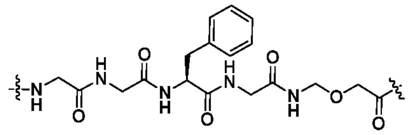,
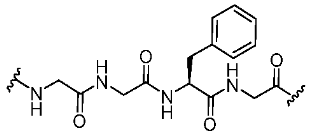,
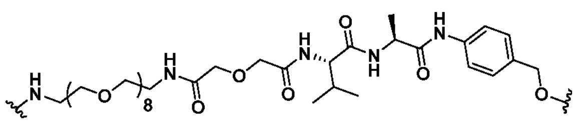,
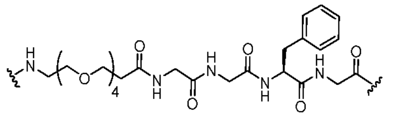,
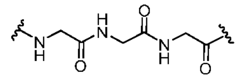,
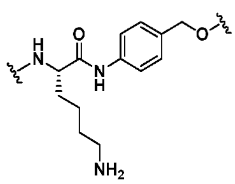,
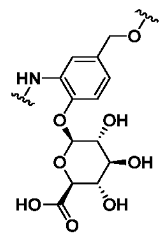,
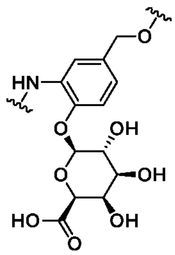,
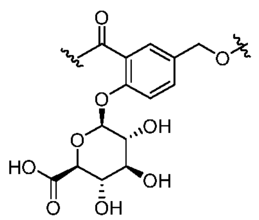 and
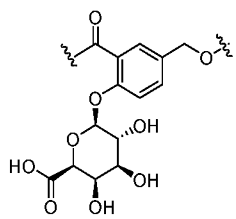.

In some embodiments, L is selected from the following structures:

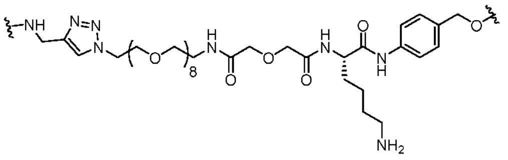,
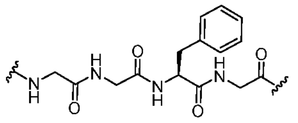,
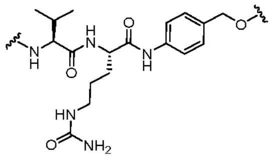,

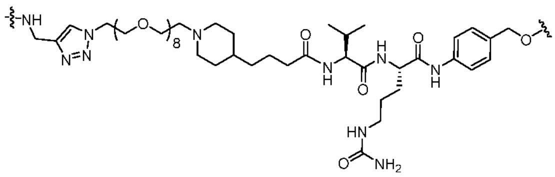,
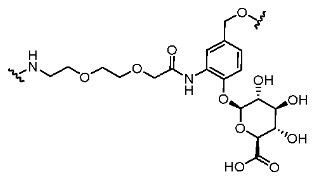,

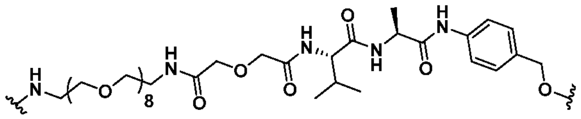

and

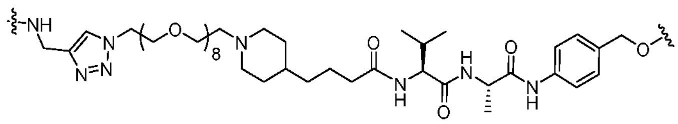.

In some embodiments, L is selected from the following structures:

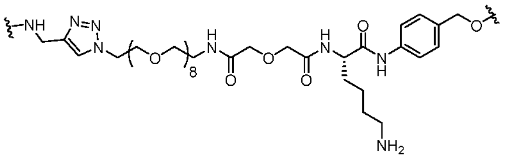,
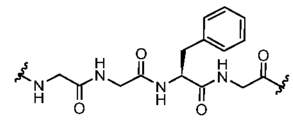,
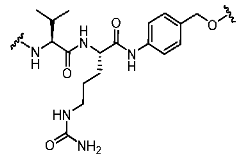,

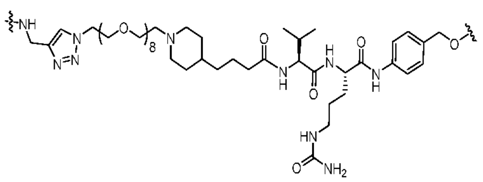,
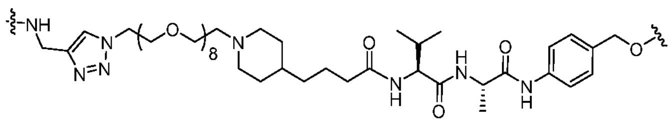,

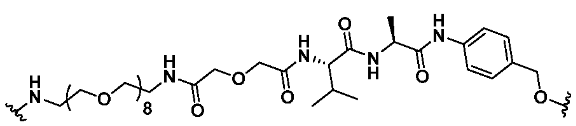,
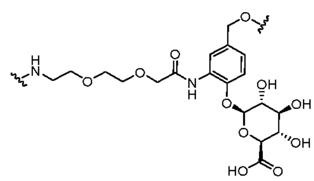,

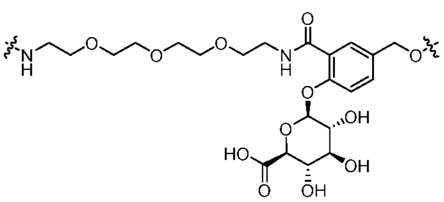

and

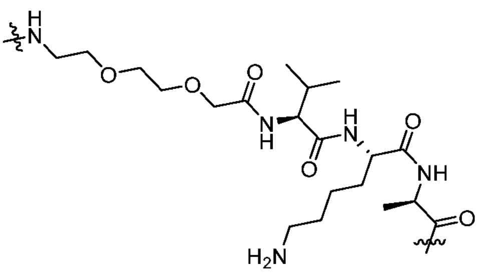.

In some embodiments, L is selected from the following structures:

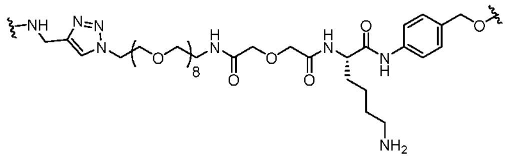, 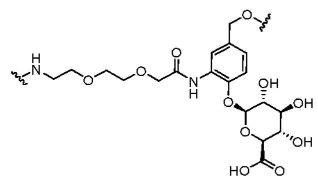, 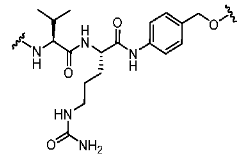,

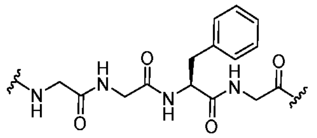, 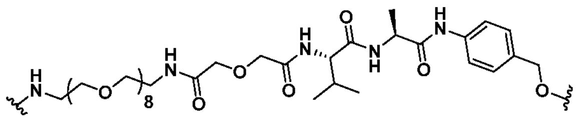,

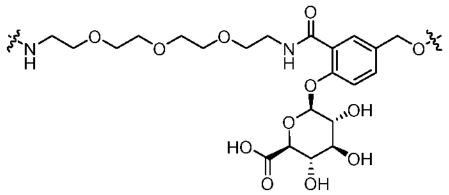 and 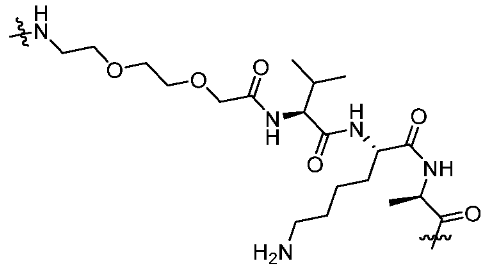.

In some embodiments, L is selected from the following structures:

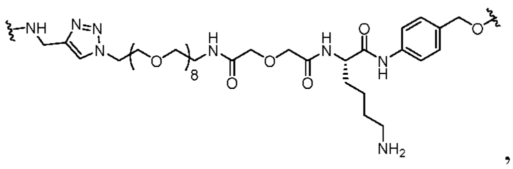, 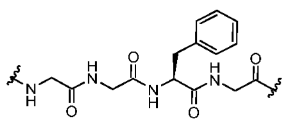, and 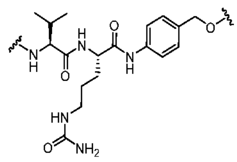.

In some embodiments, L is selected from the following structures:

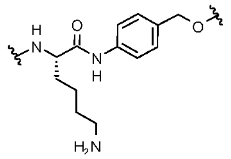, 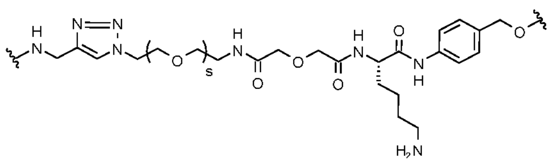, 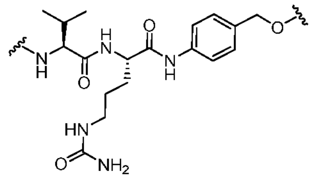,

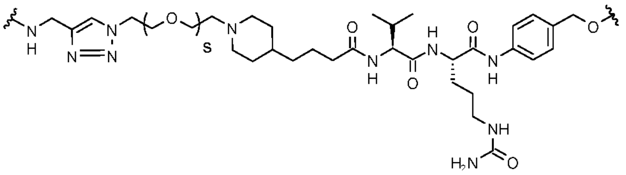, 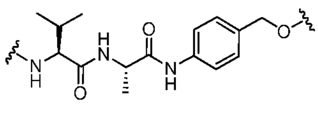,

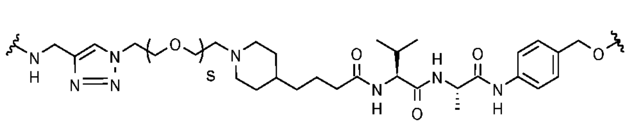, 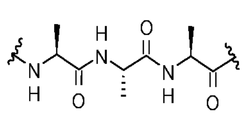, 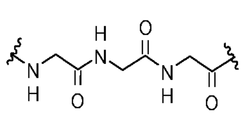,

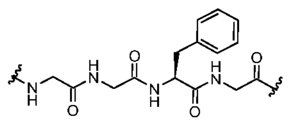, 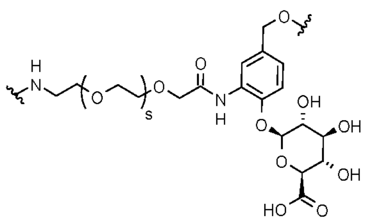 and 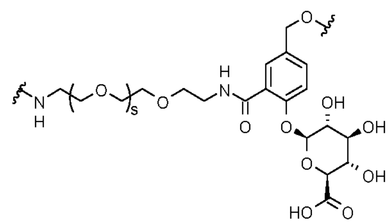 ;

wherein s is an integer selected from 1-20.

In some embodiments, L is selected from the following structures:

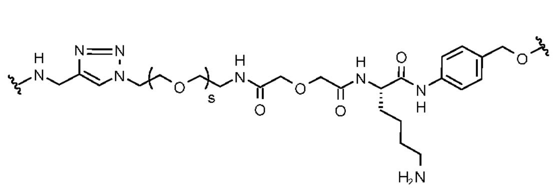 , 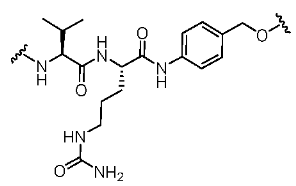 ,

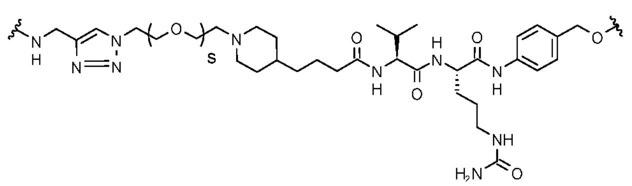 ,

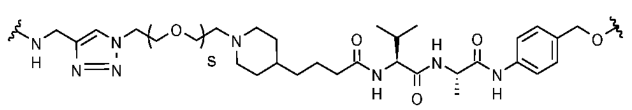 , 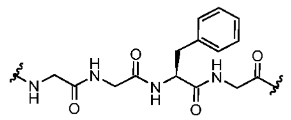 ,

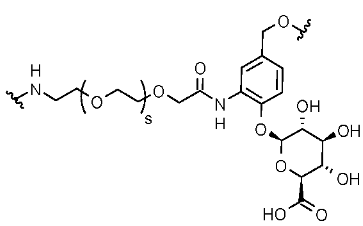 and 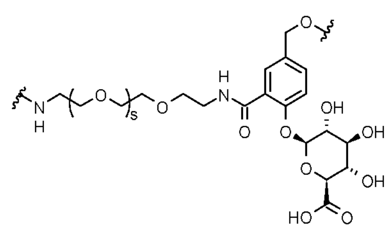 ;

wherein s is an integer selected from 1-10; preferably, s is selected from 1, 2 and 3.

In some embodiments, L is selected from the following structures:

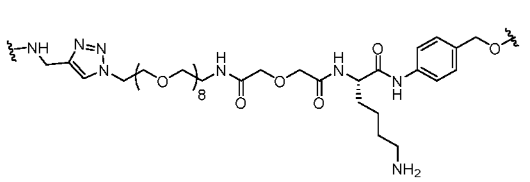 , 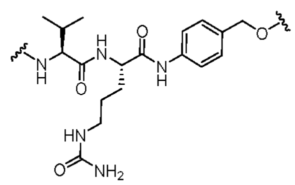 ,

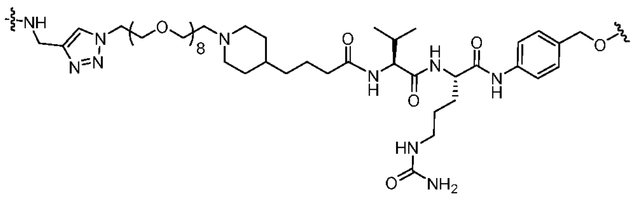

,

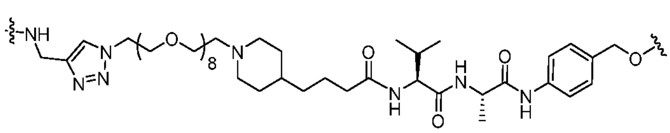

,

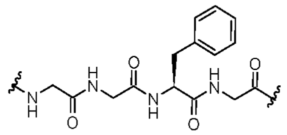

,

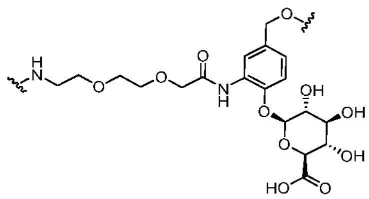

and

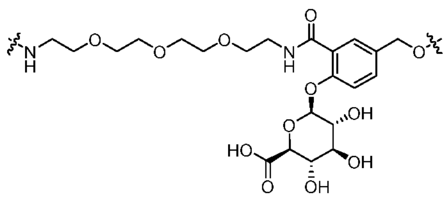

.

In some embodiments, E is a single bond or -NH-$CH_2$-, or is selected from the following structures:

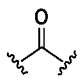

,

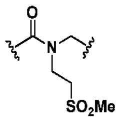

,

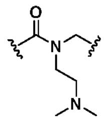

, and

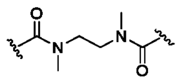

.

In some embodiments, E is a single bond, -NH-$CH_2$- or

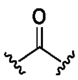

.

In some embodiments, E is-NH-$CH_2$- or

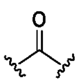

.

In some embodiments, M' is

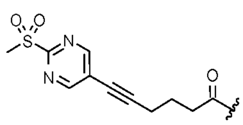

;

L is selected from the following structures:

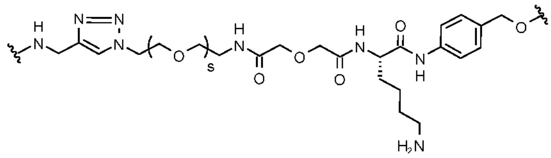

,

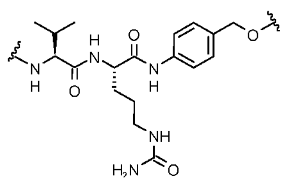

,

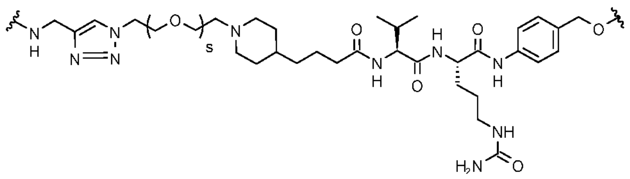, 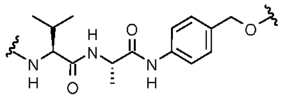,

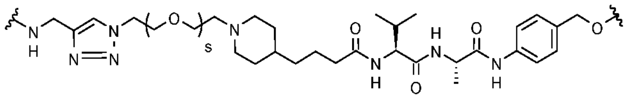, 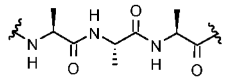, 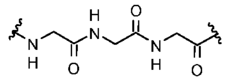,

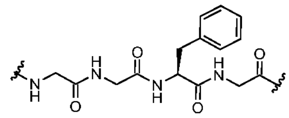, 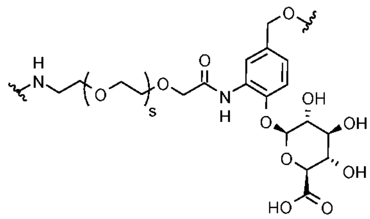 and 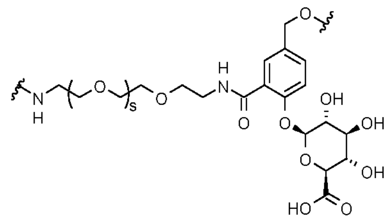;

wherein s is an integer selected from 1-10;
E is-NH-$CH_2$- or

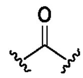.

In some embodiments,

M'—L—E—

is selected from the following structures:

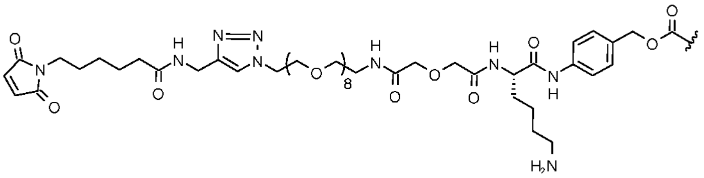,

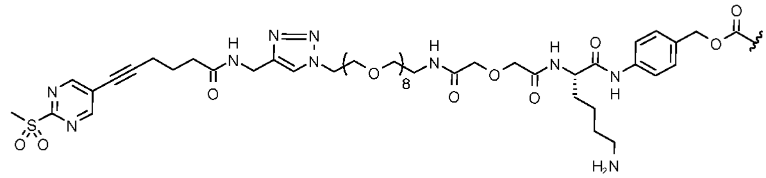,

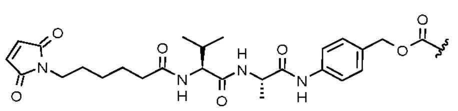, 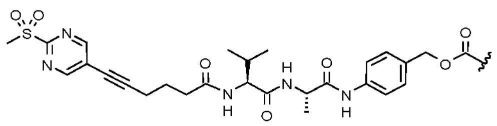,

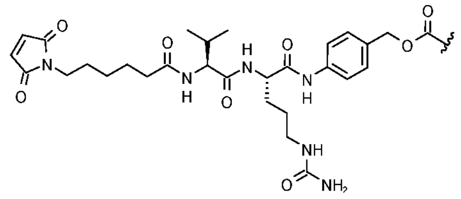
,
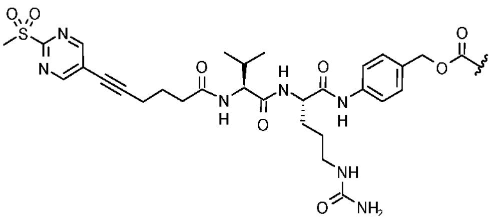
,

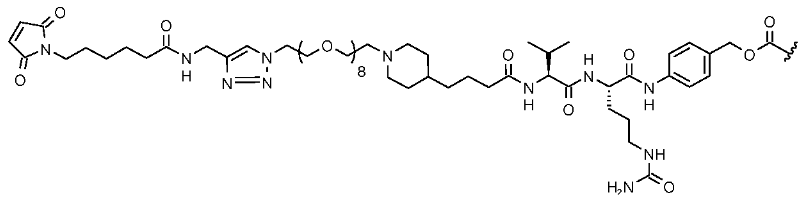
,

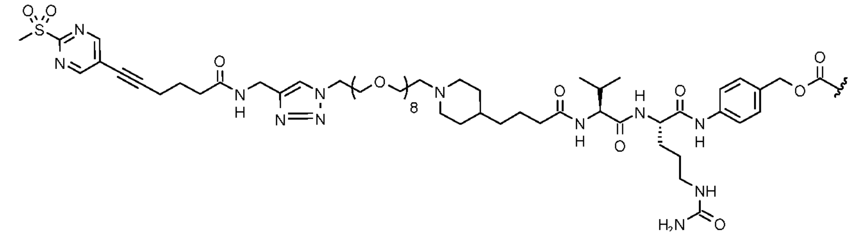
,

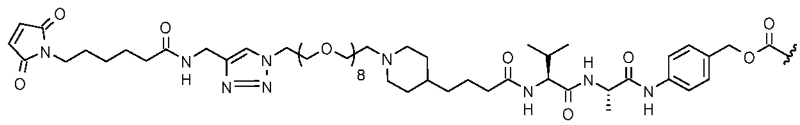
,

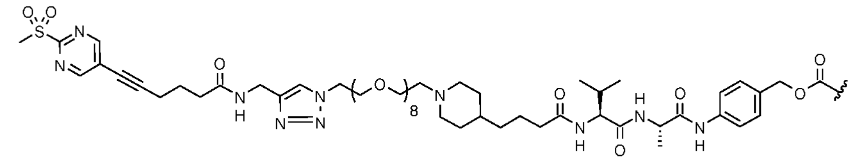
,

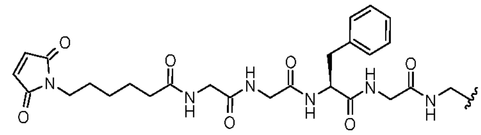
,
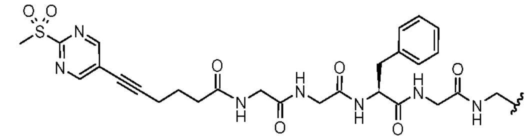
,

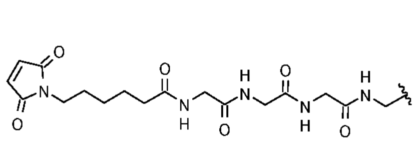
,
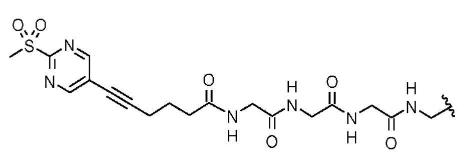
,

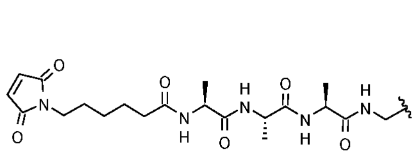
,
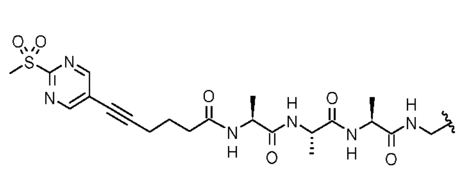
,

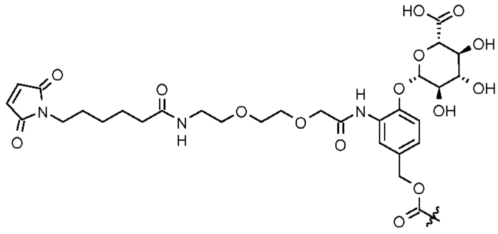,
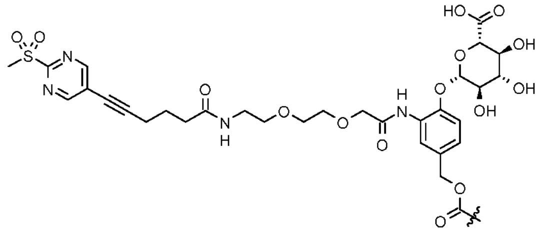,

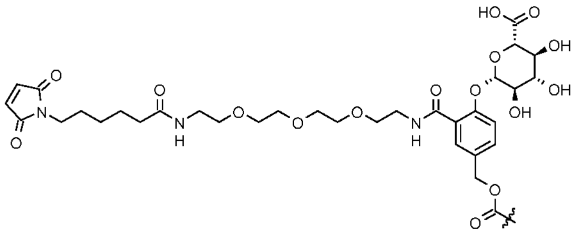

and

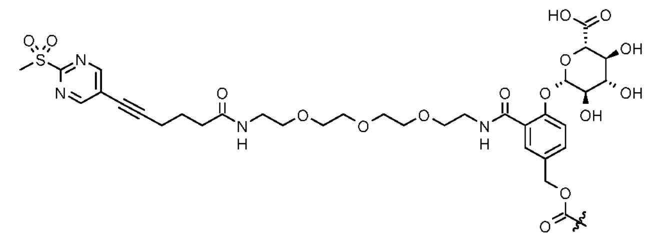.

In some embodiments,

M'—L—E—

is selected from the following structures:

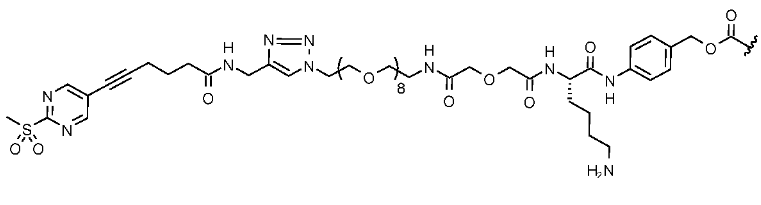,

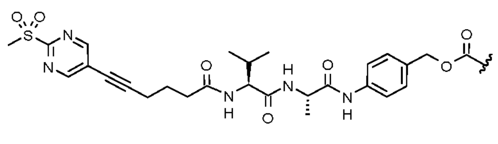,

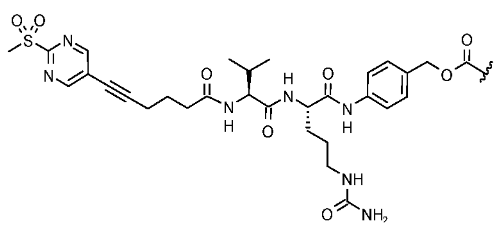,

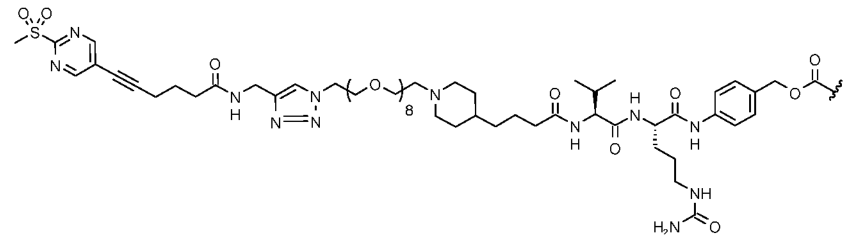

,

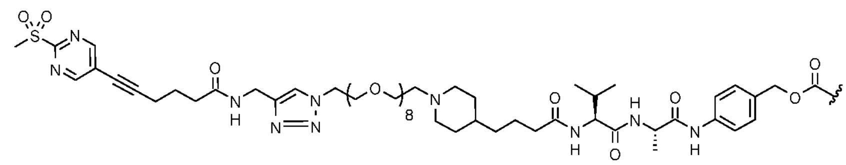

,

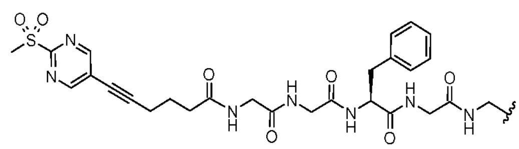

,

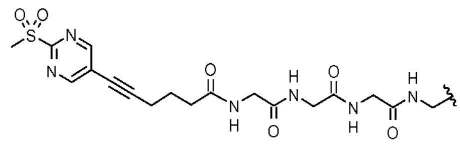

,

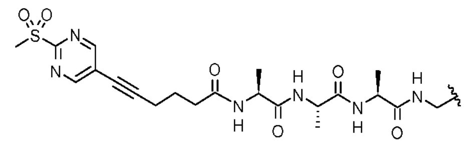

,

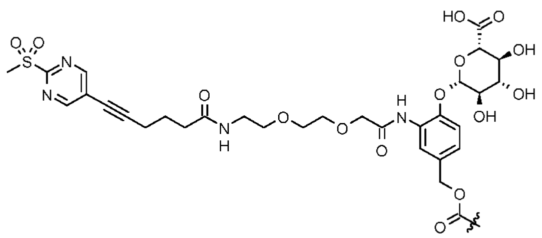

and

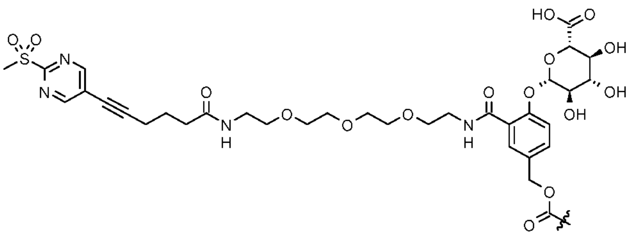

.

In some embodiments, the cytotoxic drug is selected from the group consisting of a tubulin inhibitor, a DNA intercalator, a DNA topoisomerase inhibitor, and a RNA polymerase inhibitor. In some embodiments, the tubulin inhibitor is an auristatin compound or a maytansine compound. In some embodiments, the DNA intercalator is pyrrolobenzodiazepine (PBD). In some embodiments, the DNA topoisomerase inhibitor is a topoisomerase I inhibitor (e.g., camptothecin, hydroxycamptothecin, 9-aminocamptothecin, SN-38, irinotecan, topotecan, belotecan, or rubitecan) or a topoisomerase II inhibitor (e.g., doxorubicin, PNU-159682, duocarmycin, daunorubicin, mitoxantrone, podophyllotoxin, or etoposide). In some embodiments, the RNA polymerase inhibitor is $\alpha$-amanitin or a pharmaceutically acceptable salt, ester or analog thereof.

The cytotoxic drugs disclosed in the present application usually contain a variety of functional groups, such as hydroxyl (-OH), carboxyl (-COOH), primary amino (-$NH_2$), secondary amine (-$NR_1H$), tertiary amine (-$NR_2R_3$), wherein

$R_1$, $R_2$, $R_3$ here only represent non-hydrogen substituents on N, or sulfhydryl (-SH), and these functional groups can react with a suitable functional group in the rest of the conjugate to achieve the connection with the drug molecule.

In some embodiments, the cytotoxic drug is connected to E in the antibody-drug conjugate through -OH, primary amino group, secondary amino group or tertiary amino group, or -SH thereon.

In some embodiments, the cytotoxic drug is selected from the following compounds:

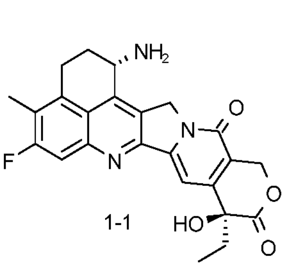
1-1

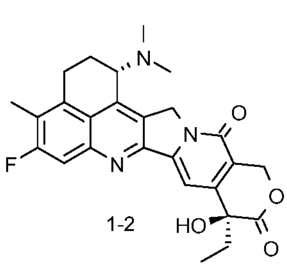
1-2

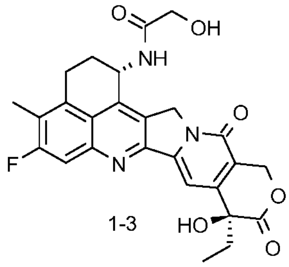
1-3

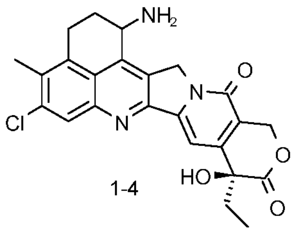
1-4

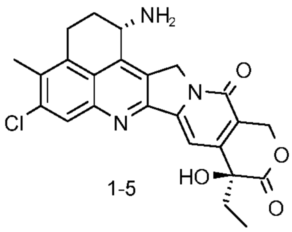
1-5

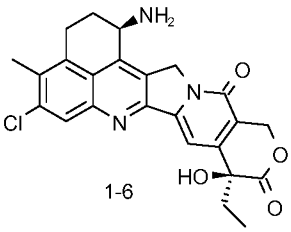
1-6

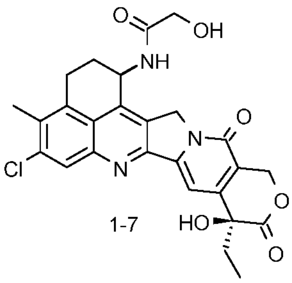
1-7

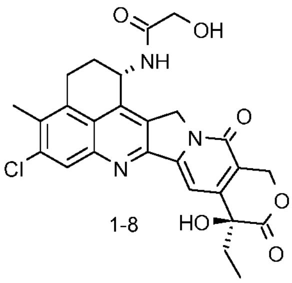
1-8

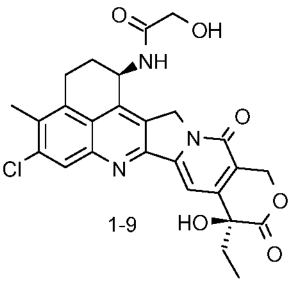
1-9

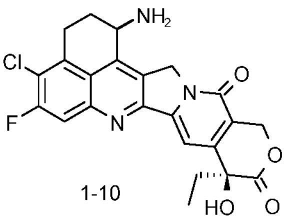
1-10

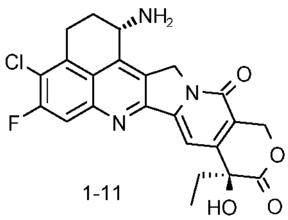
1-11

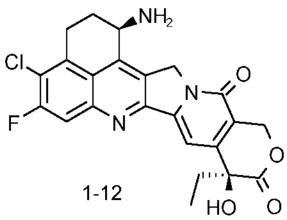
1-12

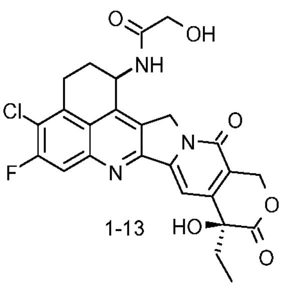
1-13

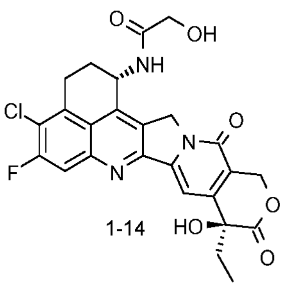
1-14

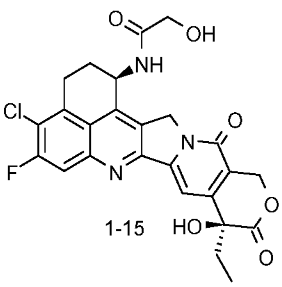
1-15

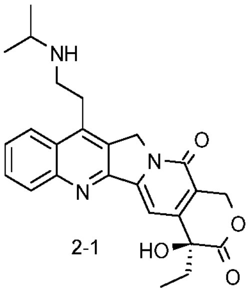
2-1
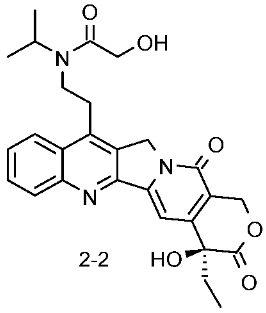
2-2
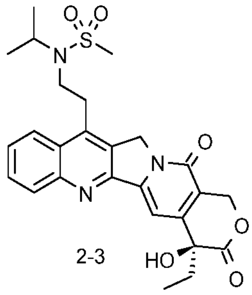
2-3
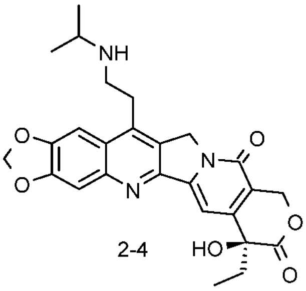
2-4

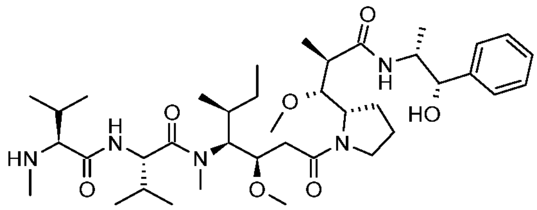
3-1

and

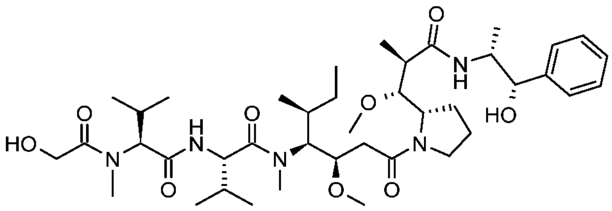
3-2

.

In some embodiments, D is selected from the following structures:

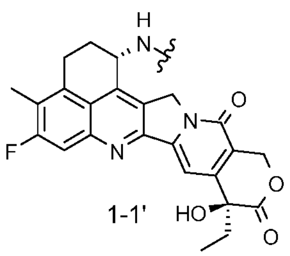
1-1'
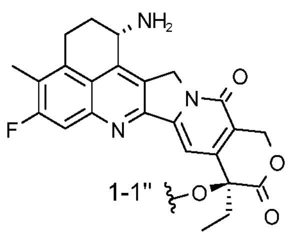
1-1''
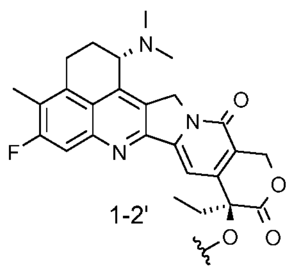
1-2'
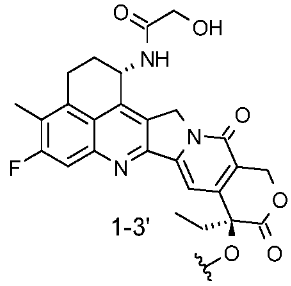
1-3'

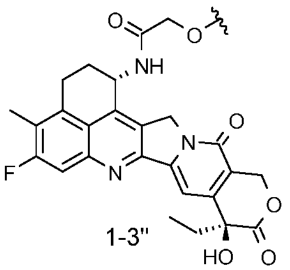
1-3''
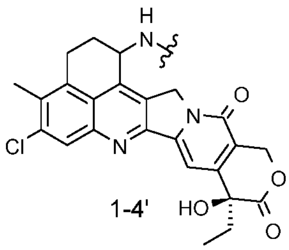
1-4'
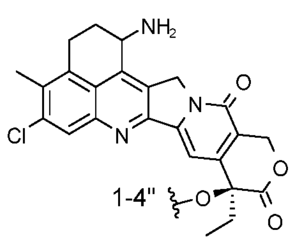
1-4''
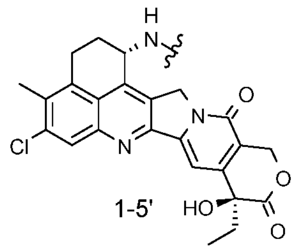
1-5'

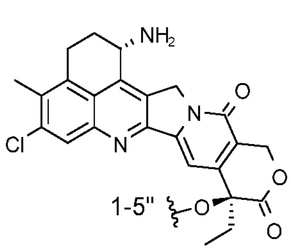
1-5''

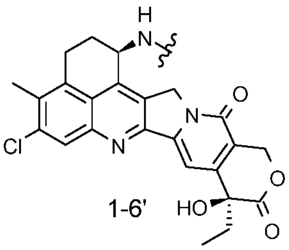
1-6'

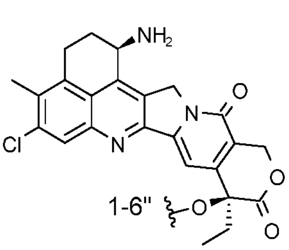
1-6''

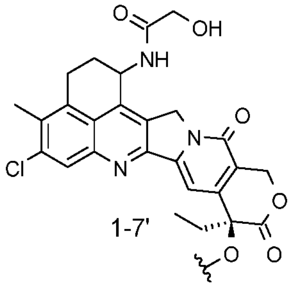
1-7'

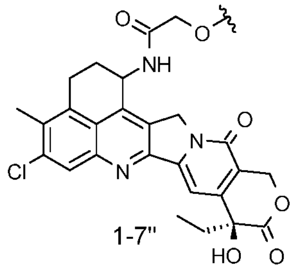
1-7''

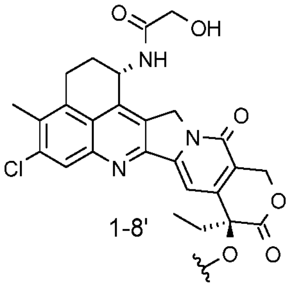
1-8'

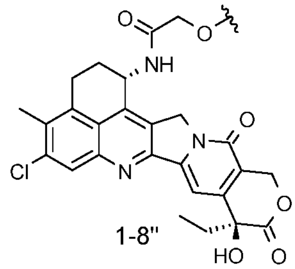
1-8''

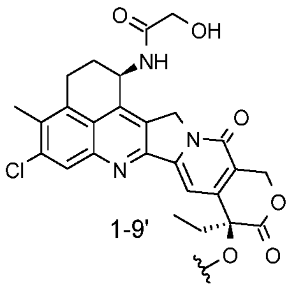
1-9'

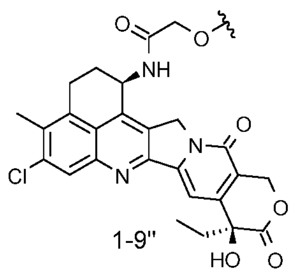
1-9''

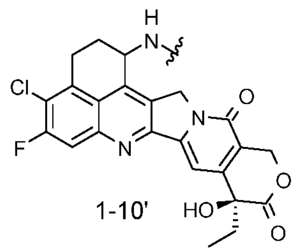
1-10'

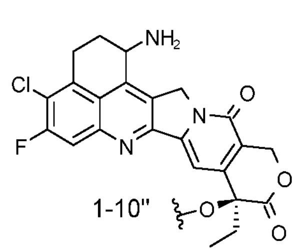
1-10''

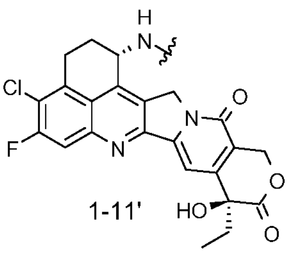
1-11'

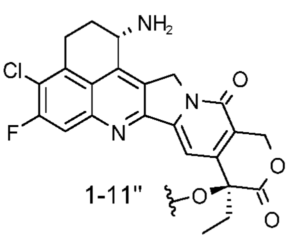
1-11''

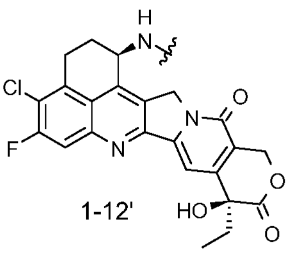
1-12'

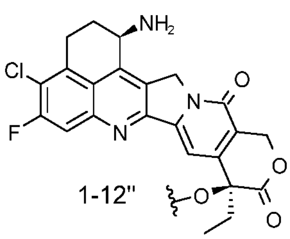
1-12''

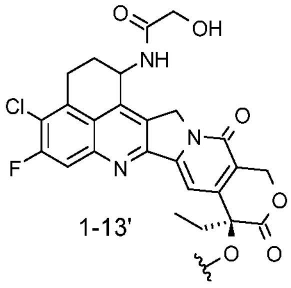
1-13'

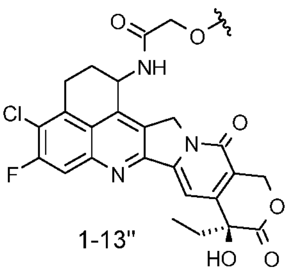
1-13''

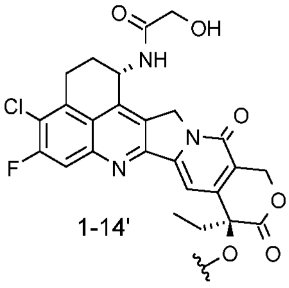
1-14'

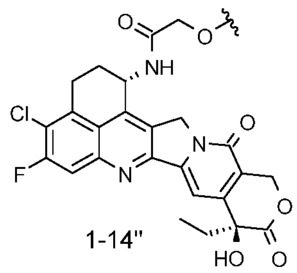
1-14''

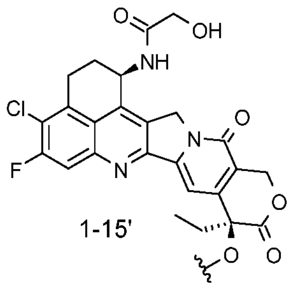

1-15'

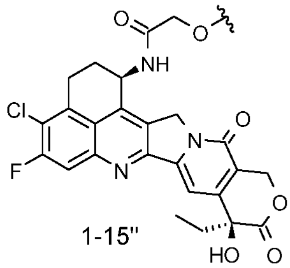

1-15''

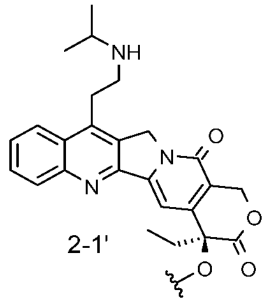

2-1'

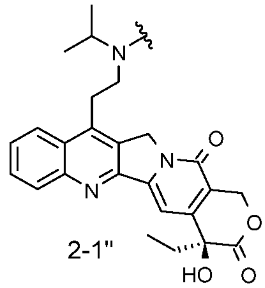

2-1''

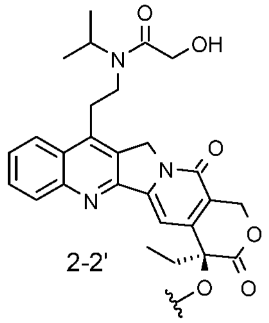

2-2'

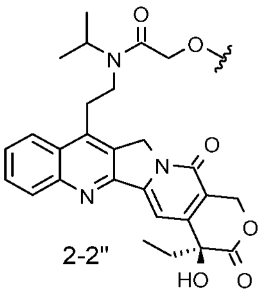

2-2''

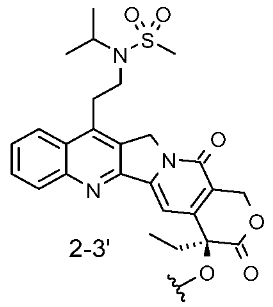

2-3'

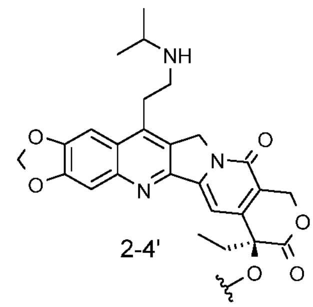

2-4'

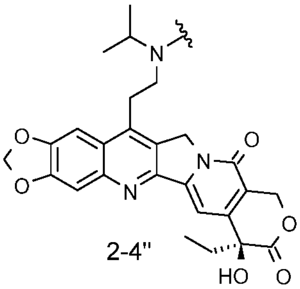

2-4''

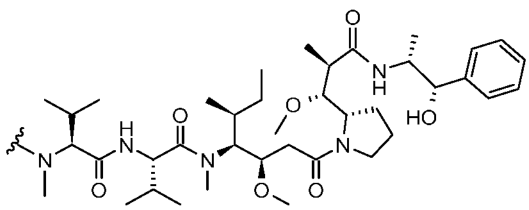

3-1'

and

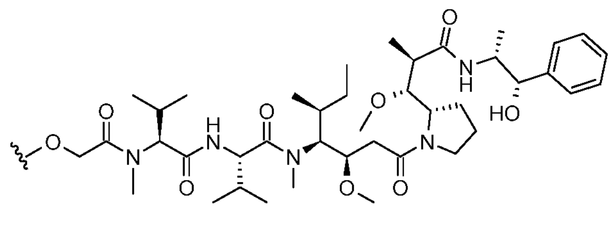

3-2' .

In some embodiments, the drug-linker is selected from A-1 to A-31 and B-1 to B-3 shown below:

A-1: 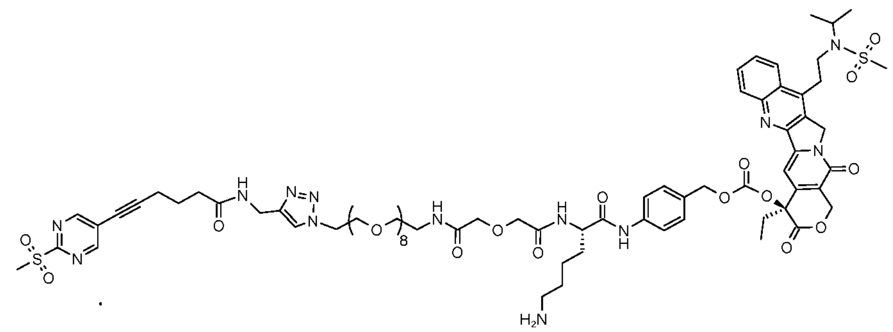 ,

A-2: 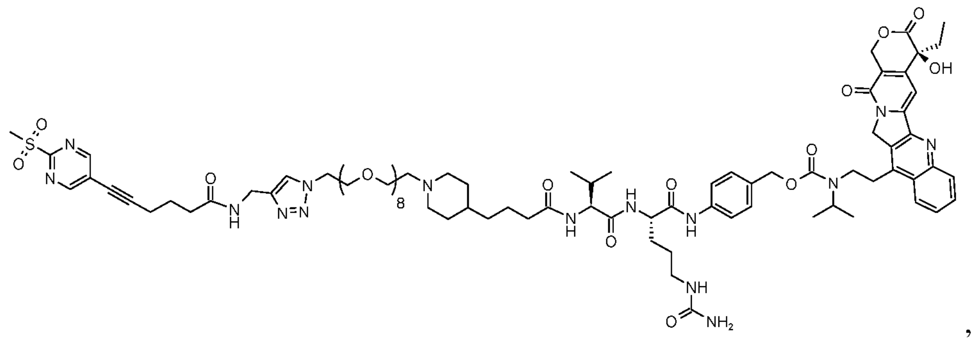 ,

A-3: 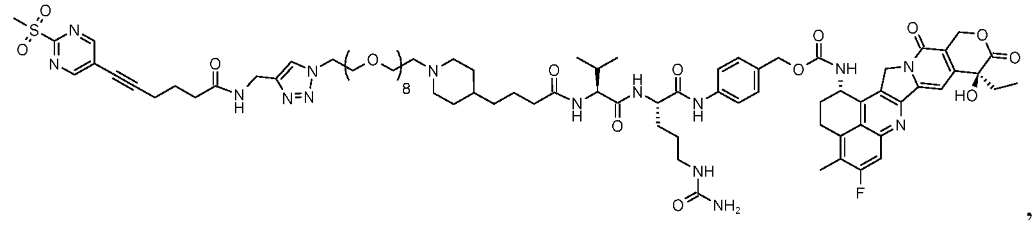 ,

A-4: 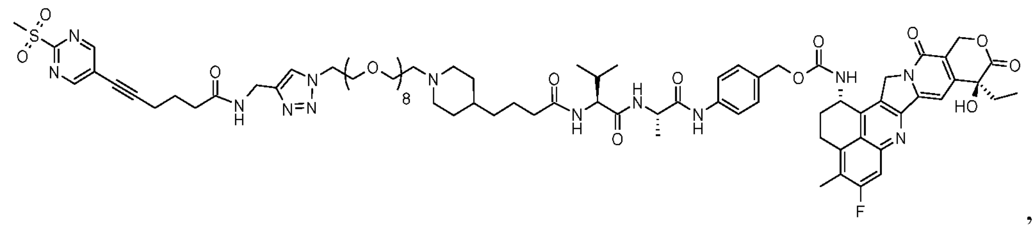 ,

A-5: 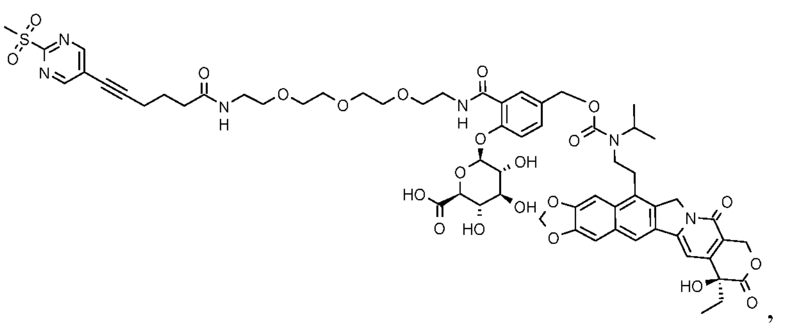 ,

A-6: 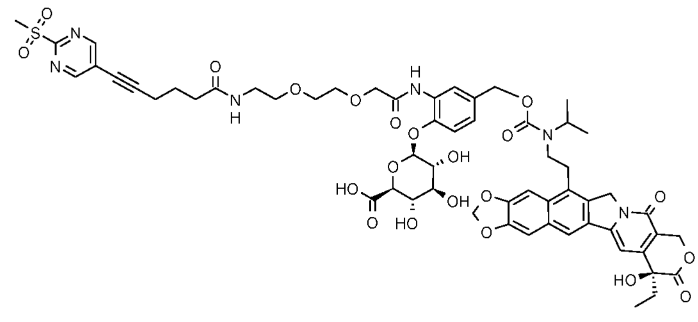 ,

A-7: 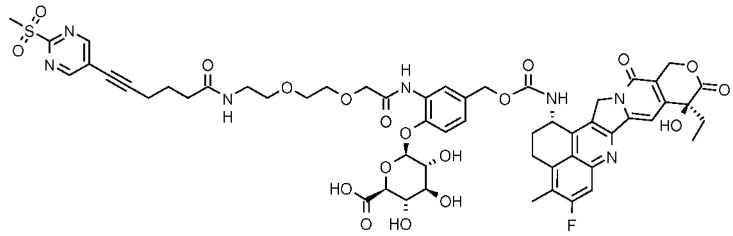 ,

A-8: 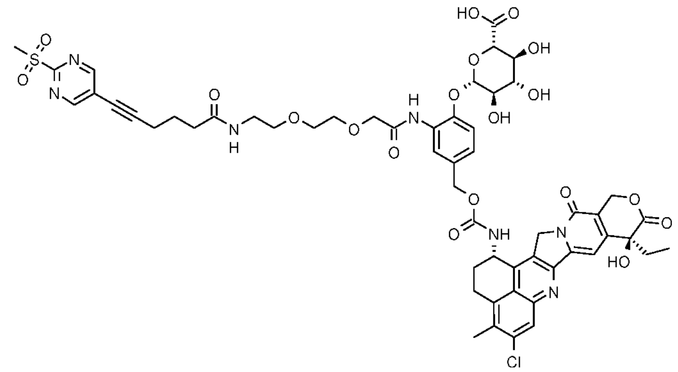 ,

A-9: 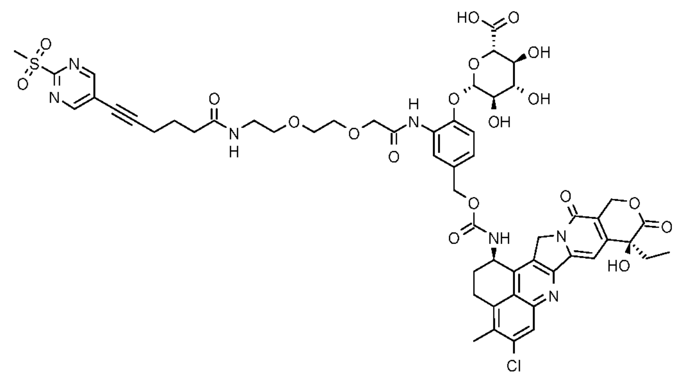 ,

A-10: 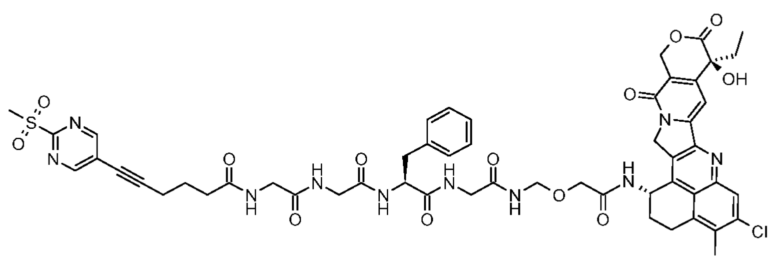 ,

A-11: 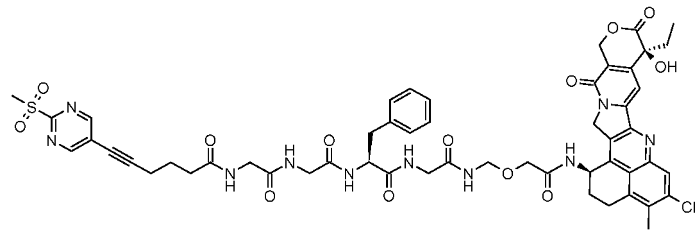 ,

A-12: 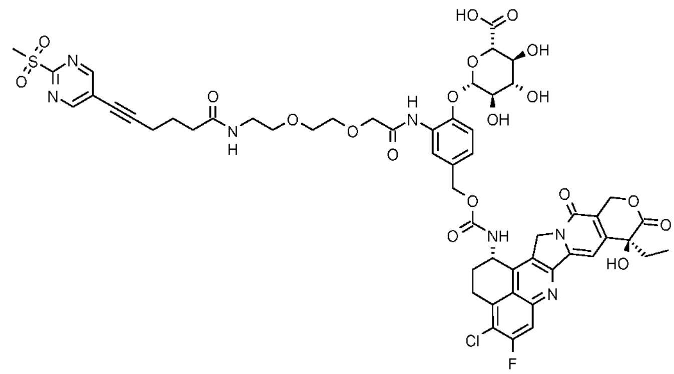 ,

A-13: 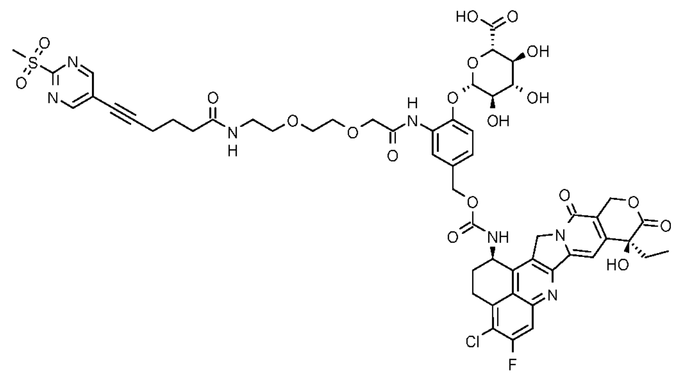 ,

A-14: 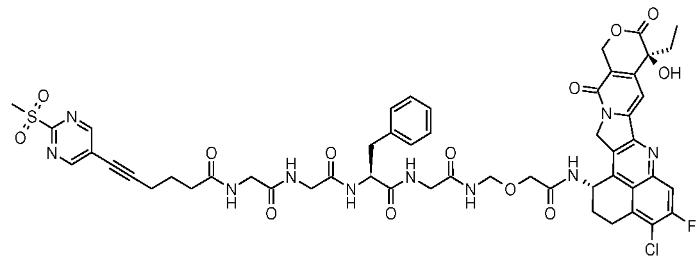 ,

A-15: 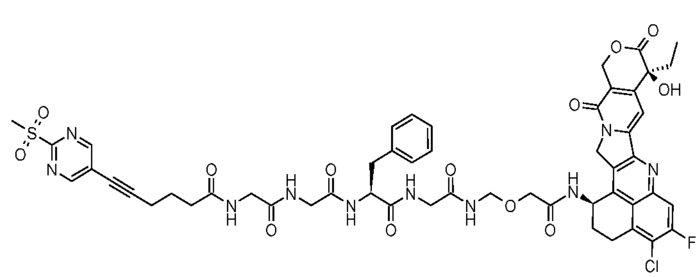 ,

A-16: 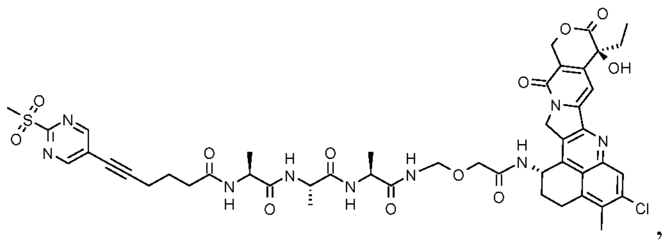 ,

A-17: 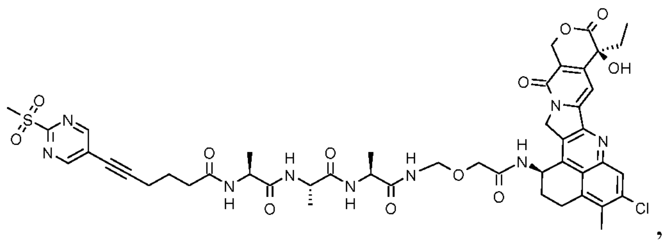 ,

A-18: 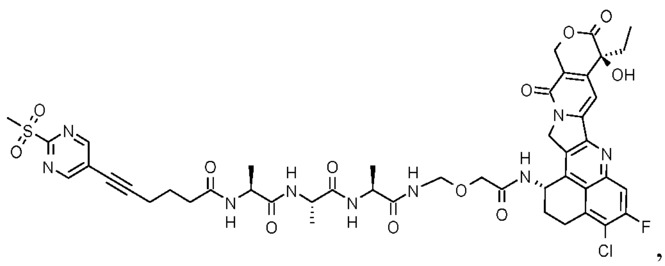 ,

A-19: 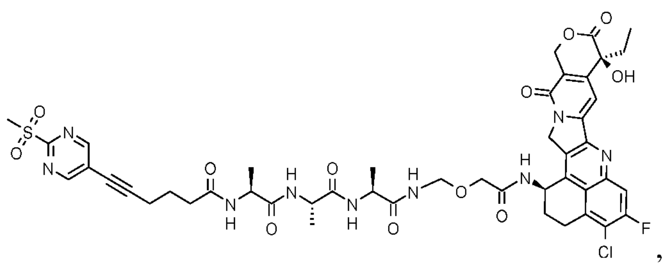 ,

A-20: 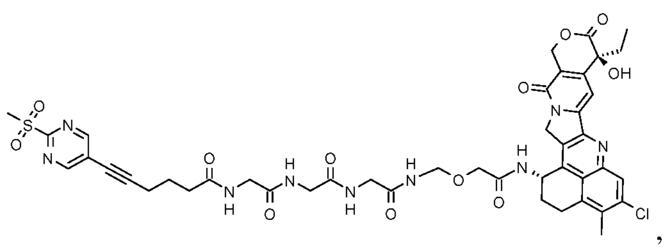 ,

A-21: 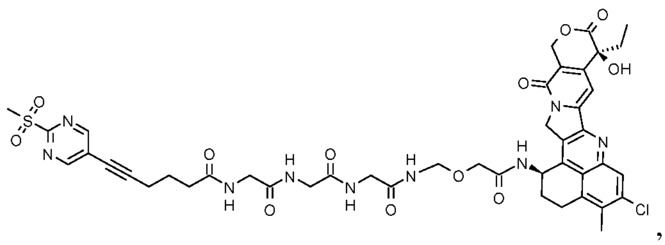 ,

A-22: 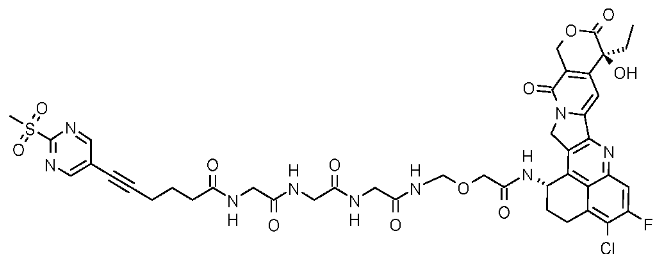 ,

A-23: 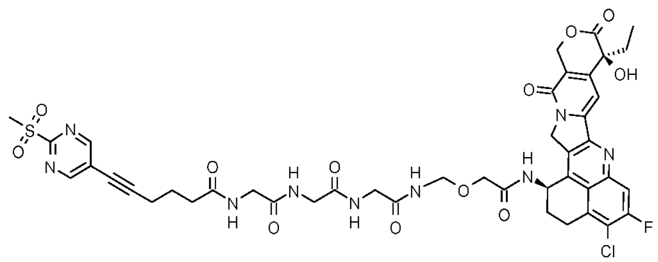 ,

A-24: 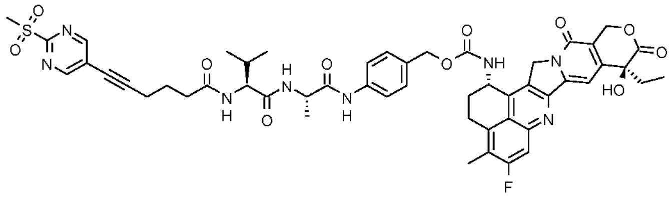 ,

A-25: 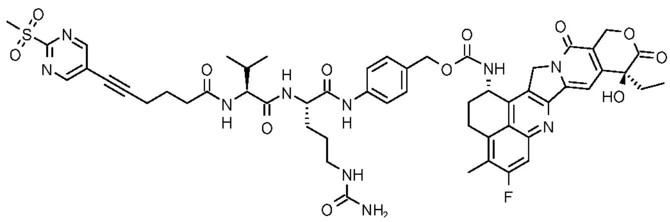 ,

A-26: 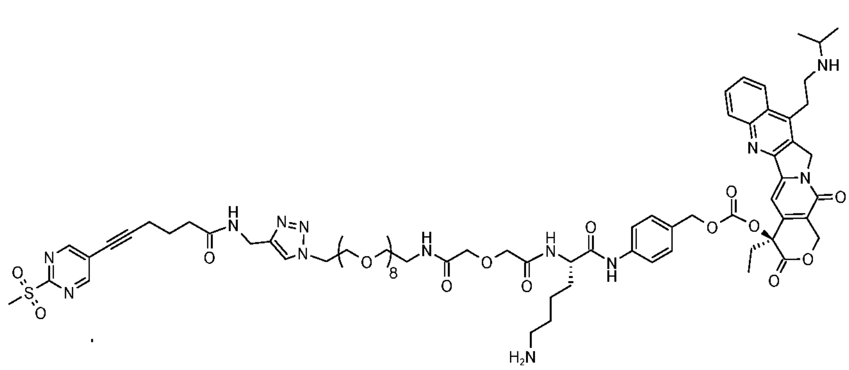 ,

A-27: 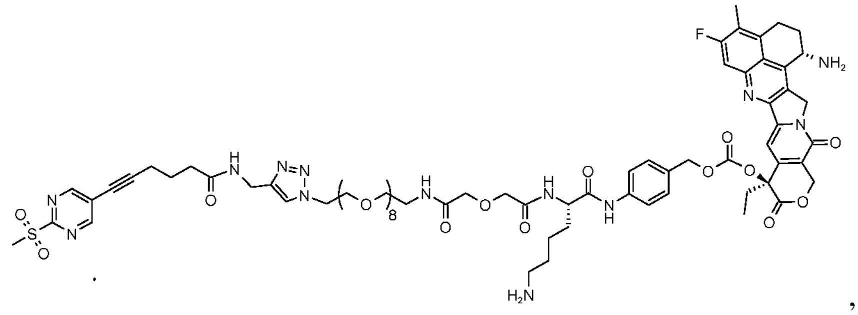 ,

A-28: 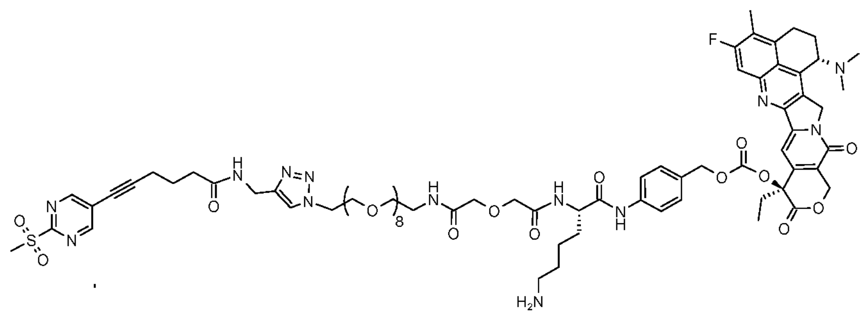 ,

A-29: 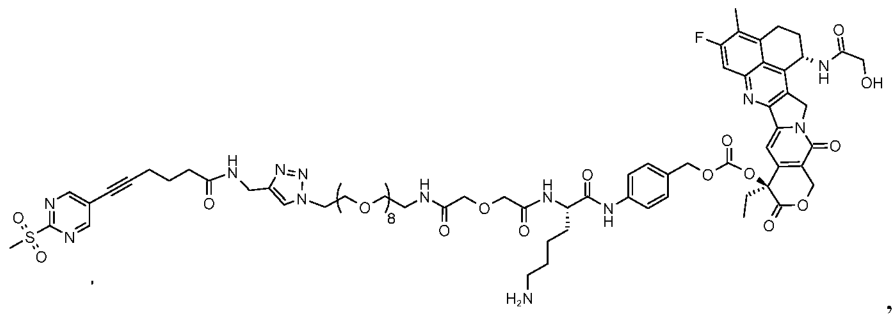 ,

A-30: 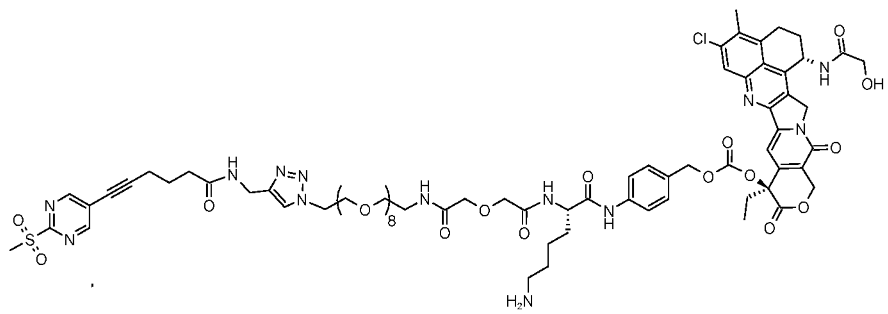 ,

A-31: 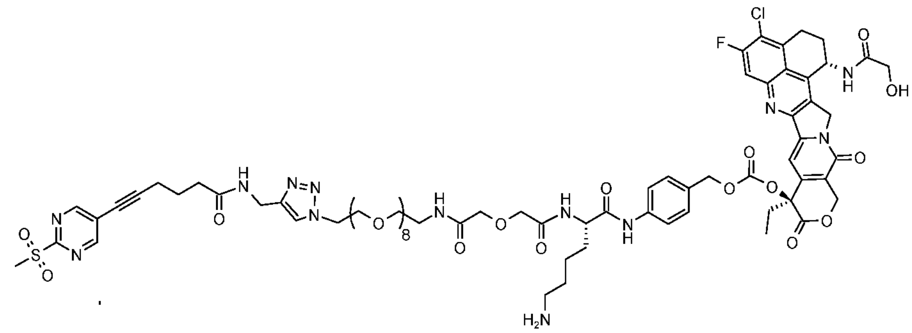,

B-1: 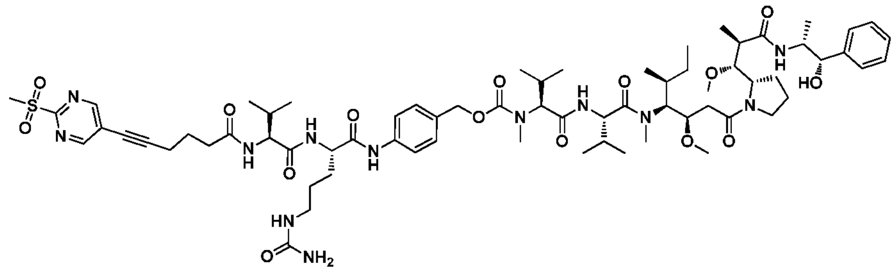,

B-2: 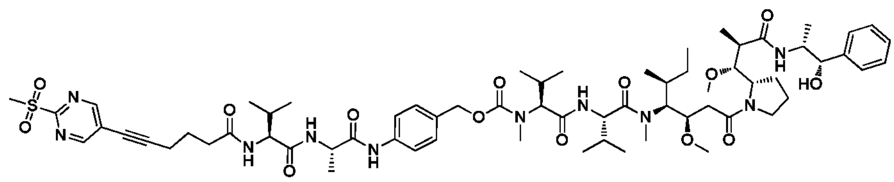,

B-3: 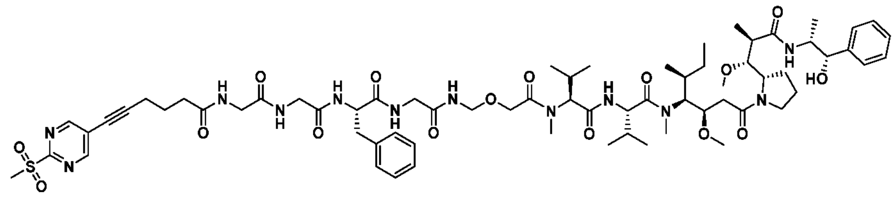.

Pharmaceutical Composition

In another aspect, the present application provides a pharmaceutical composition, which comprises the antibody-drug conjugate as described in any one of the foregoing items, and one or more pharmaceutical excipients.

The antibody-drug conjugate as described herein is commonly formulated with a pharmaceutically acceptable parenteral vehicle to form an unit injectable form for parenteral uses, such as bolus injection, intravenous injection, intratumoral injection, and the like. Optionally, the antibody-drug conjugate having the desired purity is mixed with a pharmaceutically acceptable diluent, carrier, excipient or stabilizer in the form of lyophilizate or solution (Remington's Pharmaceutical Sciences (1980) 16th edition, Osol, A. Ed.). The antibody-drug conjugate as described herein, or the pharmaceutical composition comprising the antibody-drug conjugate, can be administered via any route appropriate for the individual to be treated.

Use

The antibody-drug conjugates as described herein or pharmaceutical composition thereof can be used to treat various diseases or conditions, such as cancers with high expression of ROR1, including solid tumors or hematological malignancies, such as colon cancer, gastric cancer, breast cancer, lung cancer (e.g.,, non-small cell lung cancer, specifically lung adenocarcinoma), or lymphoma.

Therefore, the present application provides use of the antibody-drug conjugate as described in any one of the foregoing items or the pharmaceutical composition containing the same in the manufacture of a medicament for the

treatment of a cancer with high expression of ROR1.

At the same time, the present application also provides a method for treating a cancer with high expression of ROR1, which comprises a step of administering to a subject in need thereof a therapeutically effective amount of the antibody-drug conjugate as described in any one of the foregoing items or the pharmaceutical composition containing the same.

Definition

Unless defined otherwise hereinafter, all technical and scientific terms used herein have the same meaning as commonly understood by one of ordinary skill in the art. References to techniques used herein are intended to refer to techniques commonly understood in the art, including those changes in technology or substitutions of equivalent technologies obvious to the skilled person. Moreover, the laboratory operation steps of genomics, nucleic acid chemistry, and molecular biology used in herein are all routine steps widely used in the corresponding fields. Though the following terms are believed to be well understood by those skilled in the art, the following definitions are set forth to better explain the present invention.

The term "antibody" refers to an immunoglobulin molecule usually composed of two pairs of polypeptide chains (each pair has a light chain (LC) and a heavy chain (HC)). Antibody light chains can be classified into κ (kappa) and λ (lambda) light chains. Heavy chains can be classified into μ, δ, γ, α or ε heavy chains, and the isotypes of antibody are defined as IgM, IgD, IgG, IgA and IgE, respectively. Within the light and heavy chains, the variable and constant regions are connected by a "J" region of about 12 or more amino acids, and the heavy chain also includes a "D" region of about 3 or more amino acids. Each heavy chain is composed of a heavy chain variable region (VH) and a heavy chain constant region (CH). The heavy chain constant region is composed of 3 domains (CH1, CH2 and CH3). Each light chain is composed of a light chain variable region (VL) and a light chain constant region (CL). The light chain constant region consists of one domain CL. The constant domains are not directly involved in the binding of antibodies and antigens, but exhibit a variety of effector functions, such as mediating the binding of immunoglobulins to host tissues or factors, including various cells (for example, effector cells) of immune system and the first component (C1q) of classical complement system. The VH and VL regions can also be subdivided into hypervariable regions (called complementarity determining regions (CDR)), interspersed with more conservative regions (called framework regions (FR)). Each VH and VL consists of 3 CDRs and 4 FRs arranged in the following order: FR1, CDR1, FR2, CDR2, FR3, CDR3, FR4 from the amino terminus to the carboxy terminus. The variable regions (VH and VL) of each heavy chain/light chain pair form antigen binding sites respectively. The assignment of amino acids to regions or domains may follow various numbering systems known in the art.

The term "complementarity determining region" or "CDR" refers to the amino acid residues in an antibody variable region that is responsible for the antigen binding. Each of the variable regions of heavy and light chains contain 3 CDRs, named CDR1, CDR2 and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), the IMGT numbering system (Lefranc et al. al., Dev. Comparat. Immunol. 27:55-77, 2003) or the AbM numbering system (Martin ACR, Cheetham JC, Rees AR (1989) Modeling antibody hypervariable loops: A combined algorithm. Proc Natl Acad Sci USA 86:9268-9272) in the definition. For a given antibody, those skilled in the art will readily identify the CDRs defined by each numbering system. Also, the correspondence between different numbering systems is well known to those skilled in the art (e.g., see Lefranc et al., Dev. Comparat. Immunol. 27:55-77, 2003).

In the present invention, the CDRs contained in an antibody or antigen-binding fragment thereof can be identified according to various numbering systems known in the art, such as Kabat, Chothia, IMGT or AbM numbering systems. In certain embodiments, the CDRs contained in an antibody or antigen-binding fragment thereof are defined by the Chothia numbering system.

The term "framework region" or "FR" residues refers to those amino acid residues in an antibody variable region other than the CDR residues as defined above.

The term "antigen-binding fragment" of antibody refers to a polypeptide of antibody fragment, such as a polypeptide of fragment of full-length antibody, which retains the ability to specifically bind to the same antigen to which the full-length antibody binds, and/or compete with the full-length antibody for specific binding to the antigen, which is also called "antigen-binding portion". See generally, Fundamental Immunology, Ch. 7 (Paul, W., ed., 2nd edition, Raven Press, NY (1989), which is incorporated herein by reference in its entirety for all purposes. Recombinant DNA technology or enzymatic or chemical cleavage of an intact antibody can be used to produce an antigen-binding fragment of the antibody. Non-limiting examples of antigen-binding fragment include Fab, Fab', $F(ab')_2$, $F(ab')_3$, Fd, Fv, scFv, di-scFv, $(scFv)_2$, disulfide-stabilized Fv protein ("dsFv"), single-domain antibody (sdAb, nano antibody), and such polypeptides, which contain at least a portion of antibody that is sufficient to confer the polypeptide a specific antigen binding ability. Engineered

antibody variants are reviewed by Holliger et al., 2005; Nat Biotechnol, 23:1126-1136.

The term "Fd" refers to an antibody fragment composed of VH and CH1 domains; the term "dAb fragment" refers to an antibody fragment composed of VH domains (Ward et al., Nature 341:544 546 (1989)); the term "Fab fragment" refers to an antibody fragment composed of VL, VH, CL and CH1 domains; the term "$F(ab')_2$ fragment" refers to an antibody fragment comprising two Fab fragments connected by a disulfide bridge of the hinge region; the term "Fab' fragment" refers to a fragment obtained by reducing the disulfide bond connecting the two heavy chain fragments in $F(ab')_2$ fragment, consisting of an intact light chain and a heavy chain Fd fragment (consisting of VH and CH1 domains).

The term "Fv" refers to an antibody fragment consisting of VL and VH domains of a single arm of an antibody. The Fv fragment is generally considered to be the smallest antibody fragment capable of forming a complete antigen-binding site. It is generally believed that the six CDRs confer antigen-binding specificity to an antibody. However, even a variable region (e.g., Fd fragment, which contains only 3 CDRs specific for an antigen) can recognize and bind the antigen, although the affinity may be lower than that of the complete binding site.

The term "Fc" refers to an antibody fragment formed by combining the second and third constant regions of the first heavy chain of an antibody with the second and third constant regions of the second heavy chain via a disulfide bond. The antibody Fc fragment has a variety of different functions, but is not involved in antigen binding.

The term "scFv" refers to a single polypeptide chain comprising VL and VH domains, wherein the VL and VH are connected by a linker (see, for example, Bird et al., Science 242:423-426 (1988); Huston et al. Proc. Natl. Acad. Sci. USA 85:5879-5883 (1988); and Pluckthun, The Pharmacology of Monoclonal Antibodies, Vol. 113, Roseburg and Moore eds., Springer-Verlag, New York, pp. 269-315 (1994)). Such scFv molecules may have the general structure: $NH_2$-VL-linker-VH-COOH or $NH_2$-VH-linker-VL-COOH. Suitable linkers in the art may consist of the repeated GGGGS amino acid sequence or variants thereof. For example, a linker having the amino acid sequence $(GGGGS)_4$ can be used, but variants thereof can also be used (Holliger et al. (1993), Proc. Natl. Acad. Sci. USA 90: 6444-6448). Other linkers useful in the present invention are described by Alfthan et al. (1995), Protein Eng. 8:725-731, Choi et al. (2001), Eur. J. Immunol. 31: 94-106, Hu et al. (1996), Cancer Res. 56:3055-3061, Kipriyanov et al. (1999), J. Mol. Biol. 293:41-56 and Roovers et al. (2001), Cancer Immunol. In some cases, there may also be a disulfide bond between the VH and VL of scFv. In certain embodiments, the VH and VL domains may be positioned relative to each other in any suitable arrangement. For example, there is a scFv comprising $NH_2$-VH-VH-COOH, $NH_2$-VL-VL-COOH.

The term "single-domain antibody (sdAb)" has the meaning generally understood by those skilled in the art, which refers to an antibody fragment composed of a single monomer variable antibody domain (e.g., a single heavy chain variable region), which retains the ability to specifically bind the same antigen to which the full-length antibody binds (Holt, L. et al., Trends in Biotechnology, 21(11):484-490, 2003). Single-domain antibody is also called as nano antibody.

Each of the above antibody fragments maintains the ability to specifically bind to the same antigen to which the full-length antibody binds, and/or competes with the full-length antibody for specific binding to the antigen.

Herein, unless the context clearly dictates otherwise, when the term "antibody" is referred to, it includes not only an intact antibody but also an antigen-binding fragment of the antibody.

An antigen-binding fragment of an antibody (e.g., the antibody fragment described above) can be obtained from a given antibody (e.g., the antibody provided in the present invention) using a conventional technique known to those skilled in the art (e.g., recombinant DNA technique or enzymatic or chemical cleavage method), and the antigen-binding fragment of the antibody can be screened for specificity in the same manner as for the intact antibody.

The term "murine antibody" refers to an antibody obtained by the following method: fusing B cells of immunized mice to myeloma cells, selecting mouse hybrid fusion cells that can both immortalize and secrete an antibody, and then performing screening, antibody preparation and antibody purification; or refers to an antibody secreted by plasma cells formed by the differentiation and proliferation of B cells after the antigen invades the mouse body.

The term "humanized antibody" refers to a genetically engineered non-human antibody whose amino acid sequence has been modified to increase homology to the sequence of a human antibody. Generally, all or part of the CDR regions of a humanized antibody are derived from a non-human antibody (donor antibody), and all or part of the non-CDR regions (e.g., variable region FRs and/or constant region) are derived from a human immunoglobulin (receptor antibody). Humanized antibodies typically retain the desired properties of the donor antibody, including, but not limited to, antigen specificity, affinity, reactivity, ability to increase immune cell activity, ability to enhance an immune response, and the like. A donor antibody can be an antibody from mouse, rat, rabbit, or non-human primate (e.g., cynomolgus) having desirable properties (e.g., antigen specificity, affinity, reactivity, ability to increase immune cell activity and/or enhance immune response).

The term "identity" refers to the match degree between two polypeptides or between two nucleic acids. When two sequences for comparison have the same monomer sub-unit of base or amino acid at a certain site (e.g., two DNA molecules each have an adenine at a certain site, or two polypeptides each have a lysine at a certain site), the two molecules are identical at the site. The percent identity between two sequences is a function of the number of identical sites shared by the two sequences over the total number of sites for comparison x 100. For example, if 6 of 10 sites of

two sequences are matched, these two sequences have an identity of 60%. For example, DNA sequences: CTGACT and CAGGTT share an identity of 50% (3 of 6 sites are matched). Generally, the comparison of two sequences is conducted in a manner to produce maximum identity. Such alignment can be conducted by using a computer program such as Align program (DNAstar, Inc.) which is based on the method of Needleman, et al. (J. Mol. Biol. 48:443-453, 1970). The percent identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

The term "conservative substitution" refers to an amino acid substitution that does not adversely affect or alter the expected properties of a protein/polypeptide comprising an amino acid sequence. For example, conservative substitutions can be introduced by standard techniques known in the art, such as site-directed mutagenesis and PCR-mediated mutagenesis. Conservative amino acid substitutions include substitutions wherein an amino acid residue is substituted with another amino acid residue having a similar side chain, for example, a residue physically or functionally similar (e.g., having similar size, shape, charge, chemical properties, including ability of forming a covalent bond or a hydrogen bond, etc.) to the corresponding amino acid residue. A family of amino acid residues having similar side chains has been defined in the art. These families include amino acids having basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g. alanine, valine, leucine, isoleucine, valine, phenylalanine, methionine), beta branch side chains (e.g., threonine, valine, isoleucine) and aromatic side chains (e.g., tyrosine, phenylalanine, tryptophan, histidine). Therefore, it is preferred to replace the corresponding amino acid residue with another amino acid residue from the same side chain family. Methods for identifying conservative substitutions of amino acids are well known in the art (see, for example, Brummell et al, Biochem. 32: 1180-1187 (1993); Kobayashi et al., Protein Eng. 12(10): 879-884 (1999); and Burks et al. Proc. Natl Acad. Set USA 94: 412-417 (1997), which is incorporated herein by reference).

The terms "antibody-drug conjugate" and "ADC" have the same meaning and refer to a drug formed with an antibody, a cytotoxic drug, and a connector for connecting the antibody and the cytotoxic drug.

The twenty conventional amino acids involved herein are expressed in routine manners. See, for example, Immunology-A Synthesis (2nd Edition, E. S. Golub and D. R. Gren, Eds., Sinauer Associates, Sunderland, Mass. (1991)), which is incorporated herein by reference. In the present invention, amino acids are generally represented by single letter and three letter abbreviations as known in the art. For example, alanine can be represented by A or Ala.

The terms "comprising", "including", "having", "containing" or "involving" and other variations thereof herein are inclusive or open-ended and do not exclude other unlisted elements or method steps.

The term "alkyl" refers to a group obtained by removing one hydrogen atom from a linear or branched hydrocarbon group, such as "$C_{1-20}$ alkyl", "$C_{1-10}$ alkyl", "$C_{1-6}$ alkyl", "$C_{1-4}$ alkyl", "$C_{1-3}$ alkyl", etc., and specific examples include but are not limited to: methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1,2-dimethylpropyl, etc.

## Brief Description of the Drawings

The drawings described herein are used to provide a further understanding of the present invention and constitute a part of the present application. The schematic examples of the present invention and their descriptions are used to explain the present invention and do not constitute improper limitations to the present invention. In the drawings:

Figure 1 shows the identification of Ba/F3 cells overexpressing human ROR1 by flow cytometry.

Figure 2 shows the changes in tumor volume of mice in each group of the human gastric cancer cell NCI-N87 CDX model. Note: Arrows represent administration, the same below.

Figure 3 shows the changes in body weight of mice in each group of the human gastric cancer cell NCI-N87 CDX model.

Figure 4 shows the changes in tumor volume of mice in each group of the human lung adenocarcinoma cell NCI-H1975 CDX model.

Figure 5 shows the changes in body weight of mice in each group of the human lung adenocarcinoma cell NCI-H1975 CDX model.

Figure 6 shows the changes in tumor volume of mice in each group of the human colon cancer cell line HT-29 CDX model.

Figure 7 shows the changes in body weight of mice in each group of the human colon cancer cell HT-29 CDX model.

## Specific Models for Carrying Out the Invention

The technical solutions in the examples of the present invention will be clearly and completely described by referring to the drawings of the examples of the present invention. Obviously, the described examples are only some, not all, examples of the present invention. The following description of at least one exemplary example is merely illustrative in nature and in no way taken as any limitation to the present invention, its application or uses. Based on the examples of the present invention, all other examples obtained by those ordinary skilled in the art without creative efforts fall within the protection scope of the present invention.

The following description of specific examples will further illustrate the present invention, but this is not a limitation to the present invention. Those skilled in the art can make various modifications or improvements according to the teachings of the present invention without departing from the basic idea and scope of the present invention.

Information of the sequences involved in the present invention is described in the following table:

| SEQ ID NO. | Description | SEQ ID NO. | Description |
|---|---|---|---|
| 1 | 19F6_Hu35V1 VH | 25 | 19F6_Hu35Vl IMGT CDR-H2 |
| 2 | 19F6_Hu35V1 VL | 26 | 19F6_Hu35Vl IMGT CDR-H3 |
| 3 | 19F6_Hu35V1 Chothia CDR-H1 | 27 | 19F6_Hu35Vl IMGT CDR-L1 |
| 4 | 19F6_Hu35V1 Chothia CDR-H2 | 28 | 19F6_Hu35Vl IMGT CDR-L2 |
| 5 | 19F6_Hu35V1 Chothia/AbM/Kabat CDR-H3 | 29 | 19F6_Hu35Vl AbM CDR-H1 |
| 6 | 19F6_Hu35V1 AbM/Kabat/Chothia CDR-L1 | 30 | 19F6_Hu35Vl AbM CDR-H2 |
| 7 | 19F6_Hu35V1 AbM/Kabat/Chothia CDR-L2 | 31 | 19F6_Hu35Vl Kabat CDR-H1 |
| 8 | 19F6_Hu35V1 AbM/Kabat/Chothia /IMGT CDR-L3 | 32 | 19F6_Hu35Vl Kabat CDR-H2 |
| 9 | 3D8_HuC24 VH | 33 | 3D8_HuC24 IMGT CDR-H1 |
| 10 | 3D8_HuC24 VL | 34 | 3D8_HuC24 IMGT CDR-H2 |
| 11 | 3D8_HuC24 Chothia CDR-H1 | 35 | 3D8_HuC24 IMGT CDR-H3 |
| 12 | 3D8_HuC24 Chothia CDR-H2 | 36 | 3D8_HuC24 AbM CDR-H1 |
| 13 | 3D8_HuC24 AbM/Kabat/Chothia CDR-H3 | 37 | 3D8_HuC24 AbM CDR-H2 |
| 14 | 3D8_HuC24/38F8_Hu57 AbM/Kabat/Chothia CDR-L1 | 38 | 3D8_HuC24 Kabat CDR-H1 |

(continued)

| SEQ ID NO. | Description | SEQ ID NO. | Description |
|---|---|---|---|
| 15 | 3D8_HuC24/38F8_Hu57 AbM/Kabat/Chothia CDR-L2 | 39 | 3D8_HuC24 Kabat CDR-H2 |
| 16 | 3D8_HuC24/38F8_Hu57 IMGT/AbM/Kabat/Chothia CDR-L3 | 40 | 38F8_Hu57 IMGT CDR-H1 |
| 17 | 38F8_Hu57 VH | 41 | 38F8_Hu57 IMGT CDR-H2 |
| 18 | 38F8_Hu57 VL | 42 | 38F8_Hu57 IMGT CDR-H3 |
| 19 | 38F8_Hu57 Chothia CDR-H1 | 43 | 38F8_Hu57 /3D8_HuC24 IMGT CDR-L1 |
| 20 | 38F8_Hu57 Chothia CDR-H2 | 44 | 38F8_Hu57/3D8_HuC24 IMGT CDR-L2 |
| 21 | 38F8_Hu57 AbM/Kabat/Chothia CDR-H3 | 45 | 38F8_Hu57 AbM CDR-H1 |
| 22 | Human IgG1 heavy chain constant region | 46 | 38F8_Hu57 AbM CDR-H2 |
| 23 | Human kappa light chain constant region | 47 | 38F8_Hu57 Kabat CDR-H1 |
| 24 | 19F6_Hu35V1 IMGT CDR-H1 | 48 | 38F8_Hu57 Kabat CDR-H2 |

Abbreviations used in the present application have the following meanings:

| Abbreviation | Meaning | Abbreviation | Meaning |
|---|---|---|---|
| HATU | N,N,N',N'-Tetramethyl-O- (7-azabenzotriazol-1-yl)urea hexafluorophosphate | EDCI | 1-(3-Dimethylaminopropyl)- 3-ethylcarbodiimide hydrochloride |
| DIPEA | Diisopropvlethylamine | NBS | N-Bromosuccinimide |
| THF | Tetrahydrofuran | MMAF | Monomethyl Auristatin F |
| Val | Valine | Cit | Citrulline |
| PABC | p-Aminobenzvl alcohol | Boc | Tert-butoxycarbonyl |
| EEDQ | 2-Ethoxy-1-ethoxycarbonyl-1,2-dihvdroquinoline | HOBt | 1-Hydroxybenzotriazole |
| HPLC | High performance liquid chromatography | DMSO | Dimethyl sulfoxide |
| Pre-TLC | Preparative thin layer chromatography | Rf | Retardation factor |
| DMAP | 4-Dimethylaminopvridine | DMF | N,N-Dimethylformamide |
| TFA | Trifluoroacetic acid | TBAF | Tetrabutylammonium fluoride |
| LCMS | Liquid chromatography-Mass spectrometry | EA | Ethyl acetate |
| Pd/C | Palladium on carbon | Pt/C | Platinum on carbon |

The structures of the compounds described in the following Examples were identified by nuclear magnetic resonance ($^1$H-NMR) or mass spectrometry (MS).

The nuclear magnetic resonance ($^1$H-NMR) was determined by using a Bruker 400 MHz nuclear magnetic resonance instrument; the deuterated reagent was hexadeuteriodimethylsulfoxide (DMSO-d6); and the internal standard substance was tetramethylsilane (TMS).

Abbreviations used in Examples in interpretation the nuclear magnetic resonance (NMR) spectra were shown below.

s: singlet, d: doublet, t: triplet, q: quartet, m: multiplet, br: broad, J: coupling constant, Hz: Hertz, DMSO-*d6*: deuterated dimethylsulfoxide. The $\delta$ values were expressed in ppm.

The mass spectrometry (MS) was determined by using an Agilent (ESI) mass spectrometer, model Agilent 6120B.

Example 1: 4-((S)-2-(4-Aminobutyl)-35-(4-((6-(2-(methylsulfonyl)pyrimidin-5-yl)hex- 5-ynamido)methyl)-1H-1,2,3-triazol-1-yl)-4,8-dioxo-6,12,15,18,21,24,27,30,33-nonaoxa-3,9-diazapentatriacontanamido)benzyl ((S)-4-ethyl-11-(2-(N-isopropylmethylsulfonamido)ethyl)-3,14- dioxo-3,4,12,14-tetrahydro-1H-pyrano[3',4':6,7]indolizino[1,2-b]quinolin-4-yl)carbonate (A-1)

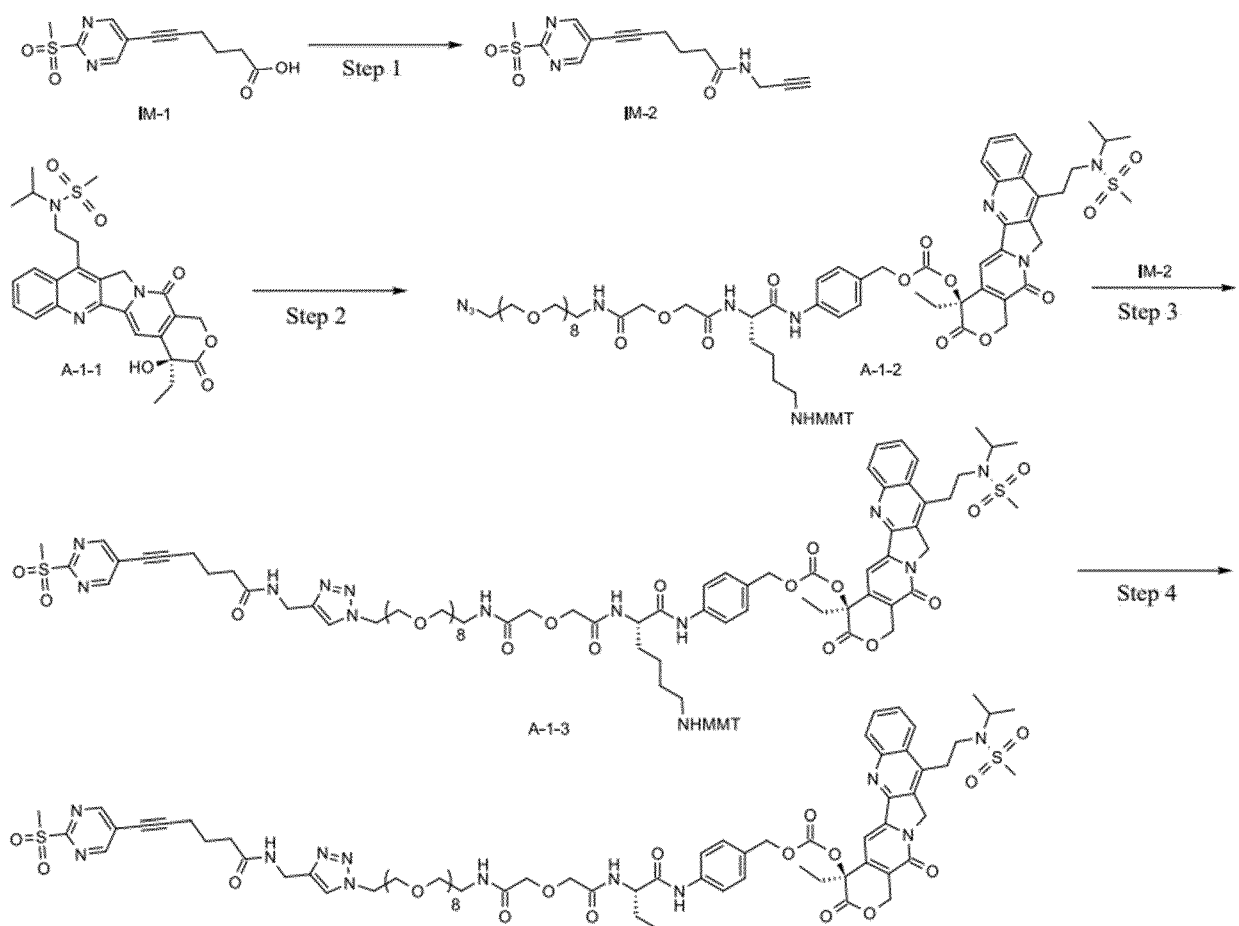


Step 1: Synthesis of 6-(2-(methylsulfonyl)pyrimidin-5-yl)-N-(prop-2-yn-1-yl)hex- 5-ynamide

Prop-2-yn-1-amine (189 mg, 3.4 mmol) and compound IM-1 (800 mg, 2.83 mmol) were dissolved in dichloromethane (10 mL) at 25°C, added sequentially with N,N-diisopropylethylamine (738 mg, 5.67 mmol), and O-(7-azabenzotriazol-1-yl)- N,N,N',N'-tetramethyluronium hexafluorophosphate (1.63 g, 4.25 mmol), stirred and reacted for 2 h. The reaction solution was concentrated under reduced pressure, and the residue was purified by flash silica gel column

(ethyl acetate/petroleum ether=3/1) to obtain 700 mg of the title compound. ESI-MS (m/z): 306.1 $[M+H]^+$.

Step 2: Synthesis of 4-((S)-35-azido-2-(4-(((4-methoxyphenyl)benzhydryl)amino)butyl)-4,8-dioxo-6,12,15,18,21,24,27,30,33-nonaoxa-3,9-diazapentatriacontanamido)benzyl ((S)-4-ethyl-11-(2-(N-isopropylmethylsulfonamido)ethyl)-3,14-dioxo-3,4,12,14-tetrahydro-2H-pyrano [2,3-b]-1H-pyrano [3',4':6,7]indolizino [1,2-b]quinolin-4-yl)carbonate

Under nitrogen protection at 25°C, **A-1-1** (250 mg, 0.49 mmol) was dissolved in dichloromethane (10 mL), cooled to 0°C, added with a solution of 4-dimethylaminopyridine (478 mg, 3.91 mmol) in dichloromethane (3 mL), then added slowly dropwise with a solution of triphosgene (72 mg, 0.24 mmol) in dichloromethane (10 mL), after the addition, the reaction was carried out under stirring at 0°C for 20 min, and the reaction solution was blown with nitrogen for 20 min. (S)-2-(32-Azido-5-oxo-3,9,12,15,18,21,24,27,30-nonaoxa- 6-azatriacetamido)-N-(4-(hydroxymethyl)phenyl)-6(((4-methoxyphenyl)benzhydryl)amino)acetamide (518 mg, 0.49 mmol) in dichloromethane (7 mL) was added, after the addition, the reaction was carried out at 0°C under stirring for 1 h. The reaction solution was concentrated under reduced pressure, and the residue was purified by preparative high performance liquid chromatography (with conditions as follows) to obtain 500 mg of the title compound. ESI-MS (m/z): 1597.5 $[M+H]^+$.

Chromatographic column: Daisogel C18 10μm 100x250mm

Mobile phase A: water; Mobile phase B: acetonitrile

| Time [min] | Mobile phase A [%] | Mobile phase B[%] | Flow rate [mL/min] |
|---|---|---|---|
| 0.00 | 70.0 | 30.0 | 30 |
| 8.00 | 70.0 | 30.0 | 30 |
| 50.00 | 20.0 | 80.0 | 30 |

Step 3: Synthesis of (S)-4-Ethyl-11-(2-(N-isopropylmethylsulfonamido)ethyl)-3,14- dioxo-3,4,12,14-tetrahydro-1H-pyrano[3',4':6,7]indolizino[1,2-b]quinolin-4-yl (4-((S)-2-(4-(((4-methoxyphenyl)benzhydryl)amino)butyl)-35-(4-((6-(2-(methylsulfonyl)pyrimidin-5-yl)hex-5-ynamido)methyl)-1H-1,2,3-triazol-1-yl)-4,8-dioxo-6,12,15,18,21,24,27,30,33-nonoxa-3,9-diazapentatriacontanamido)benzyl)carbonate

At room temperature, compound **A-1-2** (14 mg, 0.05 mmol) was dissolved in dimethyl sulfoxide and water (2.0 mL : 0.5 mL), added with copper bromide (11 mg, 0.08 mmol) and 6-(2-(methylsulfonyl)pyrimidin-5-yl)-N-(prop-2-yn-1-yl)-hex-5-ynamide **(IM-2,** 18.8 mg, 0.06 mmol), stirred and reacted for 1 h. The reaction solution was purified by preparative high performance liquid chromatography (with conditions as follows) to obtain 30 mg of the title compound. ESI-MS (m/z): 815.9$[(M-273)/2+H]^+$.

Chromatographic column: SunFire Prep C18 OBD 19 mm×150 mm×5.0 μm

Mobile phase A: acetonitrile; Mobile phase B: water

| Time [min] | Mobile phase A [%] | Mobile phase B[%] | Flow rate [mL/min] |
|---|---|---|---|
| 0.00 | 40 | 60 | 28 |
| 8.00 | 40 | 60 | 28 |
| 50.00 | 90 | 10 | 28 |

Step 4: Synthesis of 4-((S)-2-(4-aminobutyl)-35-(4-((6-(2-(methylsulfonyl)pyrimidin- 5-yl)hex-5-ynamido)methyl)-1H-1,2,3-triazol-1-yl)-4,8-dioxo-6,12,15,18,21,24,27,30,33-nonaoxa-3,9-diazapentatriacontanamido)benzyl ((S)-4-ethyl-11-(2-(N-isopropylmethylsulfonamido) ethyl)-3,14-dioxo-3,4,12,14-tetrahydro-1H-pyrano[3',4':6,7]indolizino[1,2-b]quinolin-4-yl)carbonate

Compound **A-1-3** (30 mg, 0.02 mmol) was dissolved in dichloromethane (1.0 mL), added with trifluoroacetic acid (0.2 mL), and reacted at room temperature for 30 min. After purification by preparative high-performance liquid chromatography (with conditions as follows), 20.0 mg of the trifluoroacetic acid salt of the title compound was obtained.

Chromatographic column: SunFire Prep C18 OBD 19 mm×150 mm×5.0 μm

Mobile phase A: acetonitrile; Mobile phase B: water (0.05% trifluoroacetic acid)

| Time [min] | Mobile phase A [%] | Mobile phase B[%] | Flow rate [mL/min] |
|---|---|---|---|
| 0.00 | 15 | 85 | 28 |
| 8.00 | 15 | 85 | 28 |
| 50.00 | 60 | 40 | 28 |

The structure characterization data were as follows:
$^{1}$H NMR (400 MHz, DMSO-d6) δ 10.18 (s, 1H), 9.10 (s, 2H), 8.38 (t, *J* = 5.56 Hz, 1H), 8.32 (d, *J* = 8.40 Hz, 1H), 8.22-8.20 (m, 2H), 8.09 (t, *J* = 5.68 Hz, 1H), 7.91-7.87 (m, 2H), 7.82-7.78 (m, 1H), 7.69 (brs, 3H), 7.61 (d, *J* = 8.56 Hz, 2H), 7.32 (d, *J* = 8.56 Hz, 2H), 7.06 (s, 1H), 5.56 (d, *J* = 16.96 Hz, 1H), 5.51 (d, *J* = 16.96 Hz, 1H), 5.47 (d, *J* = 19.28 Hz, 1H), 5.42 (d, *J* = 19.28 Hz, 1H), 5.14 (d, *J* = 12.20 Hz, 1H), 5.07 (d, *J* = 12.16 Hz, 1H), 4.48 (t, *J* = 5.24 Hz, 2H), 4.46-4.43 (m, 1H), 4.29 (d, *J* = 5.60 Hz, 2H), 4.08-3.95 (m, 5H), 3.79 (t, *J* = 5.28 Hz, 2H), 3.51-3.43 (m, 32H), 3.40 (s, 3H), 3.39-3.35 (m, 2H), 3.30-3.26 (m, 2H), 3.00 (s, 3H), 2.82-2.74 (m, 2H), 2.56 (t, *J* = 7.08 Hz, 2H), 2.29 (t, *J* = 7.36 Hz, 2H), 2.23-2.13 (m, 2H), 1.82 (p, *J* = 7.24 Hz, 2H), 1.78-1.63 (m, 2H), 1.61-1.49 (m, 2H), 1.42-1.27 (m, 2H), 1.15 (d, *J* = 6.80 Hz, 3H), 1.13 (d, *J* = 6.76 Hz, 3H), 0.90 (t, *J* = 7.32 Hz, 3H). ESI-MS (m/z): 816.0[M/2+H]$^{+}$.

Example 2: 4-((S)-2-((S)-3-Methyl-2-(4-(1-(26-(4-((6-(2-(methylsulfonyl))pyrimidin-5-yl) hex-5-ynamido)methyl)-1H-1,2,3-triazol-1-yl)-3,6,9,12,15,18,21,24-octaoxahexacosyl)piperidin-4-yl)butyrylamido)butyrylamido)-5-ureidopentanamido)benzyl (2-((S)-4-ethyl-4-hydroxy-3,14-dioxo-3,4,12,14-tetrahydro-1H-pyrano [3',4':6,7]indolizino [1,2-b] quinolin-11-yl)ethyl)(isopropyl)carbamate (A-2)

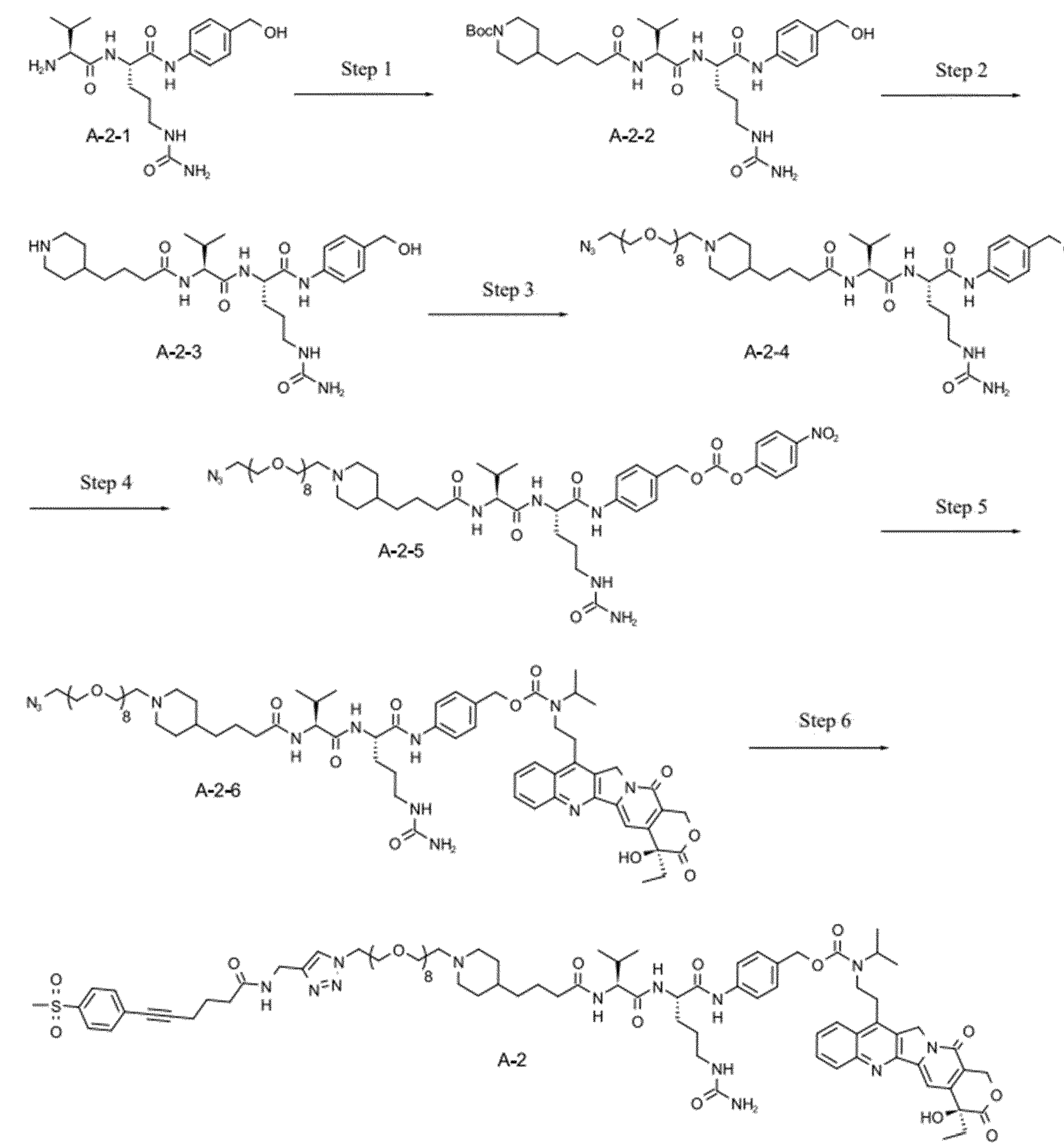


Step 1: Synthesis of tert-butyl 4-(4-(((S)-1-(((S)-1-((4-(hydroxymethyl)phenyl)amino)-1-oxo-5-ureidopentan-2-yl)amino)-3-methyl-1-oxobutan-2-yl)amino)-4-oxobutyl)piperidine-1-carboxylate

At 25°C, the compound Val-Cit-PABC **(A-2-1,** 1 g, 2.64 mmol), 4-(N-Boc-4-piperidinyl)butanoic acid (929.65 mg, 3.43 mmol) and EEDQ (977.55 mg, 3.95 mmol) were dissolved in a mixed solution of methanol (20 mL) and dichloromethane (20 mL), heated to 45°C and reacted for 2 h. The reaction solution was subjected to rotatory evaporation, poured into methyl tert-butyl ether (50ml) and stirred for 30 min, a cloudy precipitate was formed, and filtered by suction to obtain 1.3 g of the title compound.

The structure characterization data were as follows:

ESI-MS (m/z): 633.2$[M+H]^+$.

Step 2: Synthesis of (S)-N-(4-(hydroxymethyl)phenyl)-2-((S)-3-methyl-2-(4-(piperidin-4-yl)butanamido)butanamido)-5-ureidopentanamide

At 25°C, compound **A-2-2** (1.5 g, 2.37 mmol) was dissolved in dichloromethane (3.00 mL), added with trifluoroacetic acid (1.5 mL) in one portion, and reacted at 25°C for 2 h; the raw materials were consumed up monitored by

liquid chromatography-mass spectrometry. The reaction solution was evaporated to dryness under reduced pressure. The crude product was dissolved in methanol (3.00 mL), added with potassium carbonate (1.64 g, 11.85 mmol) and stirred for 30 min. The reaction solution was purified by a silica gel column (dichloromethane :methanol=10:1) to obtain 500 mg of the title compound.

The structure characterization data were as follows:
ESI-MS (m/z): 533.3$[M+H]^+$.

Step 3: Synthesis of (S)-2-((S)-2-(4-(1-(26-azido-3,6,9,12,15,18,21,24-octaoxahexacosyl)piperidin-4-yl)butyrylamido)-3-methylbutyrylamido)-N-(4-(hydroxymethyl) phenyl)-5-ureidopentanamide

At 25°C, 26-azido-3,6,9,12,15,18,21,24-octaoxahexacosyl 4-methylbenzenesulfonate (668.72 mg, 1.13 mmol) and compound **A-2-3** (1 g, 1.88 mmol) were dissolved in N,N-dimethylformamide (5 mL), added with potassium carbonate (518.89 mg, 3.75 mmol) in one portion, and heat up to 80°C for 2 h, the reaction was monitored by liquid chromatography-mass spectrometry, and the reaction solution was purified by silica gel column (dichloromethane:methanol=10:1) to obtain 600 mg of the title compound.

The structure characterization data were as follows:
ESI-MS (m/z): 954.5$[M+H]^+$.

Step 4: Synthesis of 4-((S)-2-((S)-2-(4-(1-(26-azido-3,6,9,12,15,18,21,24-octaoxahexacosyl)piperidin-4-yl)butyrylamido)-3-methylbutyrylamido)-5-ureidopentanamido)benzyl (4-nitrophenyl)carbamate

At 25°C, compound **A-2-4** (733 mg, 0.77 mmol) was dissolved in N,N-dimethylformamide (2 mL), added with DIPEA (396.40 mg, 3.07 mmol), then added dropwise with a solution of di(p-nitrophenyl) carbonate (701.10 mg, 2.30 mmol) in N,N-dimethylformamide (1 mL), stirred and reacted at 25°C for 16 h, and the reaction was monitored by liquid chromatography-mass spectrometry. The reaction solution was purified by preparative high performance liquid chromatography (with conditions as follows), and the fractions were freeze-dried to obtain 400 mg of the title compound.

Chromatographic column: SunFire Prep C18 OBD 19 mm×150 mm×5.0 μm

Mobile phase A: acetonitrile; Mobile phase B: water (0.05% trifluoroacetic acid)

| Time [min] | Mobile phase A [%] | Mobile phase B[%] | Flow rate [mL/min] |
|---|---|---|---|
| 0.00 | 20 | 80 | 70 |
| 7.00 | 20 | 80 | 70 |
| 60.00 | 80 | 20 | 70 |

The structure characterization data were as follows:
ESI-MS (m/z): 1119.5$[M+H]^+$.

Step 5: Synthesis of 4-((S)-2-((S)-2-(4-(1-(26-azido-3,6,9,12,15,18,21,24-octaoxahexacosyl) piperidin-4-yl)butyrylamido)-3-methylbutyrylamido)-5-ureidopentanamido)benzyl (2-((S)-4-ethyl-4-hydroxy-3,14-dioxo-3,4,12,14-tetrahydro-1H-pyrano[3',4':6,7]indolizino[1,2-b ]quinolin-11-yl)ethyl)(isopropyl)carbamate

At 25°C, belotecan (20 mg, 0.046 mmol), compound **A-2-5** (46.47 mg, 0.042 mmol) were dissolved in N,N-dimethylformamide (2 mL), added with HOBT (9.35 mg, 69.20 μmol), DIPEA (11.90 mg, 0.092 mmol), the reaction system was stirred at 25°C and reacted for 1 h, and the reaction was monitored by liquid chromatography-mass spectrometry. The reaction solution was purified by preparative high performance liquid chromatography (with conditions as follows), and the fractions were freeze-dried to obtain 41 mg of the title compound.

Chromatographic column: SunFire Prep C18 ODS 19 mm×150 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 10 | 90 | 28 |
| 2.00 | 10 | 90 | 28 |
| 18.00 | 90 | 10 | 28 |

The structure characterization data were as follows:
ESI-MS (m/z): 1413.5[(M+H]$^+$.

Step 6: Synthesis of 4-((S)-2-((S)-3-methyl-2-(4-(1-(26-(4-((6-(2-(methylsulfonyl) pyrimidin-5-yl)hex-5-ynamido)methyl)-1H-1,2,3-triazol-1-yl)-3,6,9,12,15,18,21,24-octaoxahexacosyl)piperidin-4-yl)butyrylamido)butyrylamido)-5-ureidopentanoylamino)benzyl (2-((S)-4- ethyl-4-hydroxy-3,14-dioxo-3,4,12,14-tetrahydro-1H-pyrano[3',4':6,7]indolizino[1,2-b]quinolin-11-yl)ethyl)(isopropyl)carbamate

At 25°C, 6-(2-(methylsulfonyl)pyrimidin-5-yl)-N-(prop-2-yn-1-yl)-hex-5-yneamide **(IM-2,** 12.96 mg, 0.042 mmol) and compound **A-2-6** (40 mg, 0.028 mmol) were dissolved in dimethyl sulfoxide (1 mL) and water (0.25 mL), added with cuprous bromide (8.20 mg, 0.057 mmol), and then reacted at 25°C for 1h, and the reaction was monitored by liquid chromatography-mass spectrometry. The reaction solution was purified by high-performance liquid chromatography (with conditions as follows), and the fractions were lyophilized to obtain 29 mg of the title compound.

Chromatographic column: SunFire Prep C18 ODS 19 mm×150 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 10 | 90 | 28 |
| 2.00 | 10 | 90 | 28 |
| 18.00 | 90 | 10 | 28 |

The structure characterization data were as follows:
ESI-MS (m/z): 1718.8[(M+H]$^+$.

Example 3: (S)-2-((S)-3-Methyl-2-(4-(1-(26-(4-((6-(2-(methylsulfonyl)pyrimidin-5-yl) hex-5-ynamido)methyl)-1H-1,2,3-triazol-1-yl)-3,6,9,12,15,18,21,24-octaoxahexacosyl)piperidin-4-yl)butanamido)butanamido)-5-ureidopentanamido)benzyl ((1S,9S)-9-ethyl-5-fluoro-9- hydroxy-4-methyl-10,13-dioxo-2,3,9,10,13,15-hexahydro-1H,12H-benzopyrano [3',4':6,7]indolizino[1,2-b]quinolin-1-yl)carbamate **(A-3)**

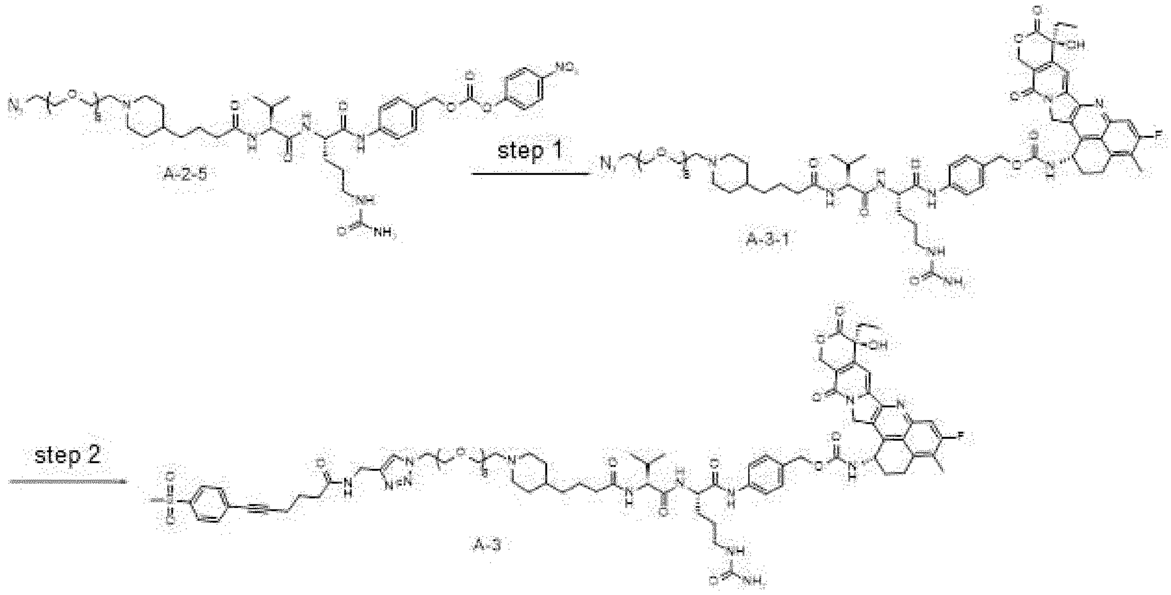


Step 1: Synthesis of 4-((S)-2-((S)-2-(4-(1-(26-azido-3,6,9,12,15,18,21,24-octaoxahexacosyl)piperidin-4-yl)butyrylamido)-3-methylbutyrylamido)-5-ureidopentanylamido)benzyl ((1S,9S)-9-ethyl-5-fluoro-9-hydroxy-4-methyl-10,13-dioxo-2,3,9,10,13,15-hexahydro- 1H,12H-benzopyrano[3',4':6,7]indolizino[1,2-b]quinolin-1-yl)carbamate

At 25°C, (1S,9S)-1-amino-9-ethyl-5-fluoro-9-hydroxy-4-methyl-1,2,3,9,12,15-hexahydro-10H,13H-benzopyrano[3',4':6,7]indolizino[1,2-b]quinoline-10,13-dione (Exatecan, 15 mg, 0.034 mmol) and compound **A-2-5** (34.70 mg, 0.031 mmol) were dissolved in N,N-dimethylformamide (1 mL), added with HOBT (6.98 mg, 51.67 μmol) and DIPEA (8.89 mg, 68.89 μmol), stirred and reacted at 25°C for 1 h, and the reaction was monitored by liquid chromatography-mass spectrometry. The reaction solution was purified by high-performance liquid chromatography (with conditions as follows), and the fractions were lyophilized to obtain 30 mg of the title compound.

Chromatographic column: SunFire Prep C18 ODS 19 mm×150 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 10 | 90 | 28 |
| 2.00 | 10 | 90 | 28 |
| 18.00 | 90 | 10 | 28 |

The structure characterization data were as follows:
ESI-MS (m/z): 1415.4[$(M+H)^+$].

Step 2: Synthesis of (S)-2-((S)-3-methyl-2-(4-(1-(26-(4-((6-(2-(methylsulfonyl)pyrimidin- 5-yl)hex-5-ynamido)methyl)-1H-1,2,3-triazol-1-yl)-3,6,9,12,15,18,21,24-octaoxahexacosyl)piperidin-4-yl)butanamido)butanamido)-5-ureidopentanamido)benzyl ((1S,9S)-9-ethyl-5-fluoro- 9-hydroxy-4-methyl-10,13-dioxo-2,3,9,10,13,15-hexahydro-1H,12H-benzopyrano[3',4':6,7]indolizino[1,2-b]quinolin-1-yl)carbamate **(A-473, A-3)**

At 25°C, 6-(2-(methylsulfonyl)pyrimidin-5-yl)-N-(prop-2-yn-1-yl)-hex-5-yneamide (**IM-2**, 9.71 mg, 0.032 mmol) and compound **A-3-1** (30 mg, 0.021 mmol) were dissolved in dimethyl sulfoxide (0.5 mL) and water (0.1 mL), then added with cuprous bromide (6.14 mg, 0.042 mmol), and reacted at 25°C for 1 h, and the reaction was monitored by liquid chromatography-mass spectrometry. The reaction solution was purified by high-performance liquid chromatography (with conditions as follows), and the fractions were freeze-dried to obtain 15.74 mg of the title compound.

Chromatographic column: SunFire Prep C18 ODS 19 mm×150 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 10 | 90 | 28 |
| 2.00 | 10 | 90 | 28 |
| 18.00 | 90 | 10 | 28 |

The structure characterization data were as follows:
ESI-MS (m/z): 861.1[$(M/2+H)^+$].

Example 4: 4-((R)-2-((S)-3-Methyl-2-(4-(1-(26-(4-((6-(2-(methylsulfonyl)pyrimidin-5-yl) hex-5-ynamide)methyl)-1H-1,2,3-triazol-1-yl)-3,6,9,12,15,18,21,24-octaoxahexacosyl)piperidin-4-yl)butanamido)butanamido)propanamido)benzyl ((1S,9S)-9-ethyl-5-fluoro-9-hydroxy- 4-methyl-10,13-dioxo-2,3,9,10,13,15-hexahydro-1H,12H-benzopyrano[3',4':6,7]indolizino[1,2-b]quinolin-1-yl)-1-yl)carbamate **(A-4)**

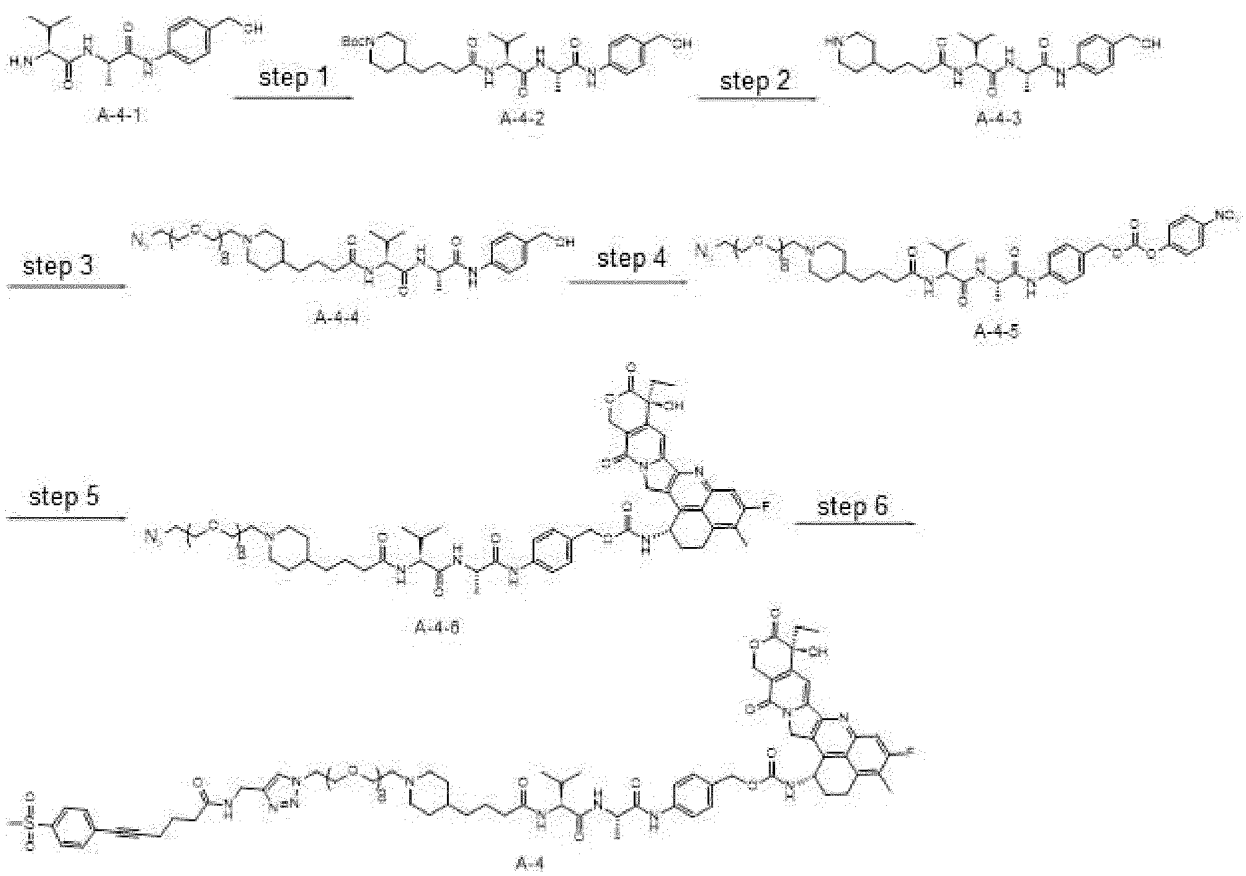


Step 1: tert-butyl 4-(4-(((S)-1-(((S)-1-((4-(hydroxymethyl)phenyl)amino)-1-oxopropan-2-yl)amino)-3-methyl-1-oxobutan-2-yl)amino)-4-oxobutyl)piperidine-1-carboxylate

At 25°C, compound **A-4-1** (1.96 g, 6.67 mmol), 4-(N-Boc-4-piperidinyl)butanoic acid (2.35 mg, 8.67 mmol) and EEDQ (2.47 mg, 10.0 mmol) were dissolved in a mixed solution of methanol (20 mL) and dichloromethane (20 mL), heated to and reacted at 45 °C for 2 h, the reaction was monitored by liquid chromatography-mass spectrometry, and

the reaction solution was concentrated and purified with a silica gel column (dichloromethane:methanol=15:1) to obtain 1.5 g of the title compound.

The structure characterization data were as follows:
ESI-MS (m/z): 547.1[M+H]$^+$.

Step 2: Synthesis of (S)-N-((S)-1-((4-(hydroxymethyl)phenyl)amino)-1-oxopropan- 2-yl)-3-methyl-2-(4-(piperidin-4-yl)butyrylamido)butanamide

At 25°C, trifluoroacetic acid (1.5 mL) was added to **A-4-2** (1.25 g, 2.37 mmol) in dichloromethane (3.00 mL), and reacted at 25°C for 2 h. The raw materials were consumed up monitored by liquid chromatography-mass spectrometry, the reaction solution was concentrated under reduced pressure to obtain a crude product, which was dissolved in methanol (3.00 mL), added with potassium carbonate (1.64 g, 11.85 mmol) and stirred for 30 min, and then detected by liquid chromatography-mass spectrometry. The reaction solution was purified with a silica gel column to obtain 750 mg of the title compound.

The structure characterization data were as follows:
ESI-MS (m/z): 447.1[M+H]$^+$.

Step 3: Synthesis of (S)-2-(4-(1-(26-azido-3,6,9,12,15,18,21,24-octaoxahexacosyl) piperidin-4-yl)butylamino)-N-((S)-1-((4-(hydroxymethyl)phenyl)amino)-1-oxopropan-2-yl-3-methylbutylamine

At 25°C, 26-azido-3,6,9,12,15,18,21,24-octaoxahexacosyl 4-methylbenzenesulfonate (1.4 mg, 2.36 mmol) and **A-4-3** (700 mg, 1.57 mmol) were dissolved in N,N-dimethylformamide (5 mL), added with potassium carbonate (433.9 mg, 3.14 mmol) in one portion, the reaction system was heated to 80°C and reacted for 2 h, the reaction was monitored by liquid chromatography-mass spectrometry, and the reaction solution was purified by silica gel column (petroleum ether: ethyl acetate = 1:3) to obtain 500 mg of the title compound.

The structure characterization data were as follows:
ESI-MS (m/z): 868.2[M+H]$^+$.

Step 4: 4-((S)-2-((S)-2-(4-(1-(26-azido-3,6,9,12,15,18,21,24-octaoxahexacosyl)piperidin-4-yl)butanamido)-3-methylbutanamido)propanamido)benzyl (4-nitrophenyl) carbonate

At 25°C, **A-4-4** (500 mg, 0.58 mmol) was dissolved in N,N-dimethylformamide (2 mL), added with DIPEA (297.77 mg, 2.30 mmol), then added dropwise with di(p-nitrophenyl) carbonate (525.67 mg, 1.73 mmol) in N,N-dimethylformamide (1 mL), and reacted under stirring at 25°C for 16 h, and the reaction was monitored by liquid chromatography-mass spectrometry. The reaction solution was purified by high performance liquid chromatography (with conditions as follows), and the fractions were lyophilized to obtain 360 mg of the title compound.

Chromatographic column: SunFire Prep C18 OBD 19 mm×150 mm×5.0 μm

Mobile phase A: acetonitrile; Mobile phase B: water (0.05% trifluoroacetic acid)

| Time [min] | Mobile phase A [%] | Mobile phase B[%] | Flow rate [mL/min] |
|---|---|---|---|
| 0.00 | 20 | 80 | 70 |
| 7.00 | 20 | 80 | 70 |
| 60.00 | 80 | 20 | 70 |

The structure characterization data were as follows:
ESI-MS (m/z): 1017.0[M+H]$^+$.

Step 5: Synthesis of 4-((S)-2-((S)-2-(4-(1-(26-azido-3,6,9,12,15,18,21,24-octaoxahexacosyl) piperidin-4-yl)butyrylamido)-3-methylbutyrylamido)propionamido)benzyl ((1S,9S)-9-ethyl-5-fluoro-9-hydroxy-4-methyl-10,13-dioxo-2,3,9,10,13,15-hexahydro-1H,12H-benzopyrano[3',4':6,7]indolizino[1,2-b]quinolin-1-yl)carbamate

At 25°C, (1S,9S)-1-amino-9-ethyl-5-fluoro-9-hydroxy-4-methyl-1,2,3,9,12,15-hexahydro-10H,13H-benzopyrano[3',4':6,7]indolizino[1,2-b]quinoline-10,13-dione (Exatecan, 20.0 mg, 0.038 mmol) and compound **A-4-5** (42.10 mg, 41.4 μmol) were dissolved in DMF (1 mL), added with HOBT (6.1 mg, 45.2 μmol), DIPEA (9.7 mg, 75.3 μmol), after the addition, the reaction system was reacted at room temperature under stirring for 1 h, and the reaction was monitored by liquid chromatography-mass spectrometry. The reaction solution was purified by high-performance liquid chromatography (with conditions as follows), and the fractions were lyophilized to obtain 32 mg of the title compound.

Chromatographic column: SunFire Prep C18 OBD 19 mm×150 mm×5.0 μm

Mobile phase A: acetonitrile; Mobile phase B: water (0.05% formic acid solution)

| Time [min] | Mobile phase A [%] | Mobile phase B[%] | Flow rate [mL/min] |
|---|---|---|---|
| 0.00 | 10 | 90 | 28 |
| 2.00 | 10 | 90 | 28 |
| 18.00 | 90 | 10 | 28 |

The structure characterization data were as follows:
ESI-MS (m/z): 1635.1[M+H]$^+$.

Step 6: Synthesis of 4-((S)-2-((S)-3-methyl-2-(4-(1-(26-(4-((6-(2-(methylsulfonyl) pyrimidin-5-yl)hex-5-ynamido)methyl)-1H-1,2,3-triazol-1-yl)-3,6,9,12,15,18,21,24-octaoxahexacosyl)piperidin-4-yl)butanamido)butanamido)propanamido)benzyl ((1S,9S)-9-ethyl-5- fluoro-9-hydroxy-4-methyl-10,13-dioxo-2,3,9,10,13,15-hexahydro-1H,12H-benzopyrano[3',4':6,7]indolizino[1,2-b]quinolin-1-yl)-1-yl)carbamate

At 25°C, compound **A-4-6** (30 mg, 0.023 mmol) was dissolved in DMSO (0.5 mL) and water (0.1ml), added with 6-(2-(methylsulfonyl)pyrimidin-5-yl)-N-(prop-2-yn-1-yl)-hex- 5-ynamide (**IM-2**, 10.3 mg, 0.034 mmol) and cuprous bromide (6.5 mg, 0.045 mmol), and reacted at room temperature for 1 h, the reaction was monitored by liquid chromatography-mass spectrometry. The reaction solution was filtered and then purified by high-performance liquid chromatography (with conditions as follows). The fractions were freeze-dried to obtain 15.40 mg of the title compound.

Chromatographic column: SunFire Prep C18 OBD 19 mm×150 mm×5.0 μm

Mobile phase A: acetonitrile; Mobile phase B: water (0.05%TFA)

| Time [min] | Mobile phase A [%] | Mobile phase B[%] | Flow rate [mL/min] |
|---|---|---|---|
| 0.00 | 10 | 90 | 28 |
| 2.00 | 10 | 90 | 28 |
| 18.00 | 90 | 10 | 28 |

The structure characterization data were as follows:
ESI-MS (m/z): 1635.1[M+H]$^+$.

Example 5: (2S,3S,4S,5R,6S)-6-(4-((((2-((S)-7-Ethyl-7-hydroxy-8,11-dioxo-8,10,11,13-tetrahydro-7H-[1,3]dioxolo[4,5-g]pyrano[3',4':6,7]indolizino[1,2-b]quinolin-14-yl)ethyl)(isopropyl)carbamoyl)oxy)methyl)-2-((18-(2-(methylsulfonyl)pyrimidin-5-yl)-13-oxo-3,6,9-trioxa-12-azaoctadec-17-yn-1-yl)carbamoyl)phenoxy)-3,4,5-trihydroxytetrahydro-2H-pyran-2-carboxylic acid **(A-5)**

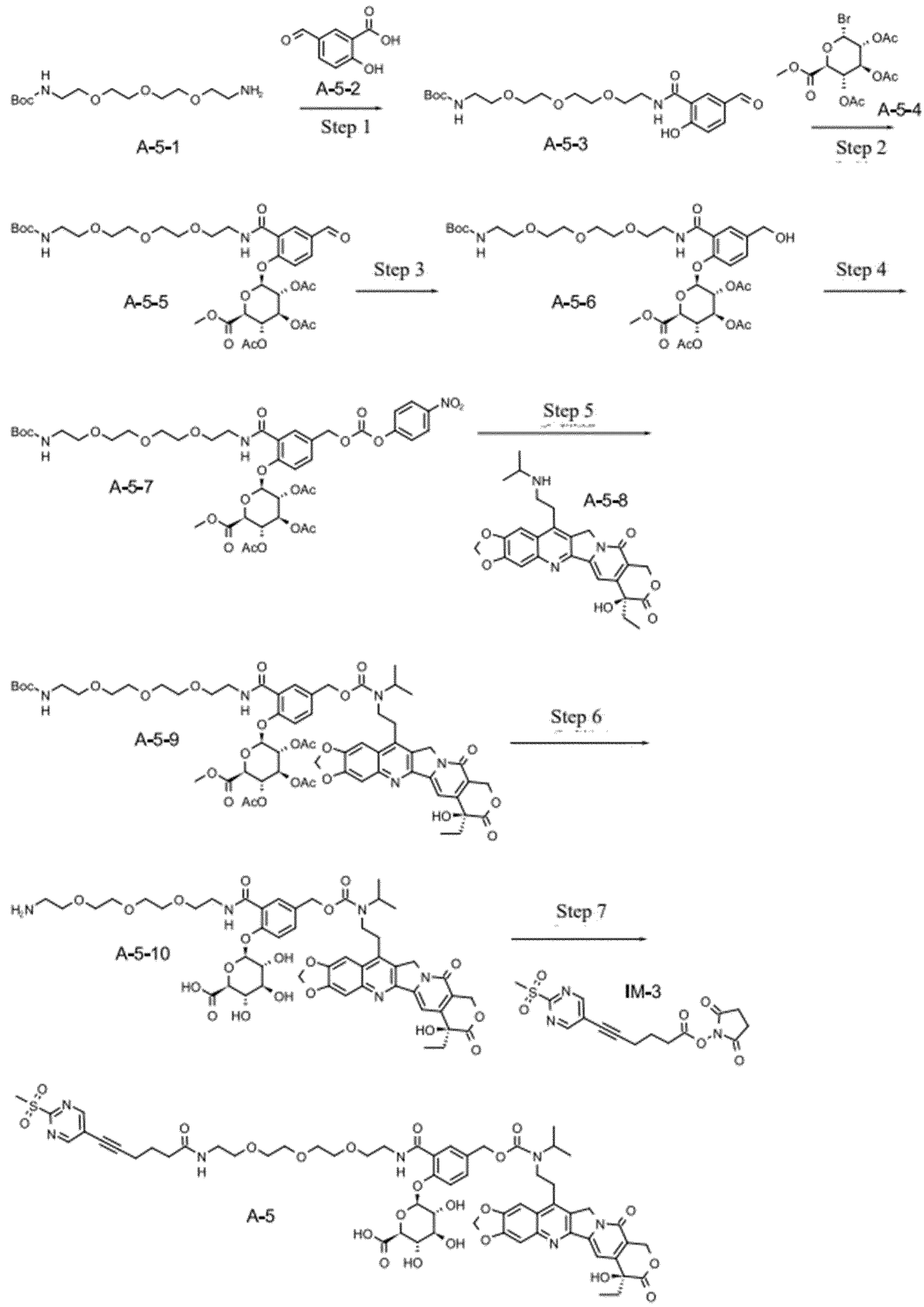


A-5-1 A-5-2 A-5-3 A-5-4 A-5-5 A-5-6 A-5-7 A-5-8 A-5-9 A-5-10 IM-3 A-5

Step 1: Synthesis of tert-butyl(1-(5-formyl-2-hydroxyphenyl)-1-oxo-5,8,11-trioxo- 2-azatridecan-13-yl)carbamate

5-Formyl-2-hydroxybenzoic acid (**A-5-2,** 625.04 mg, 3.76 mmol) was dissolved in dichloromethane (20 mL),

added with 1 drop of DMF, then added dropwise with thionyl chloride (2 mL), heated to reflux and reacted for 2 h. The reaction solution was concentrated under reduced pressure, then dissolved in dichloromethane (5 mL) to obtain an acyl chloride intermediate solution for later use. Tert-butoxycarbonyl triethylene glycol amine (**A-5-1**, 1 g, 3.42 mmol) was dissolved in dichloromethane (20 mL), cooled to 0°C, then added dropwise with the above acyl chloride solution and diisopropylethylamine (36.47 mg, 0.282 mmol), the reaction system was slowly heated to room temperature and reacted for 12 h, then concentrated under reduced pressure, and purified (with conditions as follows) to obtain **A-5-3** (271 mg).
ESI-MS (m/z): 441.2$[M+1]^+$.

Chromatographic column: Waters XBridge Prep C18OBD (5 μm*19mm*150 mm)

Mobile phase A: acetonitrile; Mobile phase B: water (0.05% Ammonium Bicarbonate)

| Time [min] | Mobile phase A [%] | Mobile phase B[%] | Flow rate [mL/min] |
|---|---|---|---|
| 0.00 | 10 | 90 | 28 |
| 2.00 | 10 | 90 | 28 |
| 18.00 | 90 | 10 | 28 |

Step 2: Synthesis of (2R,3R,4S,5S,6S)-2-(2-((2,2-dimethyl-4-oxo-3,8,11,14-tetraoxa- 5-azahexadecan-16-yl)carbamoyl)-4-formylphenoxy)-6-(methoxycarbonyl) tetrahydro-2H- pyran-3,4,5-triyl triacetate

**A-5-3** (270 mg, 0.613 mmol) and (2R,3R,5S)-2-bromo-6-(methoxycarbonyl) tetrahydro-2H-pyran-3,4,5-triacetate (**A-5-4**, 267.80 mg, 0.674 mmol) were dissolved in acetonitrile (30 mL), added with silver oxide (568.18 mg, 2.45 mmol) and 4A molecular sieve powder (1 g), reacted under stirring and nitrogen protection for 16 h. The reaction system was filtered and then concentrated under reduced pressure to obtain 460 mg of a crude product **A-5-5,** which was directly used in the next reaction.
ESI-MS (m/z): 757.4$[M+1]^+$.

Step 3: Synthesis of (2S,3R,4S,5S,6S)-2-(2-((2,2-dimethyl-4-oxo-3,8,11,14-tetraoxa-5-azahexadecan-16-yl)carbamoyl)-4-(hydroxymethyl)phenoxy)-6-(methoxycarbonyl)tetrahydro-2H-pyran-3,4,5-triyl triacetate

**A-5-5** (460 mg, 0.608 mmol) was dissolved in dichloromethane (5 mL) and isopropanol (5 mL), added with silica gel powder (1 g) and sodium borohydride (11.50 mg, 0.304 mmol) under stirring, reacted for 2 hours, filtered, and the filtrate was concentrated under reduced pressure and purified (with conditions as follows) to obtain **A-5-6** (343 mg).
ESI-MS (m/z): 759.3$[M+1]^+$.

Chromatographic column: Waters XBridge Prep C18OBD (8 μm*45mm*450 mm)

Mobile phase A: acetonitrile; Mobile phase B: water (0.05% Ammonium Bicarbonate)

| Time [min] | Mobile phase A [%] | Mobile phase B[%] | Flow rate [mL/min] |
|---|---|---|---|
| 0.00 | 25 | 75 | 70 |
| 5.00 | 25 | 75 | 70 |
| 50.0 | 90 | 10 | 70 |

Step 4: Synthesis of (2S,3R,4S,5S,6S)-2-(2-((2,2-dimethyl-4-oxo-3,8,11,14-tetraoxa-5-azahexadecan-16-yl)carbamoyl)-4-((((4-nitrophenoxy)carbonyl)oxy)methyl)phenoxy)-6-(methoxycarbonyl)tetrahydro-2H-pyran-3,4,5-triyl triacetate

**A-5-6** (343 mg, 0.452 mmol) was dissolved in anhydrous dichloromethane (25 mL), added with diisopropylethylamine (175.27 mg, 1.36 mmol), added dropwise with p-nitrophenyl chloroformate (273.35 mg, 1.36 mmol, dissolved in 25 mL of dichloromethane) under stirring, and reacted at room temperature for 12 h, the reaction system was concentrated under reduced pressure and purified on a silica gel column (eluent: 6% methanol/dichloromethane) to obtain compound **A-5-7** (342 mg).

Step 5: Synthesis of (2S,3R,4S,5S,6S)-2-(2-((2,2-dimethyl-4-oxo-3,8,11,14-tetraoxa- 5-azahexadecan-16-yl)carbamoyl)-4-(((2-((S)-7-ethyl-7-hydroxy-8,11-dioxo-8,10,11,13-tetrahydro-7H-[1,3]dioxolo[4,5-g]pyrano[3',4':6,7]indolizino[1,2-b]quinolin-14-yl)ethyl)(isopropyl)carbamoyl)oxy)methyl)phenoxy)-6-(methoxycarbonyl)tetrahydro-2H-pyran-3,4,5-triyl triacetate

**A-5-7** (53.93 mg, 0.058 mmol), (S)-7-ethyl-7-hydroxy-14-(2-(isopropylamino)ethyl)- 10,13-dihydro-11H-[1,3]dioxolo[4,5-g]pyrano[3',4':6,7]indolizino[1,2-b]quinoline-8,11(7H)-dione (**A-5-8,** 20 mg, 0.039 mmol) and HOBt (15.77 mg, 0.117 mmol) were dissolved in DMF (1 mL), added dropwise with diisopropylethylamine (15.09 mg, 0.117 mmol), stirred and reacted for 12 h, added with water and ethyl acetate and stirred, allowed to stand to separate the liquids, the organic phase was washed with saturated brine, dried, and concentrated under reduced pressure to obtain 49 mg of a crude product of **A-5-9,** which was directly used in the next reaction.
ESI-MS (m/z): 1262.5[M+1]$^+$.

Step 6: Synthesis of (2S,3S,4S,5R,6S)-6-(2-((2-(2-(2-aminoethoxy)ethoxy)ethyl) carbamoyl)-4-(((2-((S)-7-ethyl-7-hydroxy-8,11-dioxy-8,10,11,13-tetrahydro-7H-[1,3]dioxolo[4,5-g]pyrano[3',4':6,7]indolizino[1,2-b]quinolin-14-yl)ethyl)(isopropyl)carbamoyl)oxy)methyl)phenoxy)-3,4,5-trihydroxytetrahydro-2H-pyran-2-carboxylic acid

**A-5-9** (49 mg, 0.039 mmol) was dissolved in methanol (2 mL), added dropwise with lithium hydroxide (13.03 mg, 0.311 mmol, dissolved in 0.5 mL of water) aqueous solution, stirred and reacted for 1 h, and the system was concentrated under reduced pressure, then added dropwise with trifluoroacetic acid (2 mL), reacted under stirring for 1 h, and the reaction was monitored by liquid chromatography-mass spectrometry until it was completed. The reaction system was concentrated under reduced pressure, and purified (with conditions as follows) to obtain compound **A-5-10** (23 mg).
ESI-MS (m/z): 1022.0[M+1]$^+$.

Chromatographic column: Waters XBridge Prep C18OBD (5 μm*19mm*150 mm)

Mobile phase A: acetonitrile; Mobile phase B: water (0.05%TFA)

| Time [min] | Mobile phase A [%] | Mobile phase B[%] | Flow rate [mL/min] |
|---|---|---|---|
| 0.00 | 10 | 90 | 28 |
| 2.00 | 10 | 90 | 28 |
| 18.00 | 90 | 10 | 28 |

Step 7: Synthesis of (2S,3S,4S,5R,6S)-6-(4-((((2-((S)-7-ethyl-7-hydroxy-8,11-dioxo-8, 10,11,13-tetrahydro-7H-[1,3]dioxolo[4,5-g]pyrano[3',4':6,7]indolizino[1,2-b]quinolin-14-yl)ethyl)(isopropyl)carbamoyl)oxy)methyl)-2-((18-(2-(methylsulfonyl)pyrimidin-5-yl)-13-oxo-3,6,9-trioxa-12-azaoctadec-17-yn-1-yl)carbamoyl)phenoxy)-3,4,5-trihydroxytetrahydro-2H-pyran-2-carboxylic acid

**A-5-10** (23 mg, 0.020 mmol) and 6-(2-methylsulfonylpyrimidin-5-yl)-hex-5-ynoic acid 2,5-dioxopyrrolidin-1-yl ester (**IM-3,** 8.14 mg, 0.022 mmol) was dissolved in DMF (1 mL), added dropwise with diisopropylethylamine (7.85 mg, 0.061 mmol), reacted under stirring for 2 hours, and the raw materials were substantially consumed up monitored by TLC. The reaction system was purified (with conditions as follows) to obtain compound **A-5** (5.10 mg).
ESI-MS (m/z): 1272.3[M+1]$^+$.

Chromatographic column: SunFire Prep C18 OBD 19 mm×150 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 30 | 70 | 24 |
| 2.00 | 30 | 70 | 24 |
| 18.00 | 70 | 30 | 24 |

Example 6: (2S,3S,4S,5R,6S)-6-(4-(((2-((S)-7-Ethyl-7-hydroxy-8,11-dioxo-8,10,11,13-tetrahydro-7H-[1,3]dioxolo[4,5-g]pyrano[3',4':6,7]indolizino[1,2-b]quinolin-14-yl)ethyl) (isopropyl)carbamoyl)oxy)methyl)-2-(2-(2-(2-(6-(2-(methylsulfonyl)pyrimidin-5-yl)ethoxy)acetamido)acetamido)phenoxy)-3,4,5-trihydroxytetrahydro-2H-pyran-2-carboxylic acid **(A-6)**

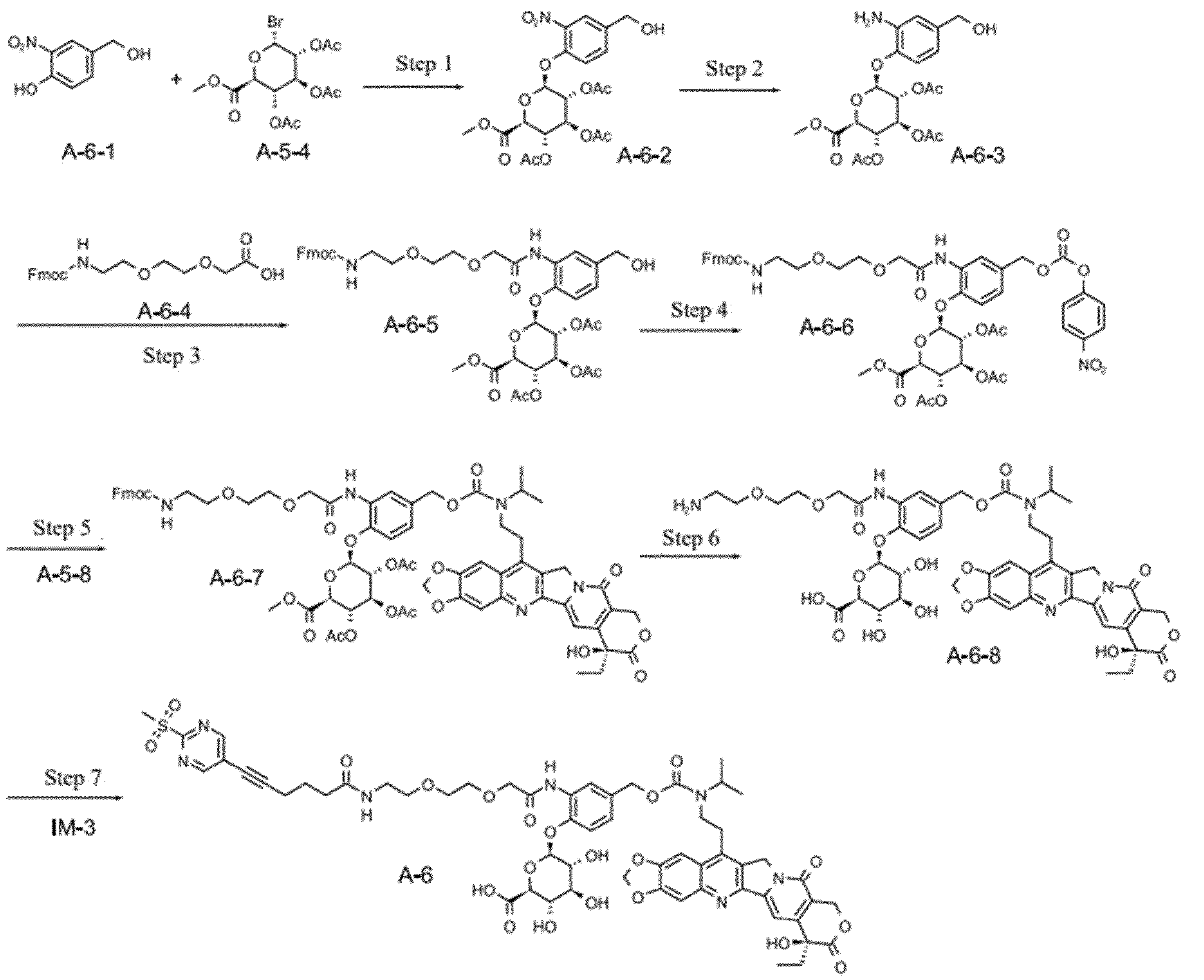


Step 1: Synthesis of (2S,3R,4S,5S,6S)-2-(4-(hydroxymethyl)-2-nitrophenoxy)-6-(methoxycarbonyl)tetrahydro-2H-pyran-3,4,5-triyl triacetate

Compound (2R,3R,4S,5S,6S)-2-bromo-6-(methoxycarbonyl)tetrahydro-2H-pyran-3,4,5-triacetic acid triester (**A-5-4,** 12.32 g, 31.02 mmol) and 4-hydroxy-3-nitrobenzyl alcohol (**A-6-1,** 5 g, 29.56 mmol) were dissolved in acetonitrile (200 mL), added with silver oxide (27.40 g, 118.25 mmol) under stirring, and reacted at room temperature for 12 hours in the dark after nitrogen replacement. The reaction was monitored by liquid chromatography-mass spectrometry until it was completed. The reaction system was filtered through diatomite. The filtrate was concentrated under reduced pressure and then purified by silica gel column chromatography (petroleum ether: ethyl acetate = 1:3) to obtain 12.8 g of the title compound.

The structure characterization data were as follows:

ESI-MS (m/z): 503[M+18]$^+$.

Step 2: Synthesis of (2S,3R,4S,5S,6S)-2-(2-amino-4-(hydroxymethyl)phenoxy)-6-(methoxycarbonyl)tetrahydro-2H-pyran-3,4,5-triyl triacetate

Compound **A-6-2** (2.2 g, 4.53 mmol) was dissolved in ethyl acetate and tetrahydrofuran (50.00 mL each), added with $PtO_2$ (200 mg), then the reaction system was subjected to replacement with a hydrogen balloon three times, and reacted under hydrogen atmosphere for 2 hours. The reaction was monitored by liquid chromatography-mass spectrometry, the reaction system was filtered, and the filtrate was concentrated to dryness under reduced pressure to obtain 2.02 g of a crude product of the title compound, which was directly used in the next reaction.
The structure characterization data were as follows:
ESI-MS (m/z): 456.1[M+1]$^+$.

Step 3: Synthesis of (2S,3R,4S,5S,6S)-2-(2-(1-(9H-fluoren-9-yl)-3-oxo-2,7,10-trioxa-4-azadodecyl)-4-(hydroxymethyl)phenoxy)-6-(methoxycarbonyl)tetrahydro-2H-pyran-3,4,5-triyl triacetate

Compound **A-6-3** (456 mg, 1.00 mmol) and (2-(2-(Fmoc-amino)ethoxy)ethoxy)acetic acid (**A-6-4,** 385.91 mg, 1.00 mmol) were dissolved in dichloromethane (10 mL), added with 2-ethoxy-1-ethoxycarbonyl-1,2-dihydroquinoline (495.22 mg, 2.00 mmol), reacted under stirring for 2 hours. The reaction was monitored by liquid chromatography-mass spectrometry. The reaction solution was concentrated under reduced pressure and then purified by silica gel column chromatography (methanol:dichloromethane=1:20) to obtain 507 mg of the title compound.
The structure characterization data were as follows:
ESI-MS (m/z): 823.3[M+1]$^+$.

Step 4: Synthesis of (2S,3R,4S,5S,6S)-2-(2-(1-(9H-fluoren-9-yl)-3-oxo-2,7,10-trioxa-4-azadodecan-12-amido)-4-((((4-nitrophenoxy)carbonyl)oxy)methyl)phenoxy)-6-(methoxycarbonyl)tetrahydro-2H-pyran-3,4,5-triyl triacetate

Nitrophenyl chloroformate (372.60 mg, 1.85 mmol) in dichloromethane (1 mL) was slowly added dropwise into Compound **A-6-5** (507 mg, 616.18 μmol) and diisopropylethylamine (238.91 mg, 1.85 mmol) in dichloromethane (20 mL), after the addition, the reaction system was reacted at room temperature for 15 h. The reaction was monitored by liquid chromatography-mass spectrometry. The reaction solution was concentrated under reduced pressure and then purified by silica gel column chromatography (methanol:dichloromethane=1:20) to obtain 496 mg of the title compound.
The structure characterization data were as follows:
ESI-MS (m/z): 988.5[M+1]$^+$.

Step 5: Synthesis of (2S,3R,4S,SS,6S)-2-(2-(1-(9H-fluoren-9-yl)-3-oxo-2,7,10-trioxa-4-azadodecan-12-amido)-4-((((2-((S)-7-ethyl-7-hydroxy-8,11-dioxo-8,10,11,13-tetrahydro-7H-[1,3]dioxolo[4,5-g]pyrano[3',4':6,7]indolizino[1,2-b]quinolin-14-yl)ethyl)(isopropyl)carbamoyl)oxy)methyl)phenoxy)-6-(methoxycarbonyl)tetrahydro-2H-pyran-3,4,5-triyl triacetate

Compound **A-6-6** (165.51 mg, 0.168mmol), compound **A-5-8** (40 mg, 0.084mmol) and 1-hydroxybenzotriazole (33.96 mg, 0.251mmol) were dissolved in DMF (4 mL), added dropwise with diisopropylethylamine (32.48 mg, 0.251mmol), and reacted under stirring for 12h. The reaction was monitored by liquid chromatography-mass spectrometry, the reaction system was added with an appropriate amount of water and ethyl acetate, stirred and then allowed to stand to separate the liquids. The organic phase was washed with saturated brine and dried, then filtered and concentrated under reduced pressure to obtain 100 mg of a crude product of the title compound, which was directly used in the next reaction.
The structure characterization data were as follows:
ESI-MS (m/z): 1326.2[M+1]$^+$.

Step 6: Synthesis of (2S,3S,4S,5R,6S)-6-(2-(2-(2-aminoethoxy)ethoxy)acetamido)- 4-(((2-((S)-7-ethyl-7-hydroxy-8,11-dioxo-8,10,11,13-tetrahydro-7H-[1,3]dioxolo[4,5-g]pyrano[3',4':6,7]indolizino[1,2-b]quinolin-14-yl)ethyl)(isopropyl)carbamoyl)oxy)methyl)phenoxy)-3,4,5-trihydroxytetrahydro-2H-pyran-2-carboxylic acid

To compound **A-6-7** (100 mg, 0.075 mmol) in MeOH (5 mL), 1 drop of dichloromethane and lithium hydroxide monohydrate (15.82 mg, 0.377 mmol) in water (1 mL) was added dropwise, reacted under stirring for 2 hours, the reaction was monitored by liquid chromatography-mass spectrometry, then the reaction solution was added with 3N hydrochloric acid aqueous solution to adjust the pH to 4, the reaction system was concentrated under reduced pressure and then

purified by high-performance liquid chromatography (with conditions as follows), and the fractions were freeze-dried to obtain 27.0 mg of the title compound.

Chromatographic column: SunFire Prep C18 OBD 19 mm×150 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 20 | 80 | 28 |
| 2.00 | 20 | 80 | 28 |
| 18.00 | 80 | 20 | 28 |

The structure characterization data were as follows:
ESI-MS (m/z): 964.2[M+1]$^+$.

Step 7: Synthesis of (2S,3S,4S,5R,6S)-6-(4-(((2-((S)-7-ethyl-7-hydroxy-8,11-dioxo-8,10, 11,13-tetrahydro-7H-[1,3]dioxolo[4,5-g]pyrano[3',4':6,7]indolizino[1,2-b]quinolin-14-yl)ethyl)(isopropyl)carbamoyl)oxy)methyl)-2-(2-(2-(2-(6-(2-(methylsulfonyl)pyrimidin-5-yl)ethoxy)acetamido)acetamido)phenoxy)-3,4,5-trihydroxytetrahydro-2H-pyran-2-carboxylic acid

Compound **A-6-8** (27 mg, 0.028mmol) and 6-(2-methylsulfonylpyrimidin-5-yl)-hex-5-ynoic acid 2,5-dioxopyrrolidin-1-yl ester (**IM-3,** 11.26 mg, 0.031mmol) were dissolved in DMF (1mL), added dropwise with diisopropylethylamine (3.62 mg, 0.028mmol) under stirring, and reacted at room temperature for 4 hours, and the reaction was monitored by liquid chromatography-mass spectrometry. The reaction solution was purified by high performance liquid chromatography (with conditions as follows), and the fractions were lyophilized to obtain 11.7 mg of the title compound.

Chromatographic column: SunFire Prep C18 OBD 19 mm×150 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 20 | 80 | 28 |
| 2.00 | 20 | 80 | 28 |
| 18.00 | 80 | 20 | 28 |

The structure characterization data were as follows:
ESI-MS (m/z): 1214.4[M+1]$^+$.

Example 7: (2S,3S,4S,5R,6S)-6-(4-(((((((((1S,9S)-9-ethyl-5-fluoro-9-hydroxy-4-methyl-10, 13-dioxo-1,2,3,9,10,12,13,15-octahydroxybenzo[de]pyrano[3',4':6,7]indolizino[1,2-b]quinolin-1-yl)carbamoyl)oxy)methyl)-2-(2-(2-(6-(2-(2-(methylsulfonyl)pyrimidin-5-yl)hex-5-ynamido)ethoxy)acetamido)phenoxy)-3,4,5-trihydroxytetrahydro-2H-pyran-2-carboxylic acid **(A-7)**

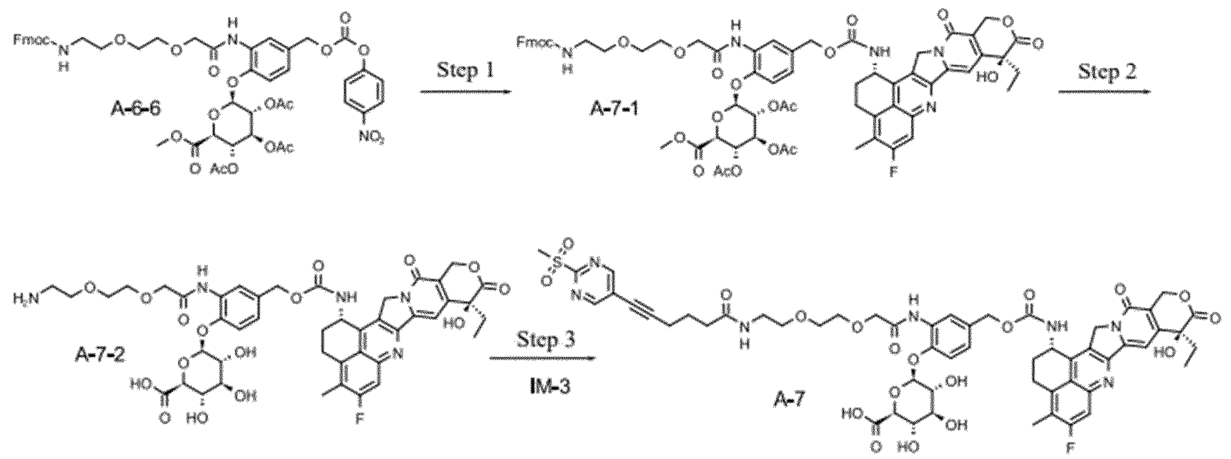


Step 1: Synthesis of (2S,3R,4S,5S,6S)-2-(2-(1-(9H-fluoren-9-yl)-3-oxo-2,7,10-trioxa-4-aza-dodecan-12-amido)-4-((((((1S,9S)-9-ethyl-5-fluoro-9-hydroxy-4-methyl-10,13-dioxo-1,2,3,9,10,12,13,15-octahydrobenzo[de]pyrano[3',4':6,7]indolizino[1,2-b]quinolin-1-yl)carbamoyl)oxy)methyl)phenoxy)-6-(methoxycarbonyl)tetrahydro-2H-pyran-3,4,5-triyl triacetate

**A-6-6** (185.85mg, 0.188mmol), Exatecan (50mg, 0.094mmol) and HOBt (38.13mg, 0.282mmol) were dissolved in DMF (4mL), added dropwise with diisopropylethylamine (36.47 mg, 0.282mmol), and reacted under stirring for 12 hours. The raw materials were consumed up monitored by LCMS. The reaction system was extracted with water and ethyl acetate, the organic phase was washed with saturated brine, then dried, and concentrated under reduced pressure to obtain 120 mg of a crude yellow oil, which was directly used in the next reaction.
ESI-MS (m/z): 1285.4[M+1]$^+$

Step 2: Synthesis of (2S,3S,4S,5R,6S)-6-(2-(2-(2-aminoethoxy)ethoxy)acetamido)-4-(((((1S, 9S)-9-ethyl-5-fluoro-9-hydroxy-4-methyl-10,13-dioxo-1,2,3,9,10,12,13,15-octahydroxybenzo[de]pyrano[3',4':6,7]indolizino[1,2-b]quinolin-1-yl)carbamoyl)oxy)phenoxy)-3,4,5-trihydroxytetrahydro-2H-pyran-2-carboxylic acid

**A-7-1** (120mg, 0.093mmol) was dissolved in methanol (5mL) and dichloromethane (about 50uL), added dropwise with lithium hydroxide aqueous solution (31.37mg, 0.747mmol, dissolved in 1mL water), and reacted under stirring for 2 hours. The raw materials were consumed up monitored by LCMS. The reaction system was added dropwise with 3N hydrochloric acid aqueous solution to adjust pH=4, concentrated under reduced pressure, and then purified to obtain 32 mg of **A-7-2** as a white solid.
ESI-MS (m/z): 922.3[M+1]$^+$

Step 3: Synthesis of (2S,3S,4S,5R,6S)-6-(4-(((((((((1S,9S)-9-ethyl-5-fluoro-9-hydroxy-4-methyl-10,13-dioxo-1,2,3,9,10,12,13,15-octahydroxybenzo[de]pyrano[3',4':6,7]indolizino[1,2-b]quinolin-1-yl)carbamoyl)oxy)methyl)-2-(2-(2-(6-(2-(2-(methylsulfonyl)pyrimidin-5-yl)hex-5-ynamido)ethoxy)acetamido)phenoxy)-3,4,5-trihydroxytetrahydro-2H-pyran-2-carboxylic acid

**A-7-2** (30 mg, 0.033mmol) and 6-(2-methylsulfonylpyrimidin-5-yl)-hex-5-ynoic acid 2,5-dioxopyrrolidin-1-yl ester (**IM-3,** 13.08mg, 0.036mmol) were dissolved in DMF (1mL), added dropwise with diisopropylethylamine (4.21 mg, 0.033mmol) under stirring, and reacted at room temperature for 4 hours. The raw materials were consumed up monitored by LCMS. The reaction system was purified to obtain 18.54 mg of **A-7** as a white solid. ESI-MS (m/z): 1171.8[M+1]$^+$.

Chromatographic column: Waters XBridge Prep C18OBD (5 μm*19mm*150 mm)

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 10 | 90 | 25 |

(continued)

| Time [min] | Mobile phase A [%] | Mobile phase B[%] | Flow rate [mL/min] |
|---|---|---|---|
| 2.00 | 10 | 90 | 25 |
| 18.00 | 90 | 10 | 25 |

Example 8: (9S)-1-amino-5-chloro-9-ethyl-9-hydroxy-4-methyl-1,2,3,9,12, 15-hexahydro-10H,13H-benzopyrano[3',4':6,7]indolizino[1,2-b]quinoline-10,13-dione **(1-4)**

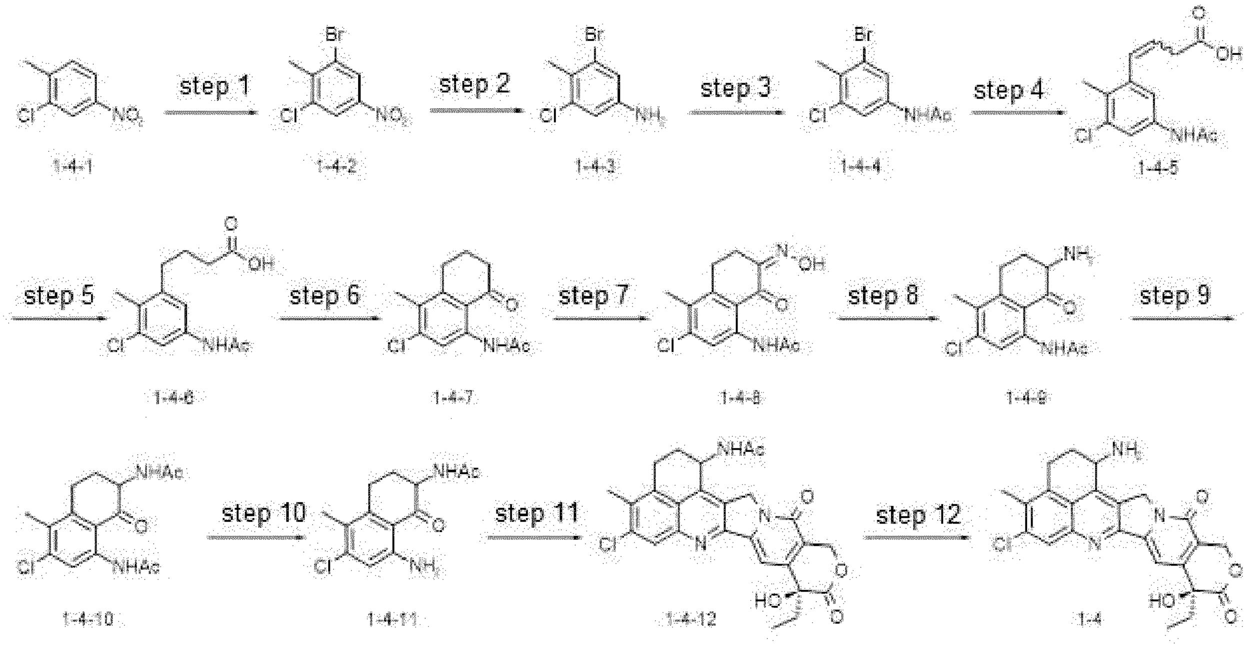


Step 1: Synthesis of 1-chloro-3-bromo-2-methyl-5-nitrobenzene

At 25°C, compound **1-4-1** (5.00 g, 29.14 mmol) was dissolved in n-heptane (25 mL), added with concentrated sulfuric acid (25 mL), heated to 50°C, added with NBS (6.22 g, 34.97 mmol) in batches at 50°C, reacted at 50°C for 2 hours, the reaction was monitored by thin layer chromatography (ethyl acetate:petroleum ether=1:10), the reaction solution was cooled to room temperature and added dropwise into ice water, extracted with toluene, the organic phases were combined, washed sequentially with sodium sulfite solution, water and saturated brine, dried over anhydrous sodium sulfate, concentrated under reduced pressure, the crude product was purified by preparative high performance liquid chromatography, and the fractions were freeze-dried to obtain 4.88 g of the title compound.

Chromatographic column: C18 ODS 45 mm×450 mm×8.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0 | 60 | 40 | 60 |
| 10 | 60 | 40 | 60 |
| 40 | 100 | 0 | 60 |

Step 2: Synthesis of 3-chloro-5-bromo-4-methylaniline

At 25°C, compound **1-4-2** (4.88 g, 19.48 mmol) was dissolved in ethyl acetate (100 mL), added with platinum on carbon (2.00 g, 19.48 mmol, content 5%), subjected to hydrogen replacement, then reacted under the protection with hydrogen balloon at 60°C for 4 hours, and the reaction was monitored by liquid chromatography-mass spectrometry. The reaction solution was filtered, and the filtrate was concentrated to obtain 3.68 g of a crude product of the title compound, which was directly used in the next reaction without further purification.

Step 3: Synthesis of N-(3-chloro-5-bromo-4-methylphenyl)acetamide

At 20°C, compound **1-4-3** (3.63 g, 14.82 mmol) was dissolved in ethyl acetate (70 mL), added with triethylamine (4.50 g, 44.45 mmol) and acetic anhydride (2.27 g, 22.23 mmol), and reacted at 20°C for 20 hours, the reaction was monitored by liquid chromatography-mass spectrometry. The reaction solution was extracted by adding water and ethyl acetate, the organic phases were combined, dried over anhydrous sodium sulfate, and concentrated under reduced pressure to obtain a crude product, which was slurried with a mixed solvent of ethyl acetate:petroleum ether=1:5 to obtain 2.86 g of the title compound.

Step 4: Synthesis of (Z)-4-(5-acetamido-3-chloro-2-methylphenyl)but-3-enoic acid

At 20°C, compound **1-4-4** (1.80 g, 6.86 mmol) was dissolved in THF (20 mL) and water (5 mL), added with vinylacetic acid (708.31 mg, 8.23 mmol), DIPEA (1.95 g, 15.08 mmol), tri(o-methylphenyl)phosphorus (62.60 mg, 0.20 mmol), the reaction system was replaced with nitrogen, then heated to 70°C and reacted for 5 hours, and the reaction was monitored by liquid chromatography-mass spectrometry. The reaction solution was added with 1N sodium hydroxide solution to adjust the pH=8, and added with ethyl acetate for extraction. The remaining aqueous phase was adjusted to pH=3 with 1N hydrochloric acid, extracted with ethyl acetate, and the organic phases were combined, dried over anhydrous sodium sulfate, and concentrated under reduced pressure to obtain 0.82 g of the title compound, which was directly used in the next reaction.

Step 5: Synthesis of 4-(5-acetamido-3-chloro-2-methylphenyl)butanoic acid

At 20°C, compound **1-4-5** (2.60 g, 9.71 mmol) was dissolved in THF (50 mL), and added with Pd/C (0.52 g, content 10%), the reaction system was replaced with hydrogen, then reacted under the protection of hydrogen balloon at 40°C for 2 hours, and the reaction was monitored by liquid chromatography-mass spectrometry. The reaction solution was filtered, and the filtrate was concentrated under reduced pressure to obtain 2.43 g of the title compound, which was directly used in the next reaction without further purification.

Step 6: Synthesis of N-(3-chloro-4-methyl-8-oxo-5,6,7,8-tetrahydronaphthalen-1-yl)acetamide

Compound **1-4-6** (2.43 g, 9.01 mmol) was dissolved in trifluoroacetic acid (10 mL), cooled to 5°C, added dropwise with trifluoroacetic anhydride (3.78 g, 18.02 mmol, 2.50 mL), and reacted at 5°C for 4 hours, and the reaction was monitored by liquid chromatography-mass spectrometry. The reaction solution was added into water, adjusted with 10N sodium hydroxide to pH=9, extracted with ethyl acetate, the organic phases were combined, dried over anhydrous sodium sulfate, concentrated under reduced pressure, and purified by a flash silica gel column (ethyl acetate: petroleum ether=0-20%) to obtain 1.53 g the title compound.

Step 7: Synthesis of (Z)-N-(3-chloro-7-(hydroxyimino)-4-methyl-8-oxo-5,6,7,8-tetrahydronaphthalen-1 -yl)acetamide

At 5°C, potassium tert-butoxide (1.50 g, 13.37 mmol) was dissolved in THF (16 mL) and tert-butanol (4 mL), added dropwise with a solution of compound **1-4-7** (1.53 g, 6.08 mmol) in THF (16 mL), after 10 minutes, added dropwise with amyl nitrite (1.14 g, 9.73 mmol), reacted at 5°C for 1 hour, and the reaction was monitored by liquid chromatography-mass spectrometry. The reaction solution was adjusted to pH=5 with 1N hydrochloric acid, extracted with ethyl acetate, the organic phases were combined, dried over anhydrous sodium sulfate, concentrated under reduced pressure, and the concentrate was slurried with methyl tert-butyl ether to obtain 1.20 g of the title compound.

Step 8: N-(7-Amino-3-chloro-4-methyl-8-oxo-5,6,7,8-tetrahydronaphthalen-1-yl)acetamide

At 20°C, compound **1-4-8** (0.50 g, 1.78 mmol) was dissolved in methanol (8 mL) and 2N hydrochloric acid (8 mL), added with Pd/C (0.15 g, content 10%), the system was subjected with hydrogen replacement, the reaction was

carried out at 5°C for 2 hours under the protection of a hydrogen balloon, and the reaction was monitored by liquid chromatography-mass spectrometry. The reaction solution was filtered, and the filtrate was concentrated under reduced pressure to obtain 0.52 g of a hydrochloride salt of the title compound, which was directly used in the next reaction without further purification.

Step 9: Synthesis of N,N'-(3-chloro-4-methyl-8-oxo-5,6,7,8-tetrahydronaphthalen- 1,7-diyl)diacetamide

At 20°C, compound **1-4-9** (0.52 g, 1.70 mmol) was dissolved in pyridine (5 mL), added with acetic anhydride (2 mL), and reacted at 20°C for 2 hours, the reaction was monitored by liquid chromatography-mass spectrometry. The reaction solution was added into water, extracted with ethyl acetate, the organic phases were washed with water, combined, dried over anhydrous sodium sulfate, concentrated under reduced pressure, and the concentrate was purified by a flash silica gel column (ethyl acetate:petroleum ether=0-30%) to obtain 0.22 g of the title compound.

Step 10: Synthesis of N-(8-amino-6-chloro-5-methyl-1-oxo-1,2,3,4-tetrahydronaphthalen- 2-yl) acetamide

At 20°C, compound **1-4-10** (0.45 g, 1.46 mmol) was dissolved in methanol (16 mL), added with 2N hydrochloric acid (16 mL), heated to 60°C and reacted for 2 hours, and the reaction was monitored by liquid chromatography-mass spectrometry. The reaction solution was cooled, adjusted to pH=8 by adding saturated sodium bicarbonate solution, extracted with ethyl acetate, the organic phases were combined, dried over anhydrous sodium sulfate, and concentrated under reduced pressure to obtain 0.23 g of the title compound, which was directly used in the next step without further purification.

Step 11: Synthesis of N-((9S)-5-chloro-9-ethyl-9-hydroxy-4-methyl-10,13-dioxo-2,3,9,10, 13,15-hexahydro-1H,12H-benzopyrano[3',4':6,7]indolizino[1,2-b]quinolin-1-yl)acetamide

Compound **1-4-11** (0.23 g, 0.78 mmol) was dissolved in toluene (10 mL), added with (S)-4-ethyl-4-hydroxy-7,8-dihydro-1H-pyrano[3,4-f]indolizine-3,6,10(4H)-trione (0.23 g, 0.87 mmol) and p-toluenesulfonic acid (26.73 mg, 0.16 mmol), heated to 140°C for 5 hours, and the reaction was monitored by LC-MS. The reaction solution was concentrated, and the crude product was purified by a flash silica gel column (methanol:dichloromethane=0-10%) to obtain 0.15 g of the title compound.

Step 12: Synthesis of (9S)-1-amino-5-chloro-9-ethyl-9-hydroxy-4-methyl-1,2,3,9,12,15-hexahydro-10H,13H-benzopyrano[3',4':6,7]indolizino[1,2-b]quinoline-10,13-dione

Compound **1-4-12** (40.00 mg, 0.08 mmol) was added into concentrated hydrochloric acid (1 mL), heated to 100°C and reacted for 5 hours, and the reaction was monitored by liquid chromatography-mass spectrometry. The reaction solution was filtered, the filtrate was purified by preparative high performance liquid chromatography, and the fractions were freeze-dried to obtain 12.00 mg of a trifluoroacetic acid salt of the title compound **1-4.**

Chromatographic column: SunFire Prep C18 OBD 19 mm×150 mm×5.0 μm

Mobile phase A: acetonitrile; Mobile phase B: water (0.05% trifluoroacetic acid)

| Time [min] | Mobile phase A [%] | Mobile phase B[%] | Flow rate [mL/min] |
|---|---|---|---|
| 0 | 5 | 95 | 28 |
| 2 | 5 | 95 | 28 |
| 18 | 50 | 50 | 28 |

The structure characterization data were as follows:
ESI-MS (m/z): 452.1[M+H]$^+$.

Example 9: N-((1S,9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)-2-hydroxyacetamide and N-((1R,9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)-2-hydroxyacetamide (**1-7-A** and **1-7-B**)

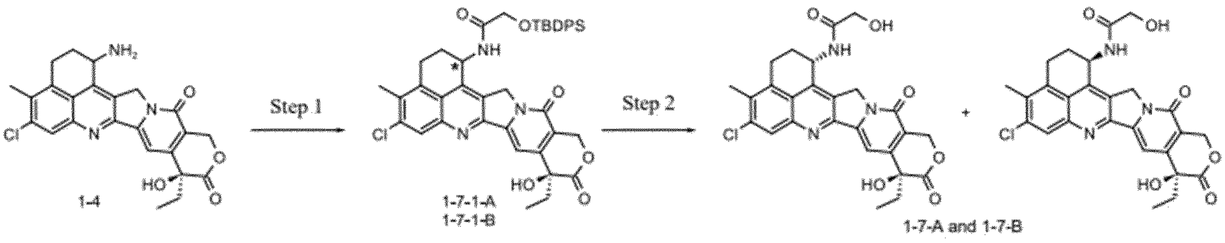


Note: * represents the labeled atom is a chiral atom

Step 1: Synthesis of 2-((tert-butyldiphenylsilyl)oxy)-N-((1S,9S)-5-chloro-9-ethyl- 9-hydroxy-4-methyl-10,13-dioxo-2,3,9,10,13,15-hexahydro-1H,12H-benzo[de]pyrano[3',4':6,7] indolizino[1,2-b]quinolin-1-yl)acetamide and 2-((tert-butyldiphenylsilyl)oxy)-N-((1R,9S)- 5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)acetamide

At 25°C, the trifluoroacetic acid salt of compound **1-4** (40.00 mg, 81.91 μmol) was dissolved in N,N-dimethylformamide (1 mL), added sequentially with 2-((tert-butyldiphenylsilyl)oxy)acetic acid (30.91 mg, 98.29 μmol), HATU (62.25 mg, 163.81 μmol) and N,N-diisopropylethylamine (42.34 mg, 327.63 μmol), reacted at 25°C for 0.5 hours, and the reaction was monitored by liquid chromatography-mass spectrometry. After the reaction was completed, the reaction solution was added with water, extracted with dichloromethane/methanol (v/v=10/1), the organic phases were combined, dried over anhydrous sodium sulfate, and concentrated under reduced pressure, and the crude product was purified and separated by preparative thin-layer chromatography (dichloromethane:methanol=20:1) to obtain two isomers, which were named as **1-7-1-A** (15.00 mg, Rf value was 0.3) and **1-7-1-B** (12.00 mg, Rf value 0.35) according to their Rf values.

Step 2: Synthesis of N-((1S,9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)-2-hydroxyacetamide and N-((1R,9S)-5-chloro-9-ethyl-9-hydroxy-4-methyl-10,13-dioxo-2,3,9,10, 13,15-hexahydro-1H,12H-benzo[de]pyrano[3',4':6,7]indolizino[1,2-b]quinolin-1-yl)-2-hydroxyacetamide

At 25°C, **1-7-1-A** (15.00 mg) and **1-7-1-B** (12.00 mg) were dissolved in tetrahydrofuran (1 mL) in two reaction flasks, respectively, and added dropwise respectively with a mixture of tetrabutylammonium fluoride (1M in tetrahydrofuran)/glacial acetic acid (v/v=13/1) (50 uL), reacted at 25°C for 0.5 hours, and the reaction was monitored by liquid chromatography-mass spectrometry. After the reaction was completed, the reaction solution was purified by preparative high performance liquid chromatography, and the fractions were freeze-dried to obtain compounds **1-7-A** (6.94 mg) and **1-7-B** (4.00 mg).

Chromatographic column: SunFire Prep C18 OBD 19 mm×150 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0 | 20 | 80 | 28 |
| 3 | 20 | 80 | 28 |
| 18 | 90 | 10 | 28 |

The structure characterization data of **1-7-A** were as follows:

$^1$H NMR (400 MHz, DMSO-*d6*) δ 8.43 (d, *J* = 8.8 Hz, 1H), 8.16 (s, 1H), 7.31 (s, 1H), 6.55 (s, 1H), 5.65-5.36 (m, 4H), 5.21 (q, *J* = 19.0 Hz, 2H), 3.95 (d, *J* = 5.7 Hz, 2H), 3.26-3.11 (m, 2H), 2.53 (s, 3H), 2.30-2.08 (m, 2H), 1.94-1.79 (m, 2H), 0.87 (t, *J* = 7.3 Hz, 3H).

ESI-MS (m/z): 510.1[M+H]$^+$.

The structure characterization data of **1-7-B** were as follows:

$^1$H NMR (400 MHz, DMSO-d6) δ 8.45 (d, *J* = 8.9 Hz, 1H), 8.15 (s, 1H), 7.31 (s, 1H), 6.54 (s, 1H), 5.64-5.35 (m, 4H), 5.19 (q, *J* = 19.0 Hz, 2H), 3.97 (d, *J* = 5.2 Hz, 2H), 3.27-3.10 (m, 2H), 2.51 (s, 3H), 2.27-2.10 (m, 2H), 1.93-1.80 (m, 2H), 0.88 (t, *J* = 7.3 Hz, 3H).
ESI-MS (m/z): 510.1[M+H]$^+$.

Example 10: (2S,3S,4S,5R,6S)-6-(4-(((((((((1S,9S)-5-chloro-9-ethyl-9-hydroxy-4-methyl-10,13-dioxo-1,2,3,9,10,12,13,15-octahydroxybenzo [de]pyrano [3',4':6,7]indolizino [1,2-b]quinolin-1-yl)carbamoyl)oxy)methyl)-2-(2-(2-(6-(2-(2-(methylsulfonyl)pyrimidin-5-yl)hex-5-acetamido)ethoxy)acetamido)phenoxy)-3,4,5-trihydroxytetrahydro-2H-pyran-2-carboxylic acid and (2S,3S, 4S,5R,6S)-6-(4-(((((((((1R,9S)-5-chloro-9-ethyl-9-hydroxy-4-methyl-10,13-dioxo-1,2,3,9,10,12,13,15-octahydroxybenzo[de]pyrano[3',4':6,7]indolizino[1,2-b]quinolin-1-yl)carbamoyl)oxy)methyl)-2-(2-(2-(6-(2-(2-(methylsulfonyl)pyrimidin-5-yl)hex-5-acetamido)ethoxy)acetamido)phenoxy)-3,4,5-trihydroxytetrahydro-2H-pyran-2-carboxylic acid (**A-8/9-A** and **A-8/9-B**)

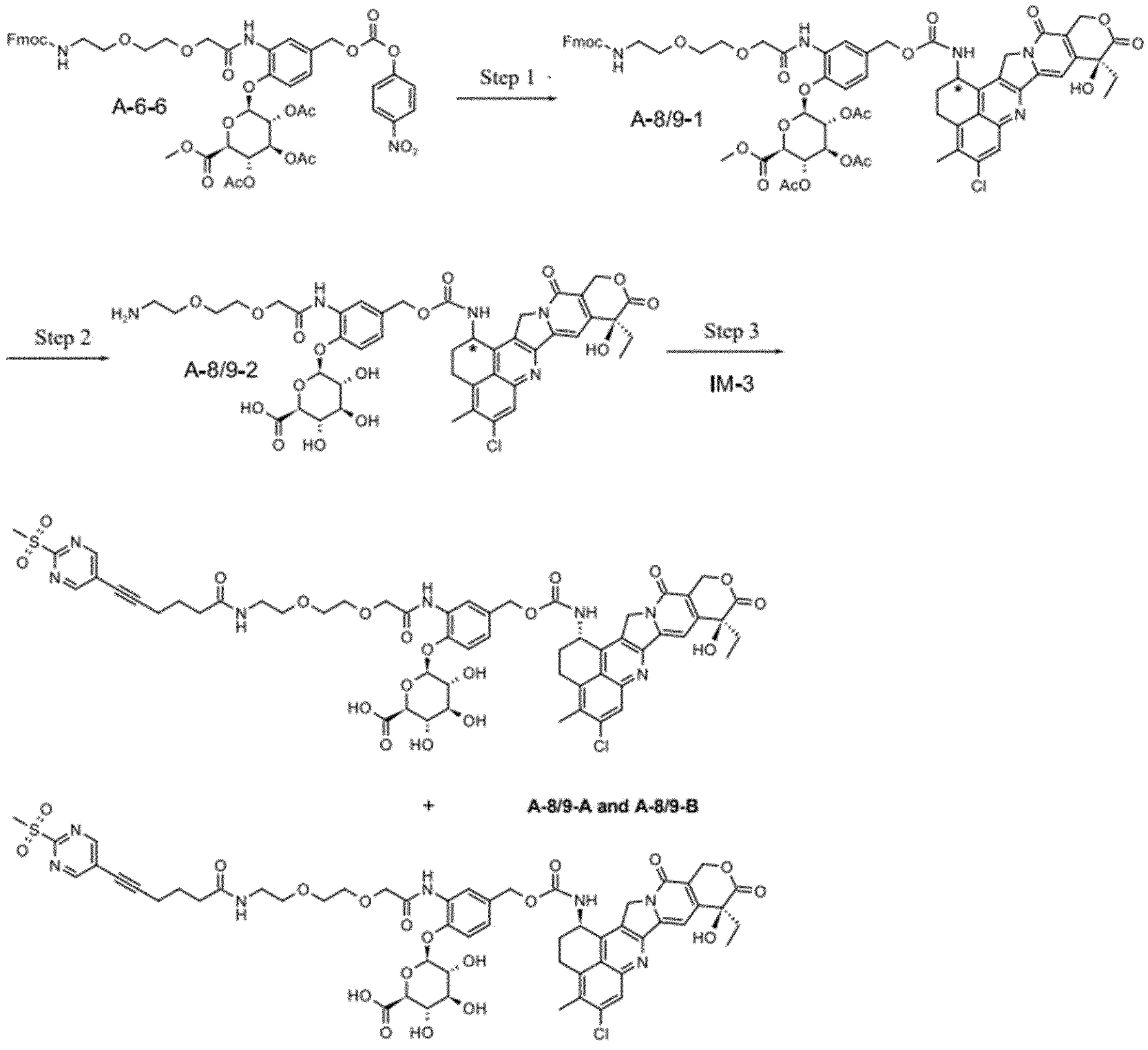


Note: * represents the labeled atom is a chiral atom

Step 1: Synthesis of (2S,3S,4S,5R,6S)-2-(2-(1-(9H-fluoren-9-yl)-3-oxo-2,7,10-trioxa-4-azadodecan-12-amido)-4-((((((((9S)-5-chloro-9-ethyl-9-hydroxy-4-methyl-10,13-dioxo-1,2,3,9,10,12,13,15-octahydrobenzo[de]pyrano[3',4':6,7]indolizino[1,2-b]quinolin-1-yl)carbamoyl)oxy)methyl)phenoxy)-6-(methoxycarbonyl)tetrahydro-2H-pyran-3,4,5-triyl triacetate

Starting materials **A-6-6** (303 mg, 0.307 mmol), **1-4** (100 mg, 0.205 mmol) and HOBt (83 mg, 0.614 mmol) were dissolved in DMF (5 mL), added dropwise with diisopropylethylamine (79 mg, 0.614 mmol), stirred and reacted for 12 hours. Water and ethyl acetate were stirred, and allowed to stand to separate the liquids. The organic phase was washed with saturated brine, dried, and concentrated under reduced pressure to obtain 260 mg of a crude product of **A-8/9-1,** which was directly used in the next reaction.
ESI-MS (m/z): 1300.7[M+1]$^+$.

Step 2: Synthesis of (2S,3S,4S,5R,6S)-6-(2-(2-(2-aminoethoxy)ethoxy)acetamido)- 4-(((((9S)-5-chloro-9-ethyl-9-hydroxy-4-methyl-10,13-dioxo-1,2,3,9,10,12,13,15-octahydroxy-benzo[de]pyrano[3',4':6,7]indolizino[1,2-b]quinolin-1-yl)carbamoyl)oxy)phenoxy)-3,4,5-trihydroxytetrahydro-2H-pyran-2-carboxylic acid

**A-8/9-1** (260 mg, 0.200 mmol) was dissolved in methanol (5 mL), then added with two drops of dichloromethane for dissolution, and added dropwise with lithium hydroxide aqueous solution (67 mg, 1.60 mmol, dissolved in 0.5 mL water), stirred and reacted for 2 hours. The raw materials were consumed up monitored by LCMS. The reaction system was added with 3N hydrochloric acid aqueous solution to adjust pH=4, concentrated under reduced pressure, and purified (with conditions as follows) to obtain **A-8/9-2** (63 mg).

ESI-MS (m/z): 938.3[M+1]+.

Column: Waters XBridge PrepC18OBD (5μm*19mm*150mm)

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 10 | 90 | 25 |
| 2.00 | 10 | 90 | 25 |
| 18.00 | 90 | 10 | 25 |

Step 3: Synthesis of (2S,3S,4S,5R,6S)-6-(4-((((((((1S,9S)-5-chloro-9-ethyl-9-hydroxy- 4-methyl-10,13-dioxo-1,2,3,9,10,12,13,15-octahydroxybenzo[de]pyrano[3',4':6,7]indolizino[1,2-b]quinolin-1-yl)carbamoyl)oxy)methyl)-2-(2-(2-(6-(2-(2-(methylsulfonyl)pyrimidin-5-yl)hex-5-acetamido)ethoxy)acetamido)phenoxy)-3,4,5-trihydroxytetrahydro-2H-pyran-2-carboxylic acid and (2S,3S,4S,5R,6S)-6-(4-((((((((1R,9S)-5-chloro-9-ethyl-9-hydroxy-4-methyl-10,13-dioxo-1,2,3,9,10,12,13,15-octahydroxybenzo[de]pyrano[3',4':6,7]indolizino[1,2-b]quinolin-1-yl)carbamoyl)oxy)methyl)-2-(2-(2-(6-(2-(2-(methylsulfonyl)pyrimidin-5-yl)hex-5-acetamido)ethoxy)acetamido)phenoxy)-3,4,5-trihydroxytetrahydro-2H-pyran-2-carboxylic acid

**A-8/9-2** (63 mg, 0.067 mmol) and 6-(2-methylsulfonylpyrimidin-5-yl)-hex-5-ynoic acid 2,5-dioxopyrrolidin-1-yl ester (**IM-3,** 27 mg, 0.074 mmol) were dissolved in DMF (1 mL), added dropwise with diisopropylethylamine (9 mg, 0.067 mmol) under stirring, and reacted at room temperature for 4 hours. The raw materials were consumed up monitored LCMS. The reaction solution was separated and purified (with conditions as follows) to obtain 10.14 mg of **A-8/9-A** and 14.60 mg of **A-8/9-B.**

Chromatographic column: Waters XBridge Prep C18OBD (5 μm*19mm*150 mm)

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 30 | 70 | 28 |
| 2.00 | 30 | 70 | 28 |
| 18.00 | 70 | 30 | 28 |

**A-8/9-A** peak retention time: 8.90 min. ESI-MS (m/z): 1188.6 [M+1]+.

**A-8/9-B** peak retention time: 9.10 min. ESI-MS (m/z): 1188.6 [M+1]+.

Example 11: N-((10S)-10-benzyl-1-(((1S,9S)-5-chloro-9-ethyl-9-hydroxy-4-methyl-10,13-dioxo-2,3,9,10,13,15-hexahydro-1H,12H-benzo[de]pyrano[3',4';6,7]indolizino[1,2-b]quinolin-1-yl)amino)-1,6,9,12,15-pentaoxo-3-oxa-5,8,11,14-tetraazahexadecan-16-yl)-6-(2-(methylsulfonyl)pyrimidin-5-yl)hexadecanamide and N-((10S)-10-benzyl-1-(((1R,9S)-5-chloro-9-ethyl-9- hydroxy-4-methyl-10,13-dioxo-2,3,9,10,13,15-hexahydro-1H,12H-benzo[de]pyrano[3',4';6,7]indolizino[1,2-b]quinolin-1-yl)amino)-1,6,9,12,15-pentoxo-3-oxa-5,8,11,14-tetraazahexadecan-16-yl)-6-(2-(methylsulfonyl)pyrimidin-5-yl)hexadecanamide (**A-10/11-A** and **A-10/11-B**)

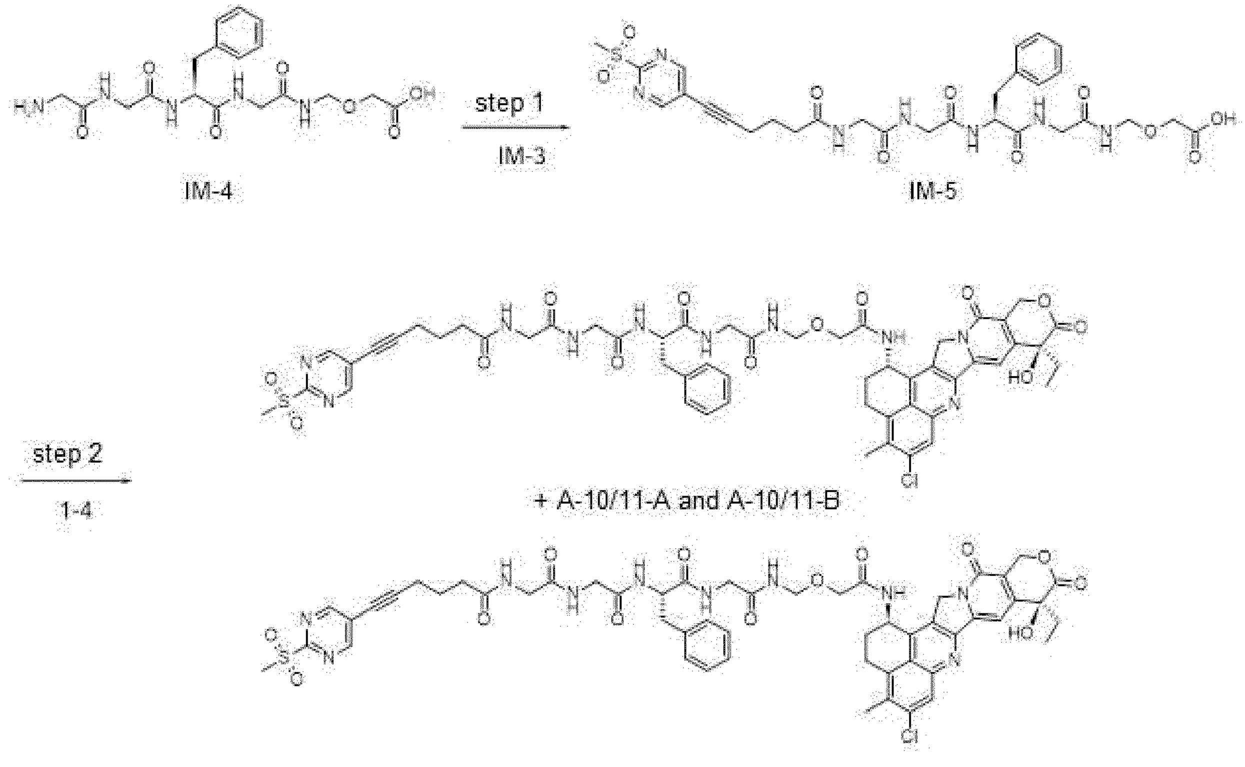


IM-4

IM-5

+ A-10/11-A and A-10/11-B

Step 1: Synthesis of (S)-10-benzyl-23-(2-(methylsulfonyl)pyrimidin-5-yl)-6,9,12,15,18-pentoxo-3-oxa-5,8,11,14,17-pentaazoctadec-22-ynoic acid

Compound **IM-4** (30.00 mg, 70.00 μmol) was dissolved in N,N-dimethylformamide (1 mL), added with 6-(2-methylsulfonylpyrimidin-5-yl)-hex-5-ynoic acid 2,5-dioxopyrrolidin-1-yl ester (**IM-3**, 28.00 mg, 77.00 μmol), reacted at room temperature for 1 hour, and the reaction was monitored by liquid chromatography-mass spectrometry. The reaction solution was directly purified by preparative high performance liquid chromatography, and the fractions were lyophilized to obtain the title compound **IM-5** (20.00 mg).

Chromatographic column: SunFire Prep C18 OBD 19 mm×150 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 10 | 90 | 28 |
| 2.00 | 10 | 90 | 28 |
| 18.00 | 90 | 10 | 28 |

The structure characterization data were as follows:
ESI-MS (m/z): 691.0[M+18]$^+$.

Step 2

At 25°C, the hydrochloride salt of **1-4** (30 mg, 61.43 μmol) was dissolved in N,N-dimethylformamide (1 mL), added sequentially with **IM-5** (49.66 mg, 73.72 μmol), HATU (35.01 mg, 92.14 μmol) and N,N-diisopropylethylamine (23.82 mg, 184.29 μmol), and reacted at 25°C for 0.5 hours, and the reaction was monitored by liquid chromatography-mass spectrometry. After the reaction was completed, the reaction solution was purified by high performance liquid chromatography (with conditions as follows) to separate two isomers, which were named as **A-10/11-A** (11.04 mg,

retention time was 7.5 min) and **A-10/11-B** (19.42 mg, retention time was 8.0 min) according to their retention time.

Chromatographic column: SunFire Prep C18 OBD 19 mm×150 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0 | 30 | 70 | 28 |
| 3 | 30 | 70 | 28 |
| 18 | 90 | 10 | 28 |

The structure characterization data were as follows:

**A-10/11-A:**
ESI-MS (m/z): 1107.3[M+H]$^+$.

**A-10/11-B:**
ESI-MS (m/z): 1107.3[M+H]$^+$.

Example 12: (1S,9S)-1-amino-4-chloro-9-ethyl-5-fluoro-9-hydroxy-1,2,3,9,12,15-hexahydro-10H,13H-benzo[de]pyrano[3',4':6,7]indolizino[1,2-b]quinoline-10,13-dione and (1R,9S)-1-amino-4-chloro-9-ethyl-5-fluoro-9-hydroxy-1,2,3,9,12,15-hexahydro-10H,13H-benzo[de]pyrano[3',4': 6,7]indolizino[1,2-b]quinoline-10,13-dione (**1-10-A** and **1-10-B**)

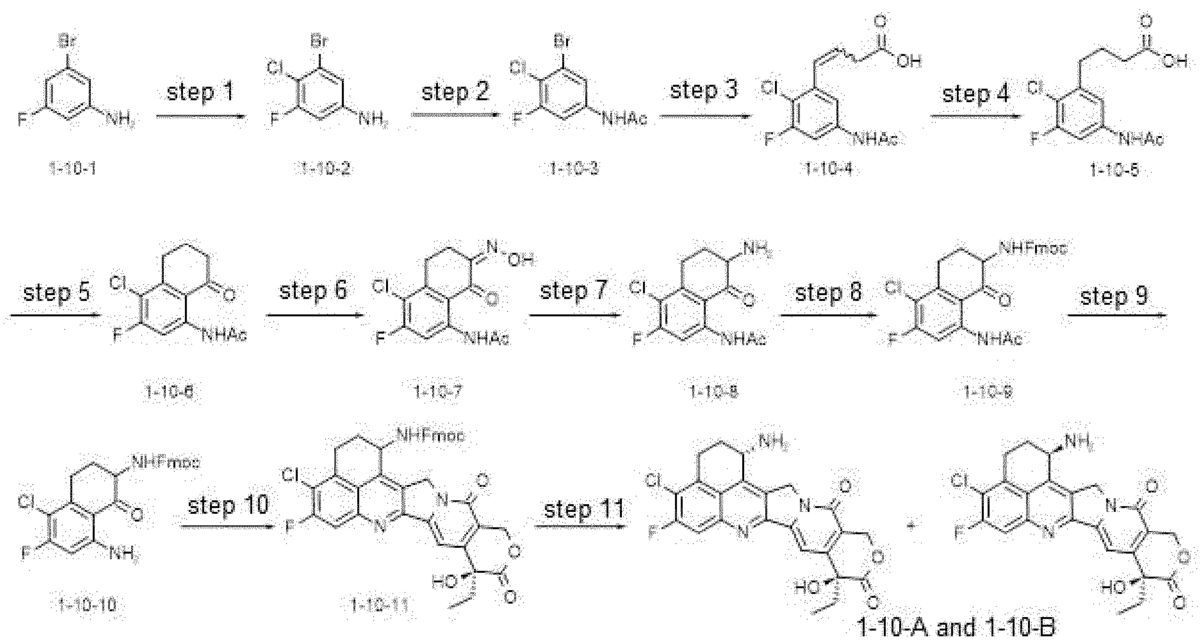


Step 1: Synthesis of 3-bromo-4-chloro-5-fluoroaniline

Compound **1-10-1** (2.00 g, 10.53 mmol) was dissolved in N,N-dimethylformamide (30 mL), then added slowly with N-chlorosuccinimide (1.69 g, 12.63 mmol), after the addition, the reaction was carried out at room temperature for 16 hours, and the reaction was monitored by liquid chromatography-mass spectrometry. The reaction solution was concentrated under reduced pressure to obtain a crude product, which was purified by flash silica gel column (ethyl

acetate:petroleum ether=0-25%) to obtain 0.95 g of the title compound.

The structure characterization data were as follows:
$^1$H NMR (400 MHz, DMSO-*d6*) δ 6.77 (dd, *J* = 2.5, 1.4 Hz, 1H), 6.51 (dd, *J* = 11.7, 2.5 Hz, 1H), 5.84 (s, 2H).

Step 2: Synthesis of N-(3-bromo-4-chloro-5-fluorophenyl)acetamide

Compound **1-10-2** (0.95 g, 4.23 mmol) was dissolved in ethyl acetate (20 mL), added with acetic anhydride (648.13 mg, 6.35 mmol) under nitrogen protection. After the addition, the temperature was raised to 50°C, the reaction was carried out for 15 hours and monitored by liquid chromatography-mass spectrometry. The reaction solution was quenched with methanol (5 mL), and directly evaporated to dryness under reduced pressure to obtain a crude product, which was purified by flash silica gel column (ethyl acetate:petroleum ether=0-40%) to obtain 1.01 g of the title compound.

The structure characterization data were as follows:
ESI-MS (m/z): 265.9[M+H]$^+$.

Step 3: Synthesis of (E)-4-(5-acetamido-2-chloro-3-fluorophenyl)but-3-enoic acid

Compound **1-10-3** and 3-butenoic acid (387.65 mg, 4.50 mmol) were dissolved in a mixed solvent of 1,4-dioxane (24 mL) and water (8 mL), and then added with N,N-diisopropylethylamine (1.45 g, 11.26 mmol), tris(o-methylphenyl)phosphine (114.21 mg, 375.24 μmol) and palladium acetate (42.12 mg, 187.62 μmol), the reaction system was subjected to nitrogen replacement three times, heated to 100°C and reacted for 16 hours under nitrogen atmosphere, and the reaction was monitored by liquid chromatography-mass spectrometry. After the reaction solution was cooled to room temperature, 1N aqueous sodium hydroxide solution (60 mL) and ethyl acetate (50 mL) were added and shaken to separate layers. the lower aqueous phase was separated and pH was adjusted to about 3 with 4 mol/L hydrochloric acid aqueous solution, then it was extracted with ethyl acetate, the organic phases were combined, washed with saturated brine, dried over anhydrous sodium sulfate, filtered, and the filtrate was evaporated to dryness under reduced pressure, to obtain 1.00 g of a crude product of the title compound.

The structure characterization data were as follows:
ESI-MS (m/z): 272.0[M+H]$^+$.

Step 4: Synthesis of 4-(5-acetamido-2-chloro-3-fluorophenyl)butanoic acid

The crude product of compound **1-10-4** (1.00 g, 3.68 mmol) was dissolved in tetrahydrofuran (15 mL), and then added with 10% palladium on carbon (0.10 g); after the addition, the reaction system was subjected to replacement with a hydrogen balloon, and the reaction was carried out under hydrogen atmosphere for 4 hours, and the reaction was monitored by liquid chromatography-mass spectrometry. The reaction solution was filtered, and the filtrate was concentrated to dryness under reduced pressure to obtain 1.00 g of a crude product of the title compound.

The structure characterization data were as follows:
ESI-MS (m/z): 274.0[M+H]$^+$.

Step 5: Synthesis of N-(4-chloro-3-fluoro-8-oxo-5,6,7,8-tetrahydronaphthalen- 1-yl)acetamide

The crude product of compound **1-10-5** (1.00 g, 3.65 mmol) was dissolved in trifluoroacetic acid (5 mL), cooled to 5°C, added slowly with trifluoroacetic anhydride (3.84 g, 18.27 mmol, 2.54 mL); after the addition, the reaction was carried out at 5°C for 2 hours, and the reaction was monitored by liquid chromatography-mass spectrometry. The reaction solution was slowly poured into water, then extracted with ethyl acetate, the organic phases were combined, washed with saturated brine, dried over anhydrous sodium sulfate, and then filtered, and the filtrate was evaporated to dryness under reduced pressure to obtain a crude product, which was purified by a flash silica gel column to obtain 0.43 g of the title compound.

The structure characterization data were as follows:
ESI-MS (m/z): 256.1[M+H]$^+$.

Step 6: Synthesis of N-(4-chloro-3-fluoro-7-(hydroxyimino)-8-oxo-5,6,7,8-tetrahydronaphthalen-1-yl)acetamide

Tetrahydrofuran (16 mL) and tert-butanol (4 mL) were added to a reaction flask, cooled to 5°C in an ice bath, added with potassium tert-butoxide (415.18 mg, 3.70 mmol), and then compound **1-10-6** (0.43 mg, 1.68 mmol) was dissolved in tetrahydrofuran (1 mL), and slowly added dropwise to the reaction solution, after 10 minutes, isoamyl nitrite (315.24 mg, 2.69 mmol) was added, and after the addition, the reaction was carried out at 5°C for 1 hour, the reaction was monitored by liquid chromatography-mass spectrometry. After the reaction solution was quenched with saturated

ammonium chloride aqueous solution, it was extracted with ethyl acetate, the organic phases were combined, washed with saturated brine, dried over anhydrous sodium sulfate, and then filtered, the filtrate was concentrated under reduced pressure to obtain 455.00 mg of a crude product of the title compound.

The structure characterization data were as follows:
ESI-MS (m/z): 285.0[M+H]$^+$.

Step 7: Synthesis of N-(7-amino-4-chloro-3-fluoro-8-oxo-5,6,7,8-tetrahydronaphthalen- 1-yl)acetamide

The crude product of compound **1-10-7** (0.40 g, 1.41 mmol) was dissolved in methanol (10 mL), then added with 3 mol/L aqueous hydrochloric acid (1 mL) and 10% palladium on carbon (40.00 mg); after the addition, the reaction system was subjected to replacement three times with a hydrogen balloon, the reaction was carried out at room temperature under hydrogen atmosphere for 1 hour, and the reaction was monitored by liquid chromatography-mass spectrometry. The reaction solution was filtered, and the filtrate was concentrated to dryness under reduced pressure to obtain 0.43 g of a crude hydrochloride of the title compound.

The structure characterization data were as follows:
ESI-MS (m/z): 271.0[M+H]$^+$.

Step 8: Synthesis of (9H-fluoren-9-yl)methyl(8-acetamido-5-chloro-6-fluoro-1-oxo-1,2,3,4-tetrahydronaphthalen-2-yl)carbamate

The crude hydrochloride of compound **1-10-8** (0.43 g, 1.19 mmol) was dissolved in 1,4-dioxane (15 mL), then added with sodium bicarbonate (400.35 mg, 4.77 mmol), water (5 mL) and 9-fluorenylmethyl-N-succinimidyl carbonate (481.81 mg, 1.43 mmol), after the addition, the reaction was carried out under stirring at room temperature for 2 hours and monitored by liquid chromatography-mass spectrometry. The reaction solution was poured into water, then extracted with ethyl acetate, the organic phases were combined, washed with saturated brine, dried over anhydrous sodium sulfate, filtered, and the filtrate was concentrated under reduced pressure to obtain a crude product. The crude product was purified by a C18 reverse-phase column (acetonitrile: 0.05% formic acid in water = 20% to 100%) to obtain 301.00 mg of the title compound.

The structure characterization data were as follows:
ESI-MS (m/z): 493.2[M+H]$^+$.

Step 9: Synthesis of (9H-fluoren-9-yl)methyl(8-amino-5-chloro-6-fluoro-1-oxo-1,2,3,4-tetrahydronaphthalen-2-yl)carbamate

Compound **1-10-9** (300.00 mg, 608.61 μmol) was dissolved in dioxane (5 mL), added with 12 mol/L concentrated hydrochloric acid (1 mL); after the addition, the temperature was raised to 60°C, the reaction was carried out for 2 hours and monitored by liquid chromatography-mass spectrometry. The reaction solution was poured into water, then extracted with ethyl acetate, the organic phases were combined, washed with saturated brine, dried over anhydrous sodium sulfate, filtered, and the filtrate was concentrated under reduced pressure to obtain a crude product. The crude product was purified by flash silica gel column (ethyl acetate:petroleum ether=0-50%) to obtain 198.00 mg of the title compound.

The structure characterization data were as follows:
ESI-MS (m/z): 451.1[M+H]$^+$.

Step 10: Synthesis of (9H-fluoren-9-yl)methyl ((9S)-4-chloro-9-ethyl-5-fluoro-9-hydroxy-10,13-dioxo-2,3,9,10,13,15-hexahydro-1H,12H-benzo[de]pyrano[3',4:6,7]indolizino[1,2-b]quinolin-1-yl)carbamate

(S)-4-Ethyl-4-hydroxy-7,8-dihydro-1H-pyrano[3,4-f]indolezine-3,6,10(4H)-trione (138.72 mg, 526.96 μmol) and compound **1-10-10** (198.00 mg, 439.13 μmol) were added in toluene (10 mL), and then added with p-toluenesulfonic acid (75.53 mg, 439.13 μmol), after addition, the temperature was raised to 140°C, and the reaction was carried out for 4 hours, the reaction solution was evaporated to dryness under reduced pressure to obtain a crude product, which was purified by flash silica gel column (methanol:dichloromethane=0-5%) to obtain 256.00 mg of the title compound.

The structure characterization data were as follows:
ESI-MS (m/z): 678.1[M+H]$^+$.

Step 11: Synthesis of (1S,9S)-1-amino-4-chloro-9-ethyl-5-fluoro-9-hydroxy-1,2,3,9,12,15-hexahydro-10H,13H-benzo[de]pyrano[3',4':6,7]indolizino[1,2-b]quinoline-10,13-dione and (1R,9S)-1-amino-4-chloro-9-ethyl-5-fluoro-9-hydroxy-1,2,3,9,12,15-hexahydro-10H,13H-benzo[de]pyrano[3',4':6,7]indolizino[1,2-b]quinoline-10,13-dione

Compound **1-10-11** (201.18 mg, 296.67 μmol) was dissolved in N,N-dimethylformamide (4 mL), then added with diethylamine (108.49 mg, 1.48 mmol), after the addition, the reaction was carried out at room temperature for 0.5 hours and monitored by liquid chromatography-mass spectrometry. After diethylamine was evaporated from the reaction solution under reduced pressure, the pH was adjusted to 2-3 with 1 mol/L hydrochloric acid aqueous solution, and the reaction solution was directly purified by preparative high performance liquid chromatography to obtain the title compound **1-10-A** (44.00 mg) and **1-10-B** (43.00 mg).

Chromatographic column: SunFire Prep C18 OBD 19 mm×150 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0 | 10 | 90 | 28 |
| 3 | 10 | 90 | 28 |
| 18 | 70 | 30 | 28 |

The structure characterization data of **1-10-A** (6 min LCMS, the earlier peak with retention time of 1.276 min) were as follows:

$^1$H NMR (400 MHz, DMSO-*d*6) δ 8.00 (d, *J* = 10.3 Hz, 1H), 7.33 (s, 1H), 6.54 (s, 1H), 5.62 (d, *J* = 19.3 Hz, 1H), 5.44 (s, 2H), 5.38 (d, *J* = 19.3 Hz, 1H), 4.43-4.38 (m, 1H), 3.28-3.10 (m, 2H), 2.22-2.12 (m, 1H), 2.12-2.02 (m, 1H), 1.93-1.80 (m, 2H), 0.87 (t, *J* = 7.3 Hz, 3H).

ESI-MS (m/z): 456.1$[M+H]^+$.

The structure characterization data of **1-10-B** (6 min LCMS, the later peak with retention time of 1.300 min) were as follows:

$^1$H NMR (400 MHz, DMSO-*d*6) δ7.98 (d, *J* = 10.3 Hz, 1H), 7.32 (s, 1H), 5.61 (d, *J* = 19.4 Hz, 1H), 5.44 (s, 2H), 5.32 (d, *J* = 19.4 Hz, 1H), 4.44-4.36 (m, 1H), 3.33-3.25 (m, 1H), 3.22-3.11 (m, 1H), 2.23- 2.13 (m, 1H), 2.11- 2.03 (m, 1H), 1.96-1.82 (m, 2H), 0.89 (t, *J* = 7.3 Hz, 3H).

ESI-MS (m/z): 456.1$[M+H]^+$.

6 min LCMS conditions:

Chromatographic column: Waters SunFire C18 OBD 4.6 mm×50 mm×5.0 μm

Mobile phase A: 0.05% acetonitrile; Mobile phase B: water (0.05% formic acid)

| Time [min] | Mobile phase A [%] | Mobile phase B[%] | Flow rate [mL/min] |
|---|---|---|---|
| 0 | 90 | 10 | 2 |
| 4.2 | 10 | 90 | 2 |
| 5.7 | 10 | 90 | 2 |
| 5.71 | 90 | 10 | 2 |
| 6.70 | 90 | 10 | 2 |

Example 13: Synthesis of (2S,3S,4S,5R,6S)-6-(4-(((((1S,9S)-4-chloro-9-ethyl-9-hydroxy-5-fluoro-10,13-dioxo-2,3,9,10,13,15-octahydroxybenzo [de]pyrano [3',4':6,7]indolizino [1,2-b] quinolin-1-yl)carbamoyl)oxy)methyl)-2-(2-(2-(6-(2-(2-(methylsulfonyl)pyrimidin-5-yl)hex-5-acetamido)ethoxy)acetamido)phenoxy)-3,4,5-trihydroxytetrahydro-2H-pyran-2-carboxylic acid **(A-12)**

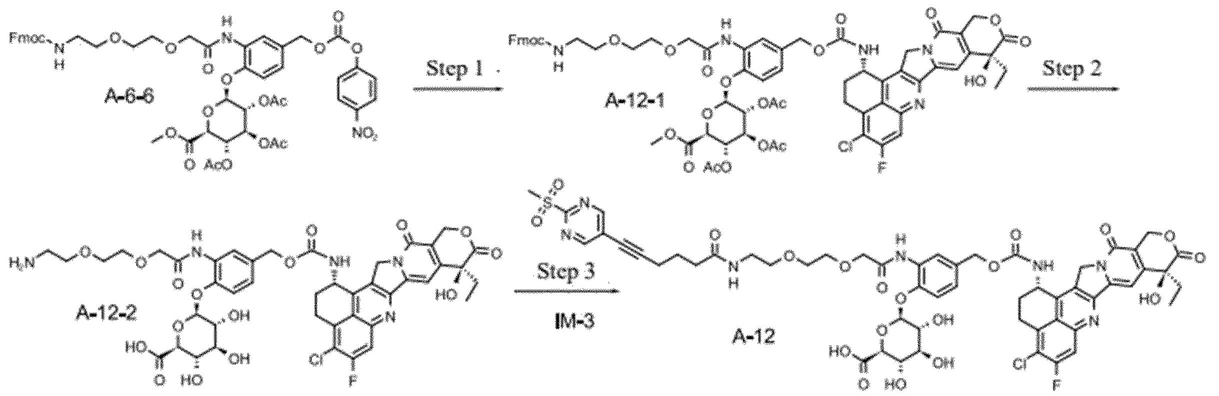


A-6-6 A-12-1 A-12-2 A-12

Step 1: Synthesis of (2S,3R,4S,SS,6S)-2-(2-(1-(9H-fluoren-9-yl)-3-oxo-2,7,10-trioxa-4-azadodecan-12-amido)-4-(((((1S,9S)-4-chloro-9-ethyl-9-hydroxy-5-fluoro-10,13-dioxo-2,3,9,10, 13,15-octahydrobenzo[de]pyrano[3',4':6,7]indolizino[1,2-b]quinolin-1-yl)carbamoyl)oxy)methyl)phenoxy)-6-(methoxycarbonyl)tetrahydro-2H-pyran-3,4,5-triyl triacetate

Starting materials **A-6-6** (56.6 mg, 0.057 mmol), compound **1-11** (30.0 mg, 0.048 mmol) and HOBt (12.9 mg, 0.095 mmol) were dissolved in DMF (1 mL), and added dropwise with diisopropylethylamine (18.5 mg, 0.143 mmol), reacted under stirring at 25°C for 4 hours, and the reaction solution was purified by preparative high performance liquid chromatography (with conditions as follows) to obtain 29.0 mg of the title compound.
ESI-MS (m/z): 1304.3[M+1]$^+$.

Chromatographic column: Waters XBridge Prep C18OBD (5 μm*19mm*150 mm)

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 40 | 60 | 30 |
| 2.00 | 400 | 60 | 30 |
| 18.00 | 90 | 10 | 30 |

Step 2: Synthesis of (2S,3S,4S,5R,6S)-6-(2-(2-(2-aminoethoxy)ethoxy)acetamido)-4-(((((1S,9S)-4-chloro-9-ethyl-9-hydroxy-5-fluoro-10,13-dioxo-2,3,9,10,13,15-octahydroxybenzo [de]pyrano[3',4':6,7]indolizino[1,2-b]quinolin-1-yl)carbamoyl)oxy)phenoxy)-3,4,5-trihydroxytetrahydro-2H-pyran-2-carboxylic acid

Compound **A-12-1** (29.0 mg, 0.022 mmol) was dissolved in methanol (1 mL)/tetrahydrofuran (1 ml), added dropwise with lithium hydroxide aqueous solution (7.7 mg, 0.184 mmol, dissolved in 0.5 mL of water), reacted at 25°C under stirring for 2 hours; the reaction system was added with water (5 ml) and adjusted to pH=2-3 with 3N hydrochloric acid, extracted with ethyl acetate (5 mL) to remove impurities, the aqueous phase was freeze-dried to obtain a crude product, and purified by preparative high performance liquid chromatography (with conditions as follows) to obtain 5.0 mg of the title compound.
ESI-MS (m/z): 942.2[M+1]$^+$.

Chromatographic column: Waters XBridge Prep C18OBD (5 μm*19mm*150 mm)

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 20 | 80 | 24 |
| 2.00 | 20 | 80 | 24 |
| 18.00 | 80 | 20 | 24 |

Step 3: Synthesis of (2S,3S,4S,5R,6S)-6-(4-(((((1S,9S)-4-chloro-9-ethyl-9-hydroxy-5-fluoro-10,13-dioxo-2,3,9,10,13,15-octahydroxybenzo[de]pyrano[3',4':6,7]indolizino[1,2-b]quinolin-1-yl)carbamoyl)oxy)methyl)-2-(2-(2-(6-(2-(2-(methylsulfonyl)pyrimidin-5-yl)hex-5-acetamido)ethoxy)acetamido)phenoxy)-3,4,5-trihydroxytetrahydro-2H-pyran-2-carboxylic acid

Compound **A-12-2** (5.0 mg, 0.0053 mmol) and 6-(2-methylsulfonylpyrimidin-5-yl)-hex-5-ynoic acid 2,5-dioxopyrrolidin-1-yl ester (**IM-3**, 2.3 mg, 0.0064 mmol) were dissolved in DMF (0.5 mL), added dropwise with diisopropylethylamine (2.0 mg, 0.016 mmol), and reacted at 25°C for 4 hours, and the reaction solution was directly purified by the preparative high performance liquid chromatography (with conditions as below) to obtain 0.90 mg of the title compound. ESI-MS (m/z): 1192.0[M+1]$^+$.

Chromatographic column: Waters XBridge Prep C18OBD (5 μm*19mm*150 mm)

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 30 | 70 | 24 |
| 2.00 | 30 | 70 | 24 |
| 18.00 | 80 | 20 | 24 |

Example 14: Synthesis of N-((S)-10-benzyl-1-((1S,9S)-4-chloro-9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-1-yl)amino)-1,6,9,12,15-pentaoxo-3-oxa-5,8,11,14-tetraazahexadecan-16-yl)-6-(2-(methylsulfonyl)pyrimidin-5-yl)hexyl-5-amide and N-((S)-10-benzyl-1-((1R,9S)-4-chloro-9-ethyl-5-fluoro-9-hydroxy-10,13-dioxo-2,3,9,10,13,15-hexahydro-1H,12H-benzo[de]pyrano[3',4':6,7]indolizino[1,2-b]quinolin-1-yl)amino)-1,6,9,12,15-pentoxo-3-oxa-5,8,11,14-tetraazahexadecan-16-yl)-6-(2-(methylsulfonyl)pyrimidin-5-yl)hexyl-5-amide (**A-14/15-A** and **A-14/15-B**)

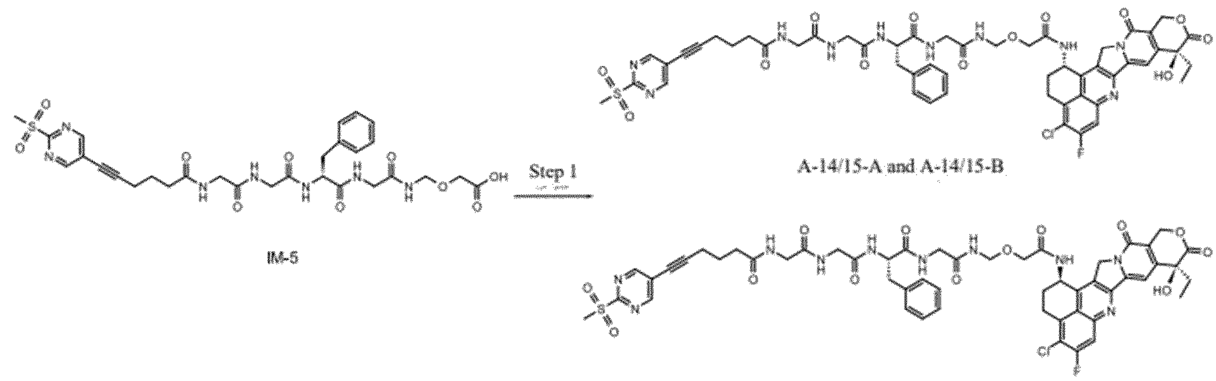


Compound **1-10-A** (36.00 mg, 79.70 μmol) in single configuration and compound **IM-5** (64.43 mg, 95.64 μmol)

were dissolved in N,N-dimethylformamide (2 mL), and then added with 4-(4,6-dimethoxytriazin-2-yl)-4-methylmorpholine hydrochloride (46.98 mg, 159.40 μmol) and triethylamine (24.19 mg, 239.10 μmol), after the addition, the reaction was carried out at room temperature for 1 hour and monitored by liquid chromatography-mass spectrometry. The reaction solution was purified by high performance liquid chromatography to obtain the title compound **A-14/15-A** (51.00 mg) in single configuration.

Chromatographic column: SunFire Prep C18 OBD 19 mm×150 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0 | 30 | 70 | 28 |
| 3 | 30 | 70 | 28 |
| 18 | 90 | 10 | 28 |

The structure characterization data were as follows:
ESI-MS (m/z): 1111.0[M+H]$^+$.

Compound **1-10-B** (36.00 mg, 79.70 μmol) in single configuration and compound **IM-5** (64.43 mg, 95.64 μmol) were dissolved in N,N-dimethylformamide (2 mL), and then added with 4-(4,6-dimethoxytriazin-2-yl)-4-methylmorpholine hydrochloride (46.98 mg, 159.40 μmol) and triethylamine (24.19 mg, 239.10 μmol), after the addition, the reaction was carried out at room temperature for 1 hour and monitored by liquid chromatography-mass spectrometry. The reaction solution was purified by high performance liquid chromatography to obtain the title compound **A-14/15-B** (52.00 mg) in single configuration.

Chromatographic column: SunFire Prep C18 OBD 19 mm×150 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0 | 30 | 70 | 28 |
| 3 | 30 | 70 | 28 |
| 18 | 90 | 10 | 28 |

The structure characterization data were as follows:
ESI-MS (m/z): 1111.0[M+H]$^+$.

Example 15: N-((1S,9S)-4-chloro-9-ethyl-5-fluoro-9-hydroxy-10,13-dioxo-2,3,9,10,13,15-hexahydro-1 H,12H-benzo [de]pyrano [3',4:6,7]indolizino [1,2-b]quinolin-1-yl)-2-hydroxyacetamide and N-((1R,9S)-4-chloro-9-ethyl-5-fluoro-9-hydroxy-10,13-dioxo-2,3,9,10,13,15-hexahydro- 1H,12H-benzo[de]pyrano[3',4:6,7]indolizino[1,2-b]quinolin-1-yl)-2-hydroxyacetamide (**1-13-A** and **1-13-B**)

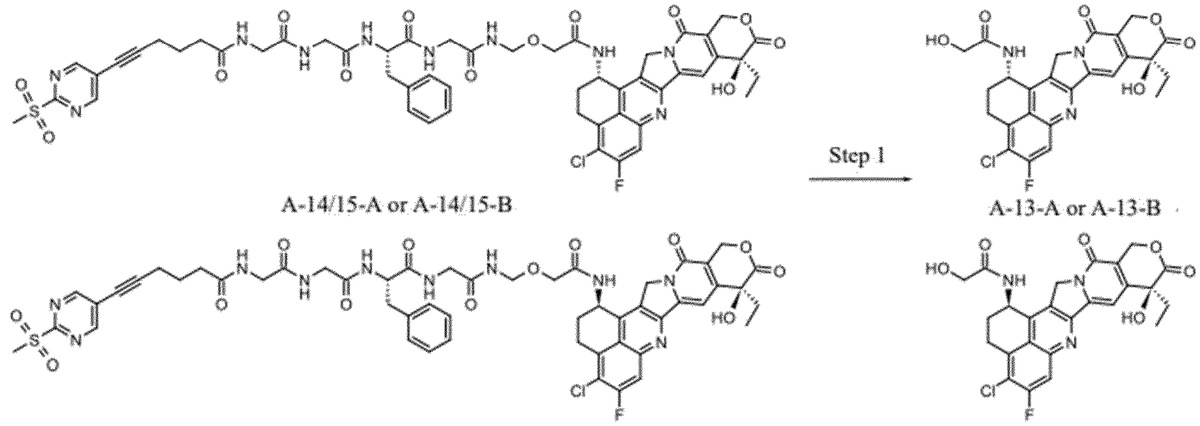


A-14/15-A or A-14/15-B

A-13-A or A-13-B

Compound **A-14/15-A** (40.00 mg, 35.99 μmol) was dissolved in a mixed solvent of dichloromethane (2 mL) and methanol (1 mL), then added with hydrogen chloride in ethyl acetate solution (4 mol/L, 1 mL), after the addition, the reaction was carried out at room temperature for 0.5 hours and monitored by liquid chromatography-mass spectrometry. The reaction solution was concentrated under reduced pressure to obtain a crude product, which was purified by high performance liquid chromatography to obtain the compound **1-13-A** (4.75 mg) in single configuration.

Chromatographic column: SunFire Prep C18 OBD 19 mm×150 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0 | 15 | 85 | 28 |
| 3 | 15 | 85 | 28 |
| 18 | 90 | 10 | 28 |

The structure characterization data were as follows:

$^1$H NMR (400 MHz, DMSO-d6) δ 8.50 (d, *J* = 8.9 Hz, 1H), 8.05 (d, *J* = 10.3 Hz, 1H), 7.33 (s, 1H), 6.55 (s, 1H), 5.67-5.60 (m, 1H), 5.49 (t, *J* = 5.8 Hz, 1H), 5.43 (s, 2H), 5.21 (s, 2H), 3.96 (d, *J* = 5.8 Hz, 2H), 3.32-3.22 (m, 2H), 2.28-2.15 (m, 2H), 1.93-1.80 (m, 2H), 0.87 (t, *J* = 7.3 Hz, 3H).

ESI-MS (m/z): 514.0[M+H]$^+$.

Compound **A-14/15-B** (40.00 mg, 35.99 μmol) was dissolved in a mixed solvent of dichloromethane (2 mL) and methanol (1 mL), then added with hydrogen chloride in ethyl acetate solution (4 mol/L, 1 mL), after the addition, the reaction was carried out at room temperature for 0.5 hours and monitored by liquid chromatography-mass spectrometry. The reaction solution was concentrated under reduced pressure to obtain a crude product, which was purified by high performance liquid chromatography to obtain the compound **1-13-B** (8.24 mg) in single configuration.

Chromatographic column: SunFire Prep C18 OBD 19 mm×150 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0 | 15 | 85 | 28 |
| 3 | 15 | 85 | 28 |

(continued)

| Time [min] | Mobile phase A [%] | Mobile phase B[%] | Flow rate [mL/min] |
|---|---|---|---|
| 18 | 90 | 10 | 28 |

The structure characterization data were as follows:

$^1$H NMR (400 MHz, DMSO-*d6*) δ 8.52 (d, *J* = 9.0 Hz, 1H), 8.05 (d, *J* = 10.3 Hz, 1H), 7.34 (s, 1H), 6.55 (s, 1H), 5.68-5.58 (m, 1H), 5.53 (t, *J* = 5.8 Hz, 1H), 5.43 (d, *J* = 2.9 Hz, 2H), 5.20 (d, *J* = 7.3 Hz, 2H), 3.97 (d, *J* = 5.7 Hz, 2H), 3.31-3.21 (m, 2H), 2.26-2.15 (m, 2H), 1.92-1.82 (m, 2H), 0.87 (t, *J* = 7.3 Hz, 3H).

ESI-MS (m/z): 514.0[M+H]$^+$.

Example 16: 4-((S)-2-(4-Aminobutyl)-35-(4-((6-(2-(methylsulfonyl)pyrimidin-5-yl) hexadec-5-ynamido)methyl)-1H-1,2,3-triazol-1-yl)-4,8-dioxo-6,12,15,18,21,24,27,30,33-nonaoxa-3,9-diazapentatriacontanamido)benzyl ((S)-4-ethyl-11-(2-(isopropylamino)ethyl)-3,14-dioxo-3, 4,12,14-tetrahydro-1H-pyrano[3',4':6,7]indolizino[1,2-b]quinolin-4-yl)carbonate **(A-26)**

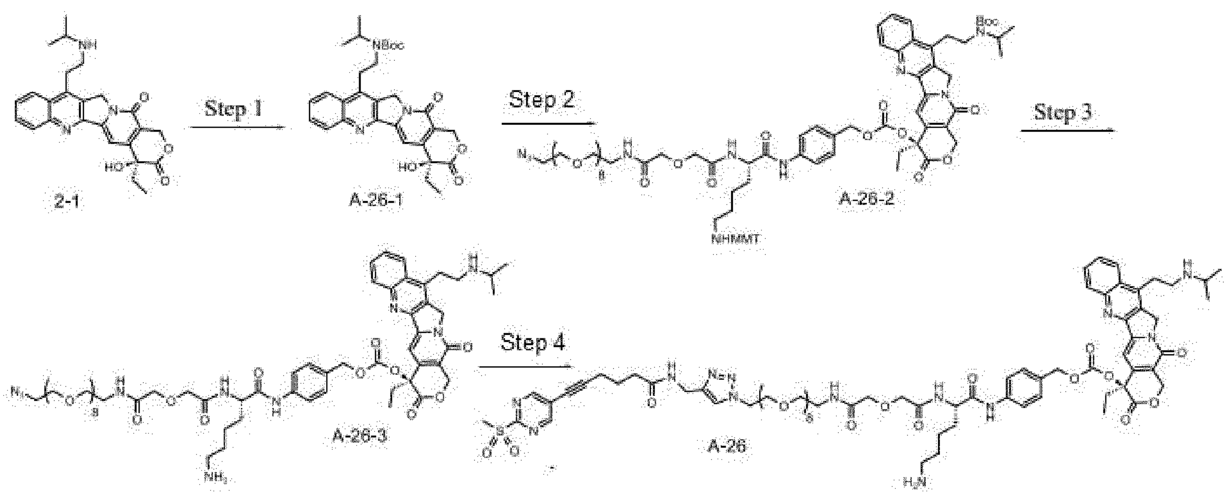


Step 1: Synthesis of tert-butyl (S)-(2-(4-ethyl-4-hydroxy-3,14-dioxo-3,4,12,14-tetrahydro-1H-pyrano[3',4':6,7]indolizino[1,2-b]quinoline-11-yl)ethyl)(isopropyl)carbamate

At 20°C, compound **2-1** (500.00 mg, 1.15 mmol) was dissolved in anhydrous dichloromethane (15 mL), added with tert-butyl tert-butyloxycarbonyl carbonate (276.90 mg, 1.27 mmol) and DIPEA (447.21 mg, 3.46 mmol), and reacted under stirring at 20°C for 16 h, the reaction was monitored by liquid chromatography-mass spectrometry, and the reaction solution was purified by silica gel column chromatography (mobile phase: dichloromethane/methanol=50/1) to obtain 400 mg of the title compound.

The structure characterization data were as follows:
ESI-MS (m/z): 534.0 [M+H]$^+$.

Step 2: Synthesis of tert-butyl (2-((S)-4-(((4-((S)-35-azido-2-(4-((4-methoxyphenyl)benzhydryl) amino)butyl)-4,8-dioxo-6,12,15,18,21,24,27,30,33-nonaoxa-3,9-diazapentatriacontanamido)benzyl)oxy)carbonyl)-4-ethyl-3,14-dioxo-3,4,12,14-tetrahydro-1H-pyrano[3',4':6,7]indolizino[1,2-b]quinoline-11-yl)ethyl)(isopropyl)carbamate

At 0°C, compound **A-26-1** (30.00 mg, 0.056 mmol) was dissolved in anhydrous dichloromethane (1 mL), added with DMAP (54.95 mg, 0.450 mmol) under nitrogen protection, then added dropwise with a solution of triphosgene (16.68 mg, 0.056 mmol) in dichloromethane (1 mL), reacted at 0°C for 30 min, the reaction solution was subjected to nitrogen replacement under reduced pressure, and added dropwise with a solution of (S)-2-(32-azido-5-oxo-3,9,12,15,18,21,24,27,30-nonaoxa-6-azapentatriacontanamido)-N-(4-(hydroxymethyl)phenyl)-6-(((4-methoxyphenyl)benzhydryl)amino)caproamide (117.39 mg, 0.111 mmol) in anhydrous dichloromethane (20 mL), the reaction solution was stirred at 20°C for 60 min, the reaction was monitored by liquid chromatography-mass spectrometry; and the reaction solution was directly used in the next step after concentration.

The structure characterization data were as follows:
ESI-MS (m/z): 1620.2$[M+H]^+$.

Step 3: Synthesis of 4-((S)-2-(4-aminobutyl)-35-azido-4,8-dioxo-6,12,15,18,21,24,27,30, 33-nonaoxa-3,9-diazapentatriacontanamido)benzyl ((S)-4-ethyl-11-(2-(isopropylamino)ethyl)-3, 14-dioxo-3,4,12,14-tetrahydro-1H-pyrano[3',4':6,7]indolizino[1,2-b]quinoline-4-yl)carbonate **(B-194-03)**

At 25°C, compound **A-26-2** (80.00 mg, 0.049 mmol) was dissolved in acetonitrile (1.0 mL), added with trifluoroacetic acid (0.4 mL), and reacted at 20°C for 1 h; the reaction was monitored by liquid chromatography-mass spectrometry; the reaction solution was purified by preparative high-performance liquid chromatography (with conditions as follows), and the fractions were freeze-dried to obtain 41.0 mg of a trifluoroacetic acid salt of the title compound.

Chromatographic column: SunFire Prep C18 OBD 19 mm×150 mm×5.0 μm

Mobile phase A: acetonitrile; Mobile phase B: water (0.05% trifluoroacetic acid)

| Time [min] | Mobile phase A [%] | Mobile phase B[%] | Flow rate [mL/min] |
|---|---|---|---|
| 0.00 | 10 | 90 | 28 |
| 2.00 | 10 | 90 | 28 |
| 18.00 | 90 | 10 | 28 |

The structure characterization data were as follows:
ESI-MS (m/z): 1248.2$[M+H]^+$.

Step 4: Synthesis of 4-((S)-2-(4-aminobutyl)-35-(4-((6-(2-(methylsulfonyl)pyrimidin-5-yl) hexadecan-5-ynamido)methyl)-1H-1,2,3-triazol-1-yl)-4,8-dioxo-6,12,15,18,21,24,27,30,33-nonaoxa-3,9-diazapentatriacontanamido)benzyl ((S)-4-ethyl-11-(2-(isopropylamino)ethyl)-3,14-dioxo-3,4,12,14-tetrahydro-1H-pyrano[3',4':6,7]indolizino[1,2-b]quinolin-4-yl)carbonate

At 25°C, compound **A-26-3** (40.00 mg, 0.032 mmol) and 6-(2-(methylsulfonyl)pyrimidin- 5-yl)-N-(prop-2-yn-1-yl)hex-5-ynamide (**IM-2,** 14.69 mg, 0.048 mmol) were dissolved in a mixed solution of DMSO (0.5 mL) and $H_2O$ (0.1 mL), added with cuprous bromide (9.20 mg, 0.064 mmol), the reaction solution was reacted under the protection of nitrogen at 20°C for 2 h; the reaction was monitored by high performance liquid chromatography-mass spectrometry; the reaction solution was purified by preparative high performance liquid chromatography (with conditions as follows), and the fractions were freeze-dried to obtain 20.0 mg of a trifluoroacetate of the title compound.

Chromatographic column: SunFire Prep C18 OBD 19 mm×150 mm×5.0 μm

Mobile phase A: acetonitrile; Mobile phase B: water (0.05% trifluoroacetic acid)

| Time [min] | Mobile phase A [%] | Mobile phase B[%] | Flow rate [mL/min] |
|---|---|---|---|
| 0.00 | 25 | 75 | 28 |
| 2.00 | 25 | 75 | 28 |
| 18.00 | 90 | 10 | 28 |

The structure characterization data were as follows:
ESI-MS (m/z): 1552.6 $[M+H]^+$.

Example 17: 4-((S)-2-(4-Aminobutyl)-35-(4-((6-(2-(methylsulfonyl)pyrimidin-5-yl)hex-5-acetamido)methyl)-1H-1,2,3-triazol-1-yl)-4,8-dioxo-6,12,15,18,21,24,27,30,33-nonaoxo-3,9-diazapentatriacontanamido)benzyl ((1S,9S)-1-(dimethylamino)-9-ethyl-5-fluoro-4-methyl-10, 13-dioxo-2,3,9,10,13,15-hexahydro-1H,12H-benzo [de]pyrano [3',4':6,7]indolizino [1,2-b]quinolin-9-yl)carbonate **(A-28)**

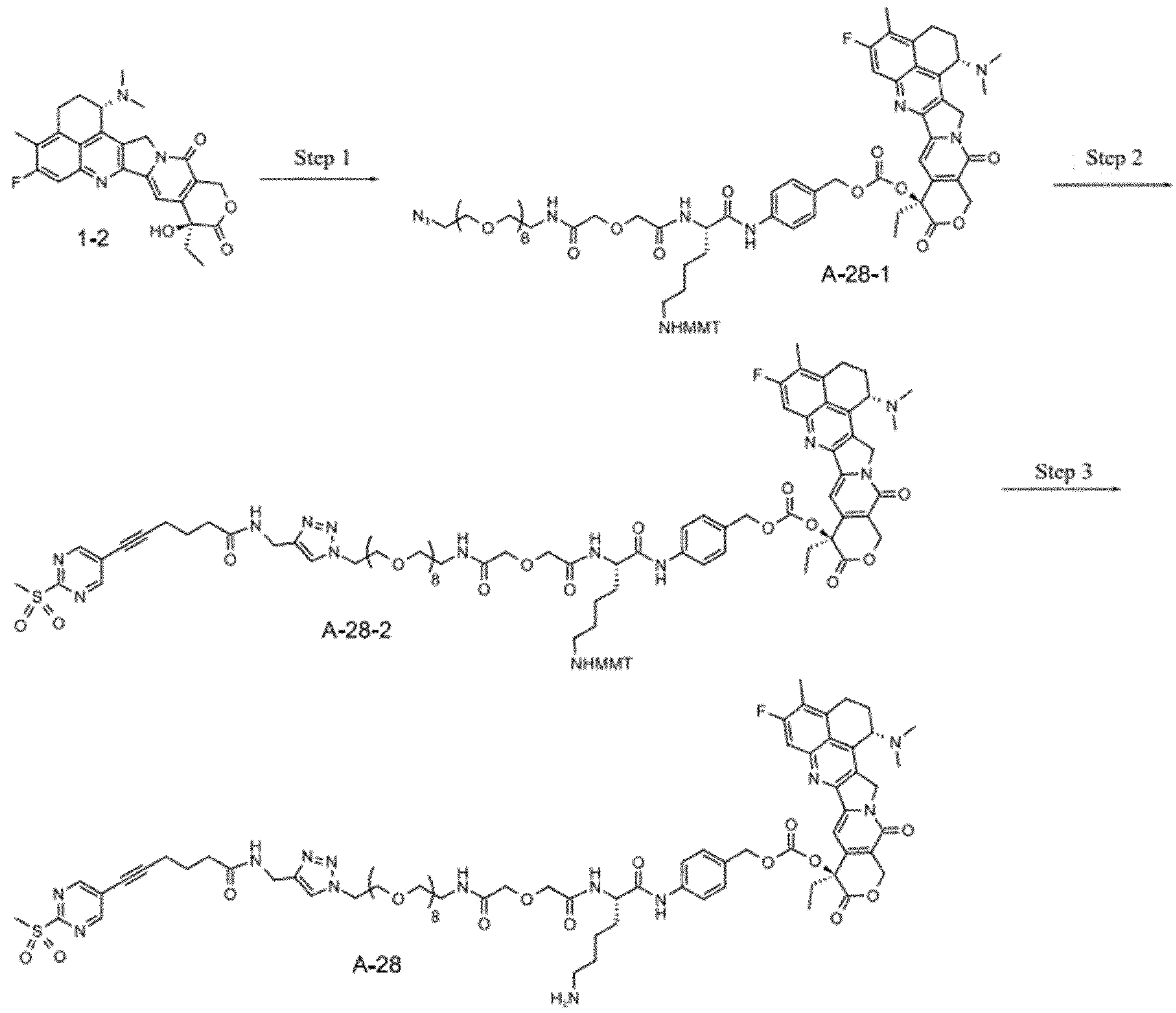


1-2

A-28-1

A-28-2

A-28

Step 1: Synthesis of 4-((S)-35-azido-2-(4-((4-methoxyphenyl) diphenylmethyl)amino)butyl)-4,8-dioxo-6,12,15,18,21,24,27,30,33-nonaoxa-3,9-diazapentatriacontanamido)benzyl ((1S,9S)-1-(dimethylamino)-9-ethyl-5-fluoro-4-methyl-10,13-dioxo-2,3,9,10,13,15-hexahydro-lH,12H-benzo[de]pyrano[3',4':6,7]indolizino[1,2-b]quinolin-9-yl)carbonate

At 0°C, compound **1-2** (20.00 mg, 0.040 mmol) was dissolved in anhydrous dichloromethane (1 mL), added with DMAP (39.10 mg, 0.320 mmol) under nitrogen protection, and added dropwise with a solution of triphosgene (11.87 mg, 0.040 mmol) in dichloromethane (1 mL), the reaction solution was reacted at 0°C for 30 min, the reaction solution was subjected to nitrogen replacement under reduced pressure, and added dropwise with a solution of (S)-2-(32-azido-5-oxo-3,9,12,15,18,21,24,27, 30-nonaoxa-6-azapentatriacontanamido)-N-(4-(hydroxymethyl)phenyl)-6-(((4-methoxyphenyl) benzhydryl)amino)hexanamide (84.66 mg, 0.080 mmol) in anhydrous dichloromethane (20 mL), the reaction solution was stirred at 20°C for 1 h, and the reaction was monitored by liquid chromatography-mass spectrometry; the reaction solution was purified by preparative high-performance liquid chromatography (with conditions as follows), and the fractions were freeze-dried to obtain 40.0 mg of the title compound.

Chromatographic column: SunFire Prep C18 OBD 19 mm×150 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 20 | 80 | 28 |
| 2.00 | 20 | 80 | 28 |
| 18.00 | 90 | 10 | 28 |

The structure characterization data were as follows:
ESI-MS (m/z): 1550.2$[M+H]^+$.

Step 2: Synthesis of (1S,9S)-1-(dimethylamino)-9-ethyl-5-fluoro-4-methyl-10,13-dioxo-2,3,9,10,13,15-hexahydro-1H,12H-benzo[de]pyrano[3',4':6,7]indolizino[1,2-b]quinolin-9-yl(4-((S)-2-(4-((4-methoxyphenyl) diphenylmethyl)amino)butyl)-35-(4-((6-(2-(methylsulfonyl)pyrimidin-5-yl)hexadec-5-ynamido)methyl)-1H-1,2,3-triazol-1-yl)-4,8-dioxo-6,12,15,18,21,24,27,30,33- nonaoxa-3,9-diazapentatriacontanamido)benzyl)carbonate

At 25°C, compound **A-28-1** (30.00 mg, 0.019 mmol) and 6-(2-(methylsulfonyl)pyrimidin- 5-yl)-N-(prop-2-yn-1-yl)hex-5-ynamide (**IM-2**, 8.87 mg, 0.029 mmol) were added in a mixture solution of DMSO (0.5 mL) and $H_2O$ (0.1 mL), and added with cuprous bromide (5.55 mg, 0.039 mmol), and the reaction solution was reacted under the protection of nitrogen at 20°C for 1 h; the reaction was monitored by liquid chromatography-mass spectrometry; the reaction solution was purified by preparative high-performance liquid chromatography (with conditions as follows), and the fractions were freeze-dried to obtain 25.0 mg of the title compound.

Chromatographic column: SunFire Prep C18 OBD 19 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 15 | 85 | 28 |
| 2.00 | 15 | 85 | 28 |
| 18.00 | 70 | 30 | 28 |

The structure characterization data were as follows:
ESI-MS (m/z): 1855.2$[M+H]^+$.

Step 3: Synthesis of 4-((S)-2-(4-aminobutyl)-35-(4-((6-(2-(methylsulfonyl)pyrimidin-5-yl) hex-5-ynamido)methyl)-1H-1,2,3-triazol-1-yl)-4, 8-dioxo-6,12,15,18,21,24,27,30,33-nonaoxa-3,9-diazapentatriacontanamido)benzyl (1S,9S)-1-(dimethylamino)-9-ethyl-5-fluoro-4-methyl-10,13- dioxo-2,3,9,10,13,15-hexahydro-1H,12H-benzo[de]pyrano[3',4':6,7]indolizino[1,2-b]quinoline-9-yl)carbonate

At 25°C, compound **A-28-2** (15.00 mg, 0.017 mmol) was dissolved in acetonitrile (0.5 mL), added with trifluoroacetic acid (0.2 mL), and the reaction solution was reacted at 20°C for 0.5 h. The reaction was monitored by liquid chromatography-mass spectrometry; the reaction solution was purified by preparative high-performance liquid chromatography (with conditions as follows), and the fractions were freeze-dried to obtain 9.0 mg of a trifluoroacetate of the title compound.

Chromatographic column: SunFire Prep C18 OBD 19 mm×5.0 μm

Mobile phase A: acetonitrile; Mobile phase B: water (0.05% trifluoroacetic acid)

| Time [min] | Mobile phase A [%] | Mobile phase B[%] | Flow rate [mL/min] |
|---|---|---|---|
| 0.00 | 15 | 85 | 28 |
| 2.00 | 15 | 85 | 28 |
| 18.00 | 80 | 20 | 28 |

The structure characterization data were as follows:
ESI-MS (m/z): 1583.1[M+H]$^+$.

Example 18: 4-((S)-2-(4-aminobutyl)-35-(4-((6-(2-(methylsulfonyl)pyrimidin-5-yl)hex- 5-ynamido)methyl)-1H-1,2,3-triazol-1-yl)-4,8-dioxo-6,12,15,18,21,24,27,30,33-nonaoxo-3,9-diazapentatriacontanamido)benzyl ((1S,9R)-9-ethyl-5-fluoro-1-(2-hydroxyacetamido)-4-methyl-10,13-dioxo-2,3,9,10,13,15-hexahydro-1H,12H-benzo [de]pyrano [3',4':6,7]indolizino [1,2-b]quinolin-9-yl)carbonate **(A-29)**

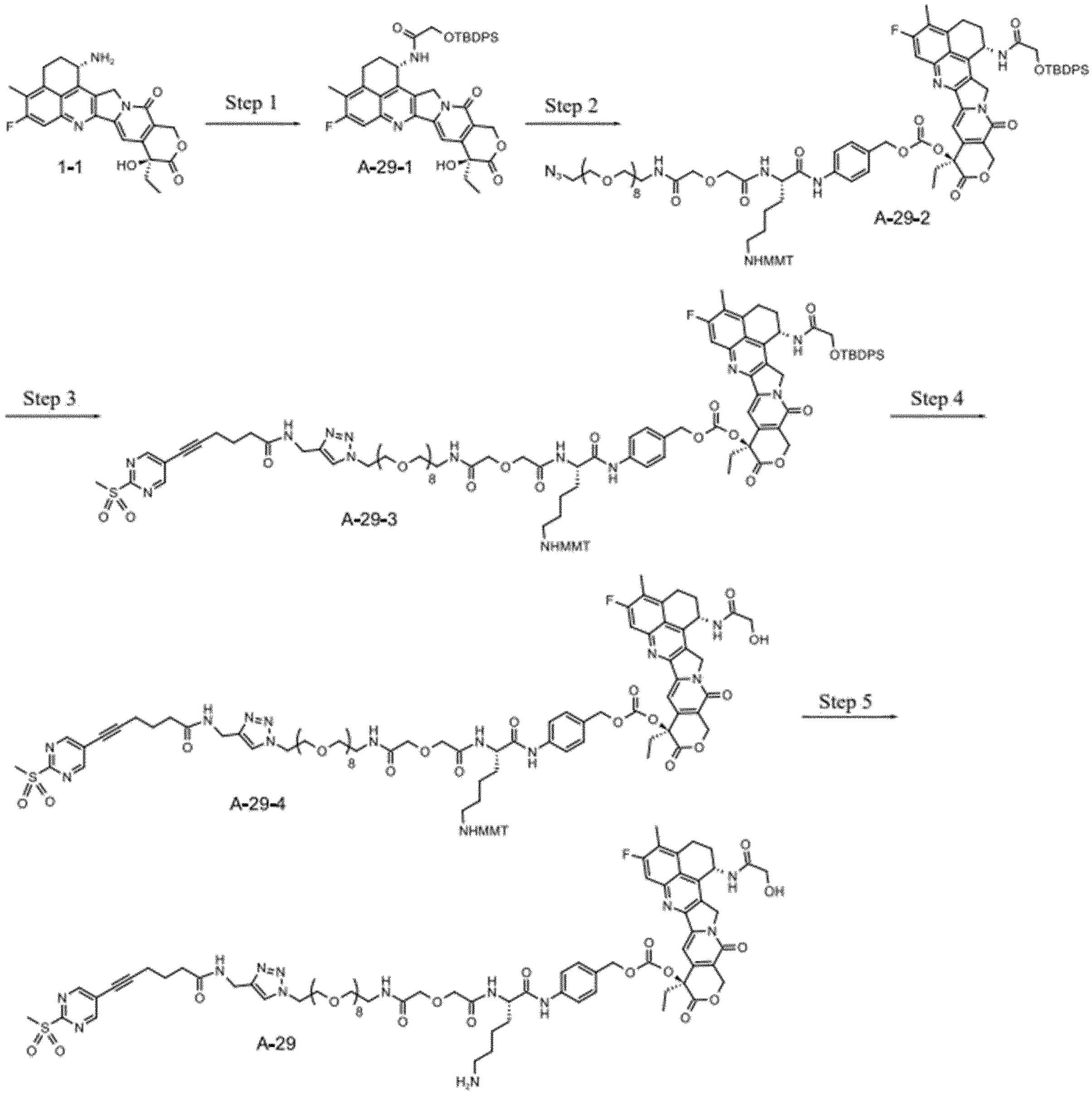

Step 1: Synthesis of 2-((tert-butyldiphenylsilyl)oxy)-N-((1S,9S)-9-ethyl-5-fluoro- 9-hydroxy-4-methyl-10,13-dioxo-2,3,9,10,13,15-hexahydro-1H,12H-benzo[de]pyrano[3',4':6,7] indolizino[1,2-b]quinolin-1-yl)acetamide

At 25°C, the mesylate salt of **1-1** (30.00 mg, 56.44 μmol) was dissolved in N,N-dimethylformamide (1 mL), added sequentially with 1H-benzotriazol-1-yloxy tripyrrolidinyl hexafluorophosphate (58.74 mg, 112.88 μmol), N,N-diisopropylethylamine (43.76 mg, 338.63 μmol) and 2-((tert-butyldiphenylsilyl)oxy)acetic acid (26.62 mg, 84.66 μmol), and reacted at 25°C for 1 hour, and the reaction was monitored by liquid chromatography-mass spectrometry. After the reaction was completed, the reaction solution was added with water, extracted with ethyl acetate, and the organic phases were combined, dried over sodium sulfate, and concentrated under reduced pressure, the crude product was separated by thin layer chromatography (dichloromethane:methanol=15:1) to obtain 27.00 mg of the title compound.

Step 2: Synthesis of 4-((S)-35-azido-2-(4-((4-methoxyphenyl) diphenylmethyl)amino)butyl)-4,8-dioxo-6,12,15,18,21,24,27,30,33-nonaoxa-3,9-diazapentatriacontanamido)benzyl ((1S,9R)-1-(2-((tert-butyldiphenylsilyloxy)acetamido)-9-ethyl-5-fluoro-4-methyl-10,13-dioxo-2,3,9,10,13,15-hexahydro-1H,12H-benzo[de]pyrano[3',4':6,7]indolizino[1,2-b]quinolin-9-yl)carbonate

At 0°C, **A-29-1** (20 mg, 27.33 μmol) was dissolved in dichloromethane (2 mL), added sequentially with 4-dimethylaminopyridine (26.71 mg, 218.61 μmol) and a solution of triphosgene (8.11 mg, 27.33 μmol) in dichloromethane (0.5 mL), reacted at 0°C for 0.5 hours; the residual triphosgene was replaced with nitrogen, followed by an addition of a solution of (S)-2-(32-azido-5-oxo-3,9,12,15,18,21,24,27, 30-nonaoxa-3,9-diazapentatriacontanamido)-N-(4-(hydroxymethyl)phenyl)-6-(((4-methoxyphenyl)benzhydryl)amino)caproamide (43.46 mg, 40.99 μmol) in dichloromethane (1 mL) in dropwise manner, reacted at 0°C for 0.5 hours; the reaction was monitored by liquid chromatography-mass spectrometry. After the reaction was completed, the reaction solution was concentrated, and the crude product was separated and purified by thin-layer chromatography (dichloromethane:methanol=15:1) to obtain 30.00 mg of the title compound.

Step 3: Synthesis of (1S,9R)-1-(2-((tert-butyldiphenylsilyl)oxy)acetamido)-9-ethyl-5-fluoro-4-methyl-10,13-dioxo-2,3,9,10,13,15-hexahydro-1H,12H-benzo[de]pyrano[3',4':6,7]indolizino[1,2-b]quinolin-9-yl(4-((4-methoxyphenyl) diphenylmethyl)amino)butyl)-35-(4-((6-(2-(methylsulfonyl) pyrimidin-5-yl)hex-5-ynamido)methyl)-1H-1,2,3-triazol-1-yl)-4,8-dioxo-6,12,15,18,21,24,27,30,33-nonaoxa-3,9-diazapentatriacontanamido)benzyl)carbonate

At 25°C, **A-29-2** (250.00 mg, 137.51 μmol) was dissolved in a mixed solvent of DMSO (2 mL) and water (0.4 mL), added with 6-(2-(methylsulfonyl)pyrimidin-5-yl)-N-(prop-2-yn- 1-yl)hex-5-ynamide (**IM-2**, 62.98 mg, 206.26 μmol) and cuprous bromide (39.45 mg, 275.01 μmol), reacted at 25°Cfor 1 hour; the reaction was monitored by liquid chromatography-mass spectrometry; after the reaction was completed, the reaction solution was purified by preparative high-performance liquid chromatography (with conditions as follows), and the fractions were lyophilized to obtain 150.00 mg of the title compound.

Chromatographic column: SunFire Prep C18 OBD 19 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0 | 15 | 85 | 28 |
| 18 | 90 | 10 | 28 |

Step 4: Synthesis of (1S,9R)-9-ethyl-5-fluoro-1-(2-hydroxyacetamido)-4-methyl-10,13-dioxo-2,3,9,10,13,15-hexahydro-1H,12H-benzo[de]pyrano[3',4':6,7]indolizino[1,2-b]quinolin-9-yl(4-((S)-2-(4-((4-methoxyphenyl) diphenylmethyl)amino)butyl)-35-(4-((6-(2-(methylsulfonyl)pyrimidin-5-yl)hex-5-ynamido)methyl)-1H-1,2,3-triazol-1-yl)-4,8-dioxo-6,12,15,18,21,24,27,30,33-nonaoxa-3,9-diazapentatriacontanamido)carbonate

At 25°C, **A-29-3** (150 mg, 49.45 μmol) was dissolved in tetrahydrofuran (1 mL), added dropwise with a mixed solution of tetrabutylammonium fluoride (1M in tetrahydrofuran)/glacial acetic acid (v/v=13/1) (50 uL), reacted at 25°C for 0.5 hours, and the reaction was monitored by liquid chromatography-mass spectrometry; after the reaction was completed, the reaction solution was purified by preparative high-performance liquid chromatography (with conditions

as follows), and the fractions were lyophilized to obtain 50.00 mg of the title compound.

Chromatographic column: SunFire Prep C18 OBD 19 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0 | 15 | 85 | 28 |
| 20 | 90 | 10 | 28 |

Step 5: Synthesis of 4-((S)-2-(4-aminobutyl)-35-(4-((6-(2-(methylsulfonyl)pyrimidin-5-yl) hex-5-ynamido)methyl)-1H-1,2,3-triazol-1-yl)-4, 8-dioxo-6,12,15,18,21,24,27,30,33-nonaoxa-3,9-diazapentatriacontanamido)benzyl ((1S,9R)-9-ethyl-5-fluoro-1-(2-hydroxyacetamido)-4-methyl-10,13-dioxo-2,3,9,10,13,15-hexahydro- 1H, 12H-benzo[de]pyrano[3',4':6,7]indolizino[1,2-b]quinolin-9-yl)carbonate

At 25°C, **A-29-4** (50 mg, 26.52 μmol) was dissolved in dichloromethane (1 mL), added with trifluoroacetic acid (60.49 mg, 530.49 μmol), reacted at 25°C for 0.5 hours; the reaction was monitored by liquid chromatography-mass spectrometry; after the reaction was completed, the reaction solution was concentrated, the crude product was purified by preparative high-performance liquid chromatography (with conditions as follows), and the fractions were lyophilized to obtain 23.69 mg of the title compound.

Chromatographic column: SunFire Prep C18 OBD 19 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0 | 15 | 85 | 28 |
| 18 | 90 | 10 | 28 |

The structure characterization data of **A-29** were as follows:
ESI-MS (m/z): 1613.6$[M+H]^+$.

Example 19: 4-((S)-2-((S)-3-methyl-2-(6-(2-(methylsulfonyl)pyrimidin-5-yl)hex-5-ynamido)butanamido)-5-ureidopentanamido)benzyl ((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate **(B-1)**

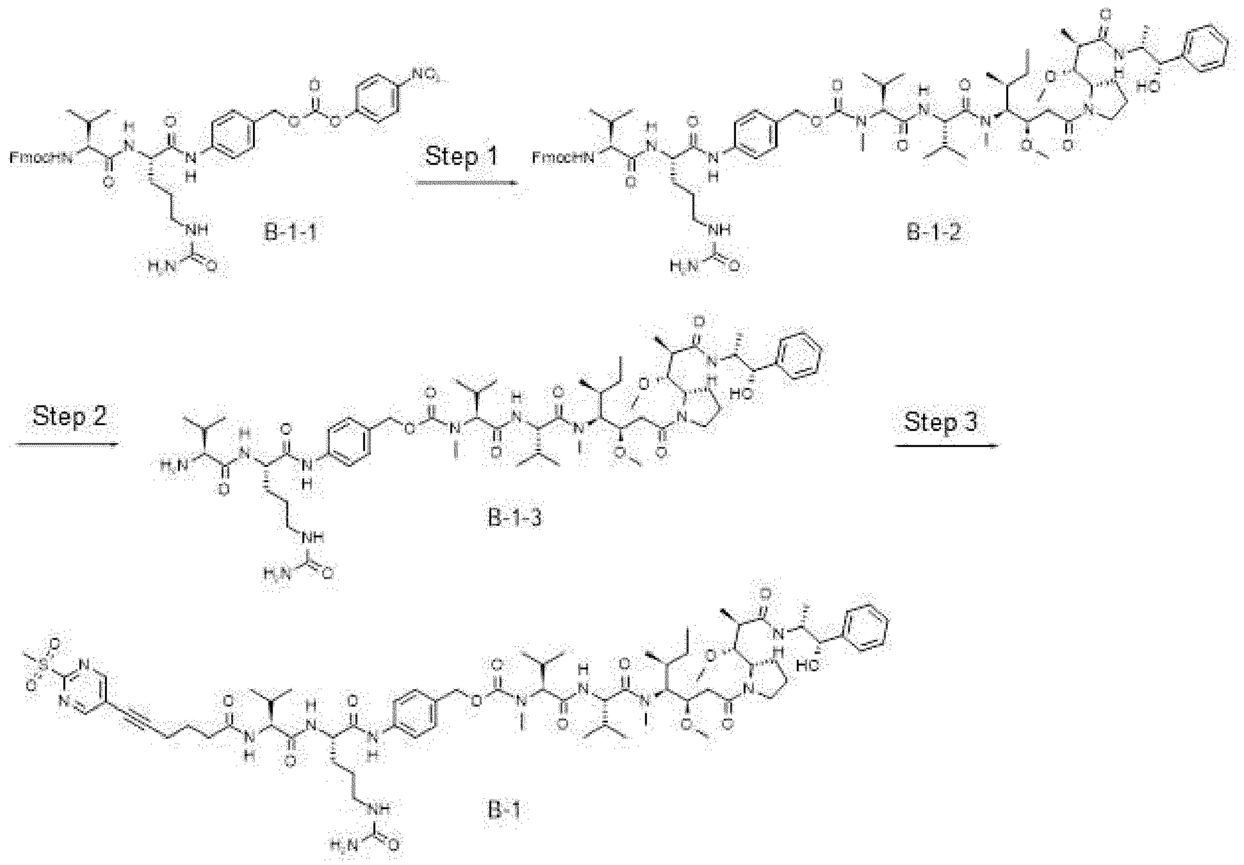


B-1-1

B-1-2

B-1-3

B-1

Step 1: Synthesis of 4-((S)-2-((S)-2-(((9H-fluoren-9-yl)methoxy)carbonyl)amino)-3-methylbutylamino)-5-ureidopentanamido)benzyl ((S)-1-((S)-1-((3R,4S,5S)-1-((S)-2-((1R,2R)- 3-((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate

At 25°C, (S)-N-((3R,4S,5S)-1-((S)-2-((1R,2R)-3-((1S,2R)-1-hydroxy-1-phenylpropan-2-yl) amino)-1-methoxy-2-methyl-3-oxopropyl)pyrrolidin-1-yl)-3-methoxy-5-methyl-1-oxohept-4-yl)-N,3-dimethyl-2-((S)-3-methyl-2-(methylamino)butanamide (**MMAE,** 10 mg, 0.014 mmol) and compound **B-1-1** (12.82 mg, 0.017 mmol) were dissolved in DMF (1.0 mL), added with HOBt (2.82 mg, 0.021 mmol) and DIPEA (3.60 mg, 0.028 mmol), reacted at 25°C for 1 h. The reaction was monitored by liquid chromatography-mass spectrometry, and the reaction solution was directly used in the next reaction without workup.

The structure characterization data were as follows:

ESI-MS (m/z): 1345.2 $[M+H]^+$.

Step 2: Synthesis of 4-((S)-2-((S)-2-amino-3-methylbutylamino)-5-ureidopentanamido) benzyl ((S)-1-((S))-1-((3R,4S,SS)-1-((S)-2-((1R,2R)-3-((1S,2R)-1-hydroxy-1-phenylpropan-2-yl) amino)-1-methoxy-2-methyl-3-oxopropyl)pyrrolidin-1-yl)-3-methoxy-5-methyl-1-oxoheptan-4-yl) (methyl)amino)-3-methyl-1-oxobutan-2-yl)amino)-3-methyl-1-oxobutan-2-yl(methyl)carbamate

At 25°C, diethylamine (0.1 mL) was added to the reaction solution of compound **B-1-2,** reacted at 25°C for 1 h, the reaction was monitored by liquid chromatography-mass spectrometry, and the reaction solution was concentrated under reduced pressure to remove the solvent and to obtain a crude product that was directly used in the next reaction.

The structure characterization data were as follows:

ESI-MS (m/z): 1123.2 $[M+H]^+$.

Step 3: Synthesis of 4-((S)-2-((S)-3-methyl-2-(6-(2-(methylsulfonyl)pyrimidin-5-yl)hex-5-ynamido)butanamido)-5-ureidopentanamido)benzyl ((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate

At 25°C, the crude product of **B-1-3** and 6-(2-(methylsulfonyl)pyrimidin-5-yl)hex-5-ynoic acid (**IM-1**, 7.16 mg, 0.027 mmol) were dissolved in DMF (0.5 mL), added with HATU (10.15 mg, 0.027 mmol) and DIPEA (3.45 mg, 0.027 mmol), reacted at 25°C for 1 h, the reaction was monitored by liquid chromatography-mass spectrometry, and the reaction solution was purified by preparative high-performance liquid chromatography (with conditions as follows) to obtain 5.0 mg of the title compound.

Chromatographic column: SunFire Prep C18 OBD 19 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 30 | 70 | 28 |
| 2.00 | 30 | 70 | 28 |
| 18.00 | 90 | 10 | 28 |

The structure characterization data were as follows:
ESI-MS (m/z): 1373.2 $[M+H]^+$.

Example 20: N-((3R,4S,7S,10S,21S)-21-Benzyl-4-((S)-sec-butyl)-3-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-7,10-diisopropyl-5,11-dimethyl-6,9,12,17,20,23,26-heptaoxo-2,14-dioxa-5,8, 11,16,19,22,25-heptazaheptoic acid-27-yl)-6-(2-(methylsulfonyl)pyrimidin-5-yl)hex-5-ynyl **(B-3)**

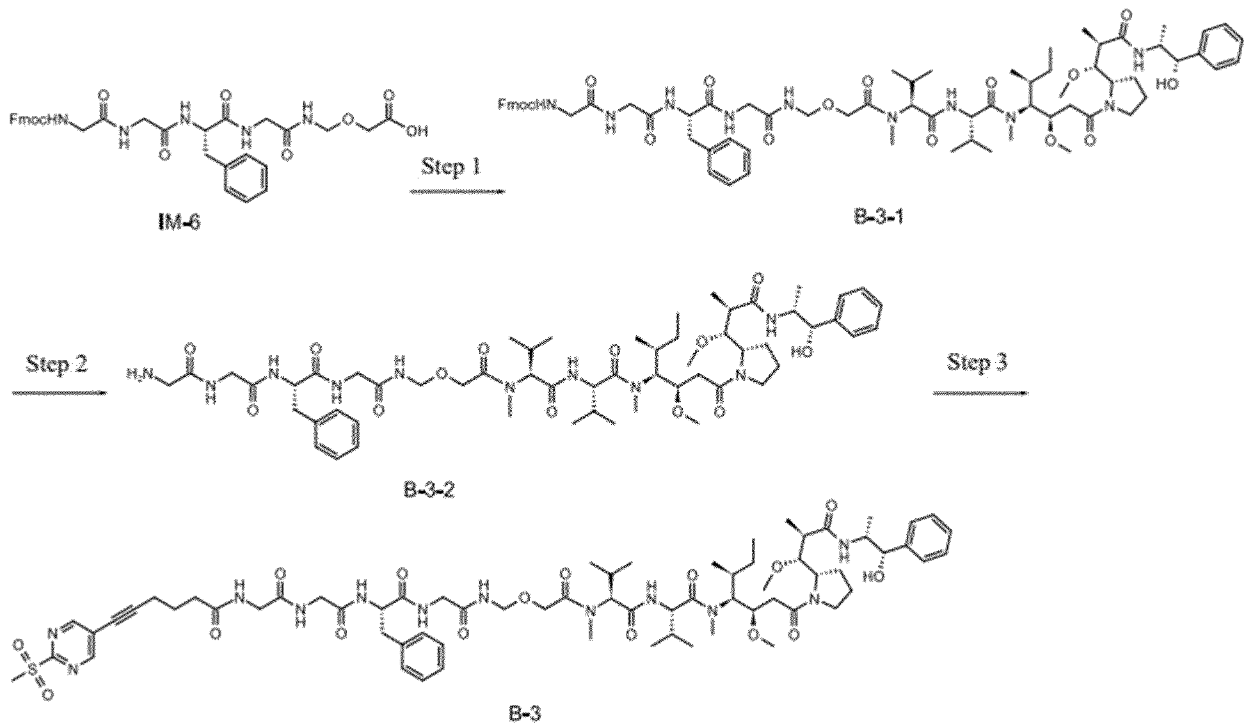


IM-6

B-3-1

B-3-2

B-3

Step 1: Synthesis of (9H-fluoren-9-yl)methyl((3R,4S,7S,10S,21S)-21-benzyl-4-((S)- sec-butyl)-3-(2-((S)-2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-7,10-diisopropyl-5,11-dimethyl-6,9,12,17,20,23,26-heptaoxo-2,14-dioxa-5,8,11,16,19,22,25-heptylheptanediamido-27-yl)carbamate

Compound **IM-6** (50.0 mg, 0.077 mmol) and (S)-N-((3R,4S,5S)-1-((S)-2-((1R,2R)- 3-(((1S,2R)-1-hydroxy-1-phenylpropan-2-yl)amino)-1-methoxy-2-methyl-3-oxypropyl)pyrrolidin-1-yl)-3-methoxy-5-methyl-1-oxoheptan-4-yl)-N,3-dimethyl-2-((S)-3-methyl-2-(methylamino) butyrylamido)butanamide (**MMAE**, 55.60 mg, 0.077 mmol) were weighed and dissolved in DMF (1 mL), then added with HATU (32.37 mg, 85.18 μmol) and DIPEA (20.02 mg, 154.88 μmol); after the addition, the reaction was carried out at room temperature for 1 h and monitored by liquid chromatography-mass spectrometry, and the reaction solution was purified by preparative high-performance liquid chromatography (with conditions as follows) to obtain 35.0 mg of the title compound.

Chromatographic column: SunFire Prep C18 OBD 19 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 40 | 60 | 28 |
| 2.00 | 40 | 60 | 28 |
| 18.00 | 90 | 10 | 28 |

The structure characterization data were as follows:
ESI-MS (m/z): 1345.1[M+H]$^+$.

Step 2: Synthesis of (S)-2-((2S,13S)-19-amino-13-benzyl-2-isopropyl-3-methyl-4,9,12, 15,18-pentaoxo-6-oxa-3,8,11,14,17-heptazaone)-N-((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethylbutanamide

Compound **B-3-1** (20.00 mg, 0.015 mmol) was dissolved in dichloromethane (2 mL), then added with diethylamine (1 mL), and reacted at room temperature for 1 h, and the reaction was monitored by liquid chromatography-mass spectrometry. The reaction solution was concentrated under reduced pressure to obtain 20.0 mg of crude product.
The structure characterization data were as follows:
ESI-MS (m/z): 1123.1[M+H]$^+$.

Step 3: Synthesis of N-((3R,4S,7S,10S,21S)-21-benzyl-4-((S)-sec-butyl)-3-(2-((S)- 2-((1R,2R)-3-(((1S,2R)-1-hydroxy-1-phenylpropan-2-yl)amino)-1-methoxy-2-methyl-3-oxopropyl)pyrrolidin-1-yl)-2-oxoethyl)-7,10-diisopropyl-5,1 1-dimethyl-6,9,12,17,20,23,26-heptaoxo-2,14-dioxa-5,8,11,16,19,22,25-heptazaheptoicacid-27-yl)-6-(2-(methylsulfonyl)pyrimidin-5-yl)hex-5-ynyl

Compound **B-3-2** (20.00 mg, 0.015 mmol) and 6-(2-(methylsulfonyl)pyrimidin-5-yl)hex-5-ynoic acid (**IM-1**, 17.00 mg, 0.015 mmol) were dissolved in DMF (1 mL), then added with HATU (6.33 mg, 16.65 μmol) and DIPEA (3.91 mg, 30.27 μmol), and reacted at room temperature for 1 h after the addition, the reaction was monitored by liquid chromatography-mass spectrometry, and the reaction solution was purified by preparative high performance liquid chromatography (with conditions as follows) to obtain 3.52 mg of the title compound.

Chromatographic column: SunFire Prep C18 OBD 19 mm×5.0 μm

Mobile phase A: acetonitrile; Mobile phase B: water (0.05% formic acid)

| Time [min] | Mobile phase A [%] | Mobile phase B[%] | Flow rate [mL/min] |
|---|---|---|---|
| 0.00 | 10 | 90 | 28 |

(continued)

| Time [min] | Mobile phase A [%] | Mobile phase B[%] | Flow rate [mL/min] |
|---|---|---|---|
| 2.00 | 10 | 90 | 28 |
| 18.00 | 90 | 10 | 28 |

The structure characterization data were as follows:
ESI-MS (m/z): 1374.1[M+H]$^+$.

II. Preparation and binding activity determination of antibody

1. Preparation and purification of antibody

In the early stage, by immunizing Balb/c, C57B1/6, NZB and A/J mice, the murine antibodies 3D8, 19F6 and 38F8 were obtained through hybridoma screening, and respectively humanized to obtain the humanized antibody sequences 3D8_HuC24 (heavy chain variable region, SEQ ID NO:9; light chain variable region, SEQ ID NO:10), 19F6_Hu35v1 (heavy chain variable region, SEQ ID NO:1; light chain variable region, SEQ ID NO:2), 38F8_Hu57 (heavy chain variable region, SEQ ID NO:17; light chain variable region, SEQ ID NO:18); the heavy chain constant regions of the above humanized antibodies were all human IgG1 heavy chain constant region (SEQ ID NO:22), and the light chain constant regions were all human kappa light chain constant region (SEQ ID NO: 23). The sequence information of 3D8_HuC24, 19F6_Hu35v1, 38F8_Hu57 is summarized in Table 1 below. After synthesis and codon-optimization of the coding DNA sequences of the above humanized antibodies, they were cloned into pcDNA3.4 plasmids, and the pcDNA3.4 plasmids corresponding to the heavy and light chains of each humanized antibody were simultaneously transfected into Expi293F cells, and Protein A was used to purify the expressed antibody in the supernatant to obtain the corresponding antibody.

Table 1: Sequence information of 3D8_HuC24, 19F6_Hu35v1, 38F8_Hu57

| Antibody | Numbering system | SEQ ID NO: | | | | | | | | | |
|---|---|---|---|---|---|---|---|---|---|---|---|
| | | CDR-H1 | CDR-H2 | CDR-H3 | CDR-L1 | CDR-L2 | CDR-L3 | V H | V L | C H | C L |
| 19F6_Hu 35V1 | Chothia | 3 | 4 | 5 | 6 | 7 | 8 | 1 | 2 | 22 | 23 |
| | AbM | 29 | 30 | 5 | 6 | 7 | 8 | | | | |
| | Kabat | 31 | 32 | 5 | 6 | 7 | 8 | | | | |
| | IMGT | 24 | 25 | 26 | 27 | 28 | 8 | | | | |
| 3D8_Hu C24 | Chothia | 11 | 12 | 13 | 14 | 15 | 16 | 9 | 10 | | |
| | AbM | 36 | 37 | 13 | 14 | 15 | 16 | | | | |
| | Kabat | 38 | 39 | 13 | 14 | 15 | 16 | | | | |
| | IMGT | 33 | 34 | 35 | 43 | 44 | 16 | | | | |
| 38F8_Hu 57 | Chothia | 19 | 20 | 21 | 14 | 15 | 16 | 17 | 18 | | |
| | AbM | 45 | 46 | 21 | 14 | 15 | 16 | | | | |
| | Kabat | 47 | 48 | 21 | 14 | 15 | 16 | | | | |
| | IMGT | 40 | 41 | 42 | 43 | 44 | 16 | | | | |

2. Affinity determination

The affinities of 19F6_Hu35V1, 3D8_HuC24 and 38F8_Hu57 to ROR1 on the surface of human cells were determined by flow cytometry.

Construction of human cell line with stable expression of ROR1: Ba/F3 cells were infected with lentivirus (G&P Biosciences) containing the complete coding sequence of human ROR1 (gene number: Q01973), positively transduced cells were screened by puromycin, and the monoclonal BA/F3-ROR1-stable cell line was obtained by monoclonal screening with limiting dilution method. The expression of ROR1 was identified by flow cytometry (Luminex, Guava easyCyte HT). Anti-human ROR1 antibody D10 (patent sequence: US Patent 9217040B2) was used as the detection antibody. As shown in Figure 1, the results of flow cytometry showed that the positive rate of Ba/F3-ROR1 was very high (close to 100%), which could be used in subsequent experiments.

In addition to Ba/F3-ROR1 overexpression cells, human mantle cell lymphoma cell line Jeko-1 (Nanjing Cobioer Biosciences), human colon cancer cell line HT-29 (the Cell Bank of the Chinese Academy of Sciences), human lung cancer cell line A549 (the Cell Bank of the China Academy of Sciences) were also used for flow cytometry analysis. Anti-ROR1 antibody UC961 (the VH and VL of antibody UC961 were SEQ ID NO: 5 and 6 disclosed in WO2018237335A1, respectively) and anti-TNP antibody were used as positive control and negative control, respectively. Ba/F3-ROR1, Jeko-1, HT-29 and A549 cells were incubated with different concentration gradients of the purified humanized antibodies on ice for 30 minutes, respectively, washed with flow buffer (PBS+2% FBS) twice, then incubated with goat anti-human fluorescent secondary antibody (Jackson ImmunoResearch) on ice for 30 minutes, and finally loaded on the machine for detection.

The results were shown in Tables 2-1, 2-2 and 2-3, in which the affinity EC50 values of 19F6_Hu35V1, 3D8_HuC24 and 38F8_Hu57 on all of the four cells were equal to or lower than that of the positive control antibody UC961, indicating that the above-mentioned anti-human ROR1 humanized antibodies had excellent binding activity to ROR1-positive cells.

Table 2-1: Cell affinity detection of 19F6-Hu35V1

| Cells / Antibody | EC50 (nM) | | | |
|---|---|---|---|---|
| | Ba/F3-ROR1 | Jeko-1 | HT-29 | A549 |
| 19F6_Hu35V1 | 0.17 | 0.12 | 0.15 | 0.08 |
| UC961 | 0.30 | 0.36 | 0.20 | 0.16 |

Table 2-2: Cell affinity detection of 3D8-HuC24

| Cells / Antibody | EC50 (nM) | | | |
|---|---|---|---|---|
| | Ba/F3-ROR1 | Jeko-1 | HT-29 | A549 |
| 3D8_HuC24 | 0.23 | 0.04 | 0.17 | 0.29 |
| UC961 | 0.31 | 0.14 | 0.21 | 0.46 |

Table 2-3: Cell affinity detection of 38F8-Hu57

| Cells / Antibody | EC50 (nM) | | | |
|---|---|---|---|---|
| | Ba/F3-ROR1 | Jeko-1 | HT-29 | A549 |
| 38F8_Hu57 | 0.75 | 0.07 | 0.33 | 0.23 |
| UC961 | 1.07 | 0.06 | 0.37 | 0.21 |

The dynamic affinities of 19F6_Hu35V1, 3D8_HuC24, 38F8_Hu57 and the positive control antibody UC961 to human ROR1 ECD protein were determined by Octet ForteBio method. The specific experimental steps were as follows: the anti-human IgG Fc AHC probe (ForteBio) was first bound to the antibody to be tested to a response signal value of 0.8-1.2nm, and the dynamic affinity was measured by immersing the antibody-coated probe into wells containing human ROR1 ECD protein at different concentrations (16, 8, 4, 2, 1, 0.5, 0.25, and 0 μg/mL), the binding was conducted for

2-4 minutes, followed by dissociation for 4-6 minutes, 1: 1 kinetic binding model was used for all fitting analysis.

As shown in Table 3, 19F6_Hu35V1 had a dissociation rate significantly slower than that of the control antibody UC961 (as shown by the Kdis value), and an affinity 5.4 times stronger than that of the control antibody UC961 (as shown by the KD value). 3D8_HuC24 and 38F8_Hu57 showed affinity comparable to that of the control antibody UC961.

Table 3: Dynamic affinity determination of antibodies to human ROR1

| Antibody | KD (M) | kon(1/Ms) | kdis(1/s) |
|---|---|---|---|
| 19F6-Hu35V1 | 1.61E-09 | 5.38E+05 | 8.63E-04 |
| 3D8-HuC24 | 2.61E-08 | 5.86E+05 | 1.53E-02 |
| 38F8-Hu57 | 1.00E-08 | 2.41E+05 | 2.42E-03 |
| UC961 | 8.73E-09* | 1.26E+06* | 1.12E-02* |
| * indicates that the value is an average of three determinations. | | | |

III. Conjugation of compound containing cell bioactive molecule and linker to antibody

The antibody 19F6 involved in the antibody-drug conjugates prepared in the following example was the 19F6_Hu35v1 antibody described in the above section II.

The conjugation and preparation of samples were as follows:

0.46ml of 19F6_Hu35v1 antibody (anti-ROR1, 11.0mg/mL) was taken, diluted with 0.1M edetate disodium solution (pH 7.7), then adjusted with 1M $Na_2HPO_4$ solution to pH 7.7, added with 10 mM TCEP (tris(2-carboxyethyl)phosphine) solution and mixed well, and allowed to stand at room temperature for 90 min. To the above system, 4.0 to 10 times the molar amount of "drug-linker" compound dissolved in dimethyl sulfoxide was added, mixed well, and allowed to stand at room temperature for 2 hours, then NAP-5 gel column (Cytiva) was used to replace the buffer solution with 10 mM histidine buffer solution with pH of 6.0, then sucrose and Tween 20 were added, and mixed well to obtain antibody-drug conjugates (i.e., ADC compounds), as shown in Table 4.

The drug-to-antibody ratio (DAR value) of the conjugation sample was determined as follows:

the molecular weight of ADC sample was determined by LC-MS, and the drug-to-antibody ratio, i.e., DAR value was calculated;

the molecular weight of ADC sample obtained by conjugation was analyzed by LC-MS.

Chromatographic conditions for determination:

Liquid chromatography column: Thermo MAbPac RP 3.0*100mm;
Mobile phase A: 0.1%FA/$H_2O$; Mobile phase B: 0.1%FA/ACN;
Flow rate: 0.25 ml/min; Sample chamber temperature: 8°C; Column temperature: 60°C; Injection volume: 2 μl;

| Time (minutes) | 2 | 20 | 22 | 25 | 26 | 30 |
|---|---|---|---|---|---|---|
| Mobile phase A (vol. %) | 75 | 60 | 5 | 5 | 75 | 75 |
| Mobile phase B (vol. %) | 25 | 40 | 95 | 95 | 25 | 25 |

Mass spectrometry conditions for determination:

Mass Spec Model: AB Sciex Triple TOF 5600+;
GS1 35; GS2 35; CUR 30; TEM 350; ISVF 5500; DP 250; CE 10; Accumulation time 0.5 s; m/z 600-4000; Time bins to sum 40.

Table 4: number of ADC and DAR

| Number of drug-linker | Number of ADC | Molar ratio of drug-linker to antibody | DAR |
|---|---|---|---|
| A-1 | 19F6-A-1 | 10:1 | 7.81 |
| A-5 | 19F6-A-5 | 10:1 | 7.73 |
| A-6 | 19F6-A-6 | 10:1 | 8.07 |
| A-7 | 19F6-A-7 | 10:1 | 7.77 |
| A-8/9-A | 19F6-A-8 | 10:1 | 7.00 |
| A-8/9-B | 19F6-A-9 | 10:1 | 7.50 |
| A-10/11-A | 19F6-A-10 | 10:1 | 7.79 |
| A-12 | 19F6-A-12 | 10:1 | 7.04 |
| A-14/15-A | 19F6-A-14 | 10:1 | 7.83 |
| A-26 | 19F6-A-26 | 10:1 | 7.30 |
| A-28 | 19F6-A-28 | 10:1 | 7.81 |
| A-29 | 19F6-A-29 | 10:1 | 7.92 |
| B-1 | 19F6-B-1 | 4.5:1 | 3.61 |

IV. Detection of inhibitory effect of antibody-drug conjugate on cell viability in vitro

Inhibitory effect of ADC compounds on cell proliferation

(1) Cell plating: First, tumor cells HCC827, NCI-H1975, HT-29 and NCI-N87 were cultured in the corresponding medium, digested with trypsin, resuspended and counted after centrifugation, and the cells were adjusted to an appropriate concentration for plating. The sources of tumor cells were shown in Table 5.

Table 5: Sources of tumor cells

| Cell name | Tumor type | Source |
|---|---|---|
| HCC827 | Non-small cell lung cancer | ATCC |
| NCI-H1975 | Human lung adenocarcinoma cells | Nanjing Cobioer Biosciences |
| HT-29 | Human colon cancer cells | Cell Bank of Chinese Academy of Sciences |
| NCI-N87 | Human gastric cancer cells | ATCC |

Co-incubation of the compound of the present invention and tumor cells: After the cells adhered to the wall, the medium was removed, and the diluted antibody-drug conjugate (ADC compound of the present invention) was added to the wells of the above plate, and incubated for 96 hours.

In vitro cell viability detection: After the incubation was completed, 50 μL of Cell Counting-Lite™ 2.0 reagent (Vazyme/Novazyme) was added to each well, shaken and mixed well in the dark, reacted for 10 minutes and then the detection was conducted, readings on microplate reader (manufacturer: BMG, Model: PHERAStar-FS) were collected. By adding Cell Counting-Lite™, the background RLU was obtained from the culture wells without cells, and the control RLU was obtained from the culture wells with cells but no compound. Cell inhibition rate = 1 - (sample RLU - background RLU) / (control RLU - background RLU) × 100%; according to the four-parameter model fitting curve, the half inhibitory concentration ($IC_{50}$) of the compound was calculated.

(2) Data results: The detection results were shown in Table 6 to Table 9.

Table 6: Killing results of ADC conjugates on NCI-H1975 cell line (96 hours)

| Number of ADC | $IC_{50}$ (μg/mL) |
|---|---|
| 19F6-A-1 | 1.35 |
| 19F6-A-6 | 73.19 |
| 19F6-A-8 | 16.84 |
| 19F6-A-9 | 13.50 |
| 19F6-A-10 | 112.6 |
| 19F6-A-26 | 0.64 |
| 19F6-A-28 | 3.26 |
| 19F6-A-29 | 5.00 |
| 19F6-B-1 | 13.20 |

Table 7: Killing results of ADC conjugates on HCC827 cell line (96 hours)

| Number of ADC | $IC_{50}$ (μg/mL) |
|---|---|
| 19F6-A-6 | 45.66 |
| 19F6-A-10 | 39.69 |
| 19F6-B-1 | 0.21 |

Table 8: Killing results of ADC conjugates on HT-29 cell line (96 hours)

| Number of ADC | $IC_{50}$ (μg/mL) |
|---|---|
| 19F6-A-1 | 0.60 |
| 19F6-A-6 | 21.00 |
| 19F6-A-10 | 34.16 |
| 19F6-A-26 | 0.78 |
| 19F6-A-28 | 3.89 |
| 19F6-A-29 | 4.56 |

Table 9: Killing results of ADC conjugates on NCI-N87 cell line (96 hours)

| Number of ADC | $IC_{50}$ (μg/mL) |
|---|---|
| 19F6-A-5 | 25.67 |
| 19F6-A-6 | 54.41 |
| 19F6-A-8 | 18.70 |
| 19F6-A-9 | 15.10 |
| 19F6-A-10 | 42.58 |

The test results show that the ADC molecules obtained by the new conjugation method had tumor cell killing effects.

It indicates that the ADC molecules formed by the new conjugation method could kill tumor cells, and the application of the new conjugation method to ADC molecules was effective.

V. Evaluation of tumor growth inhibitory effect of antibody-drug conjugate on ROR1-expressing human tumor cell line in mouse subcutaneous xenograft tumor model

The preparations containing the ADCs of the present invention were administered via tail vein injection to mouse CDX models subcutaneously implanted with human gastric cancer cells NCI-N87, human lung adenocarcinoma cells H1975, and human colon cancer cells HT-29, the changes in tumor volume and animal body weight were determined twice a week, and the tumor - inhibiting effects of the ADCs of the present invention on tumor -bearing mice were calculated.

Drugs to be tested

Drug name, source and preparation method: An appropriate amount of ADC of the present invention (Sichuan Kelun Bio-tech Biopharmaceutical Co., Ltd.) was taken, and the mother solution thereof was diluted with normal saline to obtain an administration solution according to the administration volume of 10 μl/g. Normal saline was used as vehicle control (Vehicle).

Experimental animals and cell lines

Balb/c-nu mice (Chengdu Gempharmatech Biotechnology Co., Ltd., production license number: SCXK (Sichuan) 2020-034, animal certificate number: 202112622, 202109635, 202106975)

Human gastric cancer cell NCI-N87 (ATCC)

Human lung adenocarcinoma cell NCI-H1975 (Nanjing Cobioer Biosciences)

Human colon cancer cell HT-29 (Cell Bank, Chinese Academy of Sciences)

Grouping and evaluation method

Tumor-bearing mice with an average tumor volume of 100-200 $mm^3$ were selected for random grouping (the number of groups was determined according to the number of samples). Physiological saline (hereinafter referred to as vehicle control, Vehicle) and the ADC of the present invention were administered according to the groups. The administration frequency was given in the specific experimental protocol. The administration method was tail vein injection, and the administration volume was 10 μl/g. After administration, the tumor diameter was measured twice a week with a vernier caliper, and the tumor volume was calculated according to the following formula: $V = 0.5\ a \times b^2$, wherein a and b represented the long and short diameters of the tumor, respectively. Animal death was observed and recorded daily.

The following formula was used to calculate the tumor growth inhibition rate TGI (%), which was used to evaluate the inhibitory effect of the ADC of the present invention against tumor:

$$V_{T\,end} > V_{T0},\ TGI\ (\%) = [1 - (V_{T\,end} - V_{T0}) / (V_{C\,end} - V_{C0})] * 100\%;$$

or

$$V_{T\,end} \leq V_{T0},\ TGI\ (\%) = [1 - (V_{T\,end} - V_{T0}) / V_{T0}] * 100\%.$$

wherein,

$V_{T\,end}$: mean tumor volume of treatment group at the end of experiment

$V_{T0}$: mean tumor volume of treatment group at the beginning of administration

$V_{C\,end}$: mean tumor volume of vehicle control group at the end of experiment

$V_{C0}$: mean tumor volume of vehicle control group at the beginning of administration

The following formula was used to calculate the relative tumor proliferation rate T/C (%), which was used to evaluate the inhibitory effect of the ADC of the present invention against tumor:

$$T/C = (V_{T\ end} / V_{T0}) / (V_{C\ end} / V_{C0}).$$

(1) Evaluation of pharmacological effect of anti-human ROR1 antibody-drug conjugate in NCI-N87 model

NCI-N87 cells were cultured at 37°C and 5% $CO_2$ in RPMI1640 medium containing 10% fetal bovine serum. The NCI-N87 cells in the exponential growth phase were collected, resuspended in PBS to an appropriate concentration, and inoculated subcutaneously in female Balb/c-nu mice to establish a gastric cancer model. When the average tumor volume was about 160 $mm^3$, the mice were randomly divided into groups according to tumor size, sequentially including: vehicle control group (i.e., negative control, Vehicle group), 19F6-A-1 10 mg/kg group, 19F6-A-6 10 mg /kg group and 19F6-A-10 10 mg/kg group of the present invention. Each group was administered by injection via tail vein (i.v.) on Day0, Day3, and Day7, i.e., administered three times in total. After administration, the body weight of mice was measured twice a week and the long and short diameters of tumors were measured with a vernier caliper, and the tumor volume was calculated according to the following formula: $V = 0.5\ a \times b^2$, wherein a and b represented the long and short diameters of tumors, respectively. The animal death was observed and recorded every day.

The ADCs of the present invention had significant tumor growth inhibitory effects on the NCI-N87 gastric cancer xenograft model. Compared with the Vehicle group, the tumor growth inhibition (TGI) rates of the ADC 19F6-A-1 10 mg/kg group, 19F6-A-6 10 mg/kg group and 19F6-A-10 10 mg/kg group of the present invention were 51.08%, 131.40% and 50.92%, respectively. Animal death, significant body weight reduction and obvious drug toxic reaction were not observed in each treatment group on Day28, and the mice had good tolerance to the ADCs of the present invention during the treatment period. The specific results were shown in Table 10, Figure 2 and Figure 3.

Table 10: Human gastric cancer cell NCI-N87 CDX model

| Group | Dose (mg/kg) | Day42 | | | |
|---|---|---|---|---|---|
| | | Tumor volume ($mm^3$)-($\bar{x}$±SEM) | TGI (%) | T/C (%) | P value (vs. Vehicle) |
| Vehicle | - | 1669.99±174.91 | - | - | - |
| 19F6-A-1 | 10 | 898.23±127.83 | 51.08 | 53.79 | <0.01 |
| 19F6-A-6 | 10 | 109.96±19.07 | 131.40 | 6.53 | <0.001 |
| 19F6-A-10 | 10 | 901.61±128.23 | 50.92 | 53.64 | <0.01 |

Note: TGI represents tumor growth inhibition rate, and T/C represents relative tumor proliferation rate; *P* value indicates the statistical difference of TGI between the treatment group and Vehicle.

(2) Evaluation of pharmacological effect of anti-human ROR1 antibody-drug conjugate in NCI-H1975 model

NCI-H1975 cells were cultured at 37°C and 5% $CO_2$ in RPMI1640 medium containing 10% fetal bovine serum. The NCI-H1975 cells in the exponential growth phase were collected, resuspended in PBS to an appropriate concentration, and inoculated subcutaneously in female Balb/c-nu mice to establish a human lung adenocarcinoma cell model. When the average tumor volume was about 120$mm^3$, the mice were randomly divided into groups according to tumor size (6 mice per group), respectively including: vehicle control group (Vehicle), ADC 19F6-A-6 10 mg/kg group of the present invention and ADC 19F6-A-10 10 mg/kg group of the present invention, each group was administered by injection via tail vein (i.v.) on Day0, Day4, Day7, Day10 and Day14, i.e., administered 5 times in total. After administration, the body weight of the mice was measured and the long and short diameters of the tumors were measured with a vernier caliper twice a week, and the tumor volume was calculated according to the following formula: $V = 0.5\ a \times b^2$, wherein a and b represented the long and short diameters of the tumors, respectively. The animal death was observed and recorded every day.

Compared to the vehicle control group, after 5 administrations, the Day28 data showed that the ADC 19F6-A-6 10 mg/kg group and 19F6-A-10 10 mg/kg group of the present invention significantly inhibited the tumor growth of the NCI-H1975 model, had a tumor growth inhibition rate (TGI) of 196.35% and 52.52%, respectively, and showed significant tumor growth inhibitory effects in the NCI-H1975 small cell lung cancer xenograft model; and in the ADC 19F6-A-6 10 mg/kg group of the present invention, all tumors in the mice were completely regressed. On Day 28, animal death, significant animal body weight loss and obvious drug toxic reaction were not observed in each treatment group. During the treatment period, the mice had good tolerance to the ADCs of the present invention. The specific results were shown

in Table 11, Figure 4 and Figure 5.

Table 11: Human lung adenocarcinoma cell NCI-H1975 CDX model

| Group | Dose (mg/kg) | Day28 | | | |
|---|---|---|---|---|---|
| | | Tumor volume ($mm^3$)-($\bar{x}\pm$SEM ) | TGI (%) | T/C (%) | P value (vs. Vehicle) |
| Vehicle | - | 2832.20±297.54 | - | - | - |
| 19F6-A-6 | 10 | 4.45±1.73 | 196.35 | 0.16 | <0.001 |
| 19F6-A-10 | 10 | 1407.98±223.20 | 52.52 | 50.17 | <0.01 |
| Note: P value indicates the statistical difference of TGI between the treatment group and Vehicle. | | | | | |

(3) Evaluation of pharmacological effect of anti-human ROR1 antibody-drug conjugate in HT29 model

Human colon cancer cell line HT29 was cultured at 37°C and 5% $CO_2$ in McCoy's 5a medium containing 10% fetal bovine serum. The HT29 cells in the exponential growth phase were collected, resuspended to an appropriate concentration by adding PBS and Matrigel at a final concentration of 50%, and inoculated subcutaneously in female Balb/c-nu mice to establish a human colon cancer xenograft model. When the average tumor volume was about 112 $mm^3$, the mice were randomly divided into vehicle control group (Vehicle) and ADC 19F6-A-1 10 mg/kg group of the present invention according to tumor size. After grouping, each group was administered by injection via tail vein (i.v.) on Day0, Day4, Day7, Day11, Day14 and Day18, i.e., administered 6 times in total. After administration, the body weight of the mice was measured and the long and short diameters of the tumors were measured with a vernier caliper twice a week, and the tumor volume was calculated according to the following formula: $V = 0.5\ a \times b^2$, wherein a and b represented the long and short diameters of the tumors, respectively. The animal death was observed and recorded every day.

The ADC 19F6-A-1 10 mg/kg of the present invention had a significant inhibitory effect on the tumor growth of the human colon cancer HT29 transplanted tumor model. The data of Day32 after administration showed that compared with the vehicle control group, the 19F6-A-1 10 mg/kg group had a TGI of 59.14%. In each treatment group, animal death, significant animal body weight loss, and obvious drug toxic reaction were not observed. During the treatment period, the mice had good tolerance to the ADC of the present invention. The specific results were shown in Table 12, Figure 6 and Figure 7.

Table 12. Human colon cancer cell HT29 CDX model

| Group | Dose (mg/kg) | Day32 | | | |
|---|---|---|---|---|---|
| | | Tumor volume ($mm^3$) ($\bar{x}\pm$ SEM) | TGI (%) | T/C (%) | P value (vs. Vehicle) |
| Vehicle | - | 3273.19 ± 315.24 | - | - | - |
| 19F6-A-1 | 10 | 1404.54±233.12 | 59.14 | 42.71 | <0.001 |
| Note: P value indicates the statistical difference of TGI between the treatment group and Vehicle. | | | | | |

Although the specific models of the present invention have been described in detail, those skilled in the art will understand that, according to all the teachings that have been disclosed, various modifications and substitutions can be made to those details, and these changes are all within the scope of the present invention. The full scope of the invention is given by the appended claims and any equivalents thereof.

## Claims

**1.** An antibody-drug conjugate, which has a structure represented by the formula Ab-[M-L-E-D]$_x$, wherein:
Ab represents an antibody or antigen-binding fragment thereof that specifically binds to receptor tyrosine kinase-like orphan receptor (ROR) family member 1 (ROR1):

M represents a linking site connecting the antibody or antigen-binding fragment thereof;
L represents a connector connecting the linking site M and E;
E represents a structural fragment connecting L and D;
D represents a cytotoxic drug moiety;

x is selected from 1 to 10.

**2.** The antibody-drug conjugate according to claim 1, wherein M is selected from the following structures:

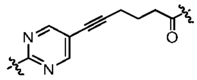 and 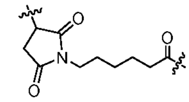.

**3.** The antibody-drug conjugate according to claim 1, wherein M is the following structure of

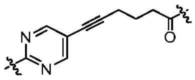.

**4.** The antibody-drug conjugate according to any one of claims 1-3, wherein L is a divalent structure composed of one or more of the following groups: $C_{1-6}$ alkylene, -N(R')-, carbonyl, -O-, Val, Cit, Phe, Lys, D-Val, Leu, Gly, Ala, Asn, Val-Cit, Val-Ala, Val-Lys, Val-Lys(Ac), Phe-Lys, Phe-Lys(Ac), D-Val-Leu-Lys, Gly-Gly-Arg, Ala-Ala-Asn, Ala-Ala-Ala, Val-Lys-Ala, Gly-Gly-Gly, Gly-Gly-Phe-Gly, Gly-Gly-Gly-Gly-Gly,

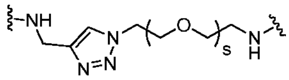, 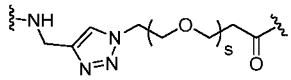,

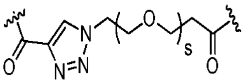, 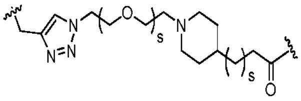, 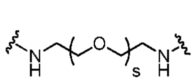, 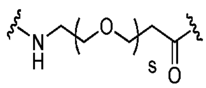, 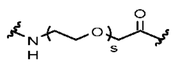,

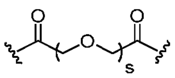, 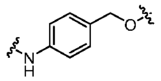, 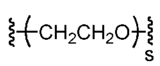, 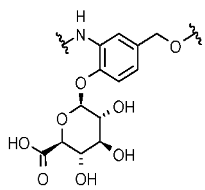 and 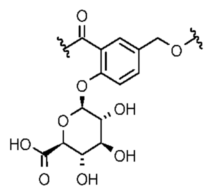,

wherein R' represents hydrogen, $C_{1-6}$ alkyl or a $-(CH_2CH_2O)_r-$-containing alkyl; r is an integer selected from 1-10; s is an integer selected from 1-20;

preferably, L is selected from the following structures:

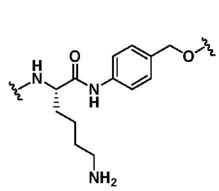, 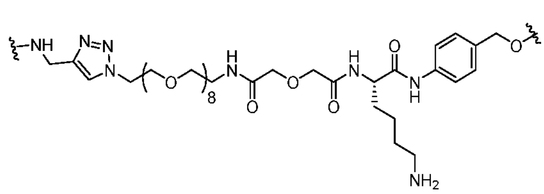, 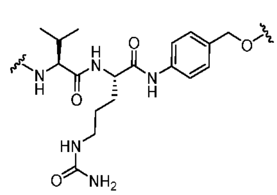,

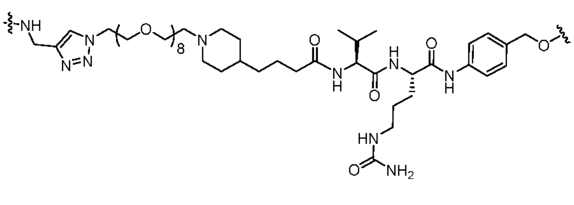, 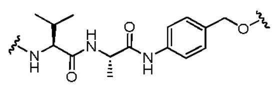,

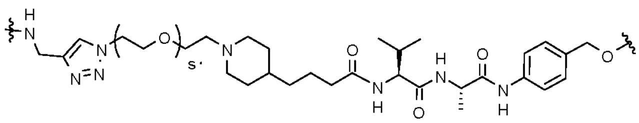, 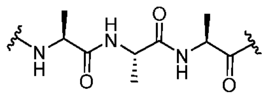, 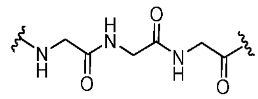,

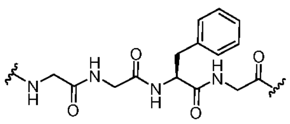, 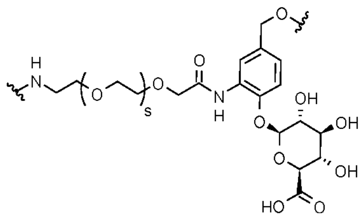 and 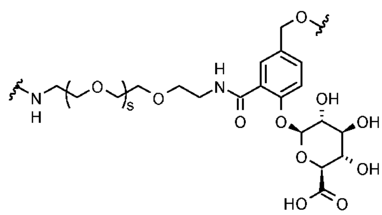;

wherein s is an integer selected from 1-20.

**5.** The antibody-drug conjugate according to any one of claims 1-4, wherein E is a single bond or $-NH-CH_2-$, or is selected from the following structures:

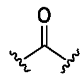, 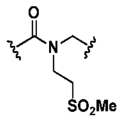, 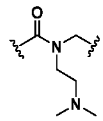, and 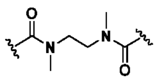;

preferably, E is a single bond, $-NH-CH_2-$, or

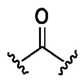.

**6.** The antibody-drug conjugate according to any one of claims 1-5, wherein

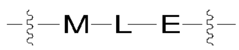

is selected from the following structures:

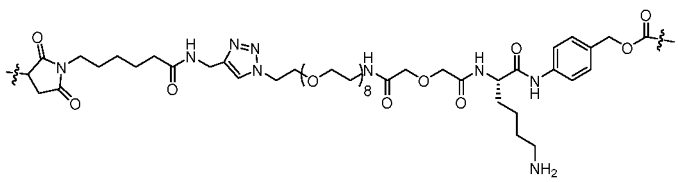,

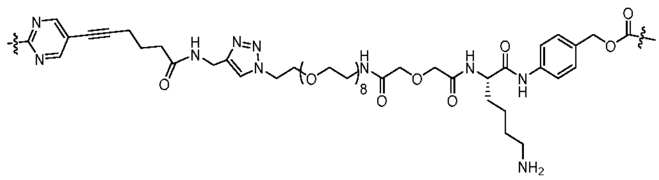,

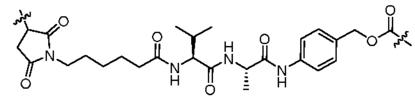,
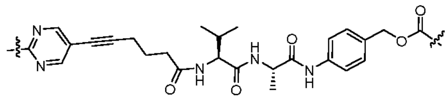,

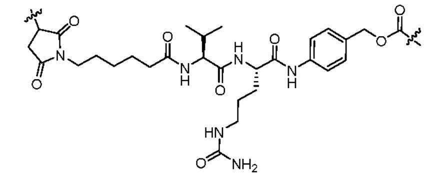,
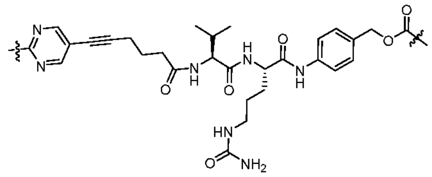,

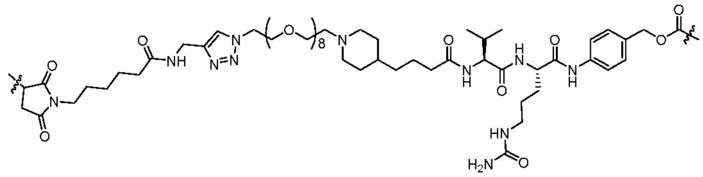,

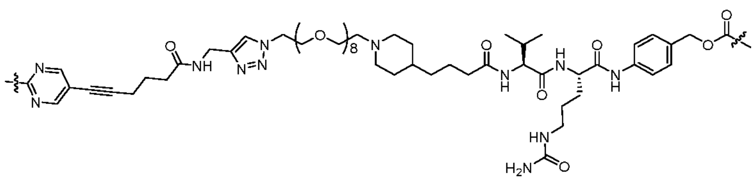,

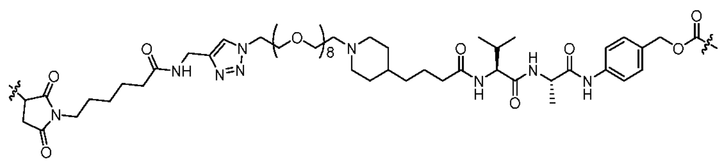,

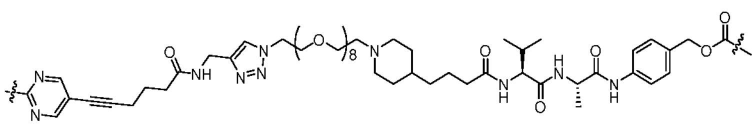,

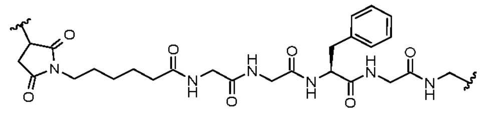,
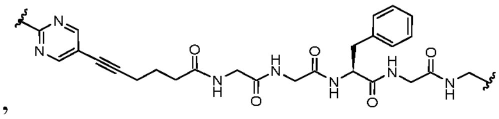,

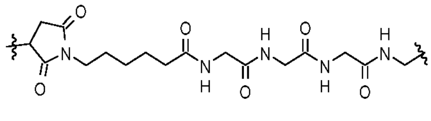,
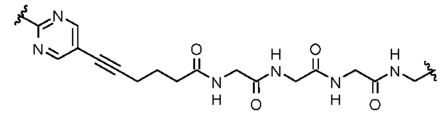,

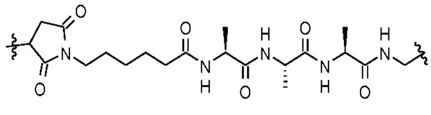,
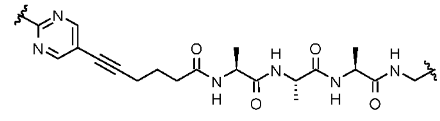,

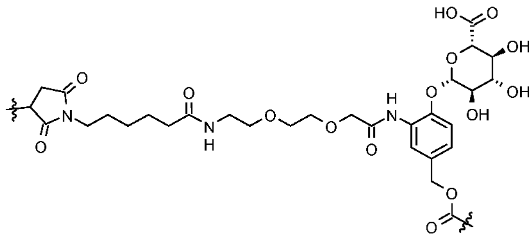
,
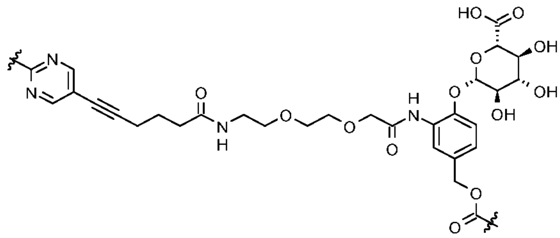
,

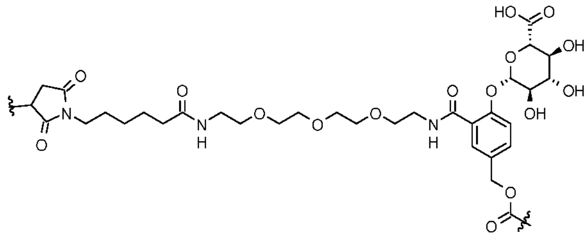

and

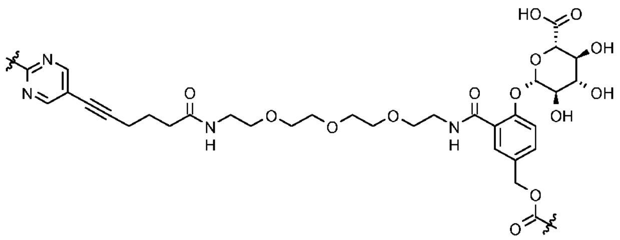
.

**7.** The antibody-drug conjugate according to any one of claims 1-6, wherein the cytotoxic drug is selected from the group consisting of a tubulin inhibitor, a DNA intercalator, a DNA topoisomerase inhibitor, and a RNA polymerase inhibitor;

preferably, the tubulin inhibitor is an auristatin compound or a maytansine compound; preferably, the DNA intercalator is pyrrolobenzodiazepine (PBD); preferably, the DNA topoisomerase inhibitor is a topoisomerase I inhibitor (e.g., camptothecin, hydroxycamptothecin, 9-aminocamptothecin, SN-38, irinotecan, topotecan, belotecan, or rubitecan) or a topoisomerase II inhibitor (e.g., doxorubicin, PNU-159682, duocarmycin, daunorubicin, mitoxantrone, podophyllotoxin, or etoposide); the RNA polymerase inhibitor is α-amanitin or a pharmaceutically acceptable salt, ester or analog thereof;
preferably, the cytotoxic drug is selected from the group consisting of the following compounds:

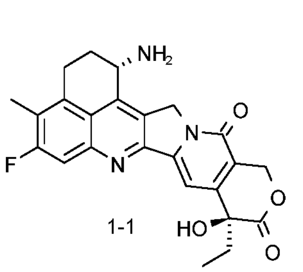
1-1

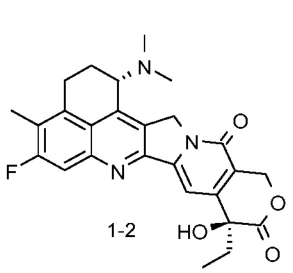
1-2

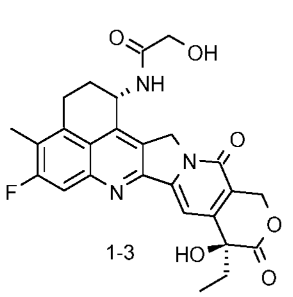
1-3

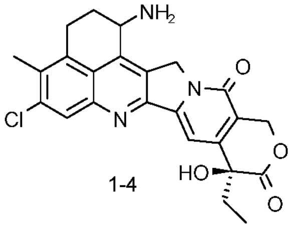
1-4

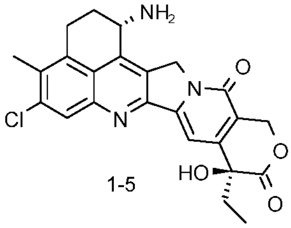
1-5

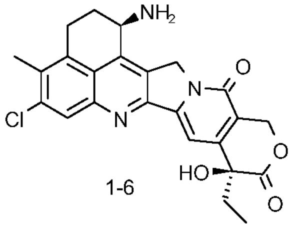
1-6

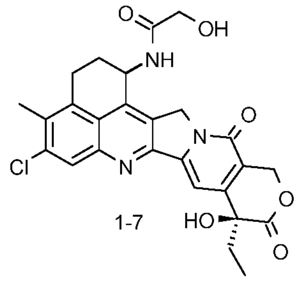
1-7

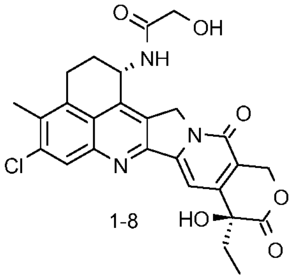
1-8

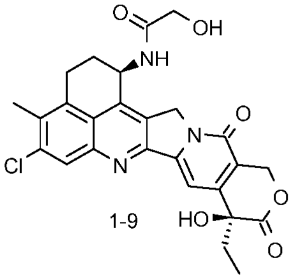
1-9

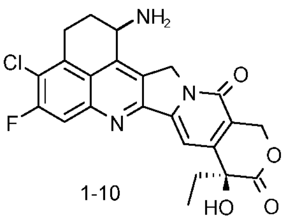
1-10

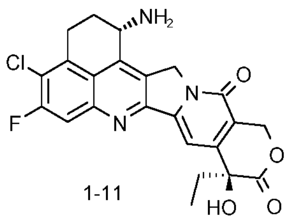
1-11

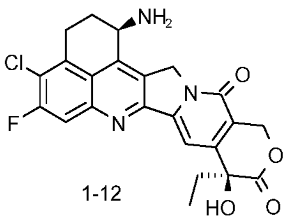
1-12

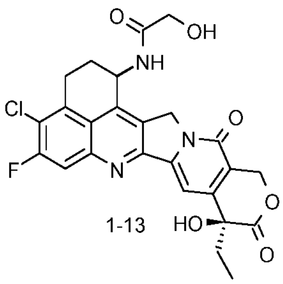
1-13

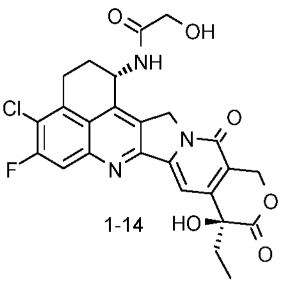
1-14

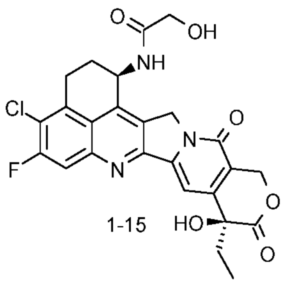
1-15

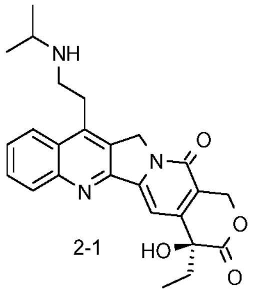
2-1

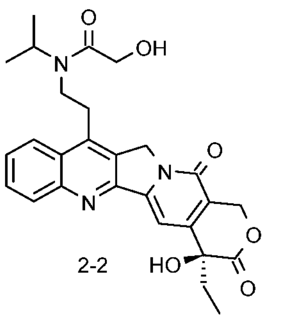
2-2

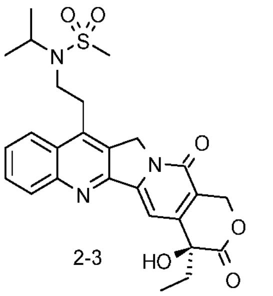
2-3

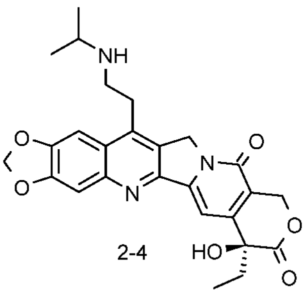
2-4

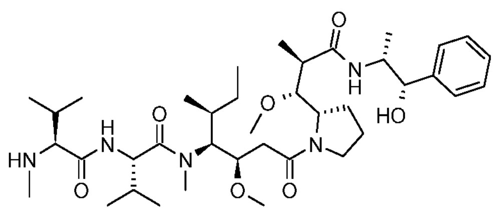
3-1

and

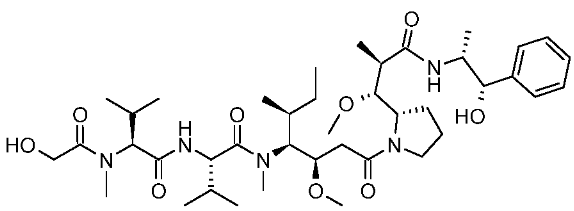
3-2

;

preferably, the cytotoxic drug is connected to E in the antibody-drug conjugate through -OH, primary amino group, secondary amino group or tertiary amino group thereon.

**8.** The antibody-drug conjugate according to any one of claims 1-7, wherein the antibody or antigen-binding fragment thereof comprises:

(1) the following heavy chain variable region (VH) and/or light chain variable region (VL), wherein the CDRs are defined by the Chothia numbering system:

(1a) a heavy chain variable region (VH) comprising the following 3 CDRs: CDR-H1 having a sequence as set forth in SEQ ID NO: 3 or a variant thereof, CDR-H2 having a sequence as set forth in SEQ ID NO: 4 or a variant thereof, CDR-H3 having a sequence as set forth in SEQ ID NO: 5 or a variant thereof; and/or, a light chain variable region (VL) comprising the following 3 CDRs: CDR-L1 having a sequence as set forth in SEQ ID NO: 6 or a variant thereof, CDR-L2 having a sequence as set forth in SEQ ID NO: 7 or a variant thereof, CDR-L3 having a sequence as set forth in SEQ ID NO: 8 or a variant thereof; or,
(1b) a heavy chain variable region (VH) comprising the following 3 CDRs: CDR-H1 having a sequence as set forth in SEQ ID NO: 11 or a variant thereof, CDR-H2 having a sequence as set forth in SEQ ID NO: 12 or a variant thereof, CDR-H3 having a sequence as set forth in SEQ ID NO: 13 or a variant thereof; and/or, a light chain variable region (VL) comprising the following 3 CDRs: CDR-L1 having a sequence as set forth in SEQ ID NO: 14 or a variant thereof, CDR-L2 having a sequence as set forth in SEQ ID NO: 15 or a variant thereof, CDR-L3 having a sequence as set forth in SEQ ID NO: 16 or a variant thereof; or,
(1c) a heavy chain variable region (VH) comprising the following 3 CDRs: CDR-H1 having a sequence as set forth in SEQ ID NO: 19 or a variant thereof, CDR-H2 having a sequence as set forth in SEQ ID NO: 20 or a variant thereof, CDR-H3 having a sequence as set forth in SEQ ID NO: 21 or a variant thereof; and/or, a light chain variable region (VL) comprising the following 3 CDRs: CDR-L1 having a sequence as set forth in SEQ ID NO: 14 or a variant thereof, CDR-L2 having a sequence as set forth in SEQ ID NO: 15 or a variant thereof, and CDR-L3 having a sequence as set forth in SEQ ID NO: 16 or a variant thereof;

wherein, the variant described in any one of items (1a), (1b) and (1c) has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletion or addition of one or several amino acids (e.g., a substitution, deletion or addition of 1, 2 or 3 amino acids) as compared to the sequence from which it is derived; preferably, the substitution is a conservative substitution;
or,
(2) the following heavy chain variable region (VH) and/or light chain variable region (VL), wherein the CDRs are defined by the AbM numbering system:

(2a) a heavy chain variable region (VH) comprising the following 3 CDRs: CDR-H1 having a sequence as set forth in SEQ ID NO: 29 or a variant thereof, CDR-H2 having a sequence as set forth in SEQ ID NO: 30 or a variant thereof, CDR-H3 having a sequence as set forth in SEQ ID NO: 5 or a variant thereof; and/or, a light chain variable region (VL) comprising the following 3 CDRs: CDR-L1 having a sequence as set forth in SEQ ID NO: 6 or a variant thereof, CDR-L2 having a sequence as set forth in SEQ ID NO: 7 or a variant thereof, CDR-L3 having a sequence as set forth in SEQ ID NO: 8 or a variant thereof; or,
(2b) a heavy chain variable region (VH) comprising the following 3 CDRs: CDR-H1 having a sequence as set forth in SEQ ID NO: 36 or a variant thereof, CDR-H2 having a sequence as set forth in SEQ ID NO: 37 or a variant thereof, CDR-H3 having a sequence as set forth in SEQ ID NO: 13 or a variant thereof; and/or, a light chain variable region (VL) comprising the following 3 CDRs: CDR-L1 having a sequence as set forth in SEQ ID NO: 14 or a variant thereof, CDR-L2 having a sequence as set forth in SEQ ID NO: 15 or a variant thereof, CDR-L3 having a sequence as set forth in SEQ ID NO: 16 or a variant thereof; or,
(2c) a heavy chain variable region (VH) comprising the following 3 CDRs: CDR-H1 having a sequence as set forth in SEQ ID NO: 45 or a variant thereof, CDR-H2 having a sequence as set forth in SEQ ID NO: 46 or a variant thereof, CDR-H3 having a sequence as set forth in SEQ ID NO: 21 or a variant thereof; and/or, a light chain variable region (VL) comprising the following 3 CDRs: CDR-L1 having a sequence as set forth in SEQ ID NO: 14 or a variant thereof, CDR-L2 having a sequence as set forth in SEQ ID NO: 15 or a variant thereof, and CDR-L3 having a sequence as set forth in SEQ ID NO: 16 or a variant thereof;

wherein, the variant described in any one of items (2a), (2b) and (2c) has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletion or addition of one or several amino acids (e.g., a substitution,

deletion or addition of 1, 2 or 3 amino acids) as compared to the sequence from which it is derived; preferably, the substitution is a conservative substitution;
or,
(3) the following heavy chain variable region (VH) and/or light chain variable region (VL), wherein the CDRs are defined according to the Kabat numbering system:

(3a) a heavy chain variable region (VH) comprising the following 3 CDRs: CDR-H1 having a sequence as set forth in SEQ ID NO: 31 or a variant thereof, CDR-H2 having a sequence as set forth in SEQ ID NO: 32 or a variant thereof, CDR-H3 having a sequence as set forth in SEQ ID NO: 5 or a variant thereof; and/or, a light chain variable region (VL) comprising the following 3 CDRs: CDR-L1 having a sequence as set forth in SEQ ID NO: 6 or a variant thereof, CDR-L2 having a sequence as set forth in SEQ ID NO: 7 or a variant thereof, CDR-L3 having a sequence as set forth in SEQ ID NO: 8 or a variant thereof; or,
(3b) a heavy chain variable region (VH) comprising the following 3 CDRs: CDR-H1 having a sequence as set forth in SEQ ID NO: 38 or a variant thereof, CDR-H2 having a sequence as set forth in SEQ ID NO: 39 or a variant thereof, CDR-H3 having a sequence as set forth in SEQ ID NO: 13 or a variant thereof; and/or, a light chain variable region (VL) comprising the following 3 CDRs: CDR-L1 having a sequence as set forth in SEQ ID NO: 14 or a variant thereof, CDR-L2 having a sequence as set forth in SEQ ID NO: 15 or a variant thereof, CDR-L3 having a sequence as set forth in SEQ ID NO: 16 or a variant thereof; or,
(3c) a heavy chain variable region (VH) comprising the following 3 CDRs: CDR-H1 having a sequence as set forth in SEQ ID NO: 47 or a variant thereof, CDR-H2 having a sequence as set forth in SEQ ID NO: 48 or a variant thereof, CDR-H3 having a sequence as set forth in SEQ ID NO: 21 or a variant thereof; and/or, a light chain variable region (VL) comprising the following 3 CDRs: CDR-L1 having a sequence as set forth in SEQ ID NO: 14 or a variant thereof, CDR-L2 having a sequence as set forth in SEQ ID NO: 15 or a variant thereof, and CDR-L3 having a sequence as set forth in SEQ ID NO: 16 or a variant thereof;

wherein, the variant described in any one of items (3a), (3b) and (3c) has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletion or addition of one or several amino acids (e.g., a substitution, deletion or addition of 1, 2 or 3 amino acids) as compared to the sequence from which it is derived; preferably, the substitution is a conservative substitution;
or,
(4) the following heavy chain variable region (VH) and/or light chain variable region (VL), wherein the CDRs are defined by the IMGT numbering system:

(4a) a heavy chain variable region (VH) comprising the following 3 CDRs: CDR-H1 having a sequence as set forth in SEQ ID NO: 24 or a variant thereof, CDR-H2 having a sequence as set forth in SEQ ID NO: 25 or a variant thereof, CDR-H3 having a sequence as set forth in SEQ ID NO: 26 or a variant thereof; and/or, a light chain variable region (VL) comprising the following 3 CDRs: CDR-L1 having a sequence as set forth in SEQ ID NO: 27 or a variant thereof, CDR-L2 having a sequence as set forth in SEQ ID NO: 28 or a variant thereof, CDR-L3 having a sequence as set forth in SEQ ID NO: 8 or a variant thereof; or,
(4b) a heavy chain variable region (VH) comprising the following 3 CDRs: CDR-H1 having a sequence as set forth in SEQ ID NO: 33 or a variant thereof, CDR-H2 having a sequence as set forth in SEQ ID NO: 34 or a variant thereof, CDR-H3 having a sequence as set forth in SEQ ID NO: 35 or a variant thereof; and/or, a light chain variable region (VL) comprising the following 3 CDRs: CDR-L1 having a sequence as set forth in SEQ ID NO: 43 or a variant thereof, CDR-L2 having a sequence as set forth in SEQ ID NO: 44 or a variant thereof, CDR-L3 having a sequence as set forth in SEQ ID NO: 16 or a variant thereof; or,
(4c) a heavy chain variable region (VH) comprising the following 3 CDRs: CDR-H1 having a sequence as set forth in SEQ ID NO: 40 or a variant thereof, CDR-H2 having a sequence as set forth in SEQ ID NO: 41 or a variant thereof, CDR-H3 having a sequence as set forth in SEQ ID NO: 42 or a variant thereof; and/or, a light chain variable region (VL) comprising the following 3 CDRs: CDR-L1 having a sequence as set forth in SEQ ID NO: 43 or a variant thereof, CDR-L2 having a sequence as set forth in SEQ ID NO: 44 or a variant thereof, and CDR-L3 having a sequence as set forth in SEQ ID NO: 16 or a variant thereof;

Wherein, the variant described in any one of items (4a), (4b) of (4c) has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletion or addition of one or several amino acids (e.g., a substitution,

deletion or addition of 1, 2 or 3 amino acids) as compared to the sequence from which it is derived; preferably, the substitution is a conservative substitution.

**9.** The antibody-drug conjugate according to claim 1 or 8, wherein the antibody or antigen-binding fragment thereof comprises:

(a) a VH as set forth in SEQ ID NO: 1 or a variant thereof, and/or, a VL as set forth in SEQ ID NO: 2 or a variant thereof;
(b) a VH as set forth in SEQ ID NO: 9 or a variant thereof, and/or, a VL as set forth in SEQ ID NO: 10 or a variant thereof; or
(c) a VH as set forth in SEQ ID NO: 17 or a variant thereof, and/or, a VL as set forth in SEQ ID NO: 18 or a variant thereof;

wherein the variant has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or the variant has a substitution, deletion or addition of one or several amino acids (e.g., a substitution, deletion or addition of 1, 2, 3, 4 or 5 amino acids) as compared to the sequence from which it is derived; preferably, the substitution is a conservative substitution.

**10.** The antibody-drug conjugate according to claim 8 or 9, wherein the antibody or antigen-binding fragment thereof further comprises:

(a) a human immunoglobulin heavy chain constant region (CH) or a variant thereof, the variant has a substitution, deletion or addition of one or more amino acids (e.g., a substitution, deletion or addition of up to 20, up to 15, up to 10, or up to 5 amino acids; for example, a substitution, deletion or addition of 1, 2, 3, 4 or 5 amino acids) as compared to the wild-type sequence from which it is derived; and
(b) a human immunoglobulin light chain constant region (CL) or a variant thereof, the variant has a substitution, deletion or addition of one or more amino acids (e.g., a substitution, deletion or addition of up to 20, up to 15, up to 10, or up to 5 amino acids; for example, a substitution, deletion or addition of 1, 2, 3, 4 or 5 amino acids) as compared to the wild-type sequence from which it is derived;
preferably, the heavy chain constant region is an IgG heavy chain constant region, such as an IgG1, IgG2, IgG3 or IgG4 heavy chain constant region, such as a human IgG1 heavy chain constant region or a human IgG4 heavy chain constant region;
preferably, the antibody or antigen-binding fragment thereof comprises a heavy chain constant region (CH) as set forth in SEQ ID NO: 22 or a variant thereof, the variant has a conservative substitution of one or more amino acids (e.g., a conservative substitution of up to 20, up to 15, up to 10, or up to 5 amino acids; for example, a conservative substitution of 1, 2, 3, 4 or 5 amino acids) as compared to SEQ ID NO: 22;
preferably, the light chain constant region is a κ light chain constant region;
preferably, the antibody or antigen-binding fragment thereof comprises a light chain constant region (CL) as set forth in SEQ ID NO: 23 or a variant thereof, the variant has a conservative substitution of one or more amino acids (e.g., a conservative substitution of up to 20, up to 15, up to 10, or up to 5 amino acids; for example, a conservative substitution of 1, 2, 3, 4 or 5 amino acids) as compared to SEQ ID NO: 23;
preferably, the antibody or antigen-binding fragment thereof comprises a heavy chain constant region (CH) as set forth in SEQ ID NO:22 and a light chain constant region (CL) as set forth in SEQ ID NO:23.

**11.** The antibody-drug conjugate according to any one of claims 1 and 8-10, wherein the antibody or antigen-binding fragment thereof comprises:

(1) a heavy chain comprising a VH having a sequence as set forth in SEQ ID NO: 1 and a heavy chain constant region (CH) as set forth in SEQ ID NO: 22, and, a light chain comprising a VL having a sequence as set forth in SEQ ID NO: 2 and a light chain constant region (CL) as set forth in SEQ ID NO: 23;
(2) a heavy chain comprising a VH having a sequence as set forth in SEQ ID NO: 9 and a heavy chain constant region (CH) as set forth in SEQ ID NO: 22, and, a light chain comprising a VL having a sequence as set forth in SEQ ID NO: 10 and a light chain constant region (CL) as set forth in SEQ ID NO: 23; or
(3) a heavy chain comprising a VH having a sequence as set forth in SEQ ID NO: 17 and a heavy chain constant region (CH) as set forth in SEQ ID NO: 22, and, a light chain comprising a VL having a sequence as set forth in SEQ ID NO: 18 and a light chain constant region (CL) as set forth in SEQ ID NO: 23.

**12.** The antibody-drug conjugate according to any one of claims 1-11, M is linked to a sulfhydryl group (-SH) or amino group ($-NH_2$) on the Ab.

**13.** The antibody-drug conjugate according to any one of claims 1-12, wherein the antibody or antigen-binding fragment thereof is selected from the antibody or antigen-binding fragment thereof according to claim 11; -M-L-E-D is selected from the structure as defined in any one of claims 1-7; x is 1 to 10; preferably, x is 1 to 8, or x is 1 to 4.

**14.** The antibody-drug conjugate according to any one of claims 1 to 13, which is selected from the group consisting of:

ADC A-1:

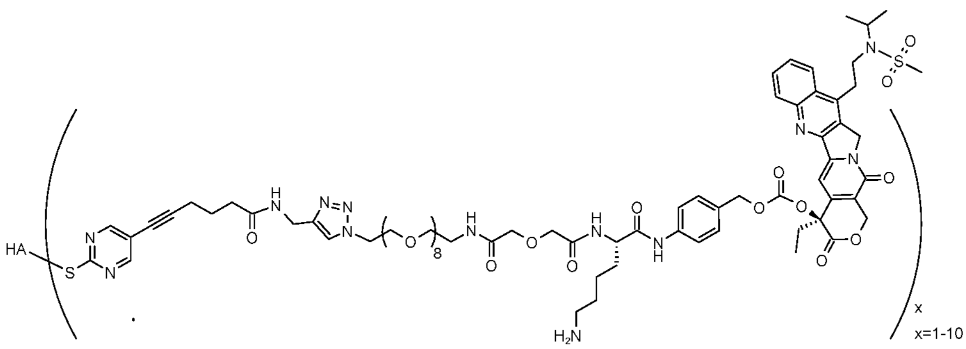

,

ADC A-2:

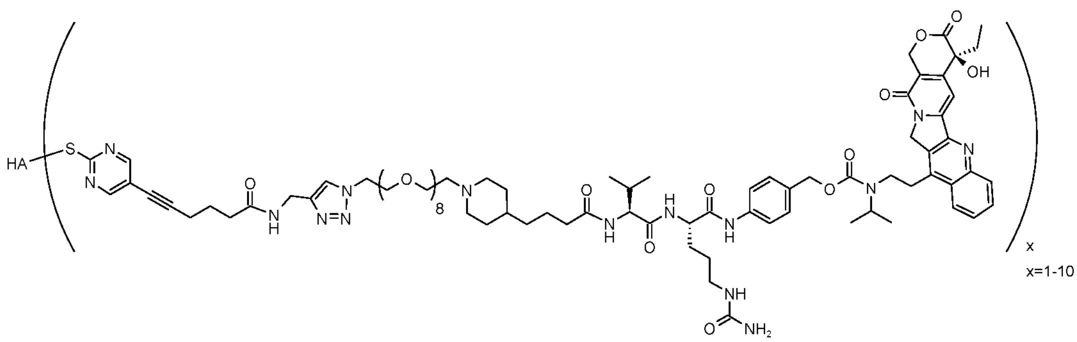

,

ADC A-3:

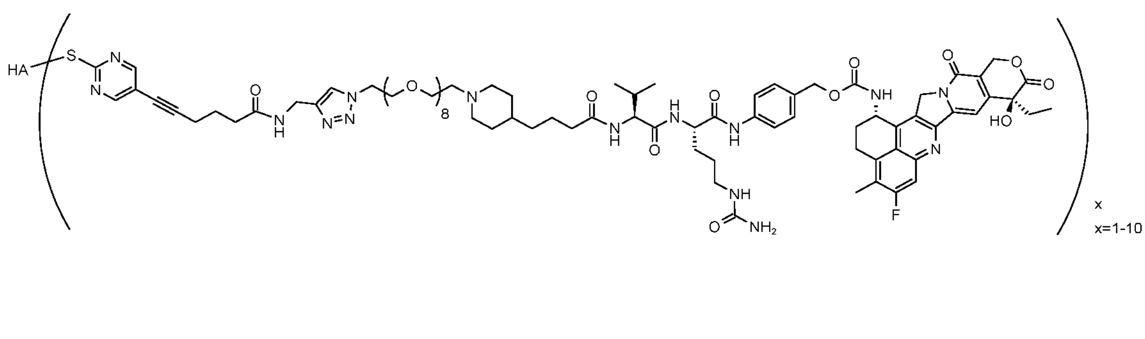

,

ADC A-4:

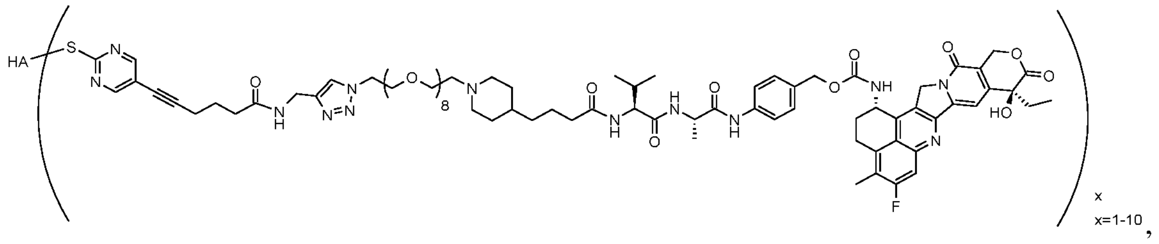

ADC A-5:

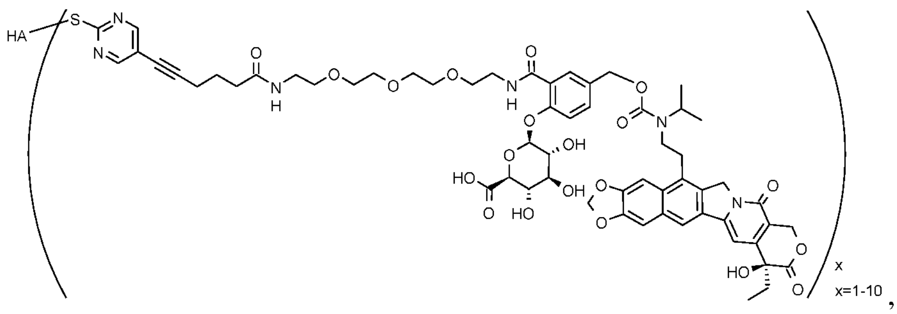

ADC A-6:

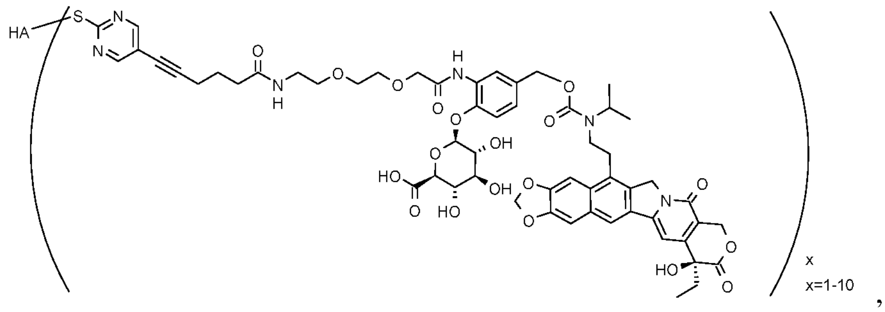

ADC A-7:

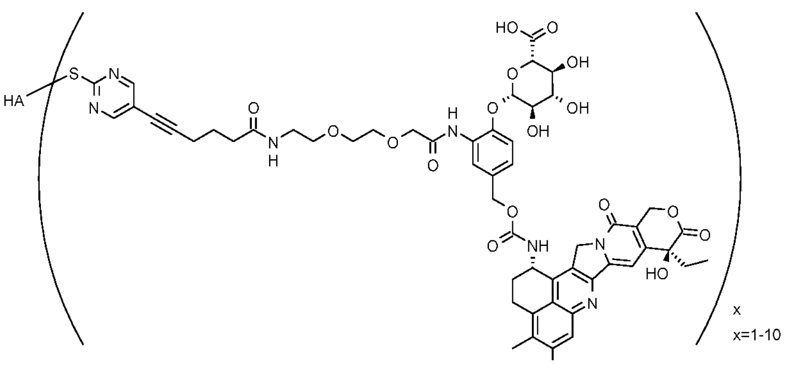

ADC A-8:

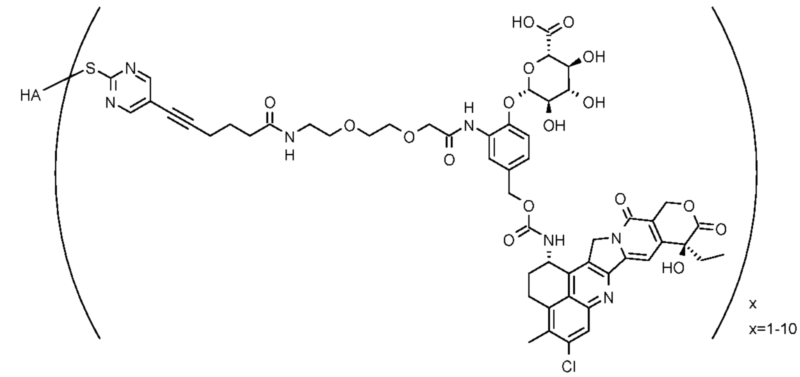

,

ADC A-9:

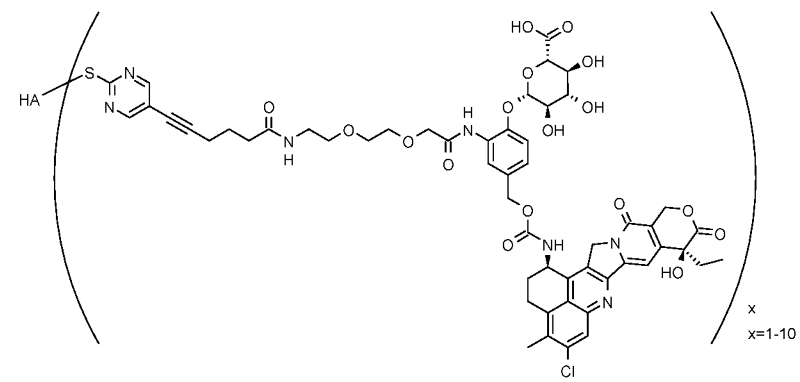

,

ADC A-10:

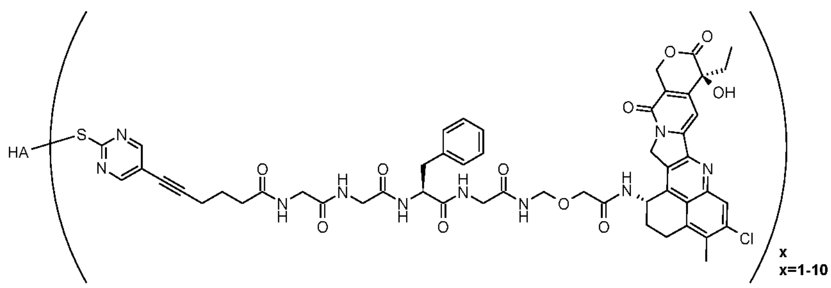

,

ADC A-11:

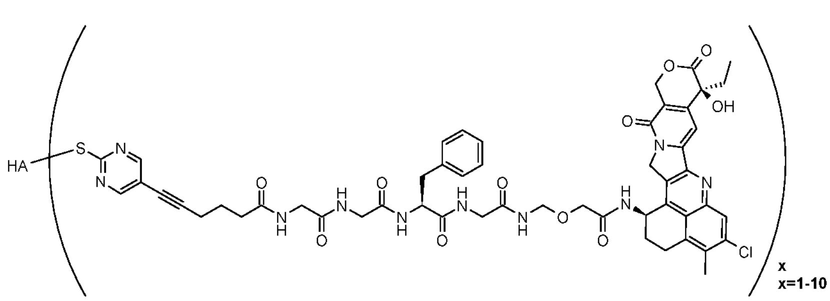

,

ADC A-12:

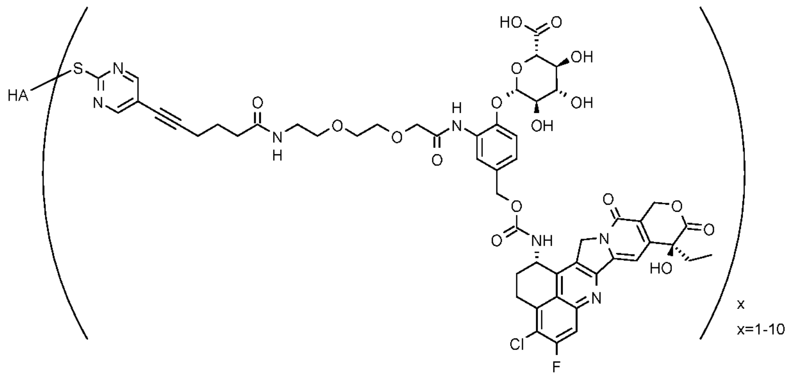

,

ADC A-13:

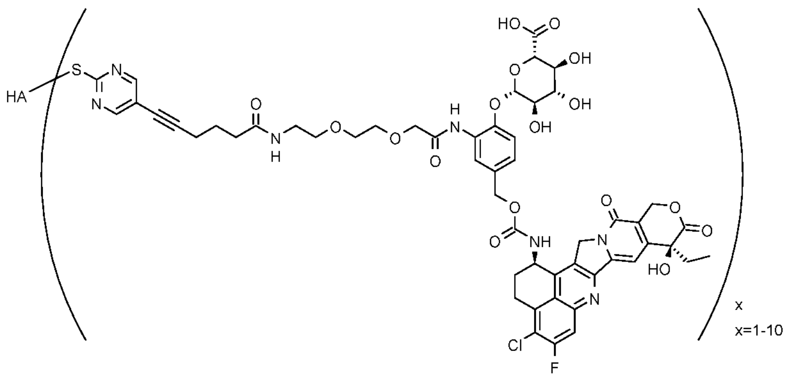

,

ADC A-14:

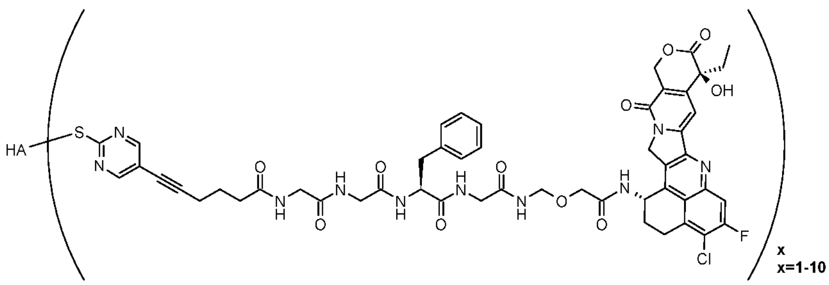

,

ADC A-15:

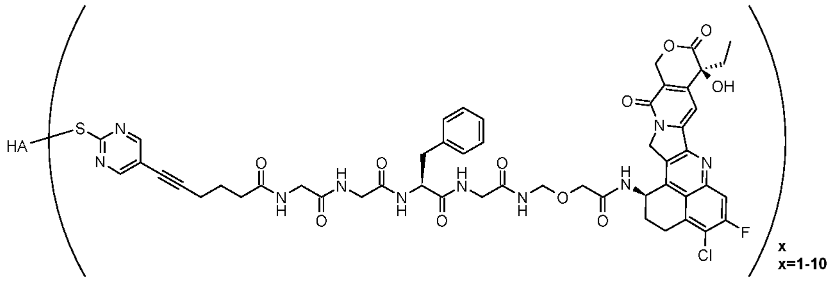

,

ADC A-16:

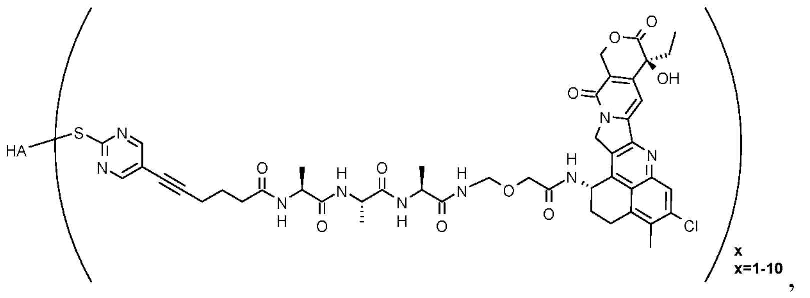

ADC A-17:

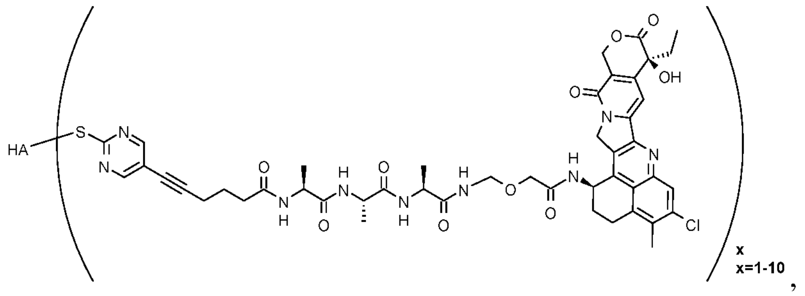

ADC A-18:

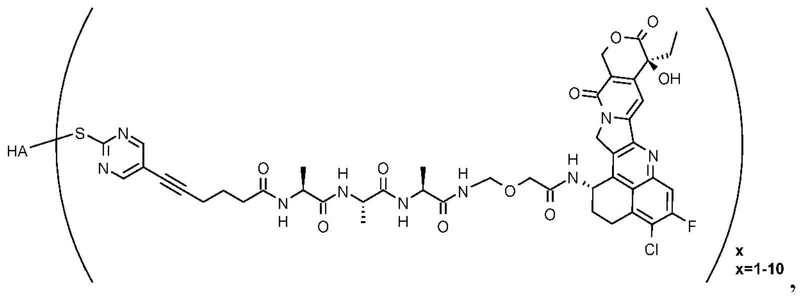

ADC A-19:

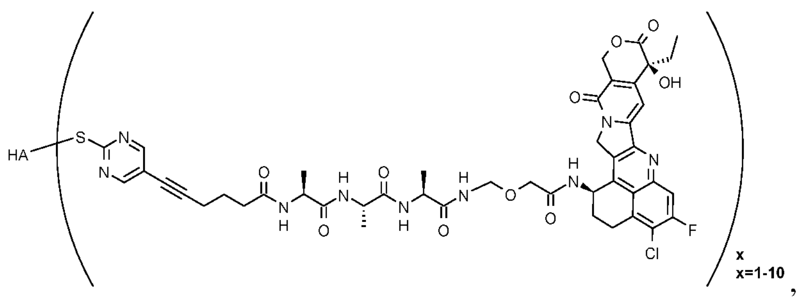

ADC A-20:

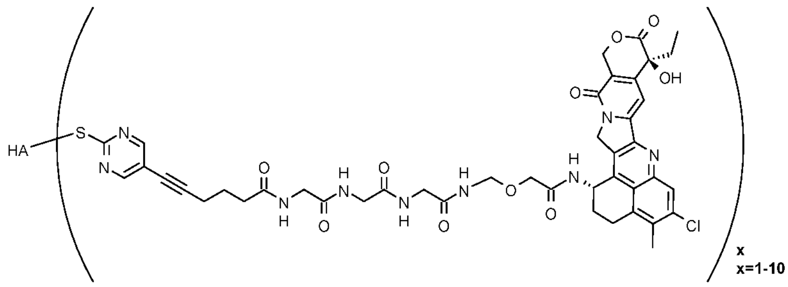

,

ADC A-21:

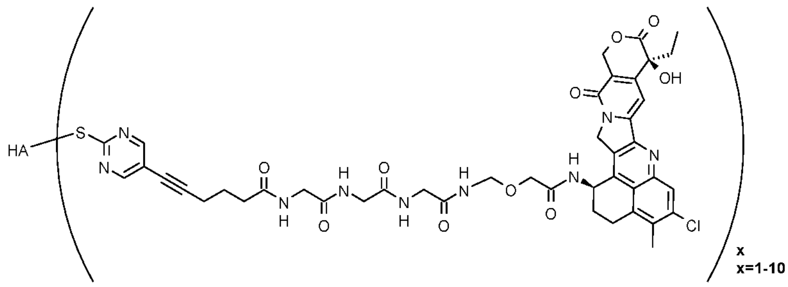

,

ADC A-22:

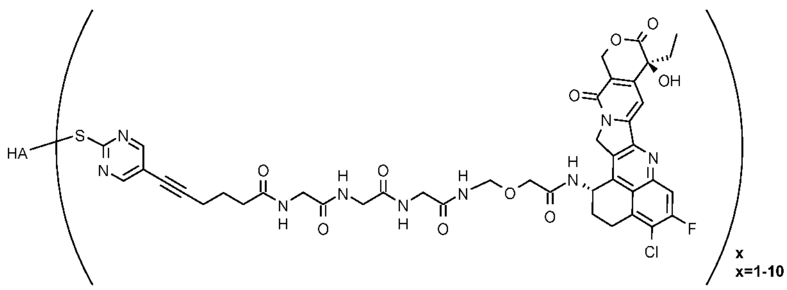

,

ADC A-23:

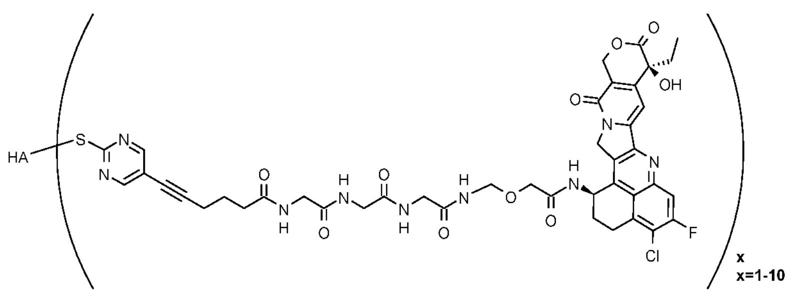

,

ADC A-24:

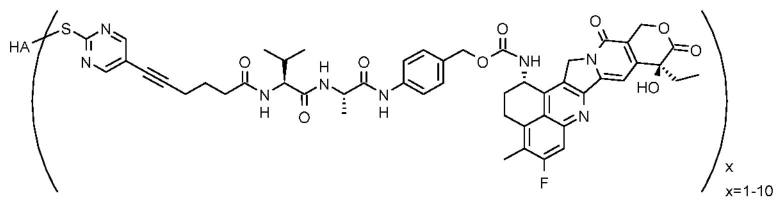
,

ADC A-25:

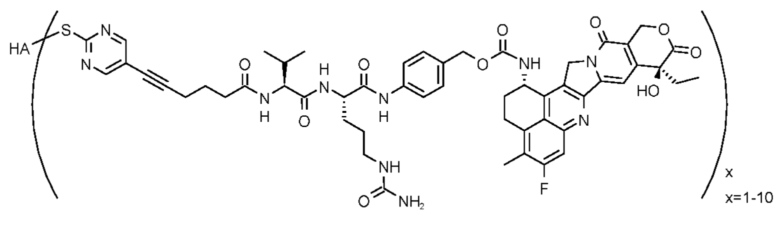
,

ADC A-26:

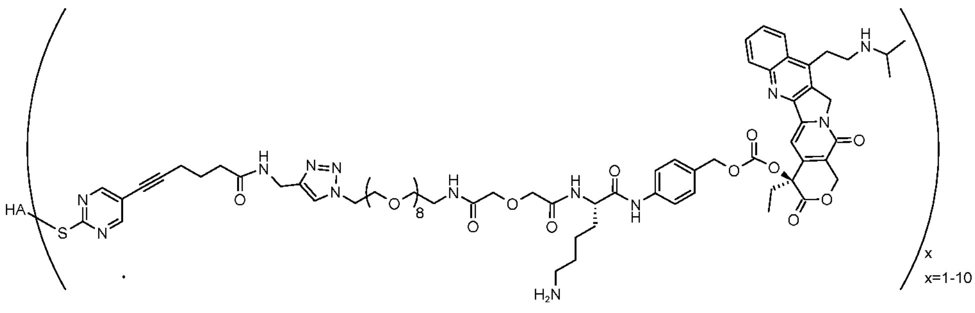
,

ADC A-27:

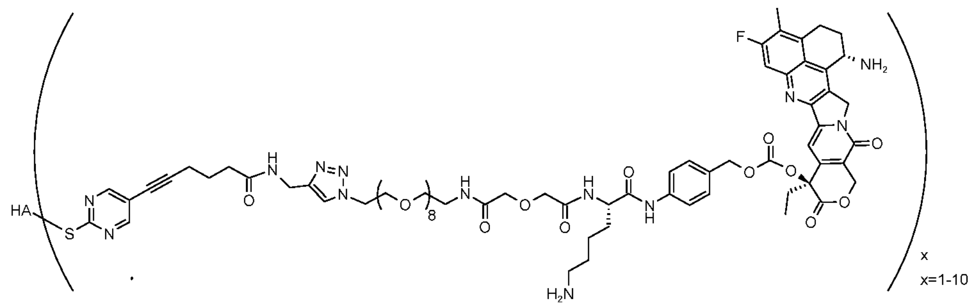
,

ADC A-28:

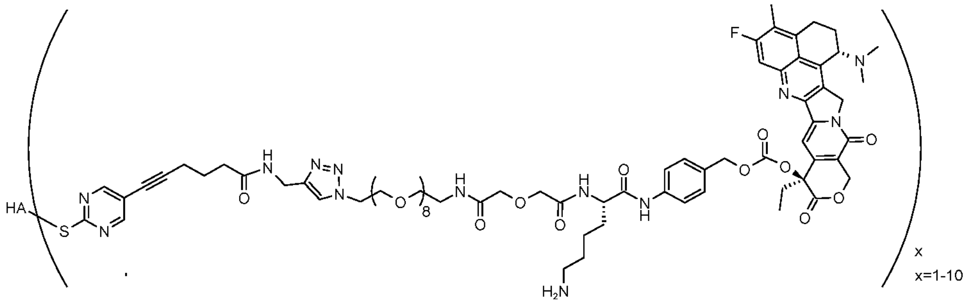

,

ADC A-29:

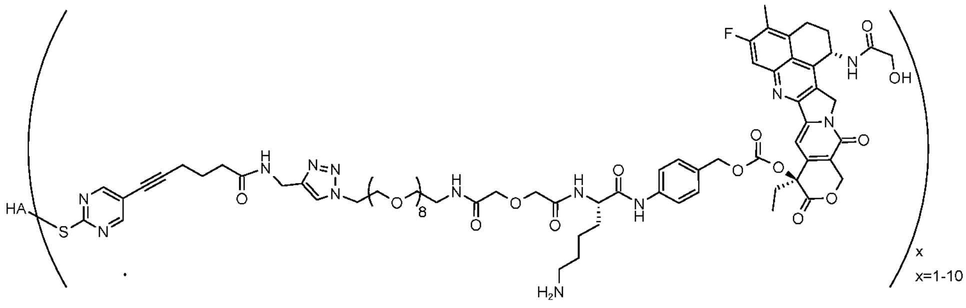

,

ADC A-30:

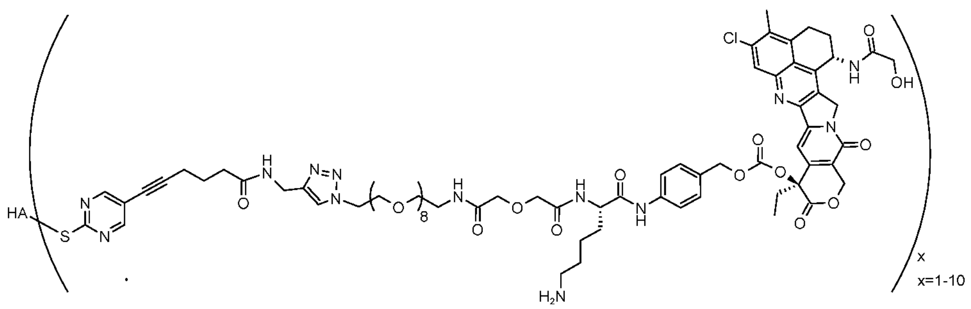

,

ADC A-31:

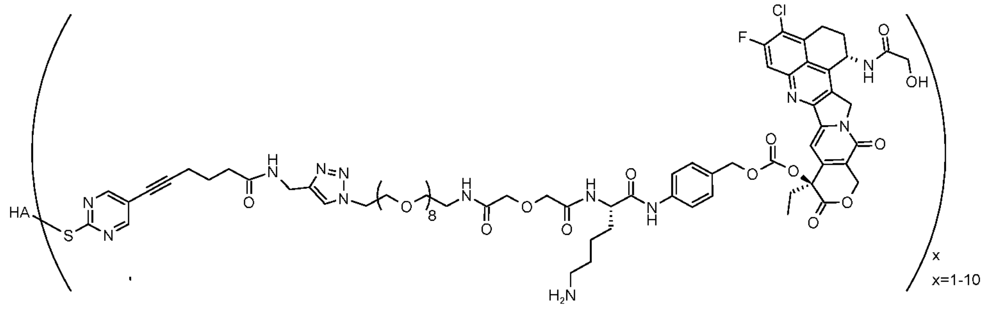

,

ADC B-1:

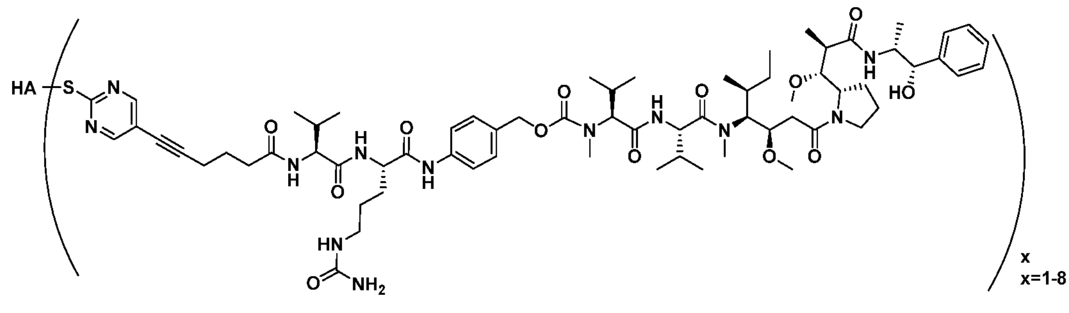

,

ADC B-2:

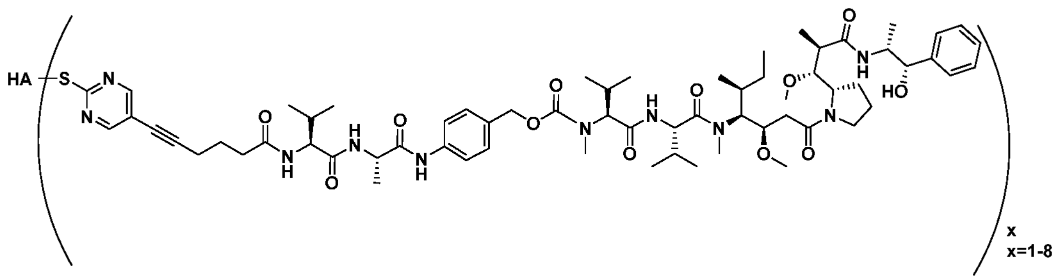

,

ADC B-3:

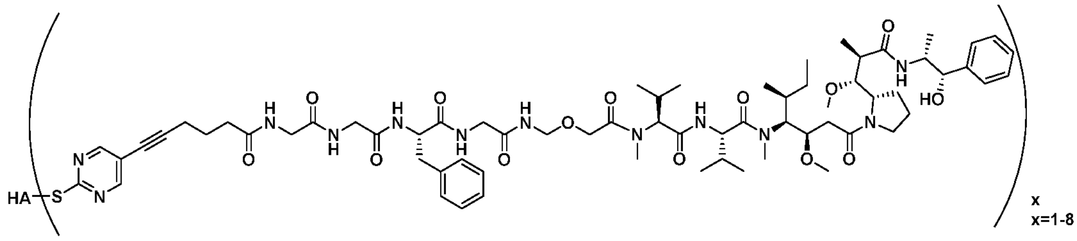

,

wherein, HA in each antibody-drug conjugate represents an antibody or antigen-binding fragment thereof comprising a VH as set forth in SEQ ID NO: 1 and a VL as set forth in SEQ ID NO: 2, for example, 19F6_Hu35v1 (which comprises a heavy chain comprising a VH as set forth in SEQ ID NO: 1 and a CH as set forth in SEQ ID NO: 22, and a light chain comprising a VL as set forth in SEQ ID NO: 2 and a CL as set forth in SEQ ID NO: 23); wherein,

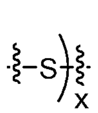

represents the specific connection mode between a sulfhydryl group of the antibody or antigen-binding fragment thereof and the connector.

**15.** The antibody-drug conjugate according to any one of claims 1-14, which has a DAR value (drug-to-antibody ratio) of 1-10, for example: 1 to 2, 1 to 3, 1 to 4, 1 to 5, 1 to 6, 1 to 7, 1 to 8, 1 to 9, 1 to 10, 2 to 3, 2 to 4, 2 to 5, 2 to 6, 2 to 7, 2 to 8, 2 to 9, 2 to 10, 3 to 4, 3 to 5, 3 to 6, 3 to 7, 3 to 8, 3 to 9, 3 to 10, 4 to 5, 4 to 6, 4 to 7, 4 to 8, 4 to 9, 4 to 10, 5 to 6, 5 to 7, 5 to 8, 5 to 9, 5 to 10, 6 to 7, 6 to 8, 6 to 9, 6 to 10, 7 to 8, 7 to 9, 7 to 10, 8 to 9, 8 to 10, or 9 to 10, preferably 3 to 8, such as 3.0 to 3.5, 3.0 to 4.0, 3.0 to 4.5, 3.0 to 5.0, 6.0 to 6.5, 6.0 to 7.0, 6.0 to 7.5, 6.0 to 8.0, 6.0 to 8.5, 6.5 to 7.0, 6.5 to 7.5, 6.5 to 8.0, 6.5 to 8.5, 7.0 to 7.5, 7.0 to 8.0 or 7.5 to 8.0.

**16.** A drug-linker, which has a structure represented by the formula M'-L-E-D, wherein:

M' represents a structure of M before it is connected to the antibody or antigen-binding fragment thereof according to any one of claims 8 to 11, and M, L, E and D are as defined in any one of claims 1 to 7; preferably, M' is selected from the following structures:

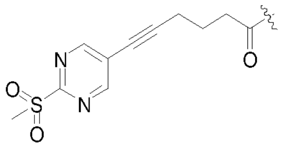

and

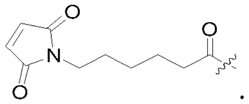

.

**17.** The drug-linker according to claim 16, which is selected from the group consisting of:

A-1: 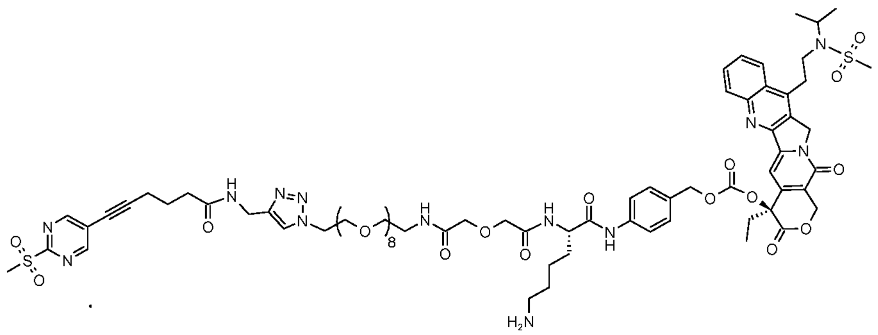

,

A-2: 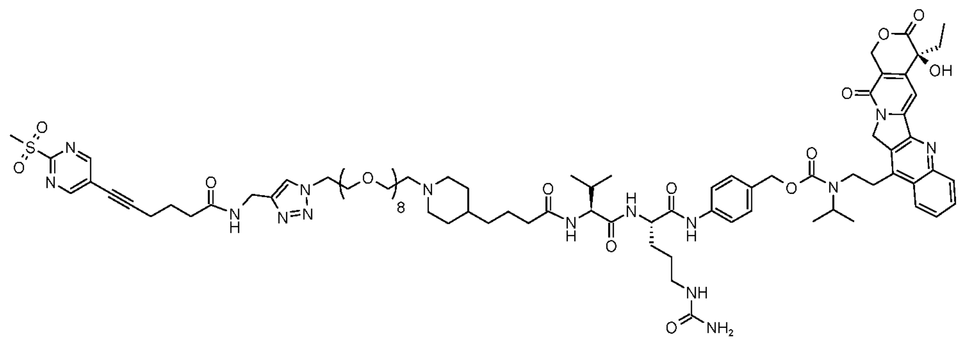

,

A-3: 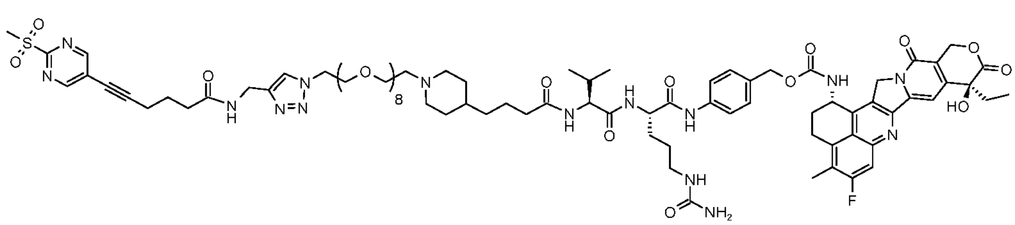

,

A-4: 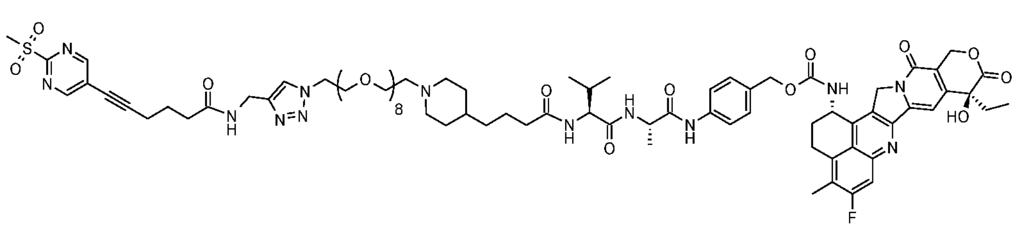

,

A-5: 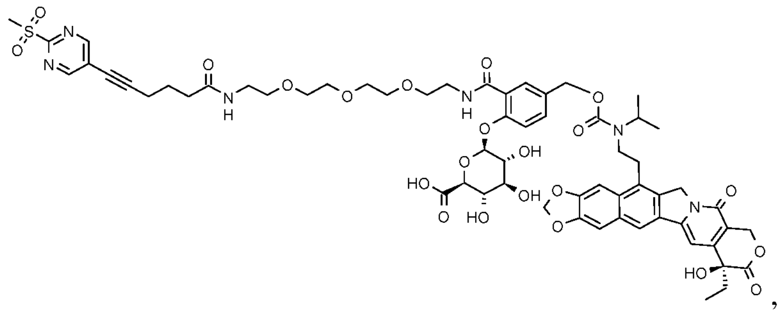 ,

A-6: 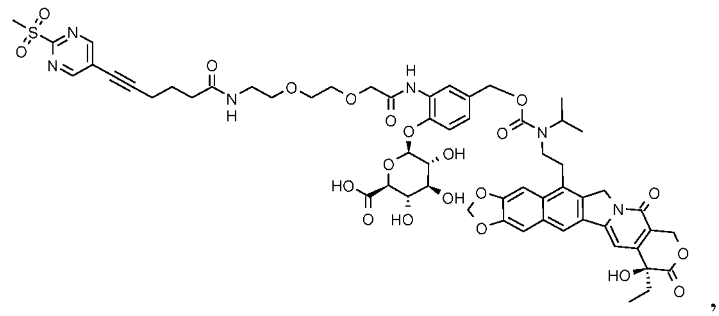 ,

A-7: 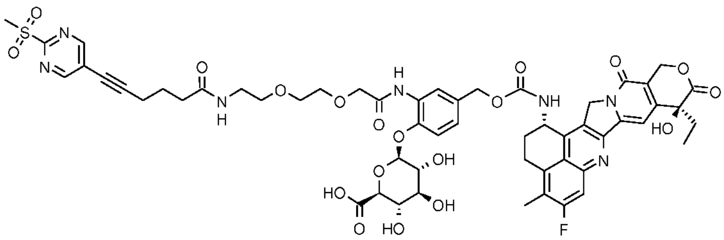 ,

A-8: 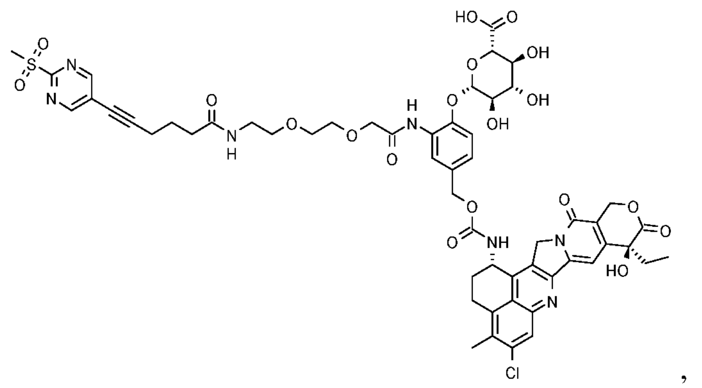 ,

A-9: 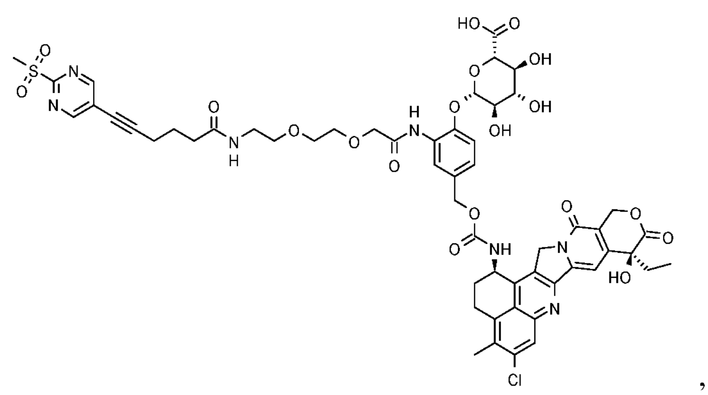 ,

A-10: 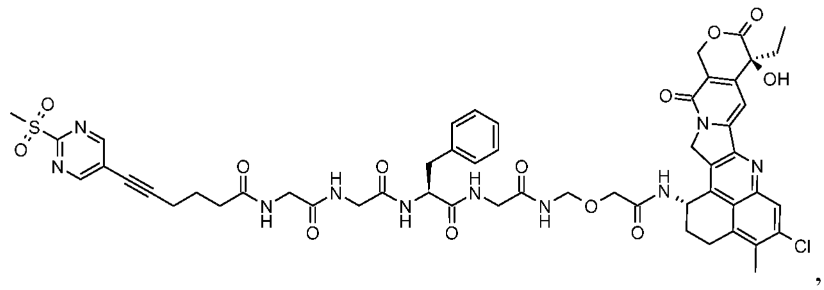 ,

A-11: 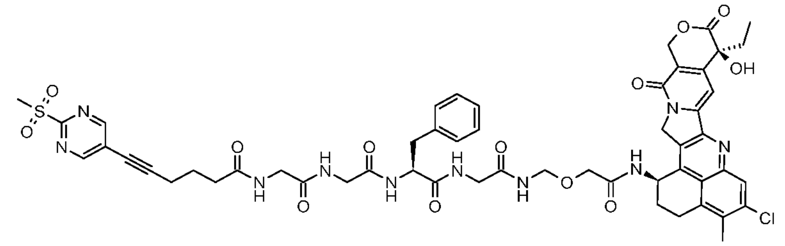 ,

A-12: 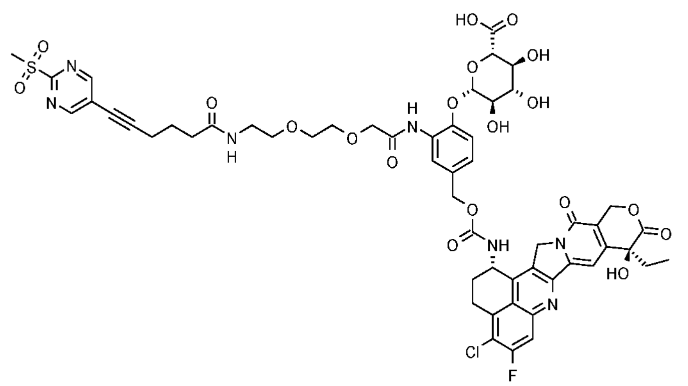 ,

A-13: 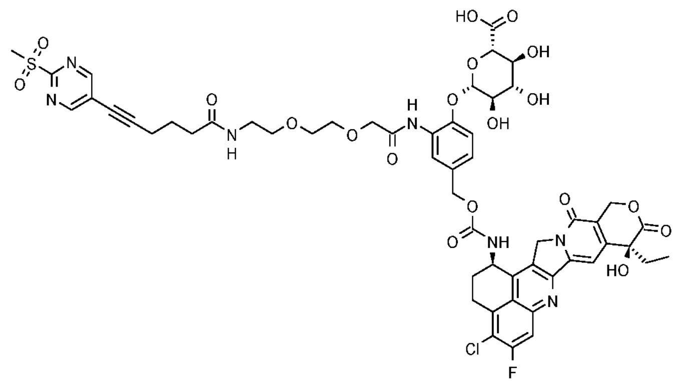 ,

A-14: 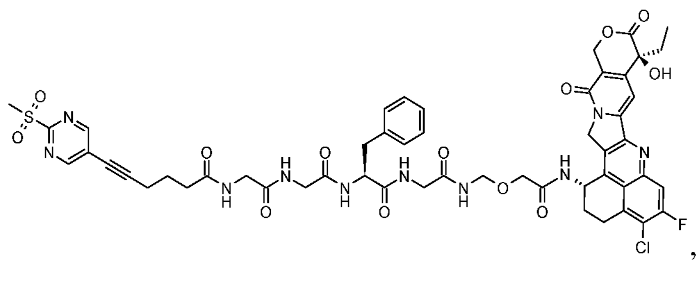 ,

A-15: 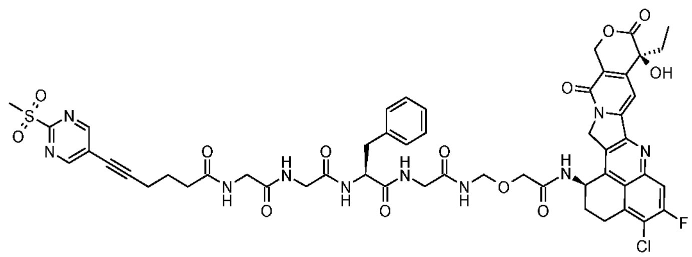 ,

A-16: 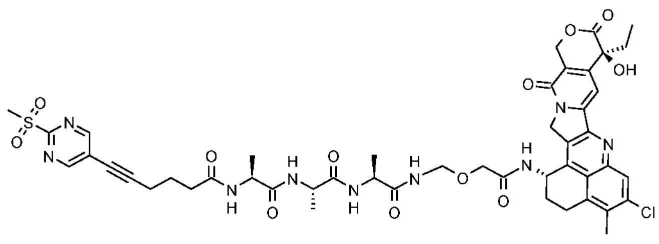 ,

A-17: 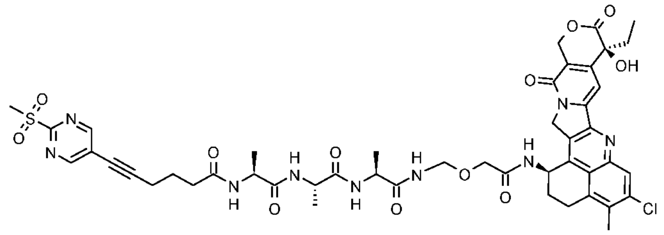 ,

A-18: 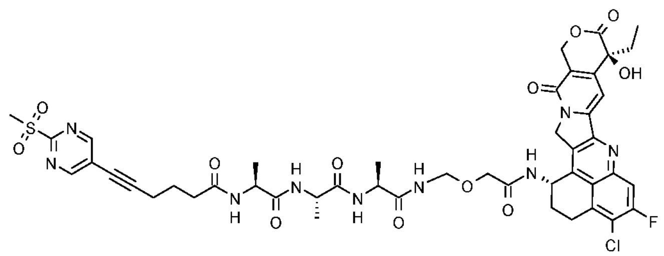 ,

A-19: 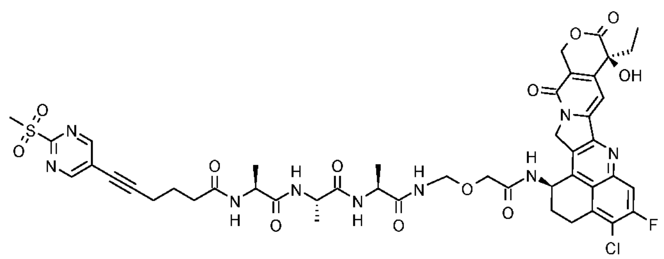 ,

A-20: 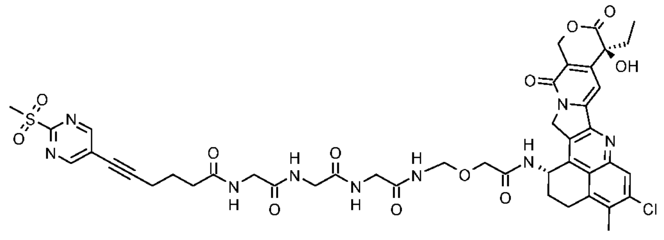 ,

A-21: 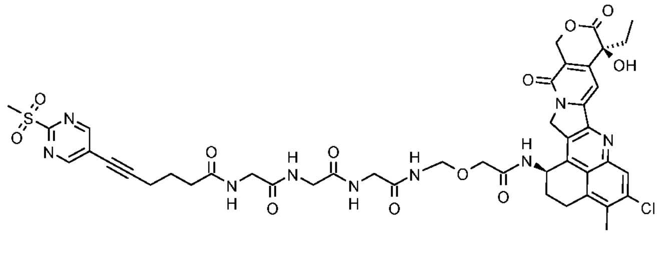 ,

A-22: 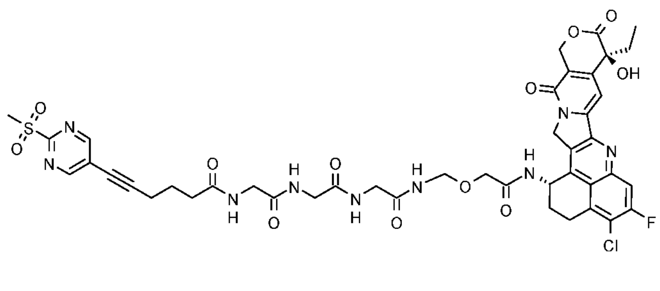 ,

A-23: 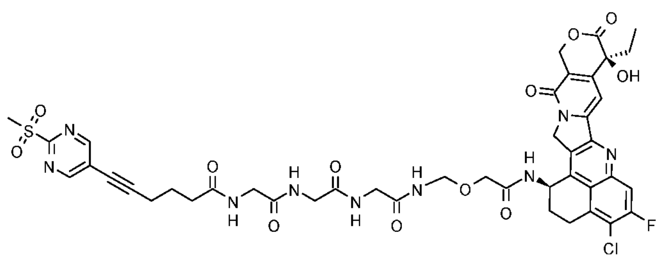 ,

A-24: 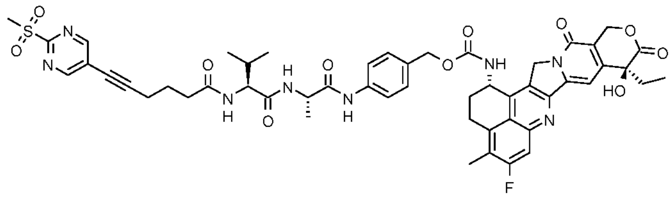 ,

A-25: 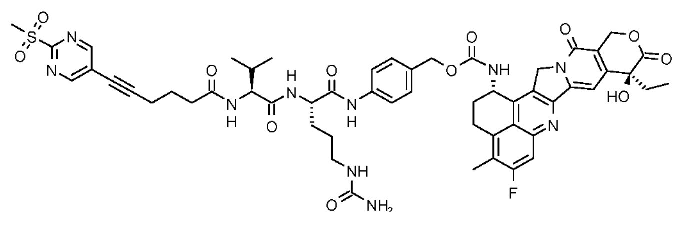 ,

A-26: 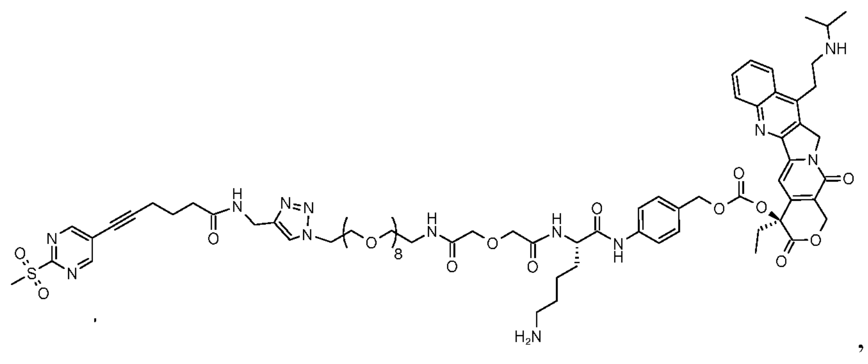 ,

A-27: 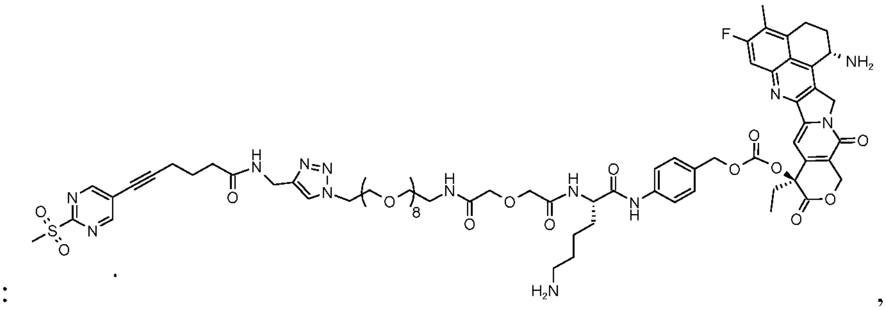 ,

A-28: 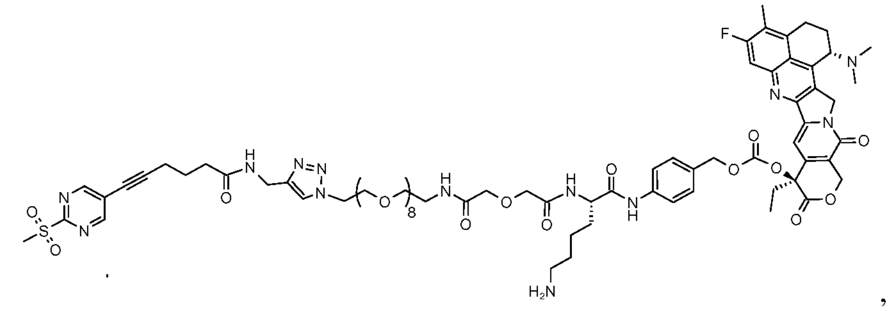 ,

A-29: 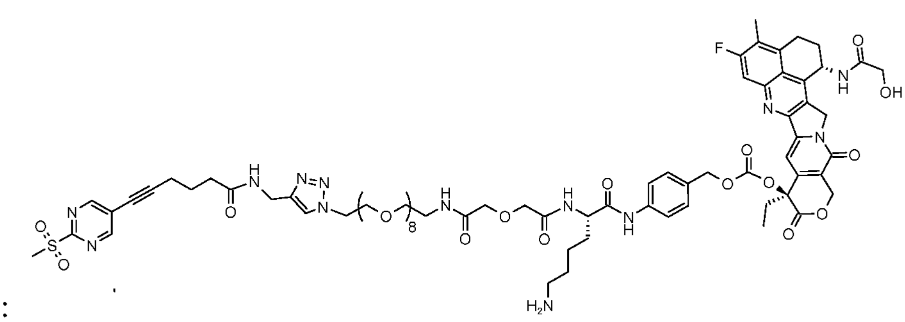 ,

A-30: 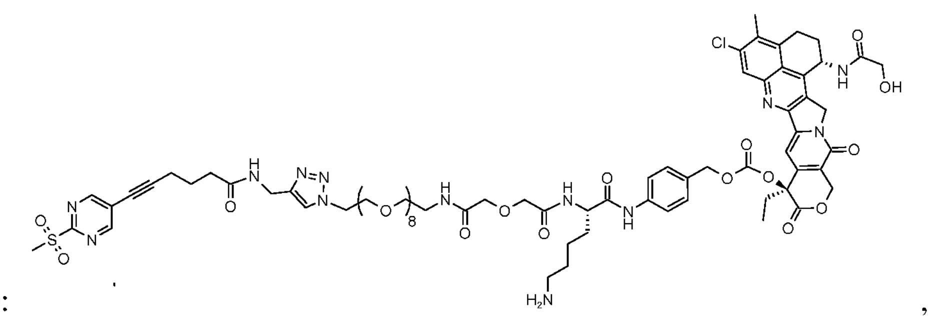 ,

A-31: 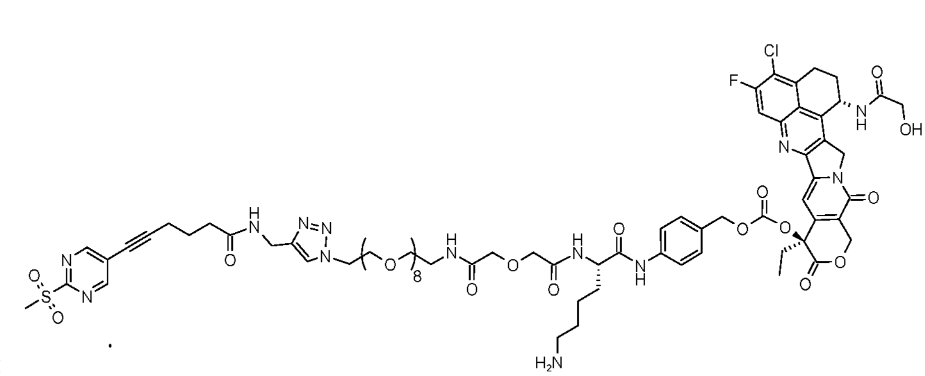 ,

B-1: 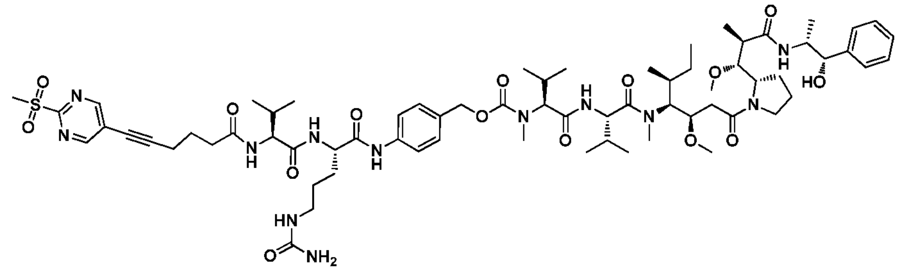 ,

B-2: 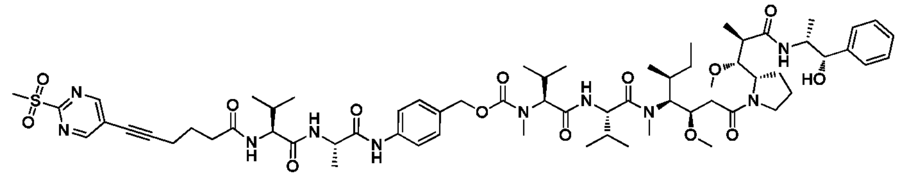 ,

B-3: 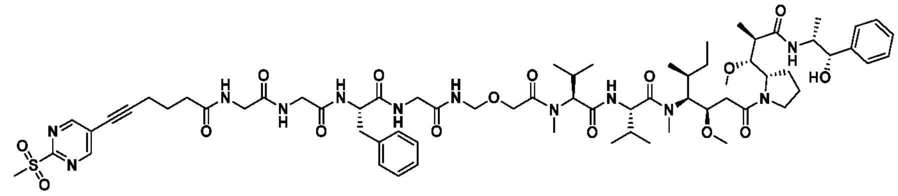 .

**18.** A pharmaceutical composition, which comprises the antibody-drug conjugate according to any one of claims 1-15 and optionally the drug-linker according to claim 16 or 17, and one or more pharmaceutical excipients.

**19.** Use of the antibody-drug conjugate according to any one of claims 1-15, the drug-linker according to claim 16 or 17, or the pharmaceutical composition according to claim 18 in the manufacture of a medicament for the treatment of a cancer with high expression of ROR1.

**20.** The use according to claim 19, wherein the cancer is selected from the group consisting of solid tumors and hematological malignancies; for example, selected from the group consisting of colon cancer, gastric cancer, breast cancer, lung cancer (e.g., non-small cell lung cancer, specifically such as lung adenocarcinoma), and lymphoma.

**21.** A method for treating a cancer with high expression of ROR1, comprising administering to a subject in need thereof a therapeutically effective amount of the antibody-drug conjugate according to any one of claims 1-15, or the drug-linker according to claim 16 or 17, or the pharmaceutical composition according to claim 18.

**22.** The antibody-drug conjugate according to any one of claims 1-15, the drug-linker according to claim 16 or 17, or the pharmaceutical composition according to claim 18, for used in the treatment of a cancer with high expression of ROR1.

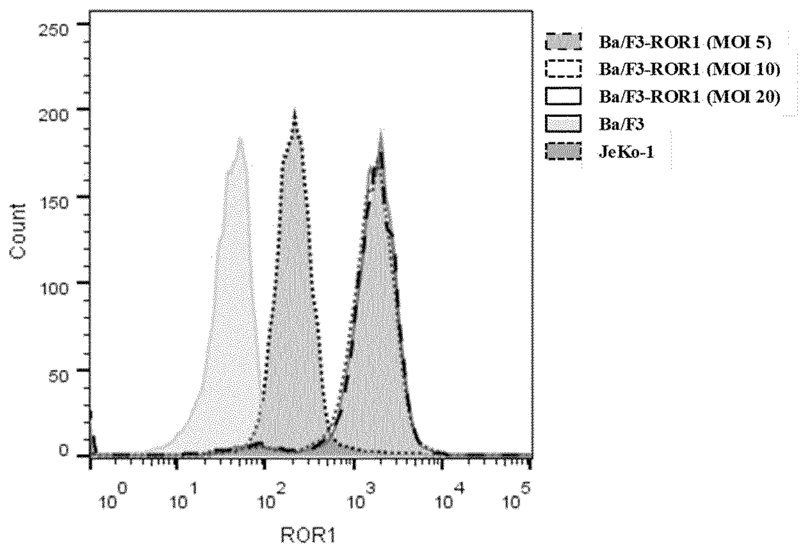
250
200
150
100
50
0
Count
10^0
10^1
10^2
10^3
10^4
10^5
ROR1
Ba/F3-ROR1 (MOI 5)
Ba/F3-ROR1 (MOI 10)
Ba/F3-ROR1 (MOI 20)
Ba/F3
JeKo-1

Fig. 1

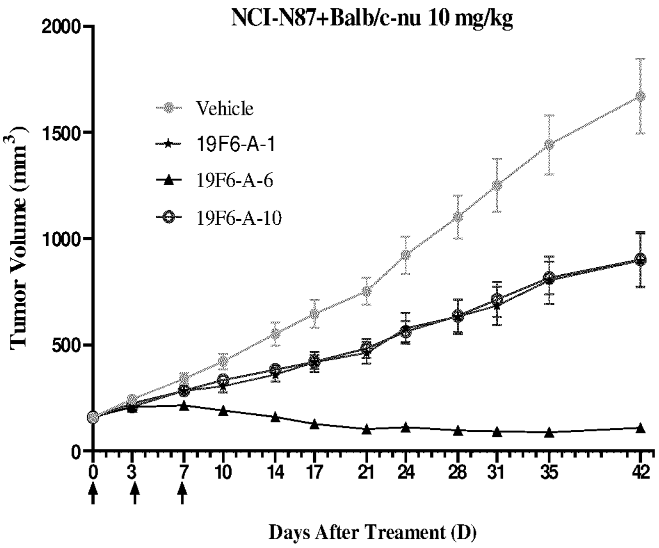
NCI-N87+Balb/c-nu 10 mg/kg
Tumor Volume (mm^3)
2000
1500
1000
500
0
Vehicle
19F6-A-1
19F6-A-6
19F6-A-10
0
3
7
10
14
17
21
24
28
31
35
42
Days After Treament (D)

Fig. 2

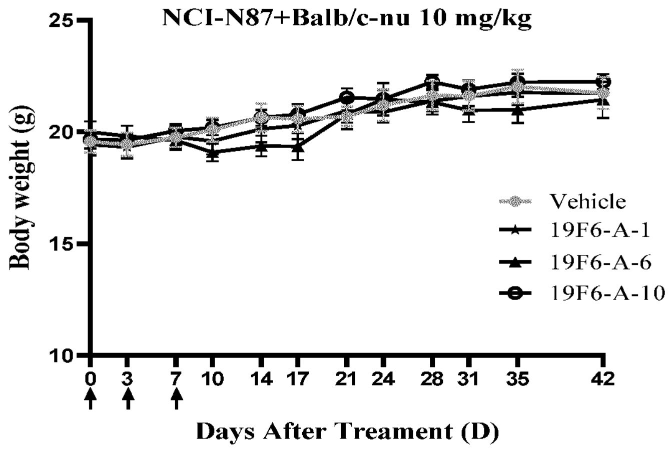
NCI-N87+Balb/c-nu 10 mg/kg
Body weight (g)
Days After Treament (D)
Vehicle
19F6-A-1
19F6-A-6
19F6-A-10

Fig. 3

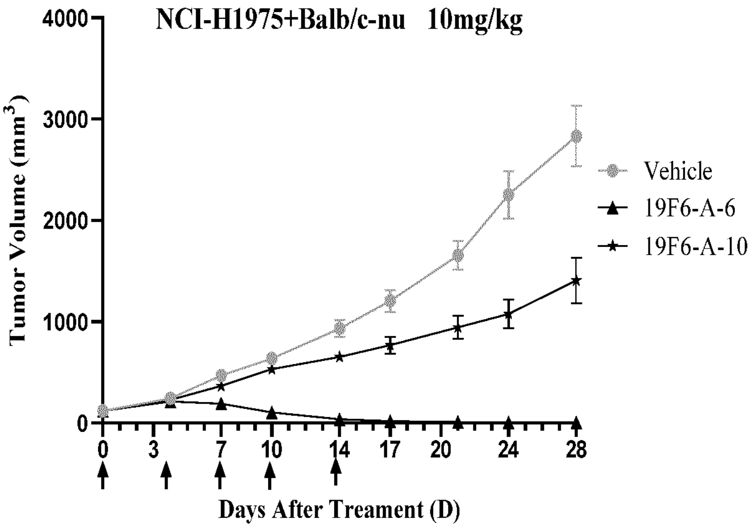
NCI-H1975+Balb/c-nu 10mg/kg
Tumor Volume (mm3)
Days After Treament (D)
Vehicle
19F6-A-6
19F6-A-10

Fig. 4

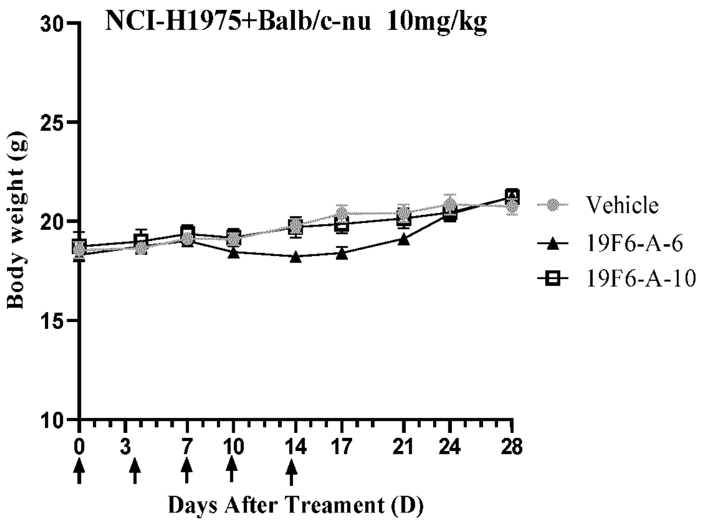
NCI-H1975+Balb/c-nu 10mg/kg
Body weight (g)
Days After Treament (D)
Vehicle
19F6-A-6
19F6-A-10

Fig. 5

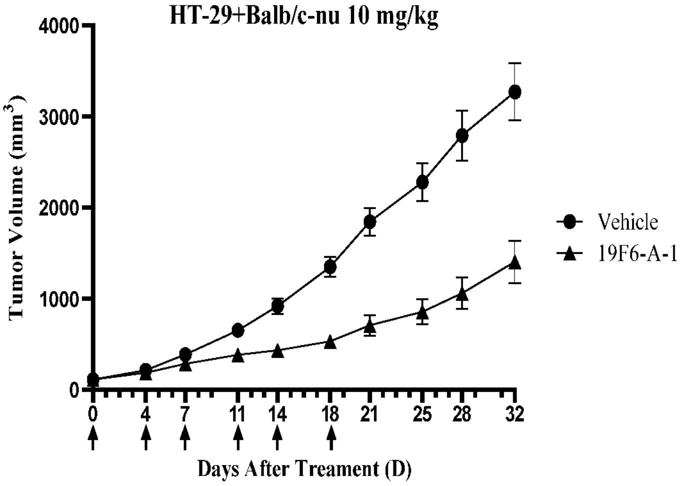


Fig. 6

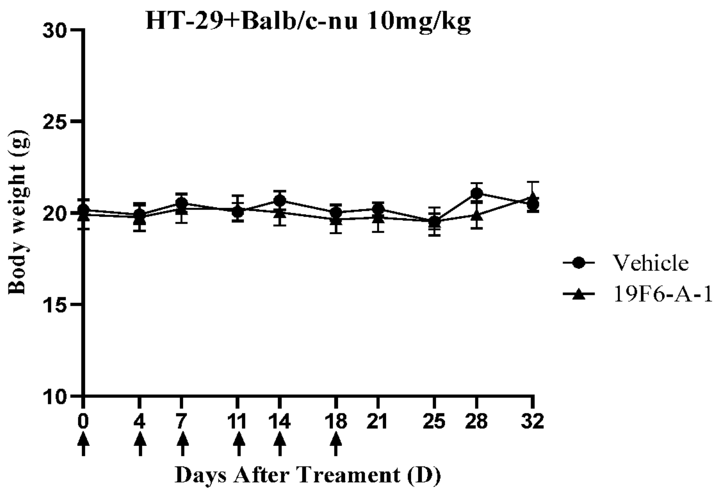


Fig. 7

**INTERNATIONAL SEARCH REPORT**

International application No.
**PCT/CN2022/094559**

**A. CLASSIFICATION OF SUBJECT MATTER**

A61K 47/68(2017.01)i； A61P 35/00(2006.01)i； C07K 16/28(2006.01)i

According to International Patent Classification (IPC) or to both national classification and IPC

**B. FIELDS SEARCHED**

Minimum documentation searched (classification system followed by classification symbols)

A61K A61P C07K

Documentation searched other than minimum documentation to the extent that such documents are included in the fields searched

Electronic data base consulted during the international search (name of data base and, where practicable, search terms used)

CNABS; CNTXT; VEN; WOTXT; EPTXT; USTXT; CNKI; 万方, WANFANG; ISI Web of Science; Genbank; STNext: 四川科伦, 序列检索, sequence search, structural formula search, 偶联物, 抗体, 酪氨酸激酶样孤儿受体, sichuan kelun, ADC, antibody, ROR

**C. DOCUMENTS CONSIDERED TO BE RELEVANT**

| Category* | Citation of document, with indication, where appropriate, of the relevant passages | Relevant to claim No. |
|---|---|---|
| X | WO 2019114666 A1 (SICHUAN KELUN BIOTECH BIOPHARMACEUTICAL CO., LTD.) 20 June 2019 (2019-06-20)<br>claims 16, 39, 40-53, 67, 78, and 89-90, and description, page 90, line 14, and page 79, lines 20 and 21 | 1-20, 22 |
| X | CN 110903395 A (SICHUAN KELUN-BIOTECH BIOPHARMACEUTICA CO., LTD.) 24 March 2020 (2020-03-24)<br>claims 16, 17, and 23 | 1-20, 22 |
| A | CN 105263962 A (BEIJING SHENOGEN PHARMACEUTICAL TECHNOLOGY CO., LTD.) 20 January 2016 (2016-01-20)<br>entire document | 1-20, 22 |

☐ Further documents are listed in the continuation of Box C.

☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **04 August 2022** | **17 August 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/094559**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   
   b. ☐ furnished together with the international application under PCT Rule 13*ter*.1(a) for the purposes of international search only in the form of an Annex C/ST.25 text file.
   
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**

International application No.
**PCT/CN2022/094559**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **21**
because they relate to subject matter not required to be searched by this Authority, namely:

[1] The subject matter of claim 21 is a method for treating cancer in which ROR1 is highly expressed, which is a method for treating a living human or animal body, and belongs to the subject matter as defined in PCT Rule 39.1 that does not warrant a search conducted by the international searching authority.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/094559**

| Patent document cited in search report | | | Publication date (day/month/year) | Patent family member(s) | | | Publication date (day/month/year) |
|---|---|---|---|---|---|---|---|
| WO | 2019114666 | A1 | 20 June 2019 | EP | 3725798 | A1 | 21 October 2020 |
| | | | | US | 2020347075 | A1 | 05 November 2020 |
| | | | | CN | 113698414 | A | 26 November 2021 |
| | | | | CN | 111295389 | A | 16 June 2020 |
| | | | | KR | 20200099123 | A | 21 August 2020 |
| | | | | CA | 3080236 | A1 | 20 June 2019 |
| | | | | CN | 113683622 | A | 23 November 2021 |
| | | | | JP | 2021506743 | A | 22 February 2021 |
| | | | | CN | 113651831 | A | 16 November 2021 |
| | | | | CN | 113603703 | A | 05 November 2021 |
| | | | | HK | 40020572 | A0 | 23 October 2020 |
| | | | | US | 2021101906 | A2 | 08 April 2021 |
| | | | | CN | 111295389 | B | 22 October 2021 |
| | | | | EP | 3725798 | A4 | 10 November 2021 |
| CN | 110903395 | A | 24 March 2020 | None | | | |
| CN | 105263962 | A | 20 January 2016 | WO | 2014146575 | A1 | 25 September 2014 |
| | | | | EP | 2976362 | A1 | 27 January 2016 |
| | | | | US | 2016046721 | A1 | 18 February 2016 |
| | | | | JP | 2016520533 | A | 14 July 2016 |
| | | | | EP | 2976362 | A4 | 22 March 2017 |
| | | | | US | 10233249 | B2 | 19 March 2019 |
| | | | | JP | 6574754 | B2 | 11 September 2019 |
| | | | | EP | 2976362 | B1 | 23 October 2019 |
| | | | | CN | 105263962 | B | 31 December 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110615214X
- CN 202110941134
- US 9217040 B2
- WO 2018237335 A1


### Non-patent literature cited in the description


- *Nat Biotechnol.,* 2012, vol. 30 (2), 184-9
- *Bioconjugate Chem.,* 2015, vol. 26, 145-152
- *Bioorg. Med. Chem. Lett.,* 2020, vol. 30, 127640
- Remington's Pharmaceutical Sciences. 1980
- **KABAT et al.** Sequences of Proteins of Immunological Interest. Public Health Service, National Institutes of Health, 1991
- **CHOTHIA ; LESK.** *J. Mol. Biol.,* 1987, vol. 196, 901-917
- **CHOTHIA et al.** *Nature,* 1989, vol. 342, 878-883
- **LEFRANC et al.** *Dev. Comparat. Immunol.,* 2003, vol. 27, 55-77
- **MARTIN ACR ; CHEETHAM JC ; REES AR.** Modeling antibody hypervariable loops: A combined algorithm. *Proc Natl Acad Sci USA,* 1989, vol. 86, 9268-9272
- Fundamental Immunology. Raven Press, 1989
- **HOLLIGER et al.** *Nat Biotechnol,* 2005, vol. 23, 1126-1136
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **PLUCKTHUN.** The Pharmacology of Monoclonal Antibodies. Springer-Verlag, 1994, vol. 113, 269-315
- **HOLLIGER et al.** *Proc. Natl. Acad. Sci. USA,* 1993, vol. 90, 6444-6448
- **ALFTHAN et al.** *Protein Eng.,* 1995, vol. 8, 725-731
- **CHOI et al.** *Eur. J. Immunol.,* 2001, vol. 31, 94-106
- **HU et al.** *Cancer Res.,* 1996, vol. 56, 3055-3061
- **KIPRIYANOV et al.** *J. Mol. Biol.,* 1999, vol. 293, 41-56
- **ROOVERS et al.** *Cancer Immunol,* 2001
- **HOLT, L. et al.** *Trends in Biotechnology,* 2003, vol. 21 (11), 484-490
- **NEEDLEMAN et al.** *J. Mol. Biol.,* 1970, vol. 48, 443-453
- **E. MEYERS ; W. MILLER.** *Comput. Appl. Biosci,* 1988, vol. 4, 11-17
- **NEEDLEMAN ; WUNSCH.** *J. Mol. Biol.,* 1970, vol. 48, 444-453
- **BRUMMELL et al.** *Biochem.,* 1993, vol. 32, 1180-1187
- **KOBAYASHI et al.** *Protein Eng.,* 1999, vol. 12 (10), 879-884
- **BURKS et al.** *Proc. Natl Acad. Set USA,* 1997, vol. 94, 412-417
- Immunology-A Synthesis. Sinauer Associates, Sunderland, Mass, 1991